# EUROPEAN PATENT SPECIFICATION

(11) **EP 2 596 098 B1**
(45) Date of publication and mention of the grant of the patent: **26.09.2018**
(21) Application number: 11809985.2
(22) Date of filing: 25.01.2011
(51) Int. Cl.: C12N 7/00

(54) **HIGH YIELD YELLOW FEVER VIRUS STRAIN WITH INCREASED PROPAGATION IN CELLS**
HOCHPRODUKTIVER GELBFIEBER-VIRUSSTAMM MIT ERHÖHTER FORTPFLANZUNG IN ZELLEN
SOUCHE DE VIRUS DE LA FIÈVRE JAUNE À RENDEMENT ÉLEVÉ AVEC PROPAGATION AUGMENTÉE DANS DES CELLULES

(30) Priority: 23.07.2010 WO PCT/US2010/004301
(43) Date of publication of application: 29.05.2013
(73) Proprietor: GE Healthcare Bio-Sciences Corp., Marlborough, MA 01752 (US)
(72) Inventor: LEE, Cynthia, K., Needham, MA 02492 (US); MONATH, Thomas, P., Harvard, MA 01451 (US); GUERTIN, Patrick, M., Mendon, MA 01756 (US); HAYMAN, Edward, G., Hanover,NH 03755 (US)
(74) Representative: Aldenbäck, Ulla Christina
(86) International application number: PCT/US2011/022347
(87) International publication number: WO 2012/011969

(56) References cited:
- WO-A2-2004/009764
- WO-A2-2011/014416
- US-A1- 2009 263 470
- US-A1- 2010 003 273
- US-A1- 2010 158 938
- SOUZA M C O ET AL: "Production of yellow fever virus in microcarrier-based Vero cell cultures", VACCINE, ELSEVIER LTD, GB, vol. 27, no. 46, 30 October 2009 (2009-10-30), pages 6420-6423, XP026704476, ISSN: 0264-410X, DOI: 10.1016/J.VACCINE.2009.06.023 [retrieved on 2009-06-24]
- MONATH T P ET AL: "Inactivated yellow fever 17D vaccine: Development and nonclinical safety, immunogenicity and protective activity", VACCINE, ELSEVIER LTD, GB, vol. 28, no. 22, 14 May 2010 (2010-05-14), pages 3827-3840, XP027057879, ISSN: 0264-410X [retrieved on 2010-05-14]
- DAVID W.C. BEASLEY ET AL: "Adaptation of yellow fever virus 17D to Vero cells is associated with mutations in structural and non-structural protein genes", VIRUS RESEARCH, vol. 176, no. 1-2, 1 September 2013 (2013-09-01), pages 280-284, XP055091118, ISSN: 0168-1702, DOI: 10.1016/j.virusres.2013.04.003
- MONATH THOMAS P ET AL: "INACTIVATED, CELL-BASED YELLOW FEVER 17D VACCINE-SAFETY AND IMMUNOGENICITY IN ANIMAL MODELS AND RESULTS OF A PHASE 1 CLINICAL TRIAL", AMERICAN JOURNAL OF TROPICAL MEDICINE AND HYGIENE, vol. 83, no. 5, Suppl. S, November 2010 (2010-11), page 16, XP8166105, & 59TH ANNUAL MEETING OF THE AMERICAN-SOCIETY-OF-TROPICAL-MEDICINE-AND- HYGIENE (ASTMH); ATLANTA, GA, USA; NOVEMBER 03 -07, 2010
- GALLER R ET AL: "THE YELLOW FEVER 17D VACCINE VIRUS: MOLECULAR BASIS OF VIRAL ATTENUATION AND ITS USE AS AN EXPRESSION VECTOR", BRAZILIAN JOURNAL OF MEDICAL AND BIOLOGICAL RESEARCH, RIBEIRAO PRETO, BR, vol. 30, 1 January 1997 (1997-01-01), pages 157-168, XP000925918, ISSN: 0100-879X, DOI: 10.1590/S0100-879X1997000200002

### SEQUENCE LISTING

<110> XCELLEREX, INC. Lee, Cynthia K. Monath, Thomas P. Guertin, Patrick M. Hayman, Edward G.
<120> HIGH YIELD YELLOW FEVER VIRUS STRAINS
   WITH
INCREASED PROPAGATION IN CELLS
<130> 4098.3003 WO
<150> PCT/US2010/043010
   <151> 2010-07-23
<160> 15
<170> FastSEQ for Windows Version 4.0
<210> 1
   <211> 6300
   <212> DNA
   <213> Flaviviridae Flavivirus Yellow Fever Virus
<400> 1
<210> 2
   <211> 6300
   <212> DNA
   <213> Flaviviridae Flavivirus Yellow Fever Virus
<400> 2
   agtaaatcct gtgtgctaat tgaggtgcat tggtctgcaa atcgagttgc taggcaataa 60
   acacatttgg attaatttta atcgttcgtt gagcgattag cagagaactg accagaacat 120
   gtctggtcgt aaagctcagg gaaaaaccct gggcgtcaat atggtacgac gaggagttcg 180
   ctccttgtca aacaaaataa aacaaaaaac gaaacaaatt ggaaacagac ctggaccttc 240
   aagaggtgtt caaggattta tctttttctt tttgttcaac attttgactg gaaaaaagat 300
   cacagcccac ctaaagaggt tgtggaaaat gctggaccca agacaaggct tggctgttct 360
   aaggaaagtc aagagagtgg tggccagttt gatgagagga ttgtcctcaa ggaaacgccg 420
   ttcccatgat gttctgactg tgcaattcct aattttggga atgctgttga tgacgggtgg 480
   agtgaccttg gtgcggaaaa acagatggtt gctcctaaat gtgacatctg aggacctcgg 540
   gaaaacattc tctgtgggca caggcaactg cacaacaaac attttggaag ccaagtactg 600
   gtgcccagac tcaatggaat acaactgtcc caatctcagt ccaagagagg agccagatga 660
   cattgattgc tggtgctatg gggtggaaaa cgttagagtc gcatatggta agtgtgactc 720
   agcaggcagg tctaggaggt caagaagggc cattgacttg cctacgcatg aaaaccatgg 780
   tttgaagacc cggcaagaaa aatggatgac tggaagaatg ggtgaaaggc aactccaaaa 840
   gattgagaga tggttcgtga ggaacccctt ttttgcagtg acggctctga ccattgccta 900
   ccttgtggga agcaacatga cgcaacgagt cgtgattgcc ctactggtct tggctgttgg 960
   tccggcctac tcagctcact gcattggaat tactgacagg gatttcattg agggggtgca 1020
   tggaggaact tgggtttcag ctaccctgga gcaagacaag tgtgtcactg ttatggcccc 1080
   tgacaagcct tcattggaca tctcactaga gacagtagcc attgatagac ctgctgaggt 1140
   gaggaaagtg tgttacaatg cagttctcac tcatgtgaag attaatgaca agtgccccag 1200
   cactggagag gcccacctag ctgaagagaa cgaaggggac aatgcgtgca agcgcactta 1260
   ttctgataga ggctggggca atggctgtgg cctatttggg aaagggagca ttgtggcatg 1320
   cgccaaattc acttgtgcca aatccatgag tttgtttgag gttgatcaga ccaaaattca 1380
   gtatgtcatc agagcacaat tgcatgtagg ggccaagcag gaaaattgga ctaccgacat 1440
   taagactctc aggtttgatg ccctgtcagg ctcccaggaa gtcgagttca ttgggtatgg 1500
   aaaagctaca ctggaatgcc aggtgcaaac tgcggtggac tttggtaaca gttacatcgc 1560
   tgagatggaa acagagagct ggatagtgga cagacagtgg gcccaggact tgaccctgcc 1620
   atggcagagt ggaagtggcg gggtgtggag agagatgcat catcttgtcg aatttgaacc 1680
   tccgcatgcc gccactatca gagtactggc cctgggaaac caggaaggct ccttgaaaac 1740
   agctcttact ggcgcaatga gggttacaaa ggacacaaat gacaacaacc tttacaaact 1800
   acatggtgga catgtttctt gcagagtgaa attgtcagct ttgacactca aggggacatc 1860
   ctacaaaata tgcactgaca aaatgttttt tgtcaagaac ccaactgaca ctggccatgg 1920
   cactgttgtg atgcaggtga aagtgtcaaa aggagccccc tgcaggattc cagtgatagt 1980
   agctgatgat cttacagcgg caatcaataa aggcattttg gttacagtta accccatcgc 2040
   ctcaaccaat gatgatgaag tgctgattga ggtgaaccca ccttttggag acagctacat 2100
   tatcgttggg agaggagatt cacgtctcac ttaccagtgg cacaaagagg gaagctcaat 2160
   aggaaagttg ttcactcaga ccatgaaagg cgtggaacgc ctggccgtca tgggagacac 2220
   cgcctgggat ttcagctccg ctggagggtt cttcacttcg gttgggaaag gaattcatac 2280
   ggtgtttggc tctgcctttc aggggctatt tggcggcttg aactggataa caaaggtcat 2340
   catgggggcg gtacttatat gggttggcat caacacaaga aacatgacaa tgtccatgag 2400
   catgatcttg gtaggagtga tcatgatgtt tttgtctcta ggagttgggg cggatcaagg 2460
   atgcgccatc aactttggca agagagagct caagtgcgga gatggtatct tcatatttag 2520
   agactctgat gactggctga acaagtactc atactatcca gaagatcctg tgaagcttgc 2580
   atcaatagtg aaagcctctt ttgaagaagg gaagtgtggc ctaaattcag ttgactccct 2640
   tgagcatgag atgtggagaa gcagggcaga tgagatcaat gccattttgg aggaaaacga 2700
   ggtggacatt tctgttgtcg tgcaggatcc aaagaatgtt taccagagag gaactcatcc 2760
   attttccaga attcgggatg gtctgcagta tggttggaag acttggggta agaaccttgt 2820
   gttctcccca gggaggaaga atggaagctt catcatagat ggaaagtcca ggaaagaatg 2880
   cccgttttca aaccgggtct ggaattcttt ccagatagag gagtttggga cgggagtgtt 2940
   caccacacgc gtgtacatgg acgcagtctt tgaatacacc atagactgcg atggatctat 3000
   cttgggtgca gcggtgaacg gaaaaaagag tgcccatggc tctccaacat tttggatggg 3060
   aagtcatgaa gtaaatggga catggatgat ccacaccttg gaggcattag attacaagga 3120
   gtgtgagtgg ccactgacac atacgattgg aacatcagtt gaagagagtg aaatgttcat 3180
   gccgagatca atcggaggcc cagttagctc tcacaatcat atccctggat acaaggttca 3240
   gacgaacgga ccttggatgc aggtaccact agaagtgaag agagaagctt gcccagggac 3300
   tagcgtgatc attgatggca actgtgatgg acggggaaaa tcaaccagat ccaccacgga 3360
   tagcgggaaa gttattcctg aatggtgttg ccgctcctgc acaatgccgc ctgtgagctt 3420
   ccatggtagt gatgggtgtt ggtatcccat ggaaattagg ccaaggaaaa cgcatgaaag 3480
   ccatctggtg cgctcctggg ttacagctgg agaaatacat gctgtccctt ttggtttggt 3540
   gagcatgatg atagcaatgg aagtggtcct aaggaaaaga cagggaccaa agcaaatgtt 3600
   ggttggagga gtagtgctct tgggagcaat gctggtcggg caagtaactc tccttgattt 3660
   gctgaaactc acagtggctg tgggattgca tttccatgag atgaacaatg gaggagacgc 3720
   catgtatatg gcgttgattg ctgccttttc aatcagacca gggctgctca tcggctttgg 3780
   gctcaggacc ctatggagcc ctcgggaacg ccttgtgctg accctaggag cagccatggt 3840
   ggagattgcc ttgggtggcg tgatgggcgg cctgtggaag tatctaaatg cagtttctct 3900
   ctgcatcctg acaataaatg ctgttgcttc taggaaagca tcaaatacca tcttgcccct 3960
   catggctctg ttgacacctg tcactatggc tgaggtgaga cttgccgcaa tgttcttttg 4020
   tgccgtggtt atcatagggg tccttcacca gaatttcaag gacacctcca tgcagaagac 4080
   tatacctctg gtggccctca cactcacatc ttacctgggc ttgacacaac cttttttggg 4140
   cctgtgtgca tttctggcaa cccgcatatt tgggcgaagg agtatcccag tgaatgaggc 4200
   actcgcagca gctggtctag tgggagtgct ggcaggactg gcttttcagg agatggagaa 4260
   cttccttggt ccgattgcag ttggaggact cctgatgatg ctggttagcg tggctgggag 4320
   ggtggatggg ctagagctca agaagcttgg tgaagtttca tgggaagagg aggcggagat 4380
   cagcgggagt tccgcccgct atgatgtggc actcagtgaa caaggggagt tcaagctgct 4440
   ttctgaagag aaagtgccat gggaccaggt tgtgatgacc tcgctggcct tggttggggc 4500
   tgccctccat ccatttgctc ttctgctggt ccttgctggg tggctgtttc atgtcagggg 4560
   agctaggaga agtggggatg tcttgtggga tattcccact cctaagatca tcgaggaatg 4620
   tgaacatctg gaggatggga tttatggcat attccagtca accttcttgg gggcctccca 4680
   gcgaggagtg ggagtggcac agggaggggt gttccacaca atgtggcatg tcacaagagg 4740
   agctttcctt gtcaggaatg gcaagaagtt gattccatct tgggcttcag taaaggaaga 4800
   ccttgtcgcc tatggtggct catggaagtt ggaaggcaga tgggatggag aggaagaggt 4860
   ccagttgatc gcggctgttc caggaaagaa cgtggtcaac gtccagacaa aaccgagctt 4920
   gttcaaagtg aggaatgggg gagaaatcgg ggctgtcgct cttgactatc cgagtggcac 4980
   ttcaggatct cctattgtta acaggaacgg agaggtgatt gggctgtacg gcaatggcat 5040
   ccttgtcggt gacaactcct tcgtgtccgc catatcccag actgaggtga aggaagaagg 5100
   aaaggaggag ctccaagaga tcccgacaat gctaaagaaa ggaatgacaa ctgtccttga 5160
   ttttcatcct ggagctggga agacaagacg tttcctccca cagatcttgg ccgagtgcgc 5220
   acggagacgc ttgcgcactc ttgtgttggc ccccaccagg gttgttcttt ctgaaatgaa 5280
   ggaggctttt cacggcctgg acgtgaaatt ccacacacag gctttttccg ctcacggcag 5340
   cgggagagaa gtcattgatg ctatgtgcca tgccacccta acttacagga tgttggaacc 5400
   aactagggtt gttaactggg aagtgatcat tatggatgaa gcccattttt tggatccagc 5460
   tagcatagcc gctagaggtt gggcagcgca cagagctagg gcaaatgaaa gtgcaacaat 5520
   cttgatgaca gccacaccgc ctgggactag tgatgaattt ccacattcaa atggtgaaat 5580
   agaagatgtt caaacggaca tacccagtga gccctggaac acagggcatg actggatcct 5640
   ggctgacaaa aggcccacgg catggttcct tccatccatc agagctgcaa atgtcatggc 5700
   tgcctctttg cgtaaggctg gaaagagtgt ggtggtcctg aacaggaaaa cctttgagag 5760
   agaatacccc acgataaagc agaagaaacc tgactttata ttggccactg acatagctga 5820
   aatgggagcc aacctttgcg tggagcgagt gctggattgc aggacggctt ttaagcctgt 5880
   gcttgtggat gaagggagga aggtggcaat aaaagggcca cttcgtatct ccgcatcctc 5940
   tgctgctcaa aggagggggc gcattgggag aaatcccaac agagatggag actcatacta 6000
   ctattctgag cctacaagtg aaaataatgc ccaccacgtc tgctggttgg aggcctcaat 6060
   gctcttggac aacatggagg tgaggggtgg aatggtcgcc ccactctatg gcgttgaagg 6120
   aactaaaaca ccagtttccc ctggtgaaat gagactgagg gatgaccaga ggaaagtctt 6180
   cagagaacta gtgaggaatt gtgacctgcc cgtttggctt tcgtggcaag tggccaaggc 6240
   tggtttgaag acgaatgatc gtaagtggtg ttttgaaggc cctgaggaac atgagatctt 6300
<210> 3
   <211> 3411
   <212> PRT
   <213> Flaviviridae Flavivirus Yellow Fever Virus
<400> 3
   Met Ser Gly Arg Lys Ala Gln Gly Lys Thr Leu Gly Val Asn Met Val
    1              5                   10                  15
   Arg Arg Gly Val Arg Ser Leu Ser Asn Lys Ile Lys Gln Lys Thr Lys
               20                  25                  30
   Gln Ile Gly Asn Arg Pro Gly Pro Ser Arg Gly Val Gln Gly Phe Ile
                   35                  40                  45
   Phe Phe Phe Leu Phe Asn Ile Leu Thr Gly Lys Lys Ile Thr Ala His
               50                  55                  60
   Leu Lys Arg Leu Trp Lys Met Leu Asp Pro Arg Gln Gly Leu Ala Val
   65                  70                  75                  80
   Leu Arg Lys Val Lys Arg Val Val Ala Ser Leu Met Arg Gly Leu Ser
                   85                  90                  95
   Ser Arg Lys Arg Arg Ser His Asp Val Leu Thr Val Gln Phe Leu Ile
               100                 105                 110
   Leu Gly Met Leu Leu Met Thr Gly Gly Val Thr Leu Val Arg Lys Asn
           115                 120                 125
   Arg Trp Leu Leu Leu Asn Val Thr Ser Glu Asp Leu Gly Lys Thr Phe
       130                 135                 140
   Ser Val Gly Thr Gly Asn Cys Thr Thr Asn Ile Leu Glu Ala Lys Tyr
   145                 150                 155                 160
   Trp Cys Pro Asp Ser Met Glu Tyr Asn Cys Pro Asn Leu Ser Pro Arg
                   165                 170                 175
   Glu Glu Pro Asp Asp Ile Asp Cys Trp Cys Tyr Gly Val Glu Asn Val
               180                 185                 190
   Arg Val Ala Tyr Gly Lys Cys Asp Ser Ala Gly Arg Ser Arg Arg Ser
           195                 200                 205
   Arg Arg Ala Ile Asp Leu Pro Thr His Glu Asn His Gly Leu Lys Thr
       210                 215                 220
   Arg Gln Glu Lys Trp Met Thr Gly Arg Met Gly Glu Arg Gln Leu Gln
   225                 230                 235                 240
   Lys Ile Glu Arg Trp Phe Val Arg Asn Pro Phe Phe Ala Val Thr Ala
                   245                 250                 255
   Leu Thr Ile Ala Tyr Leu Val Gly Ser Asn Met Thr Gln Arg Val Val
               260                 265                 270
   Ile Ala Leu Leu Val Leu Ala Val Gly Pro Ala Tyr Ser Ala His Cys
           275                 280                 285
   Ile Gly Ile Thr Asp Arg Asp Phe Ile Glu Gly Val His Gly Gly Thr
       290                 295                 300
   Trp Val Ser Ala Thr Leu Glu Gln Asp Lys Cys Val Thr Val Met Ala
   305                 310                 315                 320
   Pro Asp Lys Pro Ser Leu Asp Ile Ser Leu Glu Thr Val Ala Ile Asp
                   325                 330                 335
   Arg Pro Ala Glu Val Arg Lys Val Cys Tyr Asn Ala Val Leu Thr His
               340                 345                 350
   Val Lys Ile Asn Asp Lys Cys Pro Ser Thr Gly Glu Ala His Leu Ala
           355                 360                 365
   Glu Glu Asn Glu Gly Asp Asn Ala Cys Lys Arg Thr Tyr Ser Asp Arg
       370                 375                 380
   Gly Trp Gly Asn Gly Cys Gly Leu Phe Gly Lys Gly Ser Ile Val Ala
   385                 390                 395                 400
   Cys Ala Lys Phe Thr Cys Ala Lys Ser Met Ser Leu Phe Glu Val Asp
                   405                 410                 415
   Gln Thr Lys Ile Gln Tyr Val Ile Arg Ala Gln Leu His Val Gly Ala
               420                 425                 430
   Lys Gln Glu Asn Trp Thr Thr Asp Ile Lys Thr Leu Lys Phe Asp Ala
           435                 440                 445
   Leu Ser Gly Ser Gln Glu Val Glu Phe Ile Gly Tyr Gly Lys Ala Thr
       450                 455                 460
   Leu Glu Cys Gln Val Gln Thr Ala Val Asp Phe Gly Asn Ser Tyr Ile
   465                 470                 475                 480
   Ala Glu Met Glu Thr Glu Ser Trp Ile Val Asp Arg Gln Trp Ala Gln
                   485                 490                 495
   Asp Leu Thr Leu Pro Trp Gln Ser Gly Ser Gly Gly Val Trp Arg Glu
               500                 505                 510
   Met His His Leu Val Glu Phe Glu Pro Pro His Ala Ala Thr Ile Arg
           515                 520                 525
   Val Leu Ala Leu Gly Asn Gln Glu Gly Ser Leu Lys Thr Ala Leu Thr
       530                 535                 540
   Gly Ala Met Arg Val Thr Lys Asp Thr Asn Asp Asn Asn Leu Tyr Lys
   545                 550                 555                 560
   Leu His Gly Gly His Val Ser Cys Arg Val Lys Leu Ser Ala Leu Thr
                   565                 570                 575
   Leu Lys Gly Thr Ser Tyr Lys Ile Cys Thr Asp Lys Met Phe Phe Val
               580                 585                 590
   Lys Asn Pro Thr Asp Thr Gly His Gly Thr Val Val Met Gln Val Lys
           595                 600                 605
   Val Ser Lys Gly Ala Pro Cys Arg Ile Pro Val Ile Val Ala Asp Asp
       610                 615                 620
   Leu Thr Ala Ala Ile Asn Lys Gly Ile Leu Val Thr Val Asn Pro Ile
   625                 630                 635                 640
   Ala Ser Thr Asn Asp Asp Glu Val Leu Ile Glu Val Asn Pro Pro Phe
                   645                 650                 655
   Gly Asp Ser Tyr Ile Ile Val Gly Arg Gly Asp Ser Arg Leu Thr Tyr
               660                 665                 670
   Gln Trp His Lys Glu Gly Ser Ser Ile Gly Lys Leu Phe Thr Gln Thr
           675                 680                 685
   Met Lys Gly Val Glu Arg Leu Ala Val Met Gly Asp Thr Ala Trp Asp
       690                 695                 700
   Phe Ser Ser Ala Gly Gly Phe Phe Thr Ser Val Gly Lys Gly Ile His
   705                 710                 715                 720
   Thr Val Phe Gly Ser Ala Phe Gln Gly Leu Phe Gly Gly Leu Asn Trp
                   725                 730                 735
   Ile Thr Lys Val Ile Met Gly Ala Val Leu Ile Trp Val Gly Ile Asn
               740                 745                 750
   Thr Arg Asn Met Thr Met Ser Met Ser Met Ile Leu Val Gly Val Ile
           755                 760                 765
   Met Met Phe Leu Ser Leu Gly Val Gly Ala Asp Gln Gly Cys Ala Ile
       770                 775                 780
   Asn Phe Gly Lys Arg Glu Leu Lys Cys Gly Asp Gly Ile Phe Ile Phe
   785                 790                 795                 800
   Arg Asp Ser Asp Asp Trp Leu Asn Lys Tyr Ser Tyr Tyr Pro Glu Asp
                   805                 810                 815
   Pro Val Lys Leu Ala Ser Ile Val Lys Ala Ser Phe Glu Glu Gly Lys
               820                 825                 830
   Cys Gly Leu Asn Ser Val Asp Ser Leu Glu His Glu Met Trp Arg Ser
           835                 840                 845
   Arg Ala Asp Glu Ile Asn Ala Ile Phe Glu Glu Asn Glu Val Asp Ile
       850                 855                 860
   Ser Val Val Val Gln Asp Pro Lys Asn Val Tyr Gln Arg Gly Thr His
   865                 870                 875                 880
   Pro Phe Ser Arg Ile Arg Asp Gly Leu Gln Tyr Gly Trp Lys Thr Trp
                   885                 890                 895
   Gly Lys Asn Leu Val Phe Ser Pro Gly Arg Lys Asn Gly Ser Phe Ile
               900                 905                 910
   Ile Asp Gly Lys Ser Arg Lys Glu Cys Pro Phe Ser Asn Arg Val Trp
           915                 920                 925
   Asn Ser Phe Gln Ile Glu Glu Phe Gly Thr Gly Val Phe Thr Thr Arg
       930                 935                 940
   Val Tyr Met Asp Ala Val Phe Glu Tyr Thr Ile Asp Cys Asp Gly Ser
   945                 950                 955                 960
   Ile Leu Gly Ala Ala Val Asn Gly Lys Lys Ser Ala His Gly Ser Pro
                   965                 970                 975
   Thr Phe Trp Met Gly Ser His Glu Val Asn Gly Thr Trp Met Ile His
               980                 985                 990
   Thr Leu Glu Ala Leu Asp Tyr Lys Glu Cys Glu Trp Pro Leu Thr His
           995                 1000                1005
   Thr Ile Gly Thr Ser Val Glu Glu Ser Glu Met Phe Met Pro Arg Ser
       1010                1015                1020
   Ile Gly Gly Pro Val Ser Ser His Asn His Ile Pro Gly Tyr Lys Val
   1025                1030                1035                1040
   Gln Thr Asn Gly Pro Trp Met Gln Val Pro Leu Glu Val Lys Arg Glu
                   1045                1050                1055
   Ala Cys Pro Gly Thr Ser Val Ile Ile Asp Gly Asn Cys Asp Gly Arg
               1060                1065                1070
   Gly Lys Ser Thr Arg Ser Thr Thr Asp Ser Gly Lys Val Ile Pro Glu
   1075 1080 1085
   Trp Cys Cys Arg Ser Cys Thr Met Pro Pro Val Ser Phe His Gly Ser
   1090 1095 1100
   Asp Gly Cys Trp Tyr Pro Met Glu Ile Arg Pro Arg Lys Thr His Glu
   1105 1110 1115 1120
   Ser His Leu Val Arg Ser Trp Val Thr Ala Gly Glu Ile His Ala Val
   1125 1130 1135
   Pro Phe Gly Leu Val Ser Met Met Ile Ala Met Glu Val Val Leu Arg
   1140 1145 1150
   Lys Arg Gln Gly Pro Lys Gln Met Leu Val Gly Gly Val Val Leu Leu
   1155 1160 1165
   Gly Ala Met Leu Val Gly Gln Val Thr Leu Leu Asp Leu Leu Lys Leu
   1170 1175 1180
   Thr Val Ala Val Gly Leu His Phe His Glu Met Asn Asn Gly Gly Asp
   1185 1190 1195 1200
   Ala Met Tyr Met Ala Leu Ile Ala Ala Phe Ser Ile Arg Pro Gly Leu
   1205 1210 1215
   Leu Ile Gly Phe Gly Leu Arg Thr Leu Trp Ser Pro Arg Glu Arg Leu
   1220 1225 1230
   Val Leu Thr Leu Gly Ala Ala Met Val Glu Ile Ala Leu Gly Gly Val
   1235 1240 1245
   Met Gly Gly Leu Trp Lys Tyr Leu Asn Ala Val Ser Leu Cys Ile Leu
   1250 1255 1260
   Thr Ile Asn Ala Val Ala Ser Arg Lys Ala Ser Asn Thr Ile Leu Pro
   1265 1270 1275 1280
   Leu Met Ala Leu Leu Thr Pro Val Thr Met Ala Glu Val Arg Leu Ala
   1285 1290 1295
   Ala Met Phe Phe Cys Ala Val Val Ile Ile Gly Val Leu His Gln Asn
   1300 1305 1310
   Phe Lys Asp Thr Ser Met Gln Lys Thr Ile Pro Leu Val Ala Leu Thr
   1315 1320 1325
   Leu Thr Ser Tyr Leu Gly Leu Thr Gln Pro Phe Leu Gly Leu Cys Ala
   1330 1335 1340
   Phe Leu Ala Thr Arg Ile Phe Gly Arg Arg Ser Ile Pro Val Asn Glu
   1345 1350 1355 1360
   Ala Leu Ala Ala Ala Gly Leu Val Gly Val Leu Ala Gly Leu Ala Phe
   1365 1370 1375
   Gln Glu Met Glu Asn Phe Leu Gly Pro Ile Ala Val Gly Gly Leu Leu
   1380 1385 1390
   Met Met Leu Val Ser Val Ala Gly Arg Val Asp Gly Leu Glu Leu Lys
   1395 1400 1405
   Lys Leu Gly Glu Val Ser Trp Glu Glu Glu Ala Glu Ile Ser Gly Ser
   1410 1415 1420
   Ser Ala Arg Tyr Asp Val Ala Leu Ser Glu Gln Gly Glu Phe Lys Leu
   1425 1430 1435 1440
   Leu Ser Glu Glu Lys Val Pro Trp Asp Gln Val Val Met Thr Ser Leu
   1445 1450 1455
   Ala Leu Val Gly Ala Ala Leu His Pro Phe Ala Leu Leu Leu Val Leu
   1460 1465 1470
   Ala Gly Trp Leu Phe His Val Arg Gly Ala Arg Arg Ser Gly Asp Val
   1475 1480 1485
   Leu Trp Asp Ile Pro Thr Pro Lys Ile Ile Glu Glu Cys Glu His Leu
   1490 1495 1500
   Glu Asp Gly Ile Tyr Gly Ile Phe Gln Ser Thr Phe Leu Gly Ala Ser
   1505 1510 1515 1520
   Gln Arg Gly Val Gly Val Ala Gln Gly Gly Val Phe His Thr Met Trp
   1525 1530 1535
   His Val Thr Arg Gly Ala Phe Leu Val Arg Asn Gly Lys Lys Leu Ile
   1540 1545 1550
   Pro Ser Trp Ala Ser Val Lys Glu Asp Leu Val Ala Tyr Gly Gly Ser
   1555 1560 1565
   Trp Lys Leu Glu Gly Arg Trp Asp Gly Glu Glu Glu Val Gln Leu Ile
   1570 1575 1580
   Ala Ala Val Pro Gly Lys Asn Val Val Asn Val Gln Thr Lys Pro Ser
   1585 1590 1595 1600
   Leu Phe Lys Val Arg Asn Gly Gly Glu Ile Gly Ala Val Ala Leu Asp
                   1605                1610                1615
   Tyr Pro Ser Gly Thr Ser Gly Ser Pro Ile Val Asn Arg Asn Gly Glu
               1620                1625                1630
   Val Ile Gly Leu Tyr Gly Asn Gly Ile Leu Val Gly Asp Asn Ser Phe
           1635                1640                1645
   Val Ser Ala Ile Ser Gln Thr Glu Val Lys Glu Glu Gly Lys Glu Glu
       1650                1655                1660
   Leu Gln Glu Ile Pro Thr Met Leu Lys Lys Gly Met Thr Thr Val Leu
   1665                1670                1675                1680
   Asp Phe His Pro Gly Ala Gly Lys Thr Arg Arg Phe Leu Pro Gln Ile
                   1685                1690                1695
   Leu Ala Glu Cys Ala Arg Arg Arg Leu Arg Thr Leu Val Leu Ala Pro
               1700                1705                1710
   Thr Arg Val Val Leu Ser Glu Met Lys Glu Ala Phe His Gly Leu Asp
           1715                1720                1725
   Val Lys Phe His Thr Gln Ala Phe Ser Ala His Gly Ser Gly Arg Glu
       1730                1735                1740
   Val Ile Asp Ala Met Cys His Ala Thr Leu Thr Tyr Arg Met Leu Glu
   1745                1750                1755                1760
   Pro Thr Arg Val Val Asn Trp Glu Val Ile Ile Met Asp Glu Ala His
                   1765                1770                1775
   Phe Leu Asp Pro Ala Ser Ile Ala Ala Arg Gly Trp Ala Ala His Arg
               1780                1785                1790
   Ala Arg Ala Asn Glu Ser Ala Thr Ile Leu Met Thr Ala Thr Pro Pro
           1795                1800                1805
   Gly Thr Ser Asp Glu Phe Pro His Ser Asn Gly Glu Ile Glu Asp Val
       1810                1815                1820
   Gln Thr Asp Ile Pro Ser Glu Pro Trp Asn Thr Gly His Asp Trp Ile
   1825                1830                1835                1840
   Leu Ala Asp Lys Arg Pro Thr Ala Trp Phe Leu Pro Ser Ile Arg Ala
                   1845                1850                1855
   Ala Asn Val Met Ala Ala Ser Leu Arg Lys Ala Gly Lys Ser Val Val
               1860                1865                1870
   Val Leu Asn Arg Lys Thr Phe Glu Arg Glu Tyr Pro Thr Ile Lys Gln
           1875                1880                1885
   Lys Lys Pro Asp Phe Ile Leu Ala Thr Asp Ile Ala Glu Met Gly Ala
       1890                1895                1900
   Asn Leu Cys Val Glu Arg Val Leu Asp Cys Arg Thr Ala Phe Lys Pro
   1905                1910                1915                1920
   Val Leu Val Asp Glu Gly Arg Lys Val Ala Ile Lys Gly Pro Leu Arg
                   1925                1930                1935
   Ile Ser Ala Ser Ser Ala Ala Gln Arg Arg Gly Arg Ile Gly Arg Asn
               1940                1945                1950
   Pro Asn Arg Asp Gly Asp Ser Tyr Tyr Tyr Ser Glu Pro Thr Ser Glu
           1955                1960                1965
   Asn Asn Ala His His Val Cys Trp Leu Glu Ala Ser Met Leu Leu Asp
       1970                1975                1980
   Asn Met Glu Val Arg Gly Gly Met Val Ala Pro Leu Tyr Gly Val Glu
   1985                1990                1995                2000
   Gly Thr Lys Thr Pro Val Ser Pro Gly Glu Met Arg Leu Arg Asp Asp
                   2005                2010                2015
   Gln Arg Lys Val Phe Arg Glu Leu Val Arg Asn Cys Asp Leu Pro Val
               2020                2025                2030
   Trp Leu Ser Trp Gln Val Ala Lys Ala Gly Leu Lys Thr Asn Asp Arg
           2035                2040                2045
   Lys Trp Cys Phe Glu Gly Pro Glu Glu His Glu Ile Leu Asn Asp Ser
       2050                2055                2060
   Gly Glu Thr Val Lys Cys Arg Ala Pro Gly Gly Ala Lys Lys Pro Leu
   2065                2070                2075                2080
   Arg Pro Arg Trp Cys Asp Glu Arg Val Ser Ser Asp Gln Ser Ala Leu
                   2085                2090                2095
   Ser Glu Phe Ile Lys Phe Ala Glu Gly Arg Arg Gly Ala Ala Glu Val
               2100                2105                2110
   Leu Val Val Leu Ser Glu Leu Pro Asp Phe Leu Ala Lys Lys Gly Gly
               2115                    2120                    2125
   Glu Ala Met Asp Thr Ile Ser Val Phe Leu His Ser Glu Glu Gly Ser
           2130                    2135                    2140
   Arg Ala Tyr Arg Asn Ala Leu Ser Met Met Pro Glu Ala Met Thr Ile
   2145                    2150                    2155                    2160
   Val Met Leu Phe Ile Leu Ala Gly Leu Leu Thr Ser Gly Met Val Ile
                       2165                    2170                    2175
   Phe Phe Met Ser Pro Lys Gly Ile Ser Arg Met Ser Met Ala Met Gly
                   2180                    2185                    2190
   Thr Met Ala Gly Cys Gly Tyr Leu Met Phe Leu Gly Gly Val Lys Pro
               2195                    2200                    2205
   Thr His Ile Ser Tyr Ile Met Leu Ile Phe Phe Val Leu Met Val Val
           2210                    2215                    2220
   Val Ile Pro Glu Pro Gly Gln Gln Arg Ser Ile Gln Asp Asn Gln Val
   2225                    2230                    2235                    2240
   Ala Tyr Leu Ile Ile Gly Ile Leu Thr Leu Val Ser Ala Val Ala Ala
                       2245                    2250                    2255
   Asn Glu Leu Gly Met Leu Glu Lys Thr Lys Glu Asp Leu Phe Gly Lys
                   2260                    2265                    2270
   Lys Asn Leu Ile Pro Ser Ser Ala Ser Pro Trp Ser Trp Pro Asp Leu
               2275                    2280                    2285
   Asp Leu Lys Pro Gly Ala Ala Trp Thr Val Tyr Val Gly Ile Val Thr
           2290                    2295                    2300
   Met Leu Ser Pro Met Leu His His Trp Ile Lys Val Glu Tyr Gly Asn
   2305                    2310                    2315                    2320
   Leu Ser Leu Ser Gly Ile Ala Gln Ser Ala Ser Val Leu Ser Phe Met
                       2325                    2330                    2335
   Asp Lys Gly Ile Pro Phe Met Lys Met Asn Ile Ser Val Ile Met Leu
                   2340                    2345                    2350
   Leu Val Ser Gly Trp Asn Ser Ile Thr Val Met Pro Leu Leu Cys Gly
               2355                    2360                    2365
   Ile Gly Cys Ala Met Leu His Trp Ser Leu Ile Leu Pro Gly Ile Lys
           2370                    2375                    2380
   Ala Gln Gln Ser Lys Leu Ala Gln Arg Arg Val Phe His Gly Val Ala
   2385                    2390                    2395                    2400
   Lys Asn Pro Val Val Asp Gly Asn Pro Thr Val Asp Ile Glu Glu Ala
                       2405                    2410                    2415
   Pro Glu Met Pro Ala Leu Tyr Glu Lys Lys Leu Ala Leu Tyr Leu Leu
                   2420                    2425                    2430
   Leu Ala Leu Ser Leu Ala Ser Val Ala Met Cys Arg Thr Pro Phe Ser
               2435                    2440                    2445
   Leu Ala Glu Gly Ile Val Leu Ala Ser Ala Ala Leu Gly Pro Leu Ile
           2450                    2455                    2460
   Glu Gly Asn Thr Ser Leu Leu Trp Asn Gly Pro Met Ala Val Ser Met
   2465                    2470                    2475                    2480
   Thr Gly Val Met Arg Gly Asn His Tyr Ala Phe Val Gly Val Met Tyr
                       2485                    2490                    2495
   Asn Leu Trp Lys Met Lys Thr Gly Arg Arg Gly Ser Ala Asn Gly Lys
                   2500                    2505                    2510
   Thr Leu Gly Glu Val Trp Lys Arg Glu Leu Asn Leu Leu Asp Lys Arg
               2515                    2520                    2525
   Gln Phe Glu Leu Tyr Lys Arg Thr Asp Ile Val Glu Val Asp Arg Asp
           2530                    2535                    2540
   Thr Ala Arg Arg His Leu Ala Glu Gly Lys Val Asp Thr Gly Val Ala
   2545                    2550                    2555                    2560
   Val Ser Arg Gly Thr Ala Lys Leu Arg Trp Phe His Glu Arg Gly Tyr
                       2565                    2570                    2575
   Val Lys Leu Glu Gly Arg Val Ile Asp Leu Gly Cys Gly Arg Gly Gly
                   2580                    2585                    2590
   Trp Cys Tyr Tyr Ala Ala Ala Gln Lys Glu Val Ser Gly Val Lys Gly
               2595                    2600                    2605
   Phe Thr Leu Gly Arg Asp Gly His Glu Lys Pro Met Asn Val Gln Ser
           2610                    2615                    2620
   Leu Gly Trp Asn Ile Ile Thr Phe Lys Asp Lys Thr Asp Ile His Arg
   2625                    2630                    2635                    2640
   Leu Glu Pro Val Lys Cys Asp Thr Leu Leu Cys Asp Ile Gly Glu Ser
   2645 2650 2655
   Ser Ser Ser Ser Val Thr Glu Gly Glu Arg Thr Val Arg Val Leu Asp
   2660 2665 2670
   Thr Val Glu Lys Trp Leu Ala Cys Gly Val Asp Asn Phe Cys Val Lys
   2675 2680 2685
   Val Leu Ala Pro Tyr Met Pro Asp Val Leu Glu Lys Leu Glu Leu Leu
   2690 2695 2700
   Gln Arg Arg Phe Gly Gly Thr Val Ile Arg Asn Pro Leu Ser Arg Asn
   2705 2710 2715 2720
   Ser Thr His Glu Met Tyr Tyr Val Ser Gly Ala Arg Ser Asn Val Thr
   2725 2730 2735
   Phe Thr Val Asn Gln Thr Ser Arg Leu Leu Met Arg Arg Met Arg Arg
   2740 2745 2750
   Pro Thr Gly Lys Val Thr Leu Glu Ala Asp Val Ile Leu Pro Ile Gly
   2755 2760 2765
   Thr Arg Ser Val Glu Thr Asp Lys Gly Pro Leu Asp Lys Glu Ala Ile
   2770 2775 2780
   Glu Glu Arg Val Glu Arg Ile Lys Ser Glu Tyr Met Thr Ser Trp Phe
   2785 2790 2795 2800
   Tyr Asp Asn Asp Asn Pro Tyr Arg Thr Trp His Tyr Cys Gly Ser Tyr
   2805 2810 2815
   Val Thr Lys Thr Ser Gly Ser Ala Ala Ser Met Val Asn Gly Val Ile
   2820 2825 2830
   Lys Ile Leu Thr Tyr Pro Trp Asp Arg Ile Glu Glu Val Thr Arg Met
   2835 2840 2845
   Ala Met Thr Asp Thr Thr Pro Phe Gly Gln Gln Arg Val Phe Lys Glu
   2850 2855 2860
   Lys Val Asp Thr Arg Ala Lys Asp Pro Pro Ala Gly Thr Arg Lys Ile
   2865 2870 2875 2880
   Met Lys Val Val Asn Arg Trp Leu Phe Arg His Leu Ala Arg Glu Lys
   2885 2890 2895
   Asn Pro Arg Leu Cys Thr Lys Glu Glu Phe Ile Ala Lys Val Arg Ser
   2900 2905 2910
   His Ala Ala Ile Gly Ala Tyr Leu Glu Glu Gln Glu Gln Trp Lys Thr
   2915 2920 2925
   Ala Asn Glu Ala Val Gln Asp Pro Lys Phe Trp Glu Leu Val Asp Glu
   2930 2935 2940
   Glu Arg Lys Leu His Gln Gln Gly Arg Cys Arg Thr Cys Val Tyr Asn
   2945 2950 2955 2960
   Met Met Gly Lys Arg Glu Lys Lys Leu Ser Glu Phe Gly Lys Ala Lys
   2965 2970 2975
   Gly Ser Arg Ala Ile Trp Tyr Met Trp Leu Gly Ala Arg Tyr Leu Glu
   2980 2985 2990
   Phe Glu Ala Leu Gly Phe Leu Asn Glu Asp His Trp Ala Ser Arg Glu
   2995 3000 3005
   Asn Ser Gly Gly Gly Val Glu Gly Ile Gly Leu Gln Tyr Leu Gly Tyr
   3010 3015 3020
   Val Ile Arg Asp Leu Ala Ala Met Asp Gly Gly Gly Phe Tyr Ala Asp
   3025 3030 3035 3040
   Asp Thr Ala Gly Trp Asp Thr Arg Ile Thr Glu Ala Asp Leu Asp Asp
   3045 3050 3055
   Glu Gln Glu Ile Leu Asn Tyr Met Ser Pro His His Lys Lys Leu Ala
   3060 3065 3070
   Gln Ala Val Met Glu Met Thr Tyr Lys Asn Lys Val Val Lys Val Leu
   3075 3080 3085
   Arg Pro Ala Pro Gly Gly Lys Ala Tyr Met Asp Val Ile Ser Arg Arg
   3090 3095 3100
   Asp Gln Arg Gly Ser Gly Gln Val Val Thr Tyr Ala Leu Asn Thr Ile
   3105 3110 3115 3120
   Thr Asn Leu Lys Val Gln Leu Ile Arg Met Ala Glu Ala Glu Met Val
   3125 3130 3135
   Ile His His Gln His Val Gln Asp Cys Asp Glu Ser Val Leu Thr Arg
   3140 3145 3150
   Leu Glu Ala Trp Leu Thr Glu His Gly Cys Asn Arg Leu Lys Arg Met
           3155                3160                3165
   Ala Val Ser Gly Asp Asp Cys Val Val Arg Pro Ile Asp Asp Arg Phe
       3170                3175                3180
   Gly Leu Ala Leu Ser His Leu Asn Ala Met Ser Lys Val Arg Lys Asp
   3185                3190                3195                3200
   Ile Ser Glu Trp Gln Pro Ser Lys Gly Trp Asn Asp Trp Glu Asn Val
                   3205                3210                3215
   Pro Phe Cys Ser His His Phe His Glu Leu Gln Leu Lys Asp Gly Arg
               3220                3225                3230
   Arg Ile Val Val Pro Cys Arg Glu Gln Asp Glu Leu Ile Gly Arg Gly
           3235                3240                3245
   Arg Val Ser Pro Gly Asn Gly Trp Met Ile Lys Glu Thr Ala Cys Leu
       3250                3255                3260
   Ser Lys Ala Tyr Ala Asn Met Trp Ser Leu Met Tyr Phe His Lys Arg
   3265                3270                3275                3280
   Asp Met Arg Leu Leu Ser Leu Ala Val Ser Ser Ala Val Pro Thr Ser
                   3285                3290                3295
   Trp Val Pro Gln Gly Arg Thr Thr Trp Ser Ile His Gly Lys Gly Glu
               3300                3305                3310
   Trp Met Thr Thr Glu Asp Met Leu Glu Val Trp Asn Arg Val Trp Ile
           3315                3320                3325
   Thr Asn Asn Pro His Met Gln Asp Lys Thr Met Val Lys Lys Trp Arg
       3330                3335                3340
   Asp Val Pro Tyr Leu Thr Lys Arg Gln Asp Lys Leu Cys Gly Ser Leu
   3345                3350                3355                3360
   Ile Gly Met Thr Asn Arg Ala Thr Trp Ala Ser His Ile His Leu Val
                   3365                3370                3375
   Ile His Arg Ile Arg Thr Leu Ile Gly Gln Glu Lys Tyr Thr Asp Tyr
               3380                3385                3390
   Leu Thr Val Met Asp Arg Tyr Ser Val Asp Ala Asp Leu Gln Leu Gly
           3395                3400                3405
   Glu Leu Ile
       3410
<210> 4
   <211> 3411
   <212> PRT
   <213> Flaviviridae Flavivirus Yellow Fever Virus
<400> 4
   Met Ser Gly Arg Lys Ala Gln Gly Lys Thr Leu Gly Val Asn Met Val
    1               5                  10                  15
   Arg Arg Gly Val Arg Ser Leu Ser Asn Lys Ile Lys Gln Lys Thr Lys
               20                  25                  30
   Gln Ile Gly Asn Arg Pro Gly Pro Ser Arg Gly Val Gln Gly Phe Ile
           35                  40                  45
   Phe Phe Phe Leu Phe Asn Ile Leu Thr Gly Lys Lys Ile Thr Ala His
       50                  55                  60
   Leu Lys Arg Leu Trp Lys Met Leu Asp Pro Arg Gln Gly Leu Ala Val
   65                  70                  75                  80
   Leu Arg Lys Val Lys Arg Val Val Ala Ser Leu Met Arg Gly Leu Ser
                   85                  90                  95
   Ser Arg Lys Arg Arg Ser His Asp Val Leu Thr Val Gln Phe Leu Ile
               100                 105                 110
   Leu Gly Met Leu Leu Met Thr Gly Gly Val Thr Leu Val Arg Lys Asn
           115                 120                 125
   Arg Trp Leu Leu Leu Asn Val Thr Ser Glu Asp Leu Gly Lys Thr Phe
       130                 135                 140
   Ser Val Gly Thr Gly Asn Cys Thr Thr Asn Ile Leu Glu Ala Lys Tyr
   145                 150                 155                 160
   Trp Cys Pro Asp Ser Met Glu Tyr Asn Cys Pro Asn Leu Ser Pro Arg
                   165                 170                 175
   Glu Glu Pro Asp Asp Ile Asp Cys Trp Cys Tyr Gly Val Glu Asn Val
               180                 185                 190
   Arg Val Ala Tyr Gly Lys Cys Asp Ser Ala Gly Arg Ser Arg Arg Ser
           195                 200                 205
   Arg Arg Ala Ile Asp Leu Pro Thr His Glu Asn His Gly Leu Lys Thr
       210                 215                 220
   Arg Gln Glu Lys Trp Met Thr Gly Arg Met Gly Glu Arg Gln Leu Gln
   225                 230                 235                 240
   Lys Ile Glu Arg Trp Phe Val Arg Asn Pro Phe Phe Ala Val Thr Ala
                   245                 250                 255
   Leu Thr Ile Ala Tyr Leu Val Gly Ser Asn Met Thr Gln Arg Val Val
               260                 265                 270
   Ile Ala Leu Leu Val Leu Ala Val Gly Pro Ala Tyr Ser Ala His Cys
           275                 280                 285
   Ile Gly Ile Thr Asp Arg Asp Phe Ile Glu Gly Val His Gly Gly Thr
       290                 295                 300
   Trp Val Ser Ala Thr Leu Glu Gln Asp Lys Cys Val Thr Val Met Ala
   305                 310                 315                 320
   Pro Asp Lys Pro Ser Leu Asp Ile Ser Leu Glu Thr Val Ala Ile Asp
                   325                 330                 335
   Arg Pro Ala Glu Val Arg Lys Val Cys Tyr Asn Ala Val Leu Thr His
               340                 345                 350
   Val Lys Ile Asn Asp Lys Cys Pro Ser Thr Gly Glu Ala His Leu Ala
           355                 360                 365
   Glu Glu Asn Glu Gly Asp Asn Ala Cys Lys Arg Thr Tyr Ser Asp Arg
       370                 375                 380
   Gly Trp Gly Asn Gly Cys Gly Leu Phe Gly Lys Gly Ser Ile Val Ala
   385                 390                 395                 400
   Cys Ala Lys Phe Thr Cys Ala Lys Ser Met Ser Leu Phe Glu Val Asp
                   405                 410                 415
   Gln Thr Lys Ile Gln Tyr Val Ile Arg Ala Gln Leu His Val Gly Ala
               420                 425                 430
   Lys Gln Glu Asn Trp Thr Thr Asp Ile Lys Thr Leu Arg Phe Asp Ala
           435                 440                 445
   Leu Ser Gly Ser Gln Glu Val Glu Phe Ile Gly Tyr Gly Lys Ala Thr
       450                 455                 460
   Leu Glu Cys Gln Val Gln Thr Ala Val Asp Phe Gly Asn Ser Tyr Ile
   465                 470                 475                 480
   Ala Glu Met Glu Thr Glu Ser Trp Ile Val Asp Arg Gln Trp Ala Gln
                   485                 490                 495
   Asp Leu Thr Leu Pro Trp Gln Ser Gly Ser Gly Gly Val Trp Arg Glu
               500                 505                 510
   Met His His Leu Val Glu Phe Glu Pro Pro His Ala Ala Thr Ile Arg
           515                 520                 525
   Val Leu Ala Leu Gly Asn Gln Glu Gly Ser Leu Lys Thr Ala Leu Thr
       530                 535                 540
   Gly Ala Met Arg Val Thr Lys Asp Thr Asn Asp Asn Asn Leu Tyr Lys
   545                 550                 555                 560
   Leu His Gly Gly His Val Ser Cys Arg Val Lys Leu Ser Ala Leu Thr
                   565                 570                 575
   Leu Lys Gly Thr Ser Tyr Lys Ile Cys Thr Asp Lys Met Phe Phe Val
               580                 585                 590
   Lys Asn Pro Thr Asp Thr Gly His Gly Thr Val Val Met Gln Val Lys
           595                 600                 605
   Val Ser Lys Gly Ala Pro Cys Arg Ile Pro Val Ile Val Ala Asp Asp
       610                 615                 620
   Leu Thr Ala Ala Ile Asn Lys Gly Ile Leu Val Thr Val Asn Pro Ile
   625                 630                 635                 640
   Ala Ser Thr Asn Asp Asp Glu Val Leu Ile Glu Val Asn Pro Pro Phe
                   645                 650                 655
   Gly Asp Ser Tyr Ile Ile Val Gly Arg Gly Asp Ser Arg Leu Thr Tyr
               660                 665                 670
   Gln Trp His Lys Glu Gly Ser Ser Ile Gly Lys Leu Phe Thr Gln Thr
           675                 680                 685
   Met Lys Gly Val Glu Arg Leu Ala Val Met Gly Asp Thr Ala Trp Asp
       690                 695                 700
   Phe Ser Ser Ala Gly Gly Phe Phe Thr Ser Val Gly Lys Gly Ile His
   705 710 715 720
   Thr Val Phe Gly Ser Ala Phe Gln Gly Leu Phe Gly Gly Leu Asn Trp
   725 730 735
   Ile Thr Lys Val Ile Met Gly Ala Val Leu Ile Trp Val Gly Ile Asn
   740 745 750
   Thr Arg Asn Met Thr Met Ser Met Ser Met Ile Leu Val Gly Val Ile
   755 760 765
   Met Met Phe Leu Ser Leu Gly Val Gly Ala Asp Gln Gly Cys Ala Ile
   770 775 780
   Asn Phe Gly Lys Arg Glu Leu Lys Cys Gly Asp Gly Ile Phe Ile Phe
   785 790 795 800
   Arg Asp Ser Asp Asp Trp Leu Asn Lys Tyr Ser Tyr Tyr Pro Glu Asp
   805 810 815
   Pro Val Lys Leu Ala Ser Ile Val Lys Ala Ser Phe Glu Glu Gly Lys
   820 825 830
   Cys Gly Leu Asn Ser Val Asp Ser Leu Glu His Glu Met Trp Arg Ser
   835 840 845
   Arg Ala Asp Glu Ile Asn Ala Ile Phe Glu Glu Asn Glu Val Asp Ile
   850 855 860
   Ser Val Val Val Gln Asp Pro Lys Asn Val Tyr Gln Arg Gly Thr His
   865 870 875 880
   Pro Phe Ser Arg Ile Arg Asp Gly Leu Gln Tyr Gly Trp Lys Thr Trp
   885 890 895
   Gly Lys Asn Leu Val Phe Ser Pro Gly Arg Lys Asn Gly Ser Phe Ile
   900 905 910
   Ile Asp Gly Lys Ser Arg Lys Glu Cys Pro Phe Ser Asn Arg Val Trp
   915 920 925
   Asn Ser Phe Gln Ile Glu Glu Phe Gly Thr Gly Val Phe Thr Thr Arg
   930 935 940
   Val Tyr Met Asp Ala Val Phe Glu Tyr Thr Ile Asp Cys Asp Gly Ser
   945 950 955 960
   Ile Leu Gly Ala Ala Val Asn Gly Lys Lys Ser Ala His Gly Ser Pro
   965 970 975
   Thr Phe Trp Met Gly Ser His Glu Val Asn Gly Thr Trp Met Ile His
   980 985 990
   Thr Leu Glu Ala Leu Asp Tyr Lys Glu Cys Glu Trp Pro Leu Thr His
   995 1000 1005
   Thr Ile Gly Thr Ser Val Glu Glu Ser Glu Met Phe Met Pro Arg Ser
   1010 1015 1020
   Ile Gly Gly Pro Val Ser Ser His Asn His Ile Pro Gly Tyr Lys Val
   1025 1030 1035 1040
   Gln Thr Asn Gly Pro Trp Met Gln Val Pro Leu Glu Val Lys Arg Glu
   1045 1050 1055
   Ala Cys Pro Gly Thr Ser Val Ile Ile Asp Gly Asn Cys Asp Gly Arg
   1060 1065 1070
   Gly Lys Ser Thr Arg Ser Thr Thr Asp Ser Gly Lys Val Ile Pro Glu
   1075 1080 1085
   Trp Cys Cys Arg Ser Cys Thr Met Pro Pro Val Ser Phe His Gly Ser
   1090 1095 1100
   Asp Gly Cys Trp Tyr Pro Met Glu Ile Arg Pro Arg Lys Thr His Glu
   1105 1110 1115 1120
   Ser His Leu Val Arg Ser Trp Val Thr Ala Gly Glu Ile His Ala Val
   1125 1130 1135
   Pro Phe Gly Leu Val Ser Met Met Ile Ala Met Glu Val Val Leu Arg
   1140 1145 1150
   Lys Arg Gln Gly Pro Lys Gln Met Leu Val Gly Gly Val Val Leu Leu
   1155 1160 1165
   Gly Ala Met Leu Val Gly Gln Val Thr Leu Leu Asp Leu Leu Lys Leu
   1170 1175 1180
   Thr Val Ala Val Gly Leu His Phe His Glu Met Asn Asn Gly Gly Asp
   1185 1190 1195 1200
   Ala Met Tyr Met Ala Leu Ile Ala Ala Phe Ser Ile Arg Pro Gly Leu
   1205 1210 1215
   Leu Ile Gly Phe Gly Leu Arg Thr Leu Trp Ser Pro Arg Glu Arg Leu
   1220 1225 1230
   Val Leu Thr Leu Gly Ala Ala Met Val Glu Ile Ala Leu Gly Gly Val
   1235 1240 1245
   Met Gly Gly Leu Trp Lys Tyr Leu Asn Ala Val Ser Leu Cys Ile Leu
   1250 1255 1260
   Thr Ile Asn Ala Val Ala Ser Arg Lys Ala Ser Asn Thr Ile Leu Pro
   1265 1270 1275 1280
   Leu Met Ala Leu Leu Thr Pro Val Thr Met Ala Glu Val Arg Leu Ala
   1285 1290 1295
   Ala Met Phe Phe Cys Ala Val Val Ile Ile Gly Val Leu His Gln Asn
   1300 1305 1310
   Phe Lys Asp Thr Ser Met Gln Lys Thr Ile Pro Leu Val Ala Leu Thr
   1315 1320 1325
   Leu Thr Ser Tyr Leu Gly Leu Thr Gln Pro Phe Leu Gly Leu Cys Ala
   1330 1335 1340
   Phe Leu Ala Thr Arg Ile Phe Gly Arg Arg Ser Ile Pro Val Asn Glu
   1345 1350 1355 1360
   Ala Leu Ala Ala Ala Gly Leu Val Gly Val Leu Ala Gly Leu Ala Phe
   1365 1370 1375
   Gln Glu Met Glu Asn Phe Leu Gly Pro Ile Ala Val Gly Gly Leu Leu
   1380 1385 1390
   Met Met Leu Val Ser Val Ala Gly Arg Val Asp Gly Leu Glu Leu Lys
   1395 1400 1405
   Lys Leu Gly Glu Val Ser Trp Glu Glu Glu Ala Glu Ile Ser Gly Ser
   1410 1415 1420
   Ser Ala Arg Tyr Asp Val Ala Leu Ser Glu Gln Gly Glu Phe Lys Leu
   1425 1430 1435 1440
   Leu Ser Glu Glu Lys Val Pro Trp Asp Gln Val Val Met Thr Ser Leu
   1445 1450 1455
   Ala Leu Val Gly Ala Ala Leu His Pro Phe Ala Leu Leu Leu Val Leu
   1460 1465 1470
   Ala Gly Trp Leu Phe His Val Arg Gly Ala Arg Arg Ser Gly Asp Val
   1475 1480 1485
   Leu Trp Asp Ile Pro Thr Pro Lys Ile Ile Glu Glu Cys Glu His Leu
   1490 1495 1500
   Glu Asp Gly Ile Tyr Gly Ile Phe Gln Ser Thr Phe Leu Gly Ala Ser
   1505 1510 1515 1520
   Gln Arg Gly Val Gly Val Ala Gln Gly Gly Val Phe His Thr Met Trp
   1525 1530 1535
   His Val Thr Arg Gly Ala Phe Leu Val Arg Asn Gly Lys Lys Leu Ile
   1540 1545 1550
   Pro Ser Trp Ala Ser Val Lys Glu Asp Leu Val Ala Tyr Gly Gly Ser
   1555 1560 1565
   Trp Lys Leu Glu Gly Arg Trp Asp Gly Glu Glu Glu Val Gln Leu Ile
   1570 1575 1580
   Ala Ala Val Pro Gly Lys Asn Val Val Asn Val Gln Thr Lys Pro Ser
   1585 1590 1595 1600
   Leu Phe Lys Val Arg Asn Gly Gly Glu Ile Gly Ala Val Ala Leu Asp
   1605 1610 1615
   Tyr Pro Ser Gly Thr Ser Gly Ser Pro Ile Val Asn Arg Asn Gly Glu
   1620 1625 1630
   Val Ile Gly Leu Tyr Gly Asn Gly Ile Leu Val Gly Asp Asn Ser Phe
   1635 1640 1645
   Val Ser Ala Ile Ser Gln Thr Glu Val Lys Glu Glu Gly Lys Glu Glu
   1650 1655 1660
   Leu Gln Glu Ile Pro Thr Met Leu Lys Lys Gly Met Thr Thr Val Leu
   1665 1670 1675 1680
   Asp Phe His Pro Gly Ala Gly Lys Thr Arg Arg Phe Leu Pro Gln Ile
   1685 1690 1695
   Leu Ala Glu Cys Ala Arg Arg Arg Leu Arg Thr Leu Val Leu Ala Pro
   1700 1705 1710
   Thr Arg Val Val Leu Ser Glu Met Lys Glu Ala Phe His Gly Leu Asp
   1715 1720 1725
   Val Lys Phe His Thr Gln Ala Phe Ser Ala His Gly Ser Gly Arg Glu
   1730 1735 1740
   Val Ile Asp Ala Met Cys His Ala Thr Leu Thr Tyr Arg Met Leu Glu
   1745                1750                1755                1760
   Pro Thr Arg Val Val Asn Trp Glu Val Ile Ile Met Asp Glu Ala His
                   1765                1770                1775
   Phe Leu Asp Pro Ala Ser Ile Ala Ala Arg Gly Trp Ala Ala His Arg
               1780                1785                1790
   Ala Arg Ala Asn Glu Ser Ala Thr Ile Leu Met Thr Ala Thr Pro Pro
           1795                1800                1805
   Gly Thr Ser Asp Glu Phe Pro His Ser Asn Gly Glu Ile Glu Asp Val
       1810                1815                1820
   Gln Thr Asp Ile Pro Ser Glu Pro Trp Asn Thr Gly His Asp Trp Ile
   1825                1830                1835                1840
   Leu Ala Asp Lys Arg Pro Thr Ala Trp Phe Leu Pro Ser Ile Arg Ala
                   1845                1850                1855
   Ala Asn Val Met Ala Ala Ser Leu Arg Lys Ala Gly Lys Ser Val Val
               1860                1865                1870
   Val Leu Asn Arg Lys Thr Phe Glu Arg Glu Tyr Pro Thr Ile Lys Gln
           1875                1880                1885
   Lys Lys Pro Asp Phe Ile Leu Ala Thr Asp Ile Ala Glu Met Gly Ala
       1890                1895                1900
   Asn Leu Cys Val Glu Arg Val Leu Asp Cys Arg Thr Ala Phe Lys Pro
   1905                1910                1915                1920
   Val Leu Val Asp Glu Gly Arg Lys Val Ala Ile Lys Gly Pro Leu Arg
                   1925                1930                1935
   Ile Ser Ala Ser Ser Ala Ala Gln Arg Arg Gly Arg Ile Gly Arg Asn
               1940                1945                1950
   Pro Asn Arg Asp Gly Asp Ser Tyr Tyr Tyr Ser Glu Pro Thr Ser Glu
           1955                1960                1965
   Asn Asn Ala His His Val Cys Trp Leu Glu Ala Ser Met Leu Leu Asp
       1970                1975                1980
   Asn Met Glu Val Arg Gly Gly Met Val Ala Pro Leu Tyr Gly Val Glu
   1985                1990                1995                2000
   Gly Thr Lys Thr Pro Val Ser Pro Gly Glu Met Arg Leu Arg Asp Asp
                   2005                2010                2015
   Gln Arg Lys Val Phe Arg Glu Leu Val Arg Asn Cys Asp Leu Pro Val
               2020                2025                2030
   Trp Leu Ser Trp Gln Val Ala Lys Ala Gly Leu Lys Thr Asn Asp Arg
           2035                2040                2045
   Lys Trp Cys Phe Glu Gly Pro Glu Glu His Glu Ile Leu Asn Asp Ser
       2050                2055                2060
   Gly Glu Thr Val Lys Cys Arg Ala Pro Gly Gly Ala Lys Lys Pro Leu
   2065                2070                2075                2080
   Arg Pro Arg Trp Cys Asp Glu Arg Val Ser Ser Asp Gln Ser Ala Leu
                   2085                2090                2095
   Ser Glu Phe Ile Lys Phe Ala Glu Gly Arg Arg Gly Ala Ala Glu Val
               2100                2105                2110
   Leu Val Val Leu Ser Glu Leu Pro Asp Phe Leu Ala Lys Lys Gly Gly
           2115                2120                2125
   Glu Ala Met Asp Thr Ile Ser Val Phe Leu His Ser Glu Glu Gly Ser
       2130                2135                2140
   Arg Ala Tyr Arg Asn Ala Leu Ser Met Met Pro Glu Ala Met Thr Ile
   2145                2150                2155                2160
   Val Met Leu Phe Ile Leu Ala Gly Leu Leu Thr Ser Gly Met Val Ile
                   2165                2170                2175
   Phe Phe Met Ser Pro Lys Gly Ile Ser Arg Met Ser Met Ala Met Gly
               2180                2185                2190
   Thr Met Ala Gly Cys Gly Tyr Leu Met Phe Leu Gly Gly Val Lys Pro
           2195                2200                2205
   Thr His Ile Ser Tyr Ile Met Leu Ile Phe Phe Val Leu Met Val Val
       2210                2215                2220
   Val Ile Pro Glu Pro Gly Gln Gln Arg Ser Ile Gln Asp Asn Gln Val
   2225                2230                2235                2240
   Ala Tyr Leu Ile Ile Gly Ile Leu Thr Leu Val Ser Ala Val Ala Ala
                   2245                2250                2255
   Asn Glu Leu Gly Met Leu Glu Lys Thr Lys Glu Asp Leu Phe Gly Lys
               2260                2265                2270
   Lys Asn Leu Ile Pro Ser Ser Ala Ser Pro Trp Ser Trp Pro Asp Leu
           2275                2280                2285
   Asp Leu Lys Pro Gly Ala Ala Trp Thr Val Tyr Val Gly Ile Val Thr
       2290                2295                2300
   Met Leu Ser Pro Met Leu His His Trp Ile Lys Val Glu Tyr Gly Asn
   2305                2310                2315                2320
   Leu Ser Leu Ser Gly Ile Ala Gln Ser Ala Ser Val Leu Ser Phe Met
                   2325                2330                2335
   Asp Lys Gly Ile Pro Phe Met Lys Met Asn Ile Ser Val Ile Met Leu
               2340                2345                2350
   Leu Val Ser Gly Trp Asn Ser Ile Thr Val Met Pro Leu Leu Cys Gly
           2355                2360                2365
   Ile Gly Cys Ala Met Leu His Trp Ser Leu Ile Leu Pro Gly Ile Lys
       2370                2375                2380
   Ala Gln Gln Ser Lys Leu Ala Gln Arg Arg Val Phe His Gly Val Ala
   2385                2390                2395                2400
   Lys Asn Pro Val Val Asp Gly Asn Pro Thr Val Asp Ile Glu Glu Ala
                   2405                2410                2415
   Pro Glu Met Pro Ala Leu Tyr Glu Lys Lys Leu Ala Leu Tyr Leu Leu
               2420                2425                2430
   Leu Ala Leu Ser Leu Ala Ser Val Ala Met Cys Arg Thr Pro Phe Ser
           2435                2440                2445
   Leu Ala Glu Gly Ile Val Leu Ala Ser Ala Ala Leu Gly Pro Leu Ile
       2450                2455                2460
   Glu Gly Asn Thr Ser Leu Leu Trp Asn Gly Pro Met Ala Val Ser Met
   2465                2470                2475                2480
   Thr Gly Val Met Arg Gly Asn His Tyr Ala Phe Val Gly Val Met Tyr
                   2485                2490                2495
   Asn Leu Trp Lys Met Lys Thr Gly Arg Arg Gly Ser Ala Asn Gly Lys
               2500                2505                2510
   Thr Leu Gly Glu Val Trp Lys Arg Glu Leu Asn Leu Leu Asp Lys Arg
           2515                2520                2525
   Gln Phe Glu Leu Tyr Lys Arg Thr Asp Ile Val Glu Val Asp Arg Asp
       2530                2535                2540
   Thr Ala Arg Arg His Leu Ala Glu Gly Lys Val Asp Thr Gly Val Ala
   2545                2550                2555                2560
   Val Ser Arg Gly Thr Ala Lys Leu Arg Trp Phe His Glu Arg Gly Tyr
                   2565                2570                2575
   Val Lys Leu Glu Gly Arg Val Ile Asp Leu Gly Cys Gly Arg Gly Gly
               2580                2585                2590
   Trp Cys Tyr Tyr Ala Ala Ala Gln Lys Glu Val Ser Gly Val Lys Gly
           2595                2600                2605
   Phe Thr Leu Gly Arg Asp Gly His Glu Lys Pro Met Asn Val Gln Ser
       2610                2615                2620
   Leu Gly Trp Asn Ile Ile Thr Phe Lys Asp Lys Thr Asp Ile His Arg
   2625                2630                2635                2640
   Leu Glu Pro Val Lys Cys Asp Thr Leu Leu Cys Asp Ile Gly Glu Ser
                   2645                2650                2655
   Ser Ser Ser Ser Val Thr Glu Gly Glu Arg Thr Val Arg Val Leu Asp
               2660                2665                2670
   Thr Val Glu Lys Trp Leu Ala Cys Gly Val Asp Asn Phe Cys Val Lys
           2675                2680                2685
   Val Leu Ala Pro Tyr Met Pro Asp Val Leu Glu Lys Leu Glu Leu Leu
       2690                2695                2700
   Gln Arg Arg Phe Gly Gly Thr Val Ile Arg Asn Pro Leu Ser Arg Asn
   2705                2710                2715                2720
   Ser Thr His Glu Met Tyr Tyr Val Ser Gly Ala Arg Ser Asn Val Thr
                   2725                2730                2735
   Phe Thr Val Asn Gln Thr Ser Arg Leu Leu Met Arg Arg Met Arg Arg
               2740                2745                2750
   Pro Thr Gly Lys Val Thr Leu Glu Ala Asp Val Ile Leu Pro Ile Gly
           2755                2760                2765
   Thr Arg Ser Val Glu Thr Asp Lys Gly Pro Leu Asp Lys Glu Ala Ile
       2770                2775                2780
   Glu Glu Arg Val Glu Arg Ile Lys Ser Glu Tyr Met Thr Ser Trp Phe
   2785                2790                2795                2800
   Tyr Asp Asn Asp Asn Pro Tyr Arg Thr Trp His Tyr Cys Gly Ser Tyr
                   2805                2810                2815
   Val Thr Lys Thr Ser Gly Ser Ala Ala Ser Met Val Asn Gly Val Ile
               2820                2825                2830
   Lys Ile Leu Thr Tyr Pro Trp Asp Arg Ile Glu Glu Val Thr Arg Met
           2835                2840                2845
   Ala Met Thr Asp Thr Thr Pro Phe Gly Gln Gln Arg Val Phe Lys Glu
       2850                2855                2860
   Lys Val Asp Thr Arg Ala Lys Asp Pro Pro Ala Gly Thr Arg Lys Ile
   2865                2870                2875                2880
   Met Lys Val Val Asn Arg Trp Leu Phe Arg His Leu Ala Arg Glu Lys
                   2885                2890                2895
   Asn Pro Arg Leu Cys Thr Lys Glu Glu Phe Ile Ala Lys Val Arg Ser
               2900                2905                2910
   His Ala Ala Ile Gly Ala Tyr Leu Glu Glu Gln Glu Gln Trp Lys Thr
           2915                2920                2925
   Ala Asn Glu Ala Val Gln Asp Pro Lys Phe Trp Glu Leu Val Asp Glu
       2930                2935                2940
   Glu Arg Lys Leu His Gln Gln Gly Arg Cys Arg Thr Cys Val Tyr Asn
   2945                2950                2955                2960
   Met Met Gly Lys Arg Glu Lys Lys Leu Ser Glu Phe Gly Lys Ala Lys
                   2965                2970                2975
   Gly Ser Arg Ala Ile Trp Tyr Met Trp Leu Gly Ala Arg Tyr Leu Glu
               2980                2985                2990
   Phe Glu Ala Leu Gly Phe Leu Asn Glu Asp His Trp Ala Ser Arg Glu
           2995                3000                3005
   Asn Ser Gly Gly Gly Val Glu Gly Ile Gly Leu Gln Tyr Leu Gly Tyr
       3010                3015                3020
   Val Ile Arg Asp Leu Ala Ala Met Asp Gly Gly Gly Phe Tyr Ala Asp
   3025                3030                3035                3040
   Asp Thr Ala Gly Trp Asp Thr Arg Ile Thr Glu Ala Asp Leu Asp Asp
                   3045                3050                3055
   Glu Gln Glu Ile Leu Asn Tyr Met Ser Pro His His Lys Lys Leu Ala
               3060                3065                3070
   Gln Ala Val Met Glu Met Thr Tyr Lys Asn Lys Val Val Lys Val Leu
           3075                3080                3085
   Arg Pro Ala Pro Gly Gly Lys Ala Tyr Met Asp Val Ile Ser Arg Arg
       3090                3095                3100
   Asp Gln Arg Gly Ser Gly Gln Val Val Thr Tyr Ala Leu Asn Thr Ile
   3105                3110                3115                3120
   Thr Asn Leu Lys Val Gln Leu Ile Arg Met Ala Glu Ala Glu Met Val
                   3125                3130                3135
   Ile His His Gln His Val Gln Asp Cys Asp Glu Ser Val Leu Thr Arg
               3140                3145                3150
   Leu Glu Ala Trp Leu Thr Glu His Gly Cys Asn Arg Leu Lys Arg Met
           3155                3160                3165
   Ala Val Ser Gly Asp Asp Cys Val Val Arg Pro Ile Asp Asp Arg Phe
       3170                3175                3180
   Gly Leu Ala Leu Ser His Leu Asn Ala Met Ser Lys Val Arg Lys Asp
   3185                3190                3195                3200
   Ile Ser Glu Trp Gln Pro Ser Lys Gly Trp Asn Asp Trp Glu Asn Val
                   3205                3210                3215
   Pro Phe Cys Ser His His Phe His Glu Leu Gln Leu Lys Asp Gly Arg
               3220                3225                3230
   Arg Ile Val Val Pro Cys Arg Glu Gln Asp Glu Leu Ile Gly Arg Gly
           3235                3240                3245
   Arg Val Ser Pro Gly Asn Gly Trp Met Ile Lys Glu Thr Ala Cys Leu
       3250                3255                3260
   Ser Lys Ala Tyr Ala Asn Met Trp Ser Leu Met Tyr Phe His Lys Arg
   3265                3270                3275                3280
   Asp Met Arg Leu Leu Ser Leu Ala Val Ser Ser Ala Val Pro Thr Ser
                   3285                3290                3295
   Trp Val Pro Gln Gly Arg Thr Thr Trp Ser Ile His Gly Lys Gly Glu
               3300                3305                3310
   Trp Met Thr Thr Glu Asp Met Leu Glu Val Trp Asn Arg Val Trp Ile
   3315 3320 3325
   Thr Asn Asn Pro His Met Gln Asp Lys Thr Met Val Lys Lys Trp Arg
   3330 3335 3340
   Asp Val Pro Tyr Leu Thr Lys Arg Gln Asp Lys Leu Cys Gly Ser Leu
   3345 3350 3355 3360
   Ile Gly Met Thr Asn Arg Ala Thr Trp Ala Ser His Ile His Leu Val
   3365 3370 3375
   Ile His Arg Ile Arg Thr Leu Ile Gly Gln Glu Lys Tyr Thr Asp Tyr
   3380 3385 3390
   Leu Thr Val Met Asp Arg Tyr Ser Val Asp Ala Asp Leu Gln Leu Gly
   3395 3400 3405
   Glu Leu Ile
   3410
<210> 5
   <211> 684
   <212> PRT
   <213> Flaviviridae Flavivirus Yellow Fever Virus
<400> 5
   Ile Leu Gly Met Leu Leu Met Thr Gly Gly Val Thr Leu Val Arg Lys
    1               5                  10                  15
   Asn Arg Trp Leu Leu Leu Asn Val Thr Ser Glu Asp Leu Gly Lys Thr
               20                  25                  30
   Phe Ser Val Gly Thr Gly Asn Cys Thr Thr Asn Ile Leu Glu Ala Lys
           35                  40                  45
   Tyr Trp Cys Pro Asp Ser Met Glu Tyr Asn Cys Pro Asn Leu Ser Pro
       50                  55                  60
   Arg Glu Glu Pro Asp Asp Ile Asp Cys Trp Cys Tyr Gly Val Glu Asn
   65                  70                  75                  80
   Val Arg Val Ala Tyr Gly Lys Cys Asp Ser Ala Gly Arg Ser Arg Arg
                   85                  90                  95
   Ser Arg Arg Ala Ile Asp Leu Pro Thr His Glu Asn His Gly Leu Lys
               100                 105                 110
   Thr Arg Gln Glu Lys Trp Met Thr Gly Arg Met Gly Glu Arg Gln Leu
           115                 120                 125
   Gln Lys Ile Glu Arg Trp Phe Val Arg Asn Pro Phe Phe Ala Val Thr
       130                 135                 140
   Ala Leu Thr Ile Ala Tyr Leu Val Gly Ser Asn Met Thr Gln Arg Val
   145                 150                 155                 160
   Val Ile Ala Leu Leu Val Leu Ala Val Gly Pro Ala Tyr Ser Ala His
                   165                 170                 175
   Cys Ile Gly Ile Thr Asp Arg Asp Phe Ile Glu Gly Val His Gly Gly
               180                 185                 190
   Thr Trp Val Ser Ala Thr Leu Glu Gln Asp Lys Cys Val Thr Val Met
           195                 200                 205
   Ala Pro Asp Lys Pro Ser Leu Asp Ile Ser Leu Glu Thr Val Ala Ile
       210                 215                 220
   Asp Arg Pro Ala Glu Val Arg Lys Val Cys Tyr Asn Ala Val Leu Thr
   225                 230                 235                 240
   His Val Lys Ile Asn Asp Lys Cys Pro Ser Thr Gly Glu Ala His Leu
                   245                 250                 255
   Ala Glu Glu Asn Glu Gly Asp Asn Ala Cys Lys Arg Thr Tyr Ser Asp
               260                 265                 270
   Arg Gly Trp Gly Asn Gly Cys Gly Leu Phe Gly Lys Gly Ser Ile Val
           275                 280                 285
   Ala Cys Ala Lys Phe Thr Cys Ala Lys Ser Met Ser Leu Phe Glu Val
       290                 295                 300
   Asp Gln Thr Lys Ile Gln Tyr Val Ile Arg Ala Gln Leu His Val Gly
   305                 310                 315                 320
   Ala Lys Gln Glu Asn Trp Thr Thr Asp Ile Lys Thr Leu Lys Phe Asp
                   325                 330                 335
   Ala Leu Ser Gly Ser Gln Glu Val Glu Phe Ile Gly Tyr Gly Lys Ala
                   340                 345                 350
   Thr Leu Glu Cys Gln Val Gln Thr Ala Val Asp Phe Gly Asn Ser Tyr
           355                 360                 365
   Ile Ala Glu Met Glu Thr Glu Ser Trp Ile Val Asp Arg Gln Trp Ala
       370                 375                 380
   Gln Asp Leu Thr Leu Pro Trp Gln Ser Gly Ser Gly Gly Val Trp Arg
   385                 390                 395                 400
   Glu Met His His Leu Val Glu Phe Glu Pro Pro His Ala Ala Thr Ile
                   405                 410                 415
   Arg Val Leu Ala Leu Gly Asn Gln Glu Gly Ser Leu Lys Thr Ala Leu
               420                 425                 430
   Thr Gly Ala Met Arg Val Thr Lys Asp Thr Asn Asp Asn Asn Leu Tyr
           435                 440                 445
   Lys Leu His Gly Gly His Val Ser Cys Arg Val Lys Leu Ser Ala Leu
       450                 455                 460
   Thr Leu Lys Gly Thr Ser Tyr Lys Ile Cys Thr Asp Lys Met Phe Phe
   465                 470                 475                 480
   Val Lys Asn Pro Thr Asp Thr Gly His Gly Thr Val Val Met Gln Val
                   485                 490                 495
   Lys Val Ser Lys Gly Ala Pro Cys Arg Ile Pro Val Ile Val Ala Asp
               500                 505                 510
   Asp Leu Thr Ala Ala Ile Asn Lys Gly Ile Leu Val Thr Val Asn Pro
           515                 520                 525
   Ile Ala Ser Thr Asn Asp Asp Glu Val Leu Ile Glu Val Asn Pro Pro
       530                 535                 540
   Phe Gly Asp Ser Tyr Ile Ile Val Gly Arg Gly Asp Ser Arg Leu Thr
   545                 550                 555                 560
   Tyr Gln Trp His Lys Glu Gly Ser Ser Ile Gly Lys Leu Phe Thr Gln
                   565                 570                 575
   Thr Met Lys Gly Val Glu Arg Leu Ala Val Met Gly Asp Thr Ala Trp
               580                 585                 590
   Asp Phe Ser Ser Ala Gly Gly Phe Phe Thr Ser Val Gly Lys Gly Ile
           595                 600                 605
   His Thr Val Phe Gly Ser Ala Phe Gln Gly Leu Phe Gly Gly Leu Asn
       610                 615                 620
   Trp Ile Thr Lys Val Ile Met Gly Ala Val Leu Ile Trp Val Gly Ile
   625                 630                 635                 640
   Asn Thr Arg Asn Met Thr Met Ser Met Ser Met Ile Leu Val Gly Val
                   645                 650                 655
   Ile Met Met Phe Leu Ser Leu Gly Val Gly Ala Asp Gln Gly Cys Ala
               660                 665                 670
   Ile Asn Phe Gly Lys Arg Glu Leu Lys Cys Gly Asp
           675                 680
<210> 6
   <211> 678
   <212> PRT
   <213> Flaviviridae Flavivirus Yellow Fever Virus
<400> 6
   Gly Met Leu Leu Met Thr Gly Gly Val Thr Leu Val Arg Lys Asn Arg
    1               5                  10                  15
   Trp Leu Leu Leu Asn Val Thr Ser Glu Asp Leu Gly Lys Thr Phe Ser
               20                  25                  30
   Val Gly Thr Gly Asn Cys Thr Thr Asn Ile Leu Glu Ala Lys Tyr Trp
           35                  40                  45
   Cys Pro Asp Ser Met Glu Tyr Asn Cys Pro Asn Leu Ser Pro Arg Glu
       50                  55                  60
   Glu Pro Asp Asp Ile Asp Cys Trp Cys Tyr Gly Val Glu Asn Val Arg
   65                  70                  75                  80
   Val Ala Tyr Gly Lys Cys Asp Ser Ala Gly Arg Ser Arg Arg Ser Arg
                   85                  90                  95
   Arg Ala Ile Asp Leu Pro Thr His Glu Asn His Gly Leu Lys Thr Arg
               100                 105                 110
   Gln Glu Lys Trp Met Thr Gly Arg Met Gly Glu Arg Gln Leu Gln Lys
   115 120 125
   Ile Glu Arg Trp Phe Val Arg Asn Pro Phe Phe Ala Val Thr Ala Leu
   130 135 140
   Thr Ile Ala Tyr Leu Val Gly Ser Asn Met Thr Gln Arg Val Val Ile
   145 150 155 160
   Ala Leu Leu Val Leu Ala Val Gly Pro Ala Tyr Ser Ala His Cys Ile
   165 170 175
   Gly Ile Thr Asp Arg Asp Phe Ile Glu Gly Val His Gly Gly Thr Trp
   180 185 190
   Val Ser Ala Thr Leu Glu Gln Asp Lys Cys Val Thr Val Met Ala Pro
   195 200 205
   Asp Lys Pro Ser Leu Asp Ile Ser Leu Glu Thr Val Ala Ile Asp Arg
   210 215 220
   Pro Ala Glu Val Arg Lys Val Cys Tyr Asn Ala Val Leu Thr His Val
   225 230 235 240
   Lys Ile Asn Asp Lys Cys Pro Ser Thr Gly Glu Ala His Leu Ala Glu
   245 250 255
   Glu Asn Glu Gly Asp Asn Ala Cys Lys Arg Thr Tyr Ser Asp Arg Gly
   260 265 270
   Trp Gly Asn Gly Cys Gly Leu Phe Gly Lys Gly Ser Ile Val Ala Cys
   275 280 285
   Ala Lys Phe Thr Cys Ala Lys Ser Met Ser Leu Phe Glu Val Asp Gln
   290 295 300
   Thr Lys Ile Gln Tyr Val Ile Arg Ala Gln Leu His Val Gly Ala Lys
   305 310 315 320
   Gln Glu Asn Trp Thr Thr Asp Ile Lys Thr Leu Arg Phe Asp Ala Leu
   325 330 335
   Ser Gly Ser Gln Glu Val Glu Phe Ile Gly Tyr Gly Lys Ala Thr Leu
   340 345 350
   Glu Cys Gln Val Gln Thr Ala Val Asp Phe Gly Asn Ser Tyr Ile Ala
   355 360 365
   Glu Met Glu Thr Glu Ser Trp Ile Val Asp Arg Gln Trp Ala Gln Asp
   370 375 380
   Leu Thr Leu Pro Trp Gln Ser Gly Ser Gly Gly Val Trp Arg Glu Met
   385 390 395 400
   His His Leu Val Glu Phe Glu Pro Pro His Ala Ala Thr Ile Arg Val
   405 410 415
   Leu Ala Leu Gly Asn Gln Glu Gly Ser Leu Lys Thr Ala Leu Thr Gly
   420 425 430
   Ala Met Arg Val Thr Lys Asp Thr Asn Asp Asn Asn Leu Tyr Lys Leu
   435 440 445
   His Gly Gly His Val Ser Cys Arg Val Lys Leu Ser Ala Leu Thr Leu
   450 455 460
   Lys Gly Thr Ser Tyr Lys Ile Cys Thr Asp Lys Met Phe Phe Val Lys
   465 470 475 480
   Asn Pro Thr Asp Thr Gly His Gly Thr Val Val Met Gln Val Lys Val
   485 490 495
   Ser Lys Gly Ala Pro Cys Arg Ile Pro Val Ile Val Ala Asp Asp Leu
   500 505 510
   Thr Ala Ala Ile Asn Lys Gly Ile Leu Val Thr Val Asn Pro Ile Ala
   515 520 525
   Ser Thr Asn Asp Asp Glu Val Leu Ile Glu Val Asn Pro Pro Phe Gly
   530 535 540
   Asp Ser Tyr Ile Ile Val Gly Arg Gly Asp Ser Arg Leu Thr Tyr Gln
   545 550 555 560
   Trp His Lys Glu Gly Ser Ser Ile Gly Lys Leu Phe Thr Gln Thr Met
   565 570 575
   Lys Gly Val Glu Arg Leu Ala Val Met Gly Asp Thr Ala Trp Asp Phe
   580 585 590
   Ser Ser Ala Gly Gly Phe Phe Thr Ser Val Gly Lys Gly Ile His Thr
   595 600 605
   Val Phe Gly Ser Ala Phe Gln Gly Leu Phe Gly Gly Leu Asn Trp Ile
   610 615 620
   Thr Lys Val Ile Met Gly Ala Val Leu Ile Trp Val Gly Ile Asn Thr
   625                 630                 635                 640
   Arg Asn Met Thr Met Ser Met Ser Met Ile Leu Val Gly Val Ile Met
                   645                 650                 655
   Met Phe Leu Ser Leu Gly Val Gly Ala Asp Gln Gly Cys Ala Ile Asn
               660                 665                 670
   Phe Gly Lys Arg Glu Leu
           675
<210> 7
   <211> 3411
   <212> PRT
   <213> Flaviviridae Flavivirus Yellow Fever Virus
<400> 7
   Met Ser Gly Arg Lys Ala Gln Gly Lys Thr Leu Gly Val Asn Met Val
    1               5                   10                  15
   Arg Arg Gly Val Arg Ser Leu Ser Asn Lys Ile Lys Gln Lys Thr Lys
               20                  25                  30
   Gln Ile Gly Asn Arg Pro Gly Pro Ser Arg Gly Val Gln Gly Phe Ile
           35                  40                  45
   Phe Phe Phe Leu Phe Asn Ile Leu Thr Gly Lys Lys Ile Thr Ala His
       50                  55                  60
   Leu Lys Arg Leu Trp Lys Met Leu Asp Pro Arg Gln Gly Leu Ala Val
   65                  70                  75                  80
   Leu Arg Lys Val Lys Arg Val Val Ala Ser Leu Met Arg Gly Leu Ser
                   85                  90                  95
   Ser Arg Lys Arg Arg Ser His Asp Val Leu Thr Val Gln Phe Leu Ile
               100                 105                 110
   Leu Gly Met Leu Leu Met Thr Gly Gly Val Thr Leu Val Arg Lys Asn
           115                 120                 125
   Arg Trp Leu Leu Leu Asn Val Thr Ser Glu Asp Leu Gly Lys Thr Phe
       130                 135                 140
   Ser Val Gly Thr Gly Asn Cys Thr Thr Asn Ile Leu Glu Ala Lys Tyr
   145                 150                 155                 160
   Trp Cys Pro Asp Ser Met Glu Tyr Asn Cys Pro Asn Leu Ser Pro Arg
                   165                 170                 175
   Glu Glu Pro Asp Asp Ile Asp Cys Trp Cys Tyr Gly Val Glu Asn Val
               180                 185                 190
   Arg Val Ala Tyr Gly Lys Cys Asp Ser Ala Gly Arg Ser Arg Arg Ser
           195                 200                 205
   Arg Arg Ala Ile Asp Leu Pro Thr His Glu Asn His Gly Leu Lys Thr
       210                 215                 220
   Arg Gln Glu Lys Trp Met Thr Gly Arg Met Gly Glu Arg Gln Leu Gln
   225                 230                 235                 240
   Lys Ile Glu Arg Trp Phe Val Arg Asn Pro Phe Phe Ala Val Thr Ala
                   245                 250                 255
   Leu Thr Ile Ala Tyr Leu Val Gly Ser Asn Met Thr Gln Arg Val Val
               260                 265                 270
   Ile Ala Leu Leu Val Leu Ala Val Gly Pro Ala Tyr Ser Ala His Cys
           275                 280                 285
   Ile Gly Ile Thr Asp Arg Asp Phe Ile Glu Gly Val His Gly Gly Thr
       290                 295                 300
   Trp Val Ser Ala Thr Leu Glu Gln Asp Lys Cys Val Thr Val Met Ala
   305                 310                 315                 320
   Pro Asp Lys Pro Ser Leu Asp Ile Ser Leu Glu Thr Val Ala Ile Asp
                   325                 330                 335
   Arg Pro Ala Glu Val Arg Lys Val Cys Tyr Asn Ala Val Leu Thr His
               340                 345                 350
   Val Lys Ile Asn Asp Lys Cys Pro Ser Thr Gly Glu Ala His Leu Ala
           355                 360                 365
   Glu Glu Asn Glu Gly Asp Asn Ala Cys Lys Arg Thr Tyr Ser Asp Arg
       370                 375                 380
   Gly Trp Gly Asn Gly Cys Gly Leu Phe Gly Lys Gly Ser Ile Val Ala
   385                 390                 395                 400
   Cys Ala Lys Phe Thr Cys Ala Lys Ser Met Ser Leu Phe Glu Val Asp
               405                 410                 415
   Gln Thr Lys Ile Gln Tyr Val Ile Arg Ala Gln Leu His Val Gly Ala
           420                 425                 430
   Lys Gln Glu Asn Trp Thr Thr Asp Ile Lys Thr Leu Arg Phe Asp Ala
       435                 440                 445
   Leu Ser Gly Ser Gln Glu Val Glu Phe Ile Gly Tyr Gly Lys Ala Thr
       450                 455                 460
   Leu Glu Cys Gln Val Gln Thr Ala Val Asp Phe Gly Asn Ser Tyr Ile
   465                 470                 475                 480
   Ala Glu Met Glu Thr Glu Ser Trp Ile Val Asp Arg Gln Trp Ala Gln
                   485                 490                 495
   Asp Leu Thr Leu Pro Trp Gln Ser Gly Ser Gly Gly Val Trp Arg Glu
               500                 505                 510
   Met His His Leu Val Glu Phe Glu Pro Pro His Ala Ala Thr Ile Arg
           515                 520                 525
   Val Leu Ala Leu Gly Asn Gln Glu Gly Ser Leu Lys Thr Ala Leu Thr
       530                 535                 540
   Gly Ala Met Arg Val Thr Lys Asp Thr Asn Asp Asn Asn Leu Tyr Lys
   545                 550                 555                 560
   Leu His Gly Gly His Val Ser Cys Arg Val Lys Leu Ser Ala Leu Thr
                   565                 570                 575
   Leu Lys Gly Thr Ser Tyr Lys Ile Cys Thr Asp Lys Met Phe Phe Val
               580                 585                 590
   Lys Asn Pro Thr Asp Thr Gly His Gly Thr Val Val Met Gln Val Lys
           595                 600                 605
   Val Ser Lys Gly Ala Pro Cys Arg Ile Pro Val Ile Val Ala Asp Asp
       610                 615                 620
   Leu Thr Ala Ala Ile Asn Lys Gly Ile Leu Val Thr Val Asn Pro Ile
   625                 630                 635                 640
   Ala Ser Thr Asn Asp Asp Glu Val Leu Ile Glu Val Asn Pro Pro Phe
                   645                 650                 655
   Gly Asp Ser Tyr Ile Ile Val Gly Arg Gly Asp Ser Arg Leu Thr Tyr
               660                 665                 670
   Gln Trp His Lys Glu Gly Ser Ser Ile Gly Lys Leu Phe Thr Gln Thr
           675                 680                 685
   Met Lys Gly Val Glu Arg Leu Ala Val Met Gly Asp Thr Ala Trp Asp
       690                 695                 700
   Phe Ser Ser Ala Gly Gly Phe Phe Thr Ser Val Gly Lys Gly Ile His
   705                 710                 715                 720
   Thr Val Phe Gly Ser Ala Phe Gln Gly Leu Phe Gly Gly Leu Asn Trp
                   725                 730                 735
   Ile Thr Lys Val Ile Met Gly Ala Val Leu Ile Trp Val Gly Ile Asn
               740                 745                 750
   Thr Arg Asn Met Thr Met Ser Met Ser Met Ile Leu Val Gly Val Ile
           755                 760                 765
   Met Met Phe Leu Ser Leu Gly Val Gly Ala Asp Gln Gly Cys Ala Ile
       770                 775                 780
   Asn Phe Gly Lys Arg Glu Leu Lys Cys Gly Asp Gly Ile Phe Ile Phe
   785                 790                 795                 800
   Arg Asp Ser Asp Asp Trp Leu Asn Lys Tyr Ser Tyr Tyr Pro Glu Asp
                   805                 810                 815
   Pro Val Lys Leu Ala Ser Ile Val Lys Ala Ser Phe Glu Glu Gly Lys
               820                 825                 830
   Cys Gly Leu Asn Ser Val Asp Ser Leu Glu His Glu Met Trp Arg Ser
           835                 840                 845
   Arg Ala Asp Glu Ile Asn Ala Ile Phe Glu Glu Asn Glu Val Asp Ile
       850                 855                 860
   Ser Val Val Val Gln Asp Pro Lys Asn Val Tyr Gln Arg Gly Thr His
   865                 870                 875                 880
   Pro Phe Ser Arg Ile Arg Asp Gly Leu Gln Tyr Gly Trp Lys Thr Trp
                   885                 890                 895
   Gly Lys Asn Leu Val Phe Ser Pro Gly Arg Lys Asn Gly Ser Phe Ile
               900                 905                 910
   Ile Asp Gly Lys Ser Arg Lys Glu Cys Pro Phe Ser Asn Arg Val Trp
           915                 920                 925
   Asn Ser Phe Gln Ile Glu Glu Phe Gly Thr Gly Val Phe Thr Thr Arg
       930                 935                 940
   Val Tyr Met Asp Ala Val Phe Glu Tyr Thr Ile Asp Cys Asp Gly Ser
   945                 950                 955                 960
   Ile Leu Gly Ala Ala Val Asn Gly Lys Lys Ser Ala His Gly Ser Pro
                   965                 970                 975
   Thr Phe Trp Met Gly Ser His Glu Val Asn Gly Thr Trp Met Ile His
               980                 985                 990
   Thr Leu Glu Ala Leu Asp Tyr Lys Glu Cys Glu Trp Pro Leu Thr His
           995                 1000                1005
   Thr Ile Gly Thr Ser Val Glu Glu Ser Glu Met Phe Met Pro Arg Ser
       1010                1015                1020
   Ile Gly Gly Pro Val Ser Ser His Asn His Ile Pro Gly Tyr Lys Val
   1025                1030                1035                1040
   Gln Thr Asn Gly Pro Trp Met Gln Val Pro Leu Glu Val Lys Arg Glu
                   1045                1050                1055
   Ala Cys Pro Gly Thr Ser Val Ile Ile Asp Gly Asn Cys Asp Gly Arg
               1060                1065                1070
   Gly Lys Ser Thr Arg Ser Thr Thr Asp Ser Gly Lys Val Ile Pro Glu
           1075                1080                1085
   Trp Cys Cys Arg Ser Cys Ile Met Pro Pro Val Ser Phe His Gly Ser
       1090                1095                1100
   Asp Gly Cys Trp Tyr Pro Met Glu Ile Arg Pro Arg Lys Thr His Glu
   1105                1110                1115                1120
   Ser His Leu Val Arg Ser Trp Val Thr Ala Gly Glu Ile His Ala Val
                   1125                1130                1135
   Pro Phe Gly Leu Val Ser Met Met Ile Ala Met Glu Val Val Leu Arg
               1140                1145                1150
   Lys Arg Gln Gly Pro Lys Gln Met Leu Val Gly Gly Val Val Leu Leu
           1155                1160                1165
   Gly Ala Met Leu Val Gly Gln Val Thr Leu Leu Asp Leu Leu Lys Leu
       1170                1175                1180
   Thr Val Ala Val Gly Leu His Phe His Glu Met Asn Asn Gly Gly Asp
   1185                1190                1195                1200
   Ala Met Tyr Met Ala Leu Ile Ala Ala Phe Ser Ile Arg Pro Gly Leu
                   1205                1210                1215
   Leu Ile Gly Phe Gly Leu Arg Thr Leu Trp Ser Pro Arg Glu Arg Leu
               1220                1225                1230
   Val Leu Thr Leu Gly Ala Ala Met Val Glu Ile Ala Leu Gly Gly Val
           1235                1240                1245
   Met Gly Gly Leu Trp Lys Tyr Leu Asn Ala Val Ser Leu Cys Ile Leu
       1250                1255                1260
   Thr Ile Asn Ala Val Ala Ser Arg Lys Ala Ser Asn Thr Ile Leu Pro
   1265                1270                1275                1280
   Leu Met Ala Leu Leu Thr Pro Val Thr Met Ala Glu Val Arg Leu Ala
                   1285                1290                1295
   Ala Met Phe Leu Cys Ala Val Val Ile Ile Gly Val Leu His Gln Asn
               1300                1305                1310
   Phe Lys Asp Thr Ser Met Gln Lys Thr Ile Pro Leu Val Ala Leu Thr
           1315                1320                1325
   Leu Thr Ser Tyr Leu Gly Leu Thr Gln Pro Phe Leu Gly Leu Cys Ala
       1330                1335                1340
   Phe Leu Ala Thr Arg Ile Phe Gly Arg Arg Ser Ile Pro Val Asn Glu
   1345                1350                1355                1360
   Ala Leu Ala Ala Ala Gly Leu Val Gly Val Leu Ala Gly Leu Ala Phe
                   1365                1370                1375
   Gln Glu Met Glu Asn Phe Leu Gly Pro Ile Ala Val Gly Gly Leu Leu
               1380                1385                1390
   Met Met Leu Val Ser Val Ala Gly Arg Val Asp Gly Leu Glu Leu Lys
           1395                1400                1405
   Lys Leu Gly Glu Val Ser Trp Glu Glu Glu Ala Glu Ile Ser Gly Ser
       1410                1415                1420
   Ser Ala Arg Tyr Asp Val Ala Leu Ser Glu Gln Gly Glu Phe Lys Leu
   1425                1430                1435                1440
   Leu Ser Glu Glu Lys Val Pro Trp Asp Gln Val Val Met Thr Ser Leu
   1445 1450 1455
   Ala Leu Val Gly Ala Ala Leu His Pro Phe Ala Leu Leu Leu Val Leu
   1460 1465 1470
   Ala Gly Trp Leu Phe His Val Arg Gly Ala Arg Arg Ser Gly Asp Val
   1475 1480 1485
   Leu Trp Asp Ile Pro Thr Pro Lys Ile Ile Glu Glu Cys Glu His Leu
   1490 1495 1500
   Glu Asp Gly Ile Tyr Gly Ile Phe Gln Ser Thr Phe Leu Gly Ala Ser
   1505 1510 1515 1520
   Gln Arg Gly Val Gly Val Ala Gln Gly Gly Val Phe His Thr Met Trp
   1525 1530 1535
   His Val Thr Arg Gly Ala Phe Leu Val Arg Asn Gly Lys Lys Leu Ile
   1540 1545 1550
   Pro Ser Trp Ala Ser Val Lys Glu Asp Leu Val Ala Tyr Gly Gly Ser
   1555 1560 1565
   Trp Lys Leu Glu Gly Arg Trp Asp Gly Glu Glu Glu Val Gln Leu Ile
   1570 1575 1580
   Ala Ala Val Pro Gly Lys Asn Val Val Asn Val Gln Thr Lys Pro Ser
   1585 1590 1595 1600
   Leu Phe Lys Val Arg Asn Gly Gly Glu Ile Gly Ala Val Ala Leu Asp
   1605 1610 1615
   Tyr Pro Ser Gly Thr Ser Gly Ser Pro Ile Val Asn Arg Asn Gly Glu
   1620 1625 1630
   Val Ile Gly Leu Tyr Gly Asn Gly Ile Leu Val Gly Asp Asn Ser Phe
   1635 1640 1645
   Val Ser Ala Ile Ser Gln Thr Glu Val Lys Glu Glu Gly Lys Glu Glu
   1650 1655 1660
   Leu Gln Glu Ile Pro Thr Met Leu Lys Lys Gly Met Thr Thr Val Leu
   1665 1670 1675 1680
   Asp Phe His Pro Gly Ala Gly Lys Thr Arg Arg Phe Leu Pro Gln Ile
   1685 1690 1695
   Leu Ala Glu Cys Ala Arg Arg Arg Leu Arg Thr Leu Val Leu Ala Pro
   1700 1705 1710
   Thr Arg Val Val Leu Ser Glu Met Lys Glu Ala Phe His Gly Leu Asp
   1715 1720 1725
   Val Lys Phe His Thr Gln Ala Phe Ser Ala His Gly Ser Gly Arg Glu
   1730 1735 1740
   Val Ile Asp Ala Met Cys His Ala Thr Leu Thr Tyr Arg Met Leu Glu
   1745 1750 1755 1760
   Pro Thr Arg Val Val Asn Trp Glu Val Ile Ile Met Asp Glu Ala His
   1765 1770 1775
   Phe Leu Asp Pro Ala Ser Ile Ala Ala Arg Gly Trp Ala Ala His Arg
   1780 1785 1790
   Ala Arg Ala Asn Glu Ser Ala Thr Ile Leu Met Thr Ala Thr Pro Pro
   1795 1800 1805
   Gly Thr Ser Asp Glu Phe Pro His Ser Asn Gly Glu Ile Glu Asp Val
   1810 1815 1820
   Gln Thr Asp Ile Pro Ser Glu Pro Trp Asn Thr Gly His Asp Trp Ile
   1825 1830 1835 1840
   Leu Ala Asp Lys Arg Pro Thr Ala Trp Phe Leu Pro Ser Ile Arg Ala
   1845 1850 1855
   Ala Asn Val Met Ala Ala Ser Leu Arg Lys Ala Gly Lys Ser Val Val
   1860 1865 1870
   Val Leu Asn Arg Lys Thr Phe Glu Arg Glu Tyr Pro Thr Ile Lys Gln
   1875 1880 1885
   Lys Lys Pro Asp Phe Ile Leu Ala Thr Asp Ile Ala Glu Met Gly Ala
   1890 1895 1900
   Asn Leu Cys Val Glu Arg Val Leu Asp Cys Arg Thr Ala Phe Lys Pro
   1905 1910 1915 1920
   Val Leu Val Asp Glu Gly Arg Lys Val Ala Ile Lys Gly Pro Leu Arg
   1925 1930 1935
   Ile Ser Ala Ser Ser Ala Ala Gln Arg Arg Gly Arg Ile Gly Arg Asn
   1940 1945 1950
   Pro Asn Arg Asp Gly Asp Ser Tyr Tyr Tyr Ser Glu Pro Thr Ser Glu
           1955                1960                1965
   Asn Asn Ala His His Val Cys Trp Leu Glu Ala Ser Met Leu Leu Asp
       1970                1975                1980
   Asn Met Glu Val Arg Gly Gly Met Val Ala Pro Leu Tyr Gly Val Glu
   1985                1990                1995                2000
   Gly Thr Lys Thr Pro Val Ser Pro Gly Glu Met Arg Leu Arg Asp Asp
                   2005                2010                2015
   Gln Arg Lys Val Phe Arg Glu Leu Val Arg Asn Cys Asp Leu Pro Val
               2020                2025                2030
   Trp Leu Ser Trp Gln Val Ala Lys Ala Gly Leu Lys Thr Asn Asp Arg
           2035                2040                2045
   Lys Trp Cys Phe Glu Gly Pro Glu Glu His Glu Ile Leu Asn Asp Ser
       2050                2055                2060
   Gly Glu Thr Val Lys Cys Arg Ala Pro Gly Gly Ala Lys Lys Pro Leu
   2065                2070                2075                2080
   Arg Pro Arg Trp Cys Asp Glu Arg Val Ser Ser Asp Gln Ser Ala Leu
                   2085                2090                2095
   Ser Glu Phe Ile Lys Phe Ala Glu Gly Arg Arg Gly Ala Ala Glu Val
               2100                2105                2110
   Leu Val Val Leu Ser Glu Leu Pro Asp Phe Leu Ala Lys Lys Gly Gly
           2115                2120                2125
   Glu Ala Met Asp Thr Ile Ser Val Phe Leu His Ser Glu Glu Gly Ser
       2130                2135                2140
   Arg Ala Tyr Arg Asn Ala Leu Ser Met Met Pro Glu Ala Met Thr Ile
   2145                2150                2155                2160
   Val Met Leu Phe Ile Leu Ala Gly Leu Leu Thr Ser Gly Met Val Ile
                   2165                2170                2175
   Phe Phe Met Ser Pro Lys Gly Ile Ser Arg Met Ser Met Ala Met Gly
               2180                2185                2190
   Thr Met Ala Gly Cys Gly Tyr Leu Met Phe Leu Gly Gly Val Lys Pro
           2195                2200                2205
   Thr His Ile Ser Tyr Ile Met Leu Ile Phe Phe Val Leu Met Val Val
       2210                2215                2220
   Val Ile Pro Glu Pro Gly Gln Gln Arg Ser Ile Gln Asp Asn Gln Val
   2225                2230                2235                2240
   Ala Tyr Leu Ile Ile Gly Ile Leu Thr Leu Val Ser Ala Val Ala Ala
                   2245                2250                2255
   Asn Glu Leu Gly Met Leu Glu Lys Thr Lys Glu Asp Leu Phe Gly Lys
               2260                2265                2270
   Lys Asn Leu Ile Pro Ser Ser Ala Ser Pro Trp Ser Trp Pro Asp Leu
           2275                2280                2285
   Asp Leu Lys Pro Gly Ala Ala Trp Thr Val Tyr Val Gly Ile Val Thr
       2290                2295                2300
   Met Leu Ser Pro Met Leu His His Trp Ile Lys Val Glu Tyr Gly Asn
   2305                2310                2315                2320
   Leu Ser Leu Ser Gly Ile Ala Gln Ser Ala Ser Val Leu Ser Phe Met
                   2325                2330                2335
   Asp Lys Gly Ile Pro Phe Met Lys Met Asn Ile Ser Val Ile Met Leu
               2340                2345                2350
   Leu Val Ser Gly Trp Asn Ser Ile Thr Val Met Pro Leu Leu Cys Gly
           2355                2360                2365
   Ile Gly Cys Ala Met Leu His Trp Ser Leu Ile Leu Pro Gly Ile Lys
       2370                2375                2380
   Ala Gln Gln Ser Lys Leu Ala Gln Arg Arg Val Phe His Gly Val Ala
   2385                2390                2395                2400
   Lys Asn Pro Val Val Asp Gly Asn Pro Thr Val Asp Ile Glu Glu Ala
                   2405                2410                2415
   Pro Glu Met Pro Ala Leu Tyr Glu Lys Lys Leu Ala Leu Tyr Leu Leu
               2420                2425                2430
   Leu Ala Leu Ser Leu Ala Ser Val Ala Met Cys Arg Thr Pro Phe Ser
           2435                2440                2445
   Leu Ala Glu Gly Ile Val Leu Ala Ser Ala Ala Leu Gly Pro Leu Ile
       2450                2455                2460
   Glu Gly Asn Thr Ser Leu Leu Trp Asn Gly Pro Met Ala Val Ser Met
   2465                2470                2475                2480
   Thr Gly Val Met Arg Gly Asn His Tyr Ala Phe Val Gly Val Met Tyr
                   2485                2490                2495
   Asn Leu Trp Lys Met Lys Thr Gly Arg Arg Gly Ser Ala Asn Gly Lys
               2500                2505                2510
   Thr Leu Gly Glu Val Trp Lys Arg Glu Leu Asn Leu Leu Asp Lys Arg
           2515                2520                2525
   Gln Phe Glu Leu Tyr Lys Arg Thr Asp Ile Val Glu Val Asp Arg Asp
       2530                2535                2540
   Thr Ala Arg Arg His Leu Ala Glu Gly Lys Val Asp Thr Gly Val Ala
   2545                2550                2555                2560
   Val Ser Arg Gly Thr Ala Lys Leu Arg Trp Phe His Glu Arg Gly Tyr
                   2565                2570                2575
   Val Lys Leu Glu Gly Arg Val Ile Asp Leu Gly Cys Gly Arg Gly Gly
               2580                2585                2590
   Trp Cys Tyr Tyr Ala Ala Ala Gln Lys Glu Val Ser Gly Val Lys Gly
           2595                2600                2605
   Phe Thr Leu Gly Arg Asp Gly His Glu Lys Pro Met Asn Val Gln Ser
       2610                2615                2620
   Leu Gly Trp Asn Ile Ile Thr Phe Lys Asp Lys Thr Asp Ile His Arg
   2625                2630                2635                2640
   Leu Glu Pro Val Lys Cys Asp Thr Leu Leu Cys Asp Ile Gly Glu Ser
                   2645                2650                2655
   Ser Ser Ser Ser Val Thr Glu Gly Glu Arg Thr Val Arg Val Leu Asp
               2660                2665                2670
   Thr Val Glu Lys Trp Leu Ala Cys Gly Val Asp Asn Phe Cys Val Lys
           2675                2680                2685
   Val Leu Ala Pro Tyr Met Pro Asp Val Leu Glu Lys Leu Glu Leu Leu
       2690                2695                2700
   Gln Arg Arg Phe Gly Gly Thr Val Ile Arg Asn Pro Leu Ser Arg Asn
   2705                2710                2715                2720
   Ser Thr His Glu Met Tyr Tyr Val Ser Gly Ala Arg Ser Asn Val Thr
                   2725                2730                2735
   Phe Thr Val Asn Gln Thr Ser Arg Leu Leu Met Arg Arg Met Arg Arg
               2740                2745                2750
   Pro Thr Gly Lys Val Thr Leu Glu Ala Asp Val Ile Leu Pro Ile Gly
           2755                2760                2765
   Thr Arg Ser Val Glu Thr Asp Lys Gly Pro Leu Asp Lys Glu Ala Ile
       2770                2775                2780
   Glu Glu Arg Val Glu Arg Ile Lys Ser Glu Tyr Met Thr Ser Trp Phe
   2785                2790                2795                2800
   Tyr Asp Asn Asp Asn Pro Tyr Arg Thr Trp His Tyr Cys Gly Ser Tyr
                   2805                2810                2815
   Val Thr Lys Thr Ser Gly Ser Ala Ala Ser Met Val Asn Gly Val Ile
               2820                2825                2830
   Lys Ile Leu Thr Tyr Pro Trp Asp Arg Ile Glu Glu Val Thr Arg Met
           2835                2840                2845
   Ala Met Thr Asp Thr Thr Pro Phe Gly Gln Gln Arg Val Phe Lys Glu
       2850                2855                2860
   Lys Val Asp Thr Arg Ala Lys Asp Pro Pro Ala Gly Thr Arg Lys Ile
   2865                2870                2875                2880
   Met Lys Val Val Asn Arg Trp Leu Phe Arg His Leu Ala Arg Glu Lys
                   2885                2890                2895
   Asn Pro Arg Leu Cys Thr Lys Glu Glu Phe Ile Ala Lys Val Arg Ser
               2900                2905                2910
   His Ala Ala Ile Gly Ala Tyr Leu Glu Glu Gln Glu Gln Trp Lys Thr
           2915                2920                2925
   Ala Asn Glu Ala Val Gln Asp Pro Lys Phe Trp Glu Leu Val Asp Glu
       2930                2935                2940
   Glu Arg Lys Leu His Gln Gln Gly Arg Cys Arg Thr Cys Val Tyr Asn
   2945                2950                2955                2960
   Met Met Gly Lys Arg Glu Lys Lys Leu Ser Glu Phe Gly Lys Ala Lys
                   2965                2970                2975
   Gly Ser Arg Ala Ile Trp Tyr Met Trp Leu Gly Ala Arg Tyr Leu Glu
               2980                2985                2990
   Phe Glu Ala Leu Gly Phe Leu Asn Glu Asp His Trp Ala Ser Arg Glu
           2995                3000                3005
   Asn Ser Gly Gly Gly Val Glu Gly Ile Gly Leu Gln Tyr Leu Gly Tyr
       3010                3015                3020
   Val Ile Arg Asp Leu Ala Ala Met Asp Gly Gly Gly Phe Tyr Ala Asp
   3025                3030                3035                3040
   Asp Thr Ala Gly Trp Asp Thr Arg Ile Thr Glu Ala Asp Leu Asp Asp
                   3045                3050                3055
   Glu Gln Glu Ile Leu Asn Tyr Met Ser Pro His His Lys Lys Leu Ala
               3060                3065                3070
   Gln Ala Val Met Glu Met Thr Tyr Lys Asn Lys Val Val Lys Val Leu
           3075                3080                3085
   Arg Pro Ala Pro Gly Gly Lys Ala Tyr Met Asp Val Ile Ser Arg Arg
       3090                3095                3100
   Asp Gln Arg Gly Ser Gly Gln Val Val Thr Tyr Ala Leu Asn Thr Ile
   3105                3110                3115                3120
   Thr Asn Leu Lys Val Gln Leu Ile Arg Met Ala Glu Ala Glu Met Val
                   3125                3130                3135
   Ile His His Gln His Val Gln Asp Cys Asp Glu Ser Val Leu Thr Arg
               3140                3145                3150
   Leu Glu Ala Trp Leu Thr Glu His Gly Cys Asn Arg Leu Lys Arg Met
           3155                3160                3165
   Ala Val Ser Gly Asp Asp Cys Val Val Arg Pro Ile Asp Asp Arg Phe
       3170                3175                3180
   Gly Leu Ala Leu Ser His Leu Asn Ala Met Ser Lys Val Arg Lys Asp
   3185                3190                3195                3200
   Ile Ser Glu Trp Gln Pro Ser Lys Gly Trp Asn Asp Trp Glu Asn Val
                   3205                3210                3215
   Pro Phe Cys Ser His His Phe His Glu Leu Gln Leu Lys Asp Gly Arg
               3220                3225                3230
   Arg Ile Val Val Pro Cys Arg Glu Gln Asp Glu Leu Ile Gly Arg Gly
           3235                3240                3245
   Arg Val Ser Pro Gly Asn Gly Trp Met Ile Lys Glu Thr Ala Cys Leu
       3250                3255                3260
   Ser Lys Ala Tyr Ala Asn Met Trp Ser Leu Met Tyr Phe His Lys Arg
   3265                3270                3275                3280
   Asp Met Arg Leu Leu Ser Leu Ala Val Ser Ser Ala Val Pro Thr Ser
                   3285                3290                3295
   Trp Val Pro Gln Gly Arg Thr Thr Trp Ser Ile His Gly Lys Gly Glu
               3300                3305                3310
   Trp Met Thr Thr Glu Asp Met Leu Glu Val Trp Asn Arg Val Trp Ile
           3315                3320                3325
   Thr Asn Asn Pro His Met Gln Asp Lys Thr Met Val Lys Lys Trp Arg
       3330                3335                3340
   Asp Val Pro Tyr Leu Thr Lys Arg Gln Asp Lys Leu Cys Gly Ser Leu
   3345                3350                3355                3360
   Ile Gly Met Thr Asn Arg Ala Thr Trp Ala Ser His Ile His Leu Val
                   3365                3370                3375
   Ile His Arg Ile Arg Thr Leu Ile Gly Gln Glu Lys Tyr Thr Asp Tyr
               3380                3385                3390
   Leu Thr Val Met Asp Arg Tyr Ser Val Asp Ala Asp Leu Gln Leu Gly
           3395                3400                3405
   Glu Leu Ile
       3410
<210> 8
   <211> 3411
   <212> PRT
   <213> Flaviviridae Flavivirus Yellow Fever Virus
<400> 8
   Met Ser Gly Arg Lys Ala Gln Gly Lys Thr Leu Gly Val Asn Met Val
    1              5                   10                  15
   Arg Arg Gly Val Arg Ser Leu Ser Asn Lys Ile Lys Gln Lys Thr Lys
               20                  25                  30
   Gln Ile Gly Asn Arg Pro Gly Pro Ser Arg Gly Val Gln Gly Phe Ile
           35                  40                  45
   Phe Phe Phe Leu Phe Asn Ile Leu Thr Gly Lys Lys Ile Thr Ala His
       50                  55                  60
   Leu Lys Arg Leu Trp Lys Met Leu Asp Pro Arg Gln Gly Leu Ala Val
   65                  70                  75                  80
   Leu Arg Lys Val Lys Arg Val Val Ala Ser Leu Met Arg Gly Leu Ser
                   85                  90                  95
   Ser Arg Lys Arg Arg Ser His Asp Val Leu Thr Val Gln Phe Leu Ile
               100                 105                 110
   Leu Gly Met Leu Leu Met Thr Gly Gly Val Thr Leu Val Arg Lys Asn
           115                 120                 125
   Arg Trp Leu Leu Leu Asn Val Thr Ser Glu Asp Leu Gly Lys Thr Phe
       130                 135                 140
   Ser Val Gly Thr Gly Asn Cys Thr Thr Asn Ile Leu Glu Ala Lys Tyr
   145                 150                 155                 160
   Trp Cys Pro Asp Ser Met Glu Tyr Asn Cys Pro Asn Leu Ser Pro Arg
                   165                 170                 175
   Glu Glu Pro Asp Asp Ile Asp Cys Trp Cys Tyr Gly Val Glu Asn Val
               180                 185                 190
   Arg Val Ala Tyr Gly Lys Cys Asp Ser Ala Gly Arg Ser Arg Arg Ser
           195                 200                 205
   Arg Arg Ala Ile Asp Leu Pro Thr His Glu Asn His Gly Leu Lys Thr
       210                 215                 220
   Arg Gln Glu Lys Trp Met Thr Gly Arg Met Gly Glu Arg Gln Leu Gln
   225                 230                 235                 240
   Lys Ile Glu Arg Trp Phe Val Arg Asn Pro Phe Phe Ala Val Thr Ala
                   245                 250                 255
   Leu Thr Ile Ala Tyr Leu Val Gly Ser Asn Met Thr Gln Arg Val Val
               260                 265                 270
   Ile Ala Leu Leu Val Leu Ala Val Gly Pro Ala Tyr Ser Ala His Cys
           275                 280                 285
   Ile Gly Ile Thr Asp Arg Asp Phe Ile Glu Gly Val His Gly Gly Thr
       290                 295                 300
   Trp Val Ser Ala Thr Leu Glu Gln Asp Lys Cys Val Thr Val Met Ala
   305                 310                 315                 320
   Pro Asp Lys Pro Ser Leu Asp Ile Ser Leu Glu Thr Val Ala Ile Asp
                   325                 330                 335
   Arg Pro Ala Glu Val Arg Lys Val Cys Tyr Asn Ala Val Leu Thr His
               340                 345                 350
   Val Lys Ile Asn Asp Lys Cys Pro Ser Thr Gly Glu Ala His Leu Ala
           355                 360                 365
   Glu Glu Asn Glu Gly Asp Asn Ala Cys Lys Arg Thr Tyr Ser Asp Arg
       370                 375                 380
   Gly Trp Gly Asn Gly Cys Gly Leu Phe Gly Lys Gly Ser Ile Val Ala
   385                 390                 395                 400
   Cys Ala Lys Phe Thr Cys Ala Lys Ser Met Ser Leu Phe Glu Val Asp
                   405                 410                 415
   Gln Thr Lys Ile Gln Tyr Val Ile Arg Ala Gln Leu His Val Gly Ala
               420                 425                 430
   Lys Gln Glu Asn Trp Thr Thr Asp Ile Lys Thr Leu Lys Phe Asp Ala
           435                 440                 445
   Leu Ser Gly Ser Gln Glu Val Glu Phe Ile Gly Tyr Gly Lys Ala Thr
       450                 455                 460
   Leu Glu Cys Gln Val Gln Thr Ala Val Asp Phe Gly Asn Ser Tyr Ile
   465                 470                 475                 480
   Ala Glu Met Glu Thr Glu Ser Trp Ile Val Asp Arg Gln Trp Ala Gln
                   485                 490                 495
   Asp Leu Thr Leu Pro Trp Gln Ser Gly Ser Gly Gly Val Trp Arg Glu
               500                 505                 510
   Met His His Leu Val Glu Phe Glu Pro Pro His Ala Ala Thr Ile Arg
           515                 520                 525
   Val Leu Ala Leu Gly Asn Gln Glu Gly Ser Leu Lys Thr Ala Leu Thr
       530                 535                 540
   Gly Ala Met Arg Val Thr Lys Asp Thr Asn Asp Asn Asn Leu Tyr Lys
   545                 550                 555                 560
   Leu His Gly Gly His Val Ser Cys Arg Val Lys Leu Ser Ala Leu Thr
                   565                 570                 575
   Leu Lys Gly Thr Ser Tyr Lys Ile Cys Thr Asp Lys Met Phe Phe Val
               580                 585                 590
   Lys Asn Pro Thr Asp Thr Gly His Gly Thr Val Val Met Gln Val Lys
           595                 600                 605
   Val Ser Lys Gly Ala Pro Cys Arg Ile Pro Val Ile Val Ala Asp Asp
       610                 615                 620
   Leu Thr Ala Ala Ile Asn Lys Gly Ile Leu Val Thr Val Asn Pro Ile
   625                 630                 635                 640
   Ala Ser Thr Asn Asp Asp Glu Val Leu Ile Glu Val Asn Pro Pro Phe
                   645                 650                 655
   Gly Asp Ser Tyr Ile Ile Val Gly Arg Gly Asp Ser Arg Leu Thr Tyr
               660                 665                 670
   Gln Trp His Lys Glu Gly Ser Ser Ile Gly Lys Leu Phe Thr Gln Thr
           675                 680                 685
   Met Lys Gly Val Glu Arg Leu Ala Val Met Gly Asp Thr Ala Trp Asp
       690                 695                 700
   Phe Ser Ser Ala Gly Gly Phe Phe Thr Ser Val Gly Lys Gly Ile His
   705                 710                 715                 720
   Thr Val Phe Gly Ser Ala Phe Gln Gly Leu Phe Gly Gly Leu Asn Trp
                   725                 730                 735
   Ile Thr Lys Val Ile Met Gly Ala Val Leu Ile Trp Val Gly Ile Asn
               740                 745                 750
   Thr Arg Asn Met Thr Met Ser Met Ser Met Ile Leu Val Gly Val Ile
           755                 760                 765
   Met Met Phe Leu Ser Leu Gly Val Gly Ala Asp Gln Gly Cys Ala Ile
       770                 775                 780
   Asn Phe Gly Lys Arg Glu Leu Lys Cys Gly Asp Gly Ile Phe Ile Phe
   785                 790                 795                 800
   Arg Asp Ser Asp Asp Trp Leu Asn Lys Tyr Ser Tyr Tyr Pro Glu Asp
                   805                 810                 815
   Pro Val Lys Leu Ala Ser Ile Val Lys Ala Ser Phe Glu Glu Gly Lys
               820                 825                 830
   Cys Gly Leu Asn Ser Val Asp Ser Leu Glu His Glu Met Trp Arg Ser
           835                 840                 845
   Arg Ala Asp Glu Ile Asn Ala Ile Phe Glu Glu Asn Glu Val Asp Ile
       850                 855                 860
   Ser Val Val Val Gln Asp Pro Lys Asn Val Tyr Gln Arg Gly Thr His
   865                 870                 875                 880
   Pro Phe Ser Arg Ile Arg Asp Gly Leu Gln Tyr Gly Trp Lys Thr Trp
                   885                 890                 895
   Gly Lys Asn Leu Val Phe Ser Pro Gly Arg Lys Asn Gly Ser Phe Ile
               900                 905                 910
   Ile Asp Gly Lys Ser Arg Lys Glu Cys Pro Phe Ser Asn Arg Val Trp
           915                 920                 925
   Asn Ser Phe Gln Ile Glu Glu Phe Gly Thr Gly Val Phe Thr Thr Arg
       930                 935                 940
   Val Tyr Met Asp Ala Val Phe Glu Tyr Thr Ile Asp Cys Asp Gly Ser
   945                 950                 955                 960
   Ile Leu Gly Ala Ala Val Asn Gly Lys Lys Ser Ala His Gly Ser Pro
                   965                 970                 975
   Thr Phe Trp Met Gly Ser His Glu Val Asn Gly Thr Trp Met Ile His
               980                 985                 990
   Thr Leu Glu Ala Leu Asp Tyr Lys Glu Cys Glu Trp Pro Leu Thr His
           995                 1000                1005
   Thr Ile Gly Thr Ser Val Glu Glu Ser Glu Met Phe Met Pro Arg Ser
       1010                1015                1020
   Ile Gly Gly Pro Val Ser Ser His Asn His Ile Pro Gly Tyr Lys Val
   1025                1030                1035                1040
   Gln Thr Asn Gly Pro Trp Met Gln Val Pro Leu Glu Val Lys Arg Glu
                   1045                1050                1055
   Ala Cys Pro Gly Thr Ser Val Ile Ile Asp Gly Asn Cys Asp Gly Arg
               1060                1065                1070
   Gly Lys Ser Thr Arg Ser Thr Thr Asp Ser Gly Lys Val Ile Pro Glu
           1075                1080                1085
   Trp Cys Cys Arg Ser Cys Thr Met Pro Pro Val Ser Phe His Gly Ser
       1090                1095                1100
   Asp Gly Cys Trp Tyr Pro Met Glu Ile Arg Pro Arg Lys Thr His Glu
   1105                1110                1115                1120
   Ser His Leu Val Arg Ser Trp Val Thr Ala Gly Glu Ile His Ala Val
                   1125                1130                1135
   Pro Phe Gly Leu Val Ser Met Met Ile Ala Met Glu Val Val Leu Arg
               1140                1145                1150
   Lys Arg Gln Gly Pro Lys Gln Met Leu Val Gly Gly Val Val Leu Leu
           1155                1160                1165
   Gly Ala Met Leu Val Gly Gln Val Thr Leu Leu Asp Leu Leu Lys Leu
       1170                1175                1180
   Thr Val Ala Val Gly Leu His Phe His Glu Met Asn Asn Gly Gly Asp
   1185                1190                1195                1200
   Ala Met Tyr Met Ala Leu Ile Ala Ala Phe Ser Ile Arg Pro Gly Leu
                   1205                1210                1215
   Leu Ile Gly Phe Gly Leu Arg Thr Leu Trp Ser Pro Arg Glu Arg Leu
               1220                1225                1230
   Val Leu Thr Leu Gly Ala Ala Met Val Glu Ile Ala Leu Gly Gly Val
           1235                1240                1245
   Met Gly Gly Leu Trp Lys Tyr Leu Asn Ala Val Ser Leu Cys Ile Leu
       1250                1255                1260
   Thr Ile Asn Ala Val Ala Ser Arg Lys Ala Ser Asn Thr Ile Leu Pro
   1265                1270                1275                1280
   Leu Met Ala Leu Leu Thr Pro Val Thr Met Ala Glu Val Arg Leu Ala
                   1285                1290                1295
   Ala Met Phe Phe Cys Ala Val Val Ile Ile Gly Val Leu His Gln Asn
               1300                1305                1310
   Phe Lys Asp Thr Ser Met Gln Lys Thr Ile Pro Leu Val Ala Leu Thr
           1315                1320                1325
   Leu Thr Ser Tyr Leu Gly Leu Thr Gln Pro Phe Leu Gly Leu Cys Ala
       1330                1335                1340
   Phe Leu Ala Thr Arg Ile Phe Gly Arg Arg Ser Ile Pro Val Asn Glu
   1345                1350                1355                1360
   Ala Leu Ala Ala Ala Gly Leu Val Gly Val Leu Ala Gly Leu Ala Phe
                   1365                1370                1375
   Gln Glu Met Glu Asn Phe Leu Gly Pro Ile Ala Val Gly Gly Leu Leu
               1380                1385                1390
   Met Met Leu Val Ser Val Ala Gly Arg Val Asp Gly Leu Glu Leu Lys
           1395                1400                1405
   Lys Leu Gly Glu Val Ser Trp Glu Glu Glu Ala Glu Ile Ser Gly Ser
       1410                1415                1420
   Ser Ala Arg Tyr Asp Val Ala Leu Ser Glu Gln Gly Glu Phe Lys Leu
   1425                1430                1435                1440
   Leu Ser Glu Glu Lys Val Pro Trp Asp Gln Val Val Met Thr Ser Leu
                   1445                1450                1455
   Ala Leu Val Gly Ala Ala Leu His Pro Phe Ala Leu Leu Leu Val Leu
               1460                1465                1470
   Ala Gly Trp Leu Phe His Val Arg Gly Ala Arg Arg Ser Gly Asp Val
           1475                1480                1485
   Leu Trp Asp Ile Pro Thr Pro Lys Ile Ile Glu Glu Cys Glu His Leu
       1490                1495                1500
   Glu Asp Gly Ile Tyr Gly Ile Phe Gln Ser Thr Phe Leu Gly Ala Ser
   1505                1510                1515                1520
   Gln Arg Gly Val Gly Val Ala Gln Gly Gly Val Phe His Thr Met Trp
                   1525                1530                1535
   His Val Thr Arg Gly Ala Phe Leu Val Arg Asn Gly Lys Lys Leu Ile
               1540                1545                1550
   Pro Ser Trp Ala Ser Val Lys Glu Asp Leu Val Ala Tyr Gly Gly Ser
           1555                1560                1565
   Trp Lys Leu Glu Gly Arg Trp Asp Gly Glu Glu Glu Val Gln Leu Ile
       1570                1575                1580
   Ala Ala Val Pro Gly Lys Asn Val Val Asn Val Gln Thr Lys Pro Ser
   1585                1590                1595                1600
   Leu Phe Lys Val Arg Asn Gly Gly Glu Ile Gly Ala Val Ala Leu Asp
                   1605                1610                1615
   Tyr Pro Ser Gly Thr Ser Gly Ser Pro Ile Val Asn Arg Asn Gly Glu
               1620                1625                1630
   Val Ile Gly Leu Tyr Gly Asn Gly Ile Leu Val Gly Asp Asn Ser Phe
           1635                1640                1645
   Val Ser Ala Ile Ser Gln Thr Glu Val Lys Glu Glu Gly Lys Glu Glu
       1650                1655                1660
   Leu Gln Glu Ile Pro Thr Met Leu Lys Lys Gly Met Thr Thr Val Leu
   1665                1670                1675                1680
   Asp Phe His Pro Gly Ala Gly Lys Thr Arg Arg Phe Leu Pro Gln Ile
                   1685                1690                1695
   Leu Ala Glu Cys Ala Arg Arg Arg Leu Arg Thr Leu Val Leu Ala Pro
               1700                1705                1710
   Thr Arg Val Val Leu Ser Glu Met Lys Glu Ala Phe His Gly Leu Asp
           1715                1720                1725
   Val Lys Phe His Thr Gln Ala Phe Ser Ala His Gly Ser Gly Arg Glu
       1730                1735                1740
   Val Ile Asp Ala Met Cys His Ala Thr Leu Thr Tyr Arg Met Leu Glu
   1745                1750                1755                1760
   Pro Thr Arg Val Val Asn Trp Glu Val Ile Ile Met Asp Glu Ala His
                   1765                1770                1775
   Phe Leu Asp Pro Ala Ser Ile Ala Ala Arg Gly Trp Ala Ala His Arg
               1780                1785                1790
   Ala Arg Ala Asn Glu Ser Ala Thr Ile Leu Met Thr Ala Thr Pro Pro
           1795                1800                1805
   Gly Thr Ser Asp Glu Phe Pro His Ser Asn Gly Glu Ile Glu Asp Val
       1810                1815                1820
   Gln Thr Asp Ile Pro Ser Glu Pro Trp Asn Thr Gly His Asp Trp Ile
   1825                1830                1835                1840
   Leu Ala Asp Lys Arg Pro Thr Ala Trp Phe Leu Pro Ser Ile Arg Ala
                   1845                1850                1855
   Ala Asn Val Met Ala Ala Ser Leu Arg Lys Ala Gly Lys Ser Val Val
               1860                1865                1870
   Val Leu Asn Arg Lys Thr Phe Glu Arg Glu Tyr Pro Thr Ile Lys Gln
           1875                1880                1885
   Lys Lys Pro Asp Phe Ile Leu Ala Thr Asp Ile Ala Glu Met Gly Ala
       1890                1895                1900
   Asn Leu Cys Val Glu Arg Val Leu Asp Cys Arg Thr Ala Phe Lys Pro
   1905                1910                1915                1920
   Val Leu Val Asp Glu Gly Arg Lys Val Ala Ile Lys Gly Pro Leu Arg
                   1925                1930                1935
   Ile Ser Ala Ser Ser Ala Ala Gln Arg Arg Gly Arg Ile Gly Arg Asn
               1940                1945                1950
   Pro Asn Arg Asp Gly Asp Ser Tyr Tyr Tyr Ser Glu Pro Thr Ser Glu
           1955                1960                1965
   Asn Asn Ala His His Val Cys Trp Leu Glu Ala Ser Met Leu Leu Asp
       1970                1975                1980
   Asn Met Glu Val Arg Gly Gly Met Val Ala Pro Leu Tyr Gly Val Glu
   1985                1990                1995                2000
   Gly Thr Lys Thr Pro Val Ser Pro Gly Glu Met Arg Leu Arg Asp Asp
                   2005                2010                2015
   Gln Arg Lys Val Phe Arg Glu Leu Val Arg Asn Cys Asp Leu Pro Val
               2020                2025                2030
   Trp Leu Ser Trp Gln Val Ala Lys Ala Gly Leu Lys Thr Asn Asp Arg
           2035                2040                2045
   Lys Trp Cys Phe Glu Gly Pro Glu Glu His Glu Ile Leu Asn Asp Ser
       2050                2055                2060
   Gly Glu Thr Val Lys Cys Arg Ala Pro Gly Gly Ala Lys Lys Pro Leu
   2065                2070                2075                2080
   Arg Pro Arg Trp Cys Asp Glu Arg Val Ser Ser Asp Gln Ser Ala Leu
                   2085                2090                2095
   Ser Glu Phe Ile Lys Phe Ala Glu Gly Arg Arg Gly Ala Ala Glu Val
               2100                2105                2110
   Leu Val Val Leu Ser Glu Leu Pro Asp Phe Leu Ala Lys Lys Gly Gly
   2115 2120 2125
   Glu Ala Met Asp Thr Ile Ser Val Phe Leu His Ser Glu Glu Gly Ser
   2130 2135 2140
   Arg Ala Tyr Arg Asn Ala Leu Ser Met Met Pro Glu Ala Met Thr Ile
   2145 2150 2155 2160
   Val Met Leu Phe Ile Leu Ala Gly Leu Leu Thr Ser Gly Met Val Ile
   2165 2170 2175
   Phe Phe Met Ser Pro Lys Gly Ile Ser Arg Met Ser Met Ala Met Gly
   2180 2185 2190
   Thr Met Ala Gly Cys Gly Tyr Leu Met Phe Leu Gly Gly Val Lys Pro
   2195 2200 2205
   Thr His Ile Ser Tyr Ile Met Leu Ile Phe Phe Val Leu Met Val Val
   2210 2215 2220
   Val Ile Pro Glu Pro Gly Gln Gln Arg Ser Ile Gln Asp Asn Gln Val
   2225 2230 2235 2240
   Ala Tyr Leu Ile Ile Gly Ile Leu Thr Leu Val Ser Ala Val Ala Ala
   2245 2250 2255
   Asn Glu Leu Gly Met Leu Glu Lys Thr Lys Glu Asp Leu Phe Gly Lys
   2260 2265 2270
   Lys Asn Leu Ile Pro Ser Ser Ala Ser Pro Trp Ser Trp Pro Asp Leu
   2275 2280 2285
   Asp Leu Lys Pro Gly Ala Ala Trp Thr Val Tyr Val Gly Ile Val Thr
   2290 2295 2300
   Met Leu Ser Pro Met Leu His His Trp Ile Lys Val Glu Tyr Gly Asn
   2305 2310 2315 2320
   Leu Ser Leu Ser Gly Ile Ala Gln Ser Ala Ser Val Leu Ser Phe Met
   2325 2330 2335
   Asp Lys Gly Ile Pro Phe Met Lys Met Asn Ile Ser Val Ile Met Leu
   2340 2345 2350
   Leu Val Ser Gly Trp Asn Ser Ile Thr Val Met Pro Leu Leu Cys Gly
   2355 2360 2365
   Met Gly Cys Ala Met Leu His Trp Ser Leu Ile Leu Pro Gly Ile Lys
   2370 2375 2380
   Ala Gln Gln Ser Lys Leu Ala Gln Arg Arg Val Phe His Gly Val Ala
   2385 2390 2395 2400
   Lys Asn Pro Val Val Asp Gly Asn Pro Thr Val Asp Ile Glu Glu Ala
   2405 2410 2415
   Pro Glu Met Pro Ala Leu Tyr Glu Lys Lys Leu Ala Leu Tyr Leu Leu
   2420 2425 2430
   Leu Ala Leu Ser Leu Ala Ser Val Ala Met Cys Arg Thr Pro Phe Ser
   2435 2440 2445
   Leu Ala Glu Gly Ile Val Leu Ala Ser Ala Ala Leu Gly Pro Leu Ile
   2450 2455 2460
   Glu Gly Asn Thr Ser Leu Leu Trp Asn Gly Pro Met Ala Val Ser Met
   2465 2470 2475 2480
   Thr Gly Val Met Arg Gly Asn His Tyr Ala Phe Val Gly Val Met Tyr
   2485 2490 2495
   Asn Leu Trp Lys Met Lys Thr Gly Arg Arg Gly Ser Ala Asn Gly Lys
   2500 2505 2510
   Thr Leu Gly Glu Val Trp Lys Arg Glu Leu Asn Leu Leu Asp Lys Arg
   2515 2520 2525
   Gln Phe Glu Leu Tyr Lys Arg Thr Asp Ile Val Glu Val Asp Arg Asp
   2530 2535 2540
   Thr Ala Arg Arg His Leu Ala Glu Gly Lys Val Asp Thr Gly Val Ala
   2545 2550 2555 2560
   Val Ser Arg Gly Thr Ala Lys Leu Arg Trp Phe His Glu Arg Gly Tyr
   2565 2570 2575
   Val Lys Leu Glu Gly Arg Val Ile Asp Leu Gly Cys Gly Arg Gly Gly
   2580 2585 2590
   Trp Cys Tyr Tyr Ala Ala Ala Gln Lys Glu Val Ser Gly Val Lys Gly
   2595 2600 2605
   Phe Thr Leu Gly Arg Asp Gly His Glu Lys Pro Met Asn Val Gln Ser
   2610 2615 2620
   Leu Gly Trp Asn Ile Ile Thr Phe Lys Asp Lys Thr Asp Ile His Arg
   2625                2630                2635                2640
   Leu Glu Pro Val Lys Cys Asp Thr Leu Leu Cys Asp Ile Gly Glu Ser
                   2645                2650                2655
   Ser Ser Ser Ser Val Thr Glu Gly Glu Arg Thr Val Arg Val Leu Asp
               2660                2665                2670
   Thr Val Glu Lys Trp Leu Ala Cys Gly Val Asp Asn Phe Cys Val Lys
           2675                2680                2685
   Val Leu Ala Pro Tyr Met Pro Asp Val Leu Glu Lys Leu Glu Leu Leu
       2690                2695                2700
   Gln Arg Arg Phe Gly Gly Thr Val Ile Arg Asn Pro Leu Ser Arg Asn
   2705                2710                2715                2720
   Ser Thr His Glu Met Tyr Tyr Val Ser Gly Ala Arg Ser Asn Val Thr
                   2725                2730                2735
   Phe Thr Val Asn Gln Thr Ser Arg Leu Leu Met Arg Arg Met Arg Arg
               2740                2745                2750
   Pro Thr Gly Lys Val Thr Leu Glu Ala Asp Val Ile Leu Pro Ile Gly
           2755                2760                2765
   Thr Arg Ser Val Glu Thr Asp Lys Gly Pro Leu Asp Lys Glu Ala Ile
       2770                2775                2780
   Glu Glu Arg Val Glu Arg Ile Lys Ser Glu Tyr Met Thr Ser Trp Phe
   2785                2790                2795                2800
   Tyr Asp Asn Asp Asn Pro Tyr Arg Thr Trp His Tyr Cys Gly Ser Tyr
                   2805                2810                2815
   Val Thr Lys Thr Ser Gly Ser Ala Ala Ser Met Val Asn Gly Val Ile
               2820                2825                2830
   Lys Ile Leu Thr Tyr Pro Trp Asp Arg Ile Glu Glu Val Thr Arg Met
           2835                2840                2845
   Ala Met Thr Asp Thr Thr Pro Phe Gly Gln Gln Arg Val Phe Lys Glu
       2850                2855                2860
   Lys Val Asp Thr Arg Ala Lys Asp Pro Pro Ala Gly Thr Arg Lys Ile
   2865                2870                2875                2880
   Met Lys Val Val Asn Arg Trp Leu Phe Arg His Leu Ala Arg Glu Lys
                   2885                2890                2895
   Asn Pro Arg Leu Cys Thr Lys Glu Glu Phe Ile Ala Lys Val Arg Ser
               2900                2905                2910
   His Ala Ala Ile Gly Ala Tyr Leu Glu Glu Gln Glu Gln Trp Lys Thr
           2915                2920                2925
   Ala Asn Glu Ala Val Gln Asp Pro Lys Phe Trp Glu Leu Val Asp Glu
       2930                2935                2940
   Glu Arg Lys Leu His Gln Gln Gly Arg Cys Arg Thr Cys Val Tyr Asn
   2945                2950                2955                2960
   Met Met Gly Lys Arg Glu Lys Lys Leu Ser Glu Phe Gly Lys Ala Lys
                   2965                2970                2975
   Gly Ser Arg Ala Ile Trp Tyr Met Trp Leu Gly Ala Arg Tyr Leu Glu
               2980                2985                2990
   Phe Glu Ala Leu Gly Phe Leu Asn Glu Asp His Trp Ala Ser Arg Glu
           2995                3000                3005
   Asn Ser Gly Gly Gly Val Glu Gly Ile Gly Leu Gln Tyr Leu Gly Tyr
       3010                3015                3020
   Val Ile Arg Asp Leu Ala Ala Met Asp Gly Gly Gly Phe Tyr Ala Asp
   3025                3030                3035                3040
   Asp Thr Ala Gly Trp Asp Thr Arg Ile Thr Glu Ala Asp Leu Asp Asp
                   3045                3050                3055
   Glu Gln Glu Ile Leu Asn Tyr Met Ser Pro His His Lys Lys Leu Ala
               3060                3065                3070
   Gln Ala Val Met Glu Met Thr Tyr Lys Asn Lys Val Val Lys Val Leu
           3075                3080                3085
   Arg Pro Ala Pro Gly Gly Lys Ala Tyr Met Asp Val Ile Ser Arg Arg
       3090                3095                3100
   Asp Gln Arg Gly Ser Gly Gln Val Val Thr Tyr Ala Leu Asn Thr Ile
   3105                3110                3115                3120
   Thr Asn Leu Lys Val Gln Leu Ile Arg Met Ala Glu Ala Glu Met Val
                   3125                3130                3135
   Ile His His Gln His Val Gln Asp Cys Asp Glu Ser Val Leu Thr Arg
               3140                3145                3150
   Leu Glu Ala Trp Leu Thr Glu His Gly Cys Asn Arg Leu Lys Arg Met
           3155                3160                3165
   Ala Val Ser Gly Asp Asp Cys Val Val Arg Pro Ile Asp Asp Arg Phe
       3170                3175                3180
   Gly Leu Ala Leu Ser His Leu Asn Ala Met Ser Lys Val Arg Lys Asp
   3185                3190                3195                3200
   Ile Ser Glu Trp Gln Pro Ser Lys Gly Trp Asn Asp Trp Glu Asn Val
                   3205                3210                3215
   Pro Phe Cys Ser His His Phe His Glu Leu Gln Leu Lys Asp Gly Arg
               3220                3225                3230
   Arg Ile Val Val Pro Cys Arg Glu Gln Asp Glu Leu Ile Gly Arg Gly
           3235                3240                3245
   Arg Val Ser Pro Gly Asn Gly Trp Met Ile Lys Glu Thr Ala Cys Leu
       3250                3255                3260
   Ser Lys Ala Tyr Ala Asn Met Trp Ser Leu Met Tyr Phe His Lys Arg
   3265                3270                3275                3280
   Asp Met Arg Leu Leu Ser Leu Ala Val Ser Ser Ala Val Pro Thr Ser
                   3285                3290                3295
   Trp Val Pro Gln Gly Arg Thr Thr Trp Ser Ile His Gly Lys Gly Glu
               3300                3305                3310
   Trp Met Thr Thr Glu Asp Met Leu Glu Val Trp Asn Arg Val Trp Ile
           3315                3320                3325
   Thr Asn Asn Pro His Met Gln Asp Lys Thr Met Val Lys Lys Trp Arg
       3330                3335                3340
   Asp Val Pro Tyr Leu Thr Lys Arg Gln Asp Lys Leu Cys Gly Ser Leu
   3345                3350                3355                3360
   Ile Gly Met Thr Asn Arg Ala Thr Trp Ala Ser His Ile His Leu Val
                   3365                3370                3375
   Ile His Arg Ile Arg Thr Leu Ile Gly Gln Glu Lys Tyr Thr Asp Tyr
               3380                3385                3390
   Leu Thr Val Met Asp Arg Tyr Ser Val Asp Ala Asp Leu Gln Leu Gly
           3395                3400                3405
   Glu Leu Ile
       3410
<210> 9
   <211> 10776
   <212> DNA
   <213> Flaviviridae Flavivirus Yellow Fever Virus
<400> 9
<210> 10
   <211> 10788
   <212> DNA
   <213> Flaviviridae Flavivirus Yellow Fever Virus
<400> 10
   aatcgagttg ctaggcaata aacacatttg gattaatttt aatcgttcgt tgagcgatta 60
   gcagagaact gaccagaaca tgtctggtcg taaagctcag ggaaaaaccc tgggcgtcaa 120
   tatggtacga cgaggagttc gctccttgtc aaacaaaata aaacaaaaaa caaaacaaat 180
   tggaaacaga cctggacctt caagaggtgt tcaaggattt atctttttct ttttgttcaa 240
   cattttgact ggaaaaaaga tcacagccca cctaaagagg ttgtggaaaa tgctggaccc 300
   aagacaaggc ttggctgttc taaggaaagt caagagagtg gtggccagtt tgatgagagg 360
   attgtcctca aggaaacgcc gttcccatga tgttctgact gtgcaattcc taattttggg 420
   aatgctgttg atgacgggtg gagtgacctt ggtgcggaaa aacagatggt tgctcctaaa 480
   tgtgacatct gaggacctcg ggaaaacatt ctctgtgggc acaggcaact gcacaacaaa 540
   cattttggaa gccaagtact ggtgcccaga ctcaatggaa tacaactgtc ccaatctcag 600
   tccaagagag gagccagatg acattgattg ctggtgctat ggggtggaaa acgttagagt 660
   cgcatatggt aagtgtgact cagcaggcag gtctaggagg tcaagaaggg ccattgactt 720
   gcctacgcat gaaaaccatg gtttgaagac ccggcaagaa aaatggatga ctggaagaat 780
   gggtgaaagg caactccaaa agattgagag atggttcgtg aggaacccct tttttgcagt 840
   gacggctctg accattgcct accttgtggg aagcaacatg acgcaacgag tcgtgattgc 900
   cctactggtc ttggctgttg gtccggccta ctcagctcac tgcattggaa ttactgacag 960
   ggatttcatt gagggggtgc atggaggaac ttgggtttca gctaccctgg agcaagacaa 1020
   gtgtgtcact gttatggccc ctgacaagcc ttcattggac atctcactag agacagtagc 1080
   cattgataga cctgctgagg tgaggaaagt gtgttacaat gcagttctca ctcatgtgaa 1140
   gattaatgac aagtgcccca gcactggaga ggcccaccta gctgaagaga acgaagggga 1200
   caatgcgtgc aagcgcactt attctgatag aggctggggc aatggctgtg gcctatttgg 1260
   gaaagggagc attgtggcat gcgccaaatt cacttgtgcc aaatccatga gtttgtttga 1320
   ggttgatcag accaaaattc agtatgtcat cagagcacaa ttgcatgtag gggccaagca 1380
   ggaaaattgg actaccgaca ttaagactct caagtttgat gccctgtcag gctcccagga 1440
   agtcgagttc attgggtatg gaaaagctac actggaatgc caggtgcaaa ctgcggtgga 1500
   ctttggtaac agttacatcg ctgagatgga aacagagagc tggatagtgg acagacagtg 1560
   ggcccaggac ttgaccctgc catggcagag tggaagtggc ggggtgtgga gagagatgca 1620
   tcatcttgtc gaatttgaac ctccgcatgc cgccactatc agagtactgg ccctgggaaa 1680
   ccaggaaggc tccttgaaaa cagctcttac tggcgcaatg agggttacaa aggacacaaa 1740
   tgacaacaac ctttacaaac tacatggtgg acatgtttct tgcagagtga aattgtcagc 1800
   tttgacactc aaggggacat cctacaaaat atgcactgac aaaatgtttt ttgtcaagaa 1860
   cccaactgac actggccatg gcactgttgt gatgcaggtg aaagtgtcaa aaggagcccc 1920
   ctgcaggatt ccagtgatag tagctgatga tcttacagcg gcaatcaata aaggcatttt 1980
   ggttacagtt aaccccatcg cctcaaccaa tgatgatgaa gtgctgattg aggtgaaccc 2040
   accttttgga gacagctaca ttatcgttgg gagaggagat tcacgtctca cttaccagtg 2100
   gcacaaagag ggaagctcaa taggaaagtt gttcactcag accatgaaag gcgtggaacg 2160
   cctggccgtc atgggagaca ccgcctggga tttcagctcc gctggagggt tcttcacttc 2220
   ggttgggaaa ggaattcata cggtgtttgg ctctgccttt caggggctat ttggcggctt 2280
   gaactggata acaaaggtca tcatgggggc ggtacttata tgggttggca tcaacacaag 2340
   aaacatgaca atgtccatga gcatgatctt ggtaggagtg atcatgatgt ttttgtctct 2400
   aggagttggg gcggatcaag gatgcgccat caactttggc aagagagagc tcaagtgcgg 2460
   agatggtatc ttcatattta gagactctga tgactggctg aacaagtact catactatcc 2520
   agaagatcct gtgaagcttg catcaatagt gaaagcctct tttgaagaag ggaagtgtgg 2580
   cctaaattca gttgactccc ttgagcatga gatgtggaga agcagggcag atgagatcaa 2640
   tgccattttt gaggaaaacg aggtggacat ttctgttgtc gtgcaggatc caaagaatgt 2700
   ttaccagaga ggaactcatc cattttccag aattcgggat ggtctgcagt atggttggaa 2760
   gacttggggt aagaaccttg tgttctcccc agggaggaag aatggaagct tcatcataga 2820
   tggaaagtcc aggaaagaat gcccgttttc aaaccgggtc tggaattctt tccagataga 2880
   ggagtttggg acgggagtgt tcaccacacg cgtgtacatg gacgcagtct ttgaatacac 2940
   catagactgc gatggatcta tcttgggtgc agcggtgaac ggaaaaaaga gtgcccatgg 3000
   ctctccaaca ttttggatgg gaagtcatga agtaaatggg acatggatga tccacacctt 3060
   ggaggcatta gattacaagg agtgtgagtg gccactgaca catacgattg gaacatcagt 3120
   tgaagagagt gaaatgttca tgccgagatc aatcggaggc ccagttagct ctcacaatca 3180
   tatccctgga tacaaggttc agacgaacgg accttggatg caggtaccac tagaagtgaa 3240
   gagagaagct tgcccaggga ctagcgtgat cattgatggc aactgtgatg gacggggaaa 3300
   atcaaccaga tccaccacgg atagcgggaa agttattcct gaatggtgtt gccgctcctg 3360
   cacaatgccg cctgtgagct tccatggtag tgatgggtgt tggtatccca tggaaattag 3420
   gccaaggaaa acgcatgaaa gccatctggt gcgctcctgg gttacagctg gagaaataca 3480
   tgctgtccct tttggtttgg tgagcatgat gatagcaatg gaagtggtcc taaggaaaag 3540
   acagggacca aagcaaatgt tggttggagg agtagtgctc ttgggagcaa tgctggtcgg 3600
   gcaagtaact ctccttgatt tgctgaaact cacagtggct gtgggattgc atttccatga 3660
   gatgaacaat ggaggagacg ccatgtatat ggcgttgatt gctgcctttt caatcagacc 3720
   agggctgctc atcggctttg ggctcaggac cctatggagc cctcgggaac gccttgtgct 3780
   gaccctagga gcagccatgg tggagattgc cttgggtggc gtgatgggcg gcctgtggaa 3840
   gtatctaaat gcagtttctc tctgcatcct gacaataaat gctgttgctt ctaggaaagc 3900
   atcaaatacc atcttgcccc tcatggctct gttgacacct gtcactatgg ctgaggtgag 3960
   acttgccgca atgttctttt gtgccgtggt tatcataggg gtccttcacc agaatttcaa 4020
   ggacacctcc atgcagaaga ctatacctct ggtggccctc acactcacat cttacctggg 4080
   cttgacacaa ccttttttgg gcctgtgtgc atttctggca acccgcatat ttgggcgaag 4140
   gagtatccca gtgaatgagg cactcgcagc agctggtcta gtgggagtgc tggcaggact 4200
   ggcttttcag gagatggaga acttccttgg tccgattgca gttggaggac tcctgatgat 4260
   gctggttagc gtggctggga gggtggatgg gctagagctc aagaagcttg gtgaagtttc 4320
   atgggaagag gaggcggaga tcagcgggag ttccgcccgc tatgatgtgg cactcagtga 4380
   acaaggggag ttcaagctgc tttctgaaga gaaagtgcca tgggaccagg ttgtgatgac 4440
   ctcgctggcc ttggttgggg ctgccctcca tccatttgct cttctgctgg tccttgctgg 4500
   gtggctgttt catgtcaggg gagctaggag aagtggggat gtcttgtggg atattcccac 4560
   tcctaagatc atcgaggaat gtgaacatct ggaggatggg atttatggca tattccagtc 4620
   aaccttcttg gggggcctcc agcgaggagt gggagtggca cagggagggg tgttccacac 4680
   aatgtggcat gtcacaagag gagctttcct tgtcaggaat ggcaagaagt tgattccatc 4740
   ttgggcttca gtaaaggaag accttgtcgc ctatggtggc tcatggaagt tggaaggcag 4800
   atgggatgga gaggaagagg tccagttgat cgcggctgtt ccaggaaaga acgtggtcaa 4860
   cgtccagaca aaaccgagct tgttcaaagt gaggaatggg ggagaaatcg gggctgtcgc 4920
   tcttgactat ccgagtggca cttcaggatc tcctattgtt aacaggaacg gagaggtgat 4980
   tgggctgtac ggcaatggca tccttgtcgg tgacaactcc ttcgtgtccg ccatatccca 5040
   gactgaggtg aaggaagaag gaaaggagga gctccaagag atcccgacaa tgctaaagaa 5100
   aggaatgaca actgtccttg attttcatcc tggagctggg aagacaagac gtttcctccc 5160
   acagatcttg gccgagtgcg cacggagacg cttgcgcact cttgtgttgg cccccaccag 5220
   ggttgttctt tctgaaatga aggaggcttt tcacggcctg gacgtgaaat tccacacaca 5280
   ggctttttcc gctcacggca gcgggagaga agtcattgat gctatgtgcc atgccaccct 5340
   aacttacagg atgttggaac caactagggt tgttaactgg gaagtgatca ttatggatga 5400
   agcccatttt ttggatccag ctagcatagc cgctagaggt tgggcagcgc acagagctag 5460
   ggcaaatgaa agtgcaacaa tcttgatgac agccacaccg cctgggacta gtgatgaatt 5520
   tccacattca aatggtgaaa tagaagatgt tcaaacggac atacccagtg agccctggaa 5580
   cacagggcat gactggatcc tggctgacaa aaggcccacg gcatggttcc ttccatccat 5640
   cagagctgca aatgtcatgg ctgcctcttt gcgtaaggct ggaaagagtg tggtggtcct 5700
   gaacaggaaa acctttgaga gagaataccc cacgataaag cagaagaaac ctgactttat 5760
   attggccact gacatagctg aaatgggagc caacctttgc gtggagcgag tgctggattg 5820
   caggacggct tttaagcctg tgcttgtgga tgaagggagg aaggtggcaa taaaagggcc 5880
   acttcgtatc tccgcatcct ctgctgctca aaggaggggg cgcattggga gaaatcccaa 5940
   cagagatgga gactcatact actattctga gcctacaagt gaaaataatg cccaccacgt 6000
   ctgctggttg gaggcctcaa tgctcttgga caacatggag gtgaggggtg gaatggtcgc 6060
   cccactctat ggcgttgaag gaactaaaac accagtttcc cctggtgaaa tgagactgag 6120
   ggatgaccag aggaaagtct tcagagaact agtgaggaat tgtgacctgc ccgtttggct 6180
   ttcgtggcaa gtggccaagg ctggtttgaa gacgaatgat cgtaagtggt gttttgaagg 6240
   ccctgaggaa catgagatct tgaatgacag cggtgaaaca gtgaagtgca gggctcctgg 6300
   aggagcaaag aagcctctgc gcccaaggtg gtgtgatgaa agggtgtcat ctgaccagag 6360
   tgcgctgtct gaatttatta agtttgctga aggtaggagg ggagctgctg aagtgctagt 6420
   tgtgctgagt gaactccctg atttcctggc taaaaaaggt ggagaggcaa tggataccat 6480
   cagtgtgttt ctccactctg aggaaggctc tagggcttac cgcaatgcac tatcaatgat 6540
   gcctgaggca atgacaatag tcatgctgtt tatactggct ggactactga catcgggaat 6600
   ggtcatcttt ttcatgtctc ccaaaggcat cagtagaatg tctatggcga tgggcacaat 6660
   ggccggctgt ggatatctca tgttccttgg aggcgtcaaa cccactcaca tctcctatat 6720
   catgctcata ttctttgtcc tgatggtggt tgtgatcccc gagccagggc aacaaaggtc 6780
   catccaagac aaccaagtgg catacctcat tattggcatc ctgacgctgg tttcagcggt 6840
   ggcagccaac gagctaggca tgctggagaa aaccaaagag gacctctttg ggaagaagaa 6900
   cttaattcca tctagtgctt caccctggag ttggccggat cttgacctga agccaggagc 6960
   tgcctggaca gtgtacgttg gcattgttac aatgctctct ccaatgttgc accactggat 7020
   caaagtcgaa tatggcaacc tgtctctgtc tggaatagcc cagtcagcct cagtcctttc 7080
   tttcatggac aaggggatac cattcatgaa gatgaatatc tcggtcataa tgctgctggt 7140
   cagtggctgg aattcaataa cagtgatgcc tctgctctgt ggcatagggt gcgccatgct 7200
   ccactggtct ctcattttac ctggaatcaa agcgcagcag tcaaagcttg cacagagaag 7260
   ggtgttccat ggcgttgcca agaaccctgt ggttgatggg aatccaacag ttgacattga 7320
   ggaagctcct gaaatgcctg ccctttatga gaagaaactg gctctatatc tccttcttgc 7380
   tctcagccta gcttctgttg ccatgtgcag aacgcccttt tcattggctg aaggcattgt 7440
   cctagcatca gctgccctag ggccgctcat agagggaaac accagccttc tttggaatgg 7500
   acccatggct gtctccatga caggagtcat gagggggaat cactatgctt ttgtgggagt 7560
   catgtacaat ctatggaaga tgaaaactgg acgccggggg agcgcgaatg gaaaaacttt 7620
   gggtgaagtc tggaagaggg aactgaatct gttggacaag cgacagtttg agttgtataa 7680
   aaggaccgac attgtggagg tggatcgtga tacggcacgc aggcatttgg ccgaagggaa 7740
   ggtggacacc ggggtggcgg tctccagggg gaccgcaaag ttaaggtggt tccatgagcg 7800
   tggctatgtc aagctggaag gtagggtgat tgacctgggg tgtggccgcg gaggctggtg 7860
   ttactacgct gctgcgcaaa aggaagtgag tggggtcaaa ggatttactc ttggaagaga 7920
   cggccatgag aaacccatga atgtgcaaag tctgggatgg aacatcatca ccttcaagga 7980
   caaaactgat atccaccgcc tagaaccagt gaaatgtgac acccttttgt gtgacattgg 8040
   agagtcatca tcgtcatcgg tcacagaggg ggaaaggacc gtgagagttc ttgatactgt 8100
   agaaaaatgg ctggcttgtg gggttgacaa cttctgtgtg aaggtgttag ctccatacat 8160
   gccagatgtt ctcgagaaac tggaattgct ccaaaggagg tttggcggaa cagtgatcag 8220
   gaaccctctc tccaggaatt ccactcatga aatgtactac gtgtctggag cccgcagcaa 8280
   tgtcacattt actgtgaacc aaacatcccg cctcctgatg aggagaatga ggcgtccaac 8340
   tggaaaagtg accctggagg ctgacgtcat cctcccaatt gggacacgca gtgttgagac 8400
   agacaaggga cccctggaca aagaggccat agaagaaagg gttgagagga taaaatctga 8460
   gtacatgacc tcttggtttt atgacaatga caacccctac aggacctggc actactgtgg 8520
   ctcctatgtc acaaaaacct caggaagtgc ggcgagcatg gtaaatggtg ttattaaaat 8580
   tctgacatat ccatgggaca ggatagagga ggtcacaaga atggcaatga ctgacacaac 8640
   cccttttgga cagcaaagag tgtttaaaga aaaagttgac accagagcaa aggatccacc 8700
   agcgggaact aggaagatca tgaaagttgt caacaggtgg ctgttccgcc acctggccag 8760
   agaaaagaac cccagactgt gcacaaagga agaatttatt gcaaaagtcc gaagtcatgc 8820
   agccattgga gcttacctgg aagaacaaga acagtggaag actgccaatg aggctgtcca 8880
   agacccaaag ttctgggaac tggtggatga agaaaggaag ctgcaccaac aaggcaggtg 8940
   tcggacttgt gtgtacaaca tgatggggaa aagagagaag aagctgtcag agtttgggaa 9000
   agcaaaggga agccgtgcca tatggtatat gtggctggga gcgcggtatc ttgagtttga 9060
   ggccctggga ttcctgaatg aggaccattg ggcttccagg gaaaactcag gaggaggagt 9120
   ggaaggcatt ggcttacaat acctaggata tgtgatcaga gacctggctg caatggatgg 9180
   tggtggattc tacgcggatg acaccgctgg atgggacacg cgcatcacag aggcagacct 9240
   tgatgatgaa caggagatct tgaactacat gagcccacat cacaaaaaac tggcacaagc 9300
   agtgatggaa atgacataca agaacaaagt ggtgaaagtg ttgagaccag ccccaggagg 9360
   gaaagcctac atggatgtca taagtcgacg agaccagaga ggatccgggc aggtagtgac 9420
   ttatgctctg aacaccatca ccaacttgaa agtccaattg atcagaatgg cagaagcaga 9480
   gatggtgata catcaccaac atgttcaaga ttgtgatgaa tcagttctga ccaggctgga 9540
   ggcatggctc actgagcacg gatgtaacag actgaagagg atggcggtga gtggagacga 9600
   ctgtgtggtc cggcccatcg atgacaggtt cggcctggcc ctgtcccatc tcaacgccat 9660
   gtccaaggtt agaaaggaca tatctgaatg gcagccatca aaagggtgga atgattggga 9720
   gaatgtgccc ttctgttccc accacttcca tgaactacag ctgaaggatg gcaggaggat 9780
   tgtggtgcct tgccgagaac aggacgagct cattgggaga ggaagggtgt ctccaggaaa 9840
   cggctggatg atcaaggaaa cagcttgcct cagcaaagcc tatgccaaca tgtggtcact 9900
   gatgtatttt cacaaaaggg acatgaggct actgtcattg gctgtttcct cagctgttcc 9960
   cacctcatgg gttccacaag gacgcacaac atggtcgatt catgggaaag gggagtggat 10020
   gaccacggaa gacatgcttg aggtgtggaa cagagtatgg ataaccaaca acccacacat 10080
   gcaggacaag acaatggtga aaaaatggag agatgtccct tatctaacca agagacaaga 10140
   caagctgtgc ggatcactga ttggaatgac caatagggcc acctgggcct cccacatcca 10200
   tttggtcatc catcgtatcc gaacgctgat tggacaggag aaatacactg actacctaac 10260
   agtcatggac aggtattctg tggatgctga cctgcaactg ggtgagctta tctgaaacac 10320
   catctaacag gaataaccgg gatacaaacc acgggtggag aaccggactc cccacaacct 10380
   gaaaccggga tataaaccac ggctggagaa ccggactccg cacttaaaat gaaacagaaa 10440
   ccgggataaa aactacggat ggagaaccgg actccacaca ttgagacaga agaagttgtc 10500
   agcccagaac cccacacgag ttttgccact gctaagctgt gaggcagtgc aggctgggac 10560
   agccgacctc caggttgcga aaaacctggt ttctgggacc tcccacccca gagtaaaaag 10620
   aacggagcct ccgctaccac cctcccacgt ggtggtagaa agacggggtc tagaggttag 10680
   aggagaccct ccagggaaca aatagtggga ccatattgac gccagggaaa gaccggagtg 10740
   gttctctgct tttcctccag aggtctgtga gcacagtttg ctcaagaa              10788
<210> 11
   <211> 10811
   <212> DNA
   <213> Flaviviridae Flavivirus Yellow Fever Virus
<400> 11
   tgcattggtc tgcaaatcga gttgctaggc aataaacaca tttggattaa ttttaatcgt 60
   tcgttgagcg attagcagag aactgaccag aacatgtctg gtcgtaaagc tcagggaaaa 120
   accctgggcg tcaatatggt acgacgagga gttcgctcct tgtcaaacaa aataaaacaa 180
   aaaacgaaac aaattggaaa cagacctgga ccttcaagag gtgttcaagg atttatcttt 240
   ttctttttgt tcaacatttt gactggaaaa aagatcacag cccacctaaa gaggttgtgg 300
   aaaatgctgg acccaagaca aggcttggct gttctaagga aagtcaagag agtggtggcc 360
   agtttgatga gaggattgtc ctcaaggaaa cgccgttccc atgatgttct gactgtgcaa 420
   ttcctaattt tgggaatgct gttgatgacg ggtggagtga ccttggtgcg gaaaaacaga 480
   tggttgctcc taaatgtgac atctgaggac ctcgggaaaa cattctctgt gggcacaggc 540
   aactgcacaa caaacatttt ggaagccaag tactggtgcc cagactcaat ggaatacaac 600
   tgtcccaatc tcagtccaag agaggagcca gatgacattg attgctggtg ctatggggtg 660
   gaaaacgtta gagtcgcata tggtaagtgt gactcagcag gcaggtctag gaggtcaaga 720
   agggccattg acttgcctac gcatgaaaac catggtttga agacccggca agaaaaatgg 780
   atgactggaa gaatgggtga aaggcaactc caaaagattg agagatggtt cgtgaggaac 840
   cccttttttg cagtgacggc tctgaccatt gcctaccttg tgggaagcaa catgacgcaa 900
   cgagtcgtga ttgccctact ggtcttggct gttggtccgg cctactcagc tcactgcatt 960
   ggaattactg acagggattt cattgagggg gtgcatggag gaacttgggt ttcagctacc 1020
   ctggagcaag acaagtgtgt cactgttatg gcccctgaca agccttcatt ggacatctca 1080
   ctagagacag tagccattga tagacctgct gaggtgagga aagtgtgtta caatgcagtt 1140
   ctcactcatg tgaagattaa tgacaagtgc cccagcactg gagaggccca cctagctgaa 1200
   gagaacgaag gggacaatgc gtgcaagcgc acttattctg atagaggctg gggcaatggc 1260
   tgtggcctat ttgggaaagg gagcattgtg gcatgcgcca aattcacttg tgccaaatcc 1320
   atgagtttgt ttgaggttga tcagaccaaa attcagtatg tcatcagagc acaattgcat 1380
   gtaggggcca agcaggaaaa ttggactacc gacattaaga ctctcaggtt tgatgccctg 1440
   tcaggctccc aggaagtcga gttcattggg tatggaaaag ccacactgga atgccaggtg 1500
   caaactgcgg tggactttgg taacagttac atcgctgaga tggaaacaga gagctggata 1560
   gtggacagac agtgggccca ggacttgacc ctgccatggc agagtggaag tggcggggtg 1620
   tggagagaga tgcatcatct tgtcgaattt gaacctccgc atgccgccac tatcagagta 1680
   ctggccctgg gaaaccagga aggctccttg aaaacagctc ttactggcgc aatgagggtt 1740
   acaaaggaca caaatgacaa caacctttac aaactacatg gtggacatgt ttcttgcaga 1800
   gtgaaattgt cagctttgac actcaagggg acatcctaca aaatatgcac tgacaaaatg 1860
   ttttttgtca agaacccaac tgacactggc catggcactg ttgtgatgca ggtgaaagtg 1920
   tcaaaaggag ccccctgcag gattccagtg atagtagctg atgatcttac agcggcaatc 1980
   aataaaggca ttttggttac agttaacccc atcgcctcaa ccaatgatga tgaagtgctg 2040
   attgaggtga acccaccttt tggagacagc tacattatcg ttgggagagg agattcacgt 2100
   ctcacttacc agtggcacaa agagggaagc tcaataggaa agttgttcac tcagaccatg 2160
   aaaggcgtgg aacgcctggc cgtcatggga gacaccgcct gggatttcag ctccgctgga 2220
   gggttcttca cttcggttgg gaaaggaatt catacggtgt ttggctctgc ctttcagggg 2280
   ctatttggcg gcttgaactg gataacaaag gtcatcatgg gggcggtact tatatgggtt 2340
   ggcatcaaca caagaaacat gacaatgtcc atgagcatga tcttggtagg agtgatcatg 2400
   atgtttttgt ctctaggagt tggggcggat caaggatgcg ccatcaactt tggcaagaga 2460
   gagctcaagt gcggagatgg tatcttcata tttagagact ctgatgactg gctgaacaag 2520
   tactcatact atccagaaga tcctgtgaag cttgcatcaa tagtgaaagc ctcttttgaa 2580
   gaagggaagt gtggcctaaa ttcagttgac tcccttgagc atgagatgtg gagaagcagg 2640
   gcagatgaga tcaatgccat ttttgaggaa aacgaggtgg acatttctgt tgtcgtgcag 2700
   gatccaaaga atgtttacca gagaggaact catccatttt ccagaattcg ggatggtctg 2760
   cagtatggtt ggaagacttg gggtaagaac cttgtgttct ccccagggag gaagaatgga 2820
   agcttcatca tagatggaaa gtccaggaaa gaatgcccgt tttcaaaccg ggtctggaat 2880
   tctttccaga tagaggagtt tgggacggga gtgttcacca cacgcgtgta catggacgca 2940
   gtctttgaat acaccataga ctgcgatgga tctatcttgg gtgcagcggt gaacggaaaa 3000
   aagagtgccc atggctctcc aacattttgg atgggaagtc atgaagtaaa tgggacatgg 3060
   atgatccaca ccttggaggc attagattac aaggagtgtg agtggccact gacacatacg 3120
   attggaacat cagttgaaga gagtgaaatg ttcatgccga gatcaatcgg aggcccagtt 3180
   agctctcaca atcatatccc tggatacaag gttcagacga acggaccttg gatgcaggta 3240
   ccactagaag tgaagagaga agcttgccca gggactagcg tgatcattga tggcaactgt 3300
   gatggacggg gaaaatcaac cagatccacc acggatagcg ggaaagttat tcctgaatgg 3360
   tgttgccgct cctgcataat gccgcctgtg agcttccatg gtagtgatgg gtgttggtat 3420
   cccatggaaa ttaggccaag gaaaacgcat gaaagccatc tggtgcgctc ctgggttaca 3480
   gctggagaaa tacatgctgt cccttttggt ttggtgagca tgatgatagc aatggaagtg 3540
   gtcctaagga aaagacaggg accaaagcaa atgttggttg gaggagtagt gctcttggga 3600
   gcaatgctgg tcgggcaagt aactctcctt gatttgctga aactcacagt ggctgtggga 3660
   ttgcatttcc atgagatgaa caatggagga gacgccatgt atatggcgtt gattgctgcc 3720
   ttttcaatca gaccagggct gctcatcggc tttgggctca ggaccctatg gagccctcgg 3780
   gaacgccttg tgctgaccct aggagcagcc atggtggaga ttgccttggg tggcgtgatg 3840
   ggcggcctgt ggaagtatct aaatgcagtt tctctctgca tcctgacaat aaatgctgtt 3900
   gcttctagga aagcatcaaa taccatcttg cccctcatgg ctctgttgac acctgtcact 3960
   atggctgagg tgagacttgc cgcaatgttc ctttgtgccg tggttatcat aggggtcctt 4020
   caccagaatt tcaaggacac ctccatgcag aagactatac ctctggtggc cctcacactc 4080
   acatcttacc tgggcttgac acaacctttt ttgggcctgt gtgcatttct ggcaacccgc 4140
   atatttgggc gaaggagtat cccagtgaat gaggcactcg cagcagctgg tctagtggga 4200
   gtgctggcag gactggcttt tcaggagatg gagaacttcc ttggtccgat tgcagttgga 4260
   ggactcctga tgatgctggt tagcgtggct gggagggtgg atgggctaga gctcaagaag 4320
   cttggtgaag tttcatggga agaggaggcg gagatcagcg ggagttccgc ccgctatgat 4380
   gtggcactca gtgaacaagg ggagttcaag ctgctttctg aagagaaagt gccatgggac 4440
   caggttgtga tgacctcgct ggccttggtt ggggctgccc tccatccatt tgctcttctg 4500
   ctggtccttg ctgggtggct gtttcatgtc aggggagcta ggagaagtgg ggatgtcttg 4560
   tgggatattc ccactcctaa gatcatcgag gaatgtgaac atctggagga tgggatttat 4620
   ggcatattcc agtcaacctt cttgggggcc tcccagcgag gagtgggagt ggcacaggga 4680
   ggggtgttcc acacaatgtg gcatgtcaca agaggagctt tccttgtcag gaatggcaag 4740
   aagttgattc catcttgggc ttcagtaaag gaagaccttg tcgcctatgg tggctcatgg 4800
   aagttggaag gcagatggga tggagaggaa gaggtccagt tgatcgcggc tgttccagga 4860
   aagaacgtgg tcaacgtcca gacaaaaccg agcttgttca aagtgaggaa tgggggagaa 4920
   atcggggctg tcgctcttga ctatccgagt ggcacttcag gatctcctat tgttaacagg 4980
   aacggagagg tgattgggct gtacggcaat ggcatccttg tcggtgacaa ctccttcgtg 5040
   tccgccatat cccagactga ggtgaaggaa gaaggaaagg aggagctcca agagatcccg 5100
   acaatgctaa agaaaggaat gacaactgtc cttgattttc atcctggagc tgggaagaca 5160
   agacgtttcc tcccacagat cttggccgag tgcgcacgga gacgcttgcg cactcttgtg 5220
   ttggccccca ccagggttgt tctttctgaa atgaaggagg cttttcacgg cctggacgtg 5280
   aaattccaca cacaggcttt ttccgctcac ggcagcggga gagaagtcat tgatgctatg 5340
   tgccatgcca ccctaactta caggatgttg gaaccaacta gggttgttaa ctgggaagtg 5400
   atcattatgg atgaagccca ttttttggat ccagctagca tagccgctag aggttgggca 5460
   gcgcacagag ctagggcaaa tgaaagtgca acaatcttga tgacagccac accgcctggg 5520
   actagtgatg aatttccaca ttcaaatggt gaaatagaag atgttcaaac ggacataccc 5580
   agtgagccct ggaacacagg gcatgactgg atcctggctg acaaaaggcc cacggcatgg 5640
   ttccttccat ccatcagagc tgcaaatgtc atggctgcct ctttgcgtaa ggctggaaag 5700
   agtgtggtgg tcctgaacag gaaaaccttt gagagagaat accccacgat aaagcagaag 5760
   aaacctgact ttatattggc cactgacata gctgaaatgg gagccaacct ttgcgtggag 5820
   cgagtgctgg attgcaggac ggcttttaag cctgtgcttg tggatgaagg gaggaaggtg 5880
   gcaataaaag ggccacttcg tatctccgca tcctctgctg ctcaaaggag ggggcgcatt 5940
   gggagaaatc ccaacagaga tggagactca tactactatt ctgagcctac aagtgaaaat 6000
   aatgcccacc acgtctgctg gttggaggcc tcaatgctct tggacaacat ggaggtgagg 6060
   ggtggaatgg tcgccccact ctatggcgtt gaaggaacta aaacaccagt ttcccctggt 6120
   gaaatgagac tgagggatga ccagaggaaa gtcttcagag aactagtgag gaattgtgac 6180
   ctgcccgttt ggctttcgtg gcaagtggcc aaggctggtt tgaagacgaa tgatcgtaag 6240
   tggtgttttg aaggccctga ggaacatgag atcttgaatg acagcggtga aacagtgaag 6300
   tgcagggctc ctggaggagc aaagaagcct ctgcgcccaa ggtggtgtga tgaaagggtg 6360
   tcatctgacc agagtgcgct gtctgaattt attaagtttg ctgaaggtag gaggggagct 6420
   gctgaagtgc tagttgtgct gagtgaactc cctgatttcc tggctaaaaa aggtggagag 6480
   gcaatggata ccatcagtgt gtttctccac tctgaggaag gctctagggc ttaccgcaat 6540
   gcactatcaa tgatgcctga ggcaatgaca atagtcatgc tgtttatact ggctggacta 6600
   ctgacatcgg gaatggtcat ctttttcatg tctcccaaag gcatcagtag aatgtctatg 6660
   gcgatgggca caatggccgg ctgtggatat ctcatgttcc ttggaggcgt caaacccact 6720
   cacatctcct atatcatgct catattcttt gtcctgatgg tggttgtgat ccccgagcca 6780
   gggcaacaaa ggtccatcca agacaaccaa gtggcatacc tcattattgg catcctgacg 6840
   ctggtttcag cggtggcagc caacgagcta ggcatgctgg agaaaaccaa agaggacctc 6900
   tttgggaaga agaacttaat tccatctagt gcttcaccct ggagttggcc ggatcttgac 6960
   ctgaagccag gagctgcctg gacagtgtac gttggcattg ttacaatgct ctctccaatg 7020
   ttgcaccact ggatcaaagt cgaatatggc aacctgtctc tgtctggaat agcccagtca 7080
   gcctcagtcc tttctttcat ggacaagggg ataccattca tgaagatgaa tatctcggtc 7140
   ataatgctgc tggtcagtgg ctggaattca ataacagtga tgcctctgct ctgtggcata 7200
   gggtgcgcca tgctccactg gtctctcatt ttacctggaa tcaaagcgca gcagtcaaag 7260
   cttgcacaga gaagggtgtt ccatggcgtt gccaagaacc ctgtggttga tgggaatcca 7320
   acagttgaca ttgaggaagc tcctgaaatg cctgcccttt atgagaagaa actggctcta 7380
   tatctccttc ttgctctcag cctagcttct gttgccatgt gcagaacgcc cttttcattg 7440
   gctgaaggca ttgtcctagc atcagctgcc ctagggccgc tcatagaggg aaacaccagc 7500
   cttctttgga atggacccat ggctgtctcc atgacaggag tcatgagggg gaatcactat 7560
   gcttttgtgg gagtcatgta caatctatgg aagatgaaaa ctggacgccg ggggagcgcg 7620
   aatggaaaaa ctttgggtga agtctggaag agggaactga atctgttgga caagcgacag 7680
   tttgagttgt ataaaaggac cgacattgtg gaggtggatc gtgatacggc acgcaggcat 7740
   ttggccgaag ggaaggtgga caccggggtg gcggtctcca gggggaccgc aaagttaagg 7800
   tggttccatg agcgtggcta tgtcaagctg gaaggtaggg tgattgacct ggggtgtggc 7860
   cgcggaggct ggtgttacta cgctgctgcg caaaaggaag tgagtggggt caaaggattt 7920
   actcttggaa gagacggcca tgagaaaccc atgaatgtgc aaagtctggg atggaacatc 7980
   atcaccttca aggacaaaac tgatatccac cgcctagaac cagtgaaatg tgacaccctt 8040
   ttgtgtgaca ttggagagtc atcatcgtca tcggtcacag agggggaaag gaccgtgaga 8100
   gttcttgata ctgtagaaaa atggctggct tgtggggttg acaacttctg tgtgaaggtg 8160
   ttagctccat acatgccaga tgttctcgag aaactggaat tgctccaaag gaggtttggc 8220
   ggaacagtga tcaggaaccc tctctccagg aattccactc atgaaatgta ctacgtgtct 8280
   ggagcccgca gcaatgtcac atttactgtg aaccaaacat cccgcctcct gatgaggaga 8340
   atgaggcgtc caactggaaa agtgaccctg gaggctgacg tcatcctccc aattgggaca 8400
   cgcagtgttg agacagacaa gggacccctg gacaaagagg ccatagaaga aagggttgag 8460
   aggataaaat ctgagtacat gacctcttgg ttttatgaca atgacaaccc ctacaggacc 8520
   tggcactact gtggctccta tgtcacaaaa acctcaggaa gtgcggcgag catggtaaat 8580
   ggtgttatta aaattctgac atatccatgg gacaggatag aggaggtcac aagaatggca 8640
   atgactgaca caaccccttt tggacagcaa agagtgttta aagaaaaagt tgacaccaga 8700
   gcaaaggatc caccagcggg aactaggaag atcatgaaag ttgtcaacag gtggctgttc 8760
   cgccacctgg ccagagaaaa gaaccccaga ctgtgcacaa aggaagaatt tattgcaaaa 8820
   gtccgaagtc atgcagccat tggagcttac ctggaagaac aagaacagtg gaagactgcc 8880
   aatgaggctg tccaagaccc aaagttctgg gaactggtgg atgaagaaag gaagctgcac 8940
   caacaaggca ggtgtcggac ttgtgtgtac aacatgatgg ggaaaagaga gaagaagctg 9000
   tcagagtttg ggaaagcaaa gggaagccgt gccatatggt atatgtggct gggagcgcgg 9060
   tatcttgagt ttgaggccct gggattcctg aatgaggacc attgggcttc cagggaaaac 9120
   tcaggaggag gagtggaagg cattggctta caatacctag gatatgtgat cagagacctg 9180
   gctgcaatgg atggtggtgg attctacgcg gatgacaccg ctggatggga cacgcgcatc 9240
   acagaggcag accttgatga tgaacaggag atcttgaact acatgagccc acatcacaaa 9300
   aaactggcac aagcagtaat ggaaatgaca tacaagaaca aagtggtgaa agtgttgaga 9360
   ccagccccag gagggaaagc ctacatggat gtcataagtc gacgagacca gagaggatcc 9420
   gggcaggtag tgacttatgc tctgaacacc atcaccaact tgaaagtcca attgatcaga 9480
   atggcagaag cagagatggt gatacatcac caacatgttc aagattgtga tgaatcagtt 9540
   ctgaccaggc tggaggcatg gctcactgag cacggatgta acagactgaa gaggatggcg 9600
   gtgagtggag acgactgtgt ggtccggccc atcgatgaca ggttcggcct ggccctgtcc 9660
   catctcaacg ccatgtccaa ggttagaaag gacatatctg aatggcagcc atcaaaaggg 9720
   tggaatgatt gggagaatgt gcccttctgt tcccaccact tccatgaact acagctgaag 9780
   gatggcagga ggattgtggt gccttgccga gaacaggacg agctcattgg gagaggaagg 9840
   gtgtctccag gaaacggctg gatgatcaag gaaacagctt gcctcagcaa agcctatgcc 9900
   aacatgtggt cactgatgta ttttcacaaa agggacatga ggctactgtc attggctgtt 9960
   tcctcagctg ttcccacctc atgggttcca caaggacgca caacatggtc gattcatggg 10020
   aaaggggagt ggatgaccac ggaagacatg cttgaggtgt ggaacagagt atggataacc 10080
   aacaacccac acatgcagga caagacaatg gtgaaaaaat ggagagatgt cccttatcta 10140
   accaagagac aagacaagct gtgcggatca ctgattggaa tgaccaatag ggccacctgg 10200
   gcctcccaca tccatttggt catccatcgt atccgaacgc tgattggaca ggagaaatac 10260
   actgactacc taacagtcat ggacaggtat tctgtggatg ctgacctgca actgggtgag 10320
   cttatctgaa acaccatcta acaggaataa ccgggataca aaccacgggt ggagaaccgg 10380
   actccccaca acctgaaacc gggatataaa ccacggctgg agaaccggac tccgcactta 10440
   aaatgaaaca gaaaccggga taaaaactac ggatggagaa ccggactcca cacattgaga 10500
   cagaagaagt tgtcagccca gaaccccaca cgagttttgc cactgctaag ctgtgaggca 10560
   gtgcaggctg ggacagccga cctccaggtt gcgaaaaacc tggtttctgg gacctcccac 10620
   cccagagtaa aaagaacgga gcctccgcta ccaccctccc acgtggtggt agaaagacgg 10680
   ggtctagagg ttagaggaga ccctccaggg aacaaatagt gggaccatat tgacgccagg 10740
   gaaagaccgg agtggttctc tgcttttcct ccagaggtct gtgagcacag tttgctcaag 10800
   aataagcaga c                                                      10811
<210> 12
   <211> 10785
   <212> DNA
   <213> Flaviviridae Flavivirus Yellow Fever Virus
<400> 12
   tcgagttgct aggcaataaa cacatttgga ttaattttaa tcgttcgttg agcgattagc 60
   agagaactga ccagaacatg tctggtcgta aagctcaggg aaaaaccctg ggcgtcaata 120
   tggtacgacg aggagttcgc tccttgtcaa acaaaataaa acaaaaaaca aaacaaattg 180
   gaaacagacc tggaccttca agaggtgttc aaggatttat ctttttcttt ttgttcaaca 240
   ttttgactgg aaaaaagatc acagcccacc taaagaggtt gtggaaaatg ctggacccaa 300
   gacaaggctt ggctgttcta aggaaagtca agagagtggt ggccagtttg atgagaggat 360
   tgtcctcaag gaaacgccgt tcccatgatg ttctgactgt gcaattccta attttgggaa 420
   tgctgttgat gacgggtgga gtgaccttgg tgcggaaaaa cagatggttg ctcctaaatg 480
   tgacatctga ggacctcggg aaaacattct ctgtgggcac aggcaactgc acaacaaaca 540
   ttttggaagc caagtactgg tgcccagact caatggaata caactgtccc aatctcagtc 600
   caagagagga gccagatgac attgattgct ggtgctatgg ggtggaaaac gttagagtcg 660
   catatggtaa gtgtgactca gcaggcaggt ctaggaggtc aagaagggcc attgacttgc 720
   ctacgcatga aaaccatggt ttgaagaccc ggcaagaaaa atggatgact ggaagaatgg 780
   gtgaaaggca actccaaaag attgagagat ggttcgtgag gaaccccttt tttgcagtga 840
   cggctctgac cattgcctac cttgtgggaa gcaacatgac gcaacgagtc gtgattgccc 900
   tactggtctt ggctgttggt ccggcctact cagctcactg cattggaatt actgacaggg 960
   atttcattga gggggtgcat ggaggaactt gggtttcagc taccctggag caagacaagt 1020
   gtgtcactgt tatggcccct gacaagcctt cattggacat ctcactagag acagtagcca 1080
   ttgatagacc tgctgaggtg aggaaagtgt gttacaatgc agttctcact catgtgaaga 1140
   ttaatgacaa gtgccccagc actggagagg cccacctagc tgaagagaac gaaggggaca 1200
   atgcgtgcaa gcgcacttat tctgatagag gctggggcaa tggctgtggc ctatttggga 1260
   aagggagcat tgtggcatgc gccaaattca cttgtgccaa atccatgagt ttgtttgagg 1320
   ttgatcagac caaaattcag tatgtcatca gagcacaatt gcatgtaggg gccaagcagg 1380
   aaaattggac taccgacatt aagactctca agtttgatgc cctgtcaggc tcccaggaag 1440
   tcgagttcat tgggtatgga aaagctacac tggaatgcca ggtgcaaact gcggtggact 1500
   ttggtaacag ttacatcgct gagatggaaa cagagagctg gatagtggac agacagtggg 1560
   cccaggactt gaccctgcca tggcagagtg gaagtggcgg ggtgtggaga gagatgcatc 1620
   atcttgtcga atttgaacct ccgcatgccg ccactatcag agtactggcc ctgggaaacc 1680
   aggaaggctc cttgaaaaca gctcttactg gcgcaatgag ggttacaaag gacacaaatg 1740
   acaacaacct ttacaaacta catggtggac atgtttcttg cagagtgaaa ttgtcagctt 1800
   tgacactcaa ggggacatcc tacaaaatat gcactgacaa aatgtttttt gtcaaaaacc 1860
   caactgacac tggccatggc actgttgtga tgcaggtgaa agtgtcaaaa ggagccccct 1920
   gcaggattcc agtgatagta gctgatgatc ttacagcggc aatcaataaa ggcattttgg 1980
   ttacagttaa ccccatcgcc tcaaccaatg atgatgaagt gctgattgag gtgaacccac 2040
   cttttggaga cagctacatt atcgttggga gaggagattc acgtctcact taccagtggc 2100
   acaaagaggg aagctcaata ggaaagttgt tcactcagac catgaaaggc gtggaacgcc 2160
   tggccgtcat gggagacacc gcctgggatt tcagctccgc tggagggttc ttcacttcgg 2220
   ttgggaaagg aattcatacg gtgtttggct ctgcctttca ggggctattt ggcggcttga 2280
   actggataac aaaggtcatc atgggggcgg tacttatatg ggttggcatc aacacaagaa 2340
   acatgacaat gtccatgagc atgatcttgg taggagtgat catgatgttt ttgtctctag 2400
   gagttggggc ggatcaagga tgcgccatca actttggcaa gagagagctc aagtgcggag 2460
   atggtatctt catatttaga gactctgatg actggctgaa caagtactca tactatccag 2520
   aagatcctgt gaagcttgca tcaatagtga aagcctcttt tgaagaaggg aagtgtggcc 2580
   taaattcagt tgactccctt gagcatgaga tgtggagaag cagggcagat gagatcaatg 2640
   ccatttttga ggaaaacgag gtggacattt ctgttgtcgt gcaggatcca aagaatgttt 2700
   accagagagg aactcatcca ttttccagaa ttcgggatgg tctgcagtat ggttggaaga 2760
   cttggggtaa gaaccttgtg ttctccccag ggaggaagaa tggaagcttc atcatagatg 2820
   gaaagtccag gaaagaatgc ccgttttcaa accgggtctg gaattctttc cagatagagg 2880
   agtttgggac gggagtgttc accacacgcg tgtacatgga cgcagtcttt gaatacacca 2940
   tagactgcga tggatctatc ttgggtgcag cggtgaacgg aaaaaagagt gcccatggct 3000
   ctccaacatt ttggatggga agtcatgaag taaatgggac atggatgatc cacaccttgg 3060
   aggcattaga ttacaaggag tgtgagtggc cactgacaca tacgattgga acatcagttg 3120
   aagagagtga aatgttcatg ccgagatcaa tcggaggccc agttagctct cacaatcata 3180
   tccctggata caaggttcag acgaacggac cttggatgca ggtaccacta gaagtgaaga 3240
   gagaagcttg cccagggact agcgtgatca ttgatggcaa ctgtgatgga cggggaaaat 3300
   caaccagatc caccacggat agcgggaaag ttattcctga atggtgttgc cgctcctgca 3360
   caatgccgcc tgtgagcttc catggtagtg atgggtgttg gtatcccatg gaaattaggc 3420
   caaggaaaac gcatgaaagc catctggtgc gctcctgggt tacagctgga gaaatacatg 3480
   ctgtcccttt tggtttggtg agcatgatga tagcaatgga agtggtccta aggaaaagac 3540
   agggaccaaa gcaaatgttg gttggaggag tagtgctctt gggagcaatg ctggtcgggc 3600
   aagtaactct ccttgatttg ctgaaactca cagtggctgt gggattgcat ttccatgaga 3660
   tgaacaatgg aggagacgcc atgtatatgg cgttgattgc tgccttttca atcagaccag 3720
   ggctgctcat cggctttggg ctcaggaccc tatggagccc tcgggaacgc cttgtgctga 3780
   ccctaggagc agccatggtg gagattgcct tgggtggcgt gatgggcggc ctgtggaagt 3840
   atctaaatgc agtttctctc tgcatcctga caataaatgc tgttgcttct aggaaagcat 3900
   caaataccat cttgcccctc atggctctgt tgacacctgt cactatggct gaggtgagac 3960
   ttgccgcaat gttcttttgt gccgtggtta tcataggggt ccttcaccag aatttcaagg 4020
   acacctccat gcagaagact atacctctgg tggccctcac actcacatct tacctgggct 4080
   tgacacaacc ttttttgggc ctgtgtgcat ttctggcaac ccgcatattt gggcgaagga 4140
   gtatcccagt gaatgaggca ctcgcagcag ctggtctagt gggagtgctg gcaggactgg 4200
   cttttcagga gatggagaac ttccttggtc cgattgcagt tggaggactc ctgatgatgc 4260
   tggttagcgt ggctgggagg gtggatgggc tagagctcaa gaagcttggt gaagtttcat 4320
   gggaagagga ggcggagatc agcgggagtt ccgcccgcta tgatgtggca ctcagtgaac 4380
   aaggggagtt caagctgctt tctgaagaga aagtgccatg ggaccaggtt gtgatgacct 4440
   cgctggcctt ggttggggct gccctccatc catttgctct tctgctggtc cttgctgggt 4500
   ggctgtttca tgtcagggga gctaggagaa gtggggatgt cttgtgggat attcccactc 4560
   ctaagatcat cgaggaatgt gaacatctgg aggatgggat ttatggcata ttccagtcaa 4620
   ccttcttggg ggcctcccag cgaggagtgg gagtggcaca gggaggggtg ttccacacaa 4680
   tgtggcatgt cacaagagga gctttccttg tcaggaatgg caagaagttg attccatctt 4740
   gggcttcagt aaaggaagac cttgtcgcct atggtggctc atggaagttg gaaggcagat 4800
   gggatggaga ggaagaggtc cagttgatcg cggctgttcc aggaaagaac gtggtcaacg 4860
   tccagacaaa accgagcttg ttcaaagtga ggaatggggg agaaatcggg gctgtcgctc 4920
   ttgactatcc gagtggcact tcaggatctc ctattgttaa caggaacgga gaggtgattg 4980
   ggctgtacgg caatggcatc cttgtcggtg acaactcctt cgtgtccgcc atatcccaga 5040
   ctgaggtgaa ggaagaagga aaggaggagc tccaagagat cccgacaatg ctaaagaaag 5100
   gaatgacaac tgtccttgat tttcatcctg gagctgggaa gacaagacgt ttcctcccac 5160
   agatcttggc cgagtgcgca cggagacgct tgcgcactct tgtgttggcc cccaccaggg 5220
   ttgttctttc tgaaatgaag gaggcttttc acggcctgga cgtgaaattc cacacacagg 5280
   ctttttccgc tcacggcagc gggagagaag tcattgatgc tatgtgccat gccaccctaa 5340
   cttacaggat gttggaacca actagggttg ttaactggga agtgatcatt atggatgaag 5400
   cccatttttt ggatccagct agcatagccg ctagaggttg ggcagcgcac agagctaggg 5460
   caaatgaaag tgcaacaatc ttgatgacag ccacaccgcc tgggactagt gatgaatttc 5520
   cacattcaaa tggtgaaata gaagatgttc aaacggacat acccagtgag ccctggaaca 5580
   cagggcatga ctggatcctg gctgacaaaa ggcccacggc atggttcctt ccatccatca 5640
   gagctgcaaa tgtcatggct gcctctttgc gtaaggctgg aaagagtgtg gtggtcctga 5700
   acaggaaaac ctttgagaga gaatacccca cgataaagca gaagaaacct gactttatat 5760
   tggccactga catagctgaa atgggagcca acctttgcgt ggagcgagtg ctggattgca 5820
   ggacggcttt taagcctgtg cttgtggatg aagggaggaa ggtggcaata aaagggccac 5880
   ttcgtatctc cgcatcctct gctgctcaaa ggagggggcg cattgggaga aatcccaaca 5940
   gagatggaga ctcatactac tattctgagc ctacaagtga aaataatgcc caccacgtct 6000
   gctggttgga ggcctcaatg ctcttggaca acatggaggt gaggggtgga atggtcgccc 6060
   cactctatgg cgttgaagga actaaaacac cagtttcccc tggtgaaatg agactgaggg 6120
   atgaccagag gaaagtcttc agagaactag tgaggaattg tgacctgccc gtttggcttt 6180
   cgtggcaagt ggccaaggct ggtttgaaga cgaatgatcg taagtggtgt tttgaaggcc 6240
   ctgaggaaca tgagatcttg aatgacagcg gtgaaacagt gaagtgcagg gctcctggag 6300
   gagcaaagaa gcctctgcgc ccaaggtggt gtgatgaaag ggtgtcatct gaccagagtg 6360
   cgctgtctga atttattaag tttgctgaag gtaggagggg agctgctgaa gtgctagttg 6420
   tgctgagtga actccctgat ttcctggcta aaaaaggtgg agaggcaatg gataccatca 6480
   gtgtgtttct ccactctgag gaaggctcta gggcttaccg caatgcacta tcaatgatgc 6540
   ctgaggcaat gacaatagtc atgctgttta tactggctgg actactgaca tcgggaatgg 6600
   tcatcttttt catgtctccc aaaggcatca gtagaatgtc tatggcgatg ggcacaatgg 6660
   ccggctgtgg atatctcatg ttccttggag gcgtcaaacc cactcacatc tcctatatca 6720
   tgctcatatt ctttgtcctg atggtggttg tgatccccga gccagggcaa caaaggtcca 6780
   tccaagacaa ccaagtggca tacctcatta ttggcatcct gacgctggtt tcagcggtgg 6840
   cagccaacga gctaggcatg ctggagaaaa ccaaagagga cctctttggg aagaagaact 6900
   taattccatc tagtgcttca ccctggagtt ggccggatct tgacctgaag ccaggagctg 6960
   cctggacagt gtacgttggc attgttacaa tgctctctcc aatgttgcac cactggatca 7020
   aagtcgaata tggcaacctg tctctgtctg gaatagccca gtcagcctca gtcctttctt 7080
   tcatggacaa ggggatacca ttcatgaaga tgaatatctc ggtcataatg ctgctggtca 7140
   gtggctggaa ttcaataaca gtgatgcctc tgctctgtgg catggggtgc gccatgctcc 7200
   actggtctct cattttacct ggaatcaaag cgcagcagtc aaagcttgca cagagaaggg 7260
   tgttccatgg cgttgccaag aaccctgtgg ttgatgggaa tccaacagtt gacattgagg 7320
   aagctcctga aatgcctgcc ctttatgaga agaaactggc tctatatctc cttcttgctc 7380
   tcagcctagc ttctgttgcc atgtgcagaa cgcccttttc attggctgaa ggcattgtcc 7440
   tagcatcagc tgccctaggg ccgctcatag agggaaacac cagccttctt tggaatggac 7500
   ccatggctgt ctccatgaca ggagtcatga gggggaatca ctatgctttt gtgggagtca 7560
   tgtacaatct atggaagatg aaaactggac gccgggggag cgcgaatgga aaaactttgg 7620
   gtgaagtctg gaagagggaa ctgaatctgt tggacaagcg acagtttgag ttgtataaaa 7680
   ggaccgacat tgtggaggtg gatcgtgata cggcacgcag gcatttggcc gaagggaagg 7740
   tggacaccgg ggtggcggtc tccaggggga ccgcaaagtt aaggtggttc catgagcgtg 7800
   gctatgtcaa gctggaaggt agggtgattg acctggggtg tggccgcgga ggctggtgtt 7860
   actacgctgc tgcgcaaaag gaagtgagtg gggtcaaagg atttactctt ggaagagacg 7920
   gccatgagaa acccatgaat gtgcaaagtc tgggatggaa catcatcacc ttcaaggaca 7980
   aaactgatat ccaccgccta gaaccagtga aatgtgacac ccttttgtgt gacattggag 8040
   agtcatcatc gtcatcggtc acagaggggg aaaggaccgt gagagttctt gatactgtag 8100
   aaaaatggct ggcttgtggg gttgacaact tctgtgtgaa ggtgttagct ccatacatgc 8160
   cagatgttct cgagaaactg gaattgctcc aaaggaggtt tggcggaaca gtgatcagga 8220
   accctctctc caggaattcc actcatgaaa tgtactacgt gtctggagcc cgcagcaatg 8280
   tcacatttac tgtgaaccaa acatcccgcc tcctgatgag gagaatgagg cgtccaactg 8340
   gaaaagtgac cctggaggct gacgtcatcc tcccaattgg gacacgcagt gttgagacag 8400
   acaagggacc cctggacaaa gaggccatag aagaaagggt tgagaggata aaatctgagt 8460
   acatgacctc ttggttttat gacaatgaca acccctacag gacctggcac tactgtggct 8520
   cctatgtcac aaaaacctca ggaagtgcgg cgagcatggt aaatggtgtt attaaaattc 8580
   tgacatatcc atgggacagg atagaggagg tcacaagaat ggcaatgact gacacaaccc 8640
   cttttggaca gcaaagagtg tttaaagaaa aagttgacac cagagcaaag gatccaccag 8700
   cgggaactag gaagatcatg aaagttgtca acaggtggct gttccgccac ctggccagag 8760
   aaaagaaccc cagactgtgc acaaaggaag aatttattgc aaaagtccga agtcatgcag 8820
   ccattggagc ttacctggaa gaacaagaac agtggaagac tgccaatgag gctgtccaag 8880
   acccaaagtt ctgggaactg gtggatgaag aaaggaagct gcaccaacaa ggcaggtgtc 8940
   ggacttgtgt gtacaacatg atggggaaaa gagagaagaa gctgtcagag tttgggaaag 9000
   caaagggaag ccgtgccata tggtatatgt ggctgggagc gcggtatctt gagtttgagg 9060
   ccctgggatt cctgaatgag gaccattggg cttccaggga aaactcagga ggaggagtgg 9120
   aaggcattgg cttacaatac ctaggatatg tgatcagaga cctggctgca atggatggtg 9180
   gtggattcta cgcggatgac accgctggat gggacacgcg catcacagag gcagaccttg 9240
   atgatgaaca ggagatcttg aactacatga gcccacatca caaaaaactg gcacaagcag 9300
   tgatggaaat gacatacaag aacaaagtgg tgaaagtgtt gagaccagcc ccaggaggga 9360
   aagcctacat ggatgtcata agtcgacgag accagagagg atccgggcag gtagtgactt 9420
   atgctctgaa caccatcacc aacttgaaag tccaattgat cagaatggca gaagcagaga 9480
   tggtgataca tcaccaacat gttcaagatt gtgatgaatc agttctgacc aggctggagg 9540
   catggctcac tgagcacgga tgtaacagac tgaagaggat ggcggtgagt ggagacgact 9600
   gtgtggtccg gcccatcgat gacaggtttg gcctggccct gtcccatctc aacgccatgt 9660
   ccaaggttag aaaggacata tctgaatggc agccatcaaa agggtggaat gattgggaga 9720
   atgtgccctt ctgttcccac cacttccatg aactacagct gaaggatggc aggaggattg 9780
   tggtgccttg ccgagaacag gacgagctca ttgggagagg aagggtgtct ccaggaaacg 9840
   gctggatgat caaggaaaca gcttgcctca gcaaagccta tgccaacatg tggtcactga 9900
   tgtattttca caaaagggac atgaggctac tgtcattggc tgtttcctca gctgttccca 9960
   cctcatgggt tccacaagga cgcacaacat ggtcgattca tgggaaaggg gagtggatga 10020
   ccacggaaga catgcttgag gtgtggaaca gagtatggat aaccaacaac ccacacatgc 10080
   aggacaagac aatggtgaaa aaatggagag atgtccctta tctaaccaag agacaagaca 10140
   agctgtgcgg atcactgatt ggaatgacca atagggccac ctgggcctcc cacatccatt 10200
   tggtcatcca tcgtatccga acgctgattg gacaggagaa atacactgac tacctaacag 10260
   tcatggacag gtattctgtg gatgctgacc tgcaactggg tgagcttatc tgaaacacca 10320
   tctaacagga ataaccggga tacaaaccac gggtggagaa ccggactccc cacaacctga 10380
   aaccgggata taaaccacgg ctggagaacc ggactccgca cttaaaatga aacagaaacc 10440
   gggataaaaa ctacggatgg agaaccggac tccacacatt gagacagaag aagttgtcag 10500
   cccagaaccc cacacgagtt ttgccactgc taagctgtga ggcagtgcag gctgggacag 10560
   ccgacctcca ggttgcgaaa aacctggttt ctgggacctc ccaccccaga gtaaaaagaa 10620
   cggagcctcc gctaccaccc tcccacgtgg tggtagaaag acggggtcta gaggttagag 10680
   gagaccctcc agggaacaaa tagtgggacc atattgacgc cagggaaaga ccggagtggt 10740
   tctctgcttt tcctccagag gtctgtgagc acagtttgct caaga                 10785
<210> 13
   <211> 3411
   <212> PRT
   <213> Flaviviridae Flavivirus Yellow Fever Virus
<400> 13
   Met Ser Gly Arg Lys Ala Gln Gly Lys Thr Leu Gly Val Asn Met Val
   1 5 10 15
   Arg Arg Gly Val Arg Ser Leu Ser Asn Lys Ile Lys Gln Lys Thr Lys
   20 25 30
   Gln Ile Gly Asn Arg Pro Gly Pro Ser Arg Gly Val Gln Gly Phe Ile
   35 40 45
   Phe Phe Phe Leu Phe Asn Ile Leu Thr Gly Lys Lys Ile Thr Ala His
   50 55 60
   Leu Lys Arg Leu Trp Lys Met Leu Asp Pro Arg Gln Gly Leu Ala Val
   65 70 75 80
   Leu Arg Lys Val Lys Arg Val Val Ala Ser Leu Met Arg Gly Leu Ser
   85 90 95
   Ser Arg Lys Arg Arg Ser His Asp Val Leu Thr Val Gln Phe Leu Ile
   100 105 110
   Leu Gly Met Leu Leu Met Thr Gly Gly Val Thr Leu Val Arg Lys Asn
   115 120 125
   Arg Trp Leu Leu Leu Asn Val Thr Ser Glu Asp Leu Gly Lys Thr Phe
   130 135 140
   Ser Val Gly Thr Gly Asn Cys Thr Thr Asn Ile Leu Glu Ala Lys Tyr
   145 150 155 160
   Trp Cys Pro Asp Ser Met Glu Tyr Asn Cys Pro Asn Leu Ser Pro Arg
   165 170 175
   Glu Glu Pro Asp Asp Ile Asp Cys Trp Cys Tyr Gly Val Glu Asn Val
   180 185 190
   Arg Val Ala Tyr Gly Lys Cys Asp Ser Ala Gly Arg Ser Arg Arg Ser
   195 200 205
   Arg Arg Ala Ile Asp Leu Pro Thr His Glu Asn His Gly Leu Lys Thr
   210 215 220
   Arg Gln Glu Lys Trp Met Thr Gly Arg Met Gly Glu Arg Gln Leu Gln
   225 230 235 240
   Lys Ile Glu Arg Trp Phe Val Arg Asn Pro Phe Phe Ala Val Thr Ala
   245 250 255
   Leu Thr Ile Ala Tyr Leu Val Gly Ser Asn Met Thr Gln Arg Val Val
   260 265 270
   Ile Ala Leu Leu Val Leu Ala Val Gly Pro Ala Tyr Ser Ala His Cys
   275 280 285
   Ile Gly Ile Thr Asp Arg Asp Phe Ile Glu Gly Val His Gly Gly Thr
   290 295 300
   Trp Val Ser Ala Thr Leu Glu Gln Asp Lys Cys Val Thr Val Met Ala
   305 310 315 320
   Pro Asp Lys Pro Ser Leu Asp Ile Ser Leu Glu Thr Val Ala Ile Asp
   325 330 335
   Arg Pro Ala Glu Val Arg Lys Val Cys Tyr Asn Ala Val Leu Thr His
   340 345 350
   Val Lys Ile Asn Asp Lys Cys Pro Ser Thr Gly Glu Ala His Leu Ala
   355 360 365
   Glu Glu Asn Glu Gly Asp Asn Ala Cys Lys Arg Thr Tyr Ser Asp Arg
   370 375 380
   Gly Trp Gly Asn Gly Cys Gly Leu Phe Gly Lys Gly Ser Ile Val Ala
   385 390 395 400
   Cys Ala Lys Phe Thr Cys Ala Lys Ser Met Ser Leu Phe Glu Val Asp
   405 410 415
   Gln Thr Lys Ile Gln Tyr Val Ile Arg Ala Gln Leu His Val Gly Ala
   420 425 430
   Lys Gln Glu Asn Trp Thr Thr Asp Ile Lys Thr Leu Lys Phe Asp Ala
           435                 440                 445
   Leu Ser Gly Ser Gln Glu Val Glu Phe Ile Gly Tyr Gly Lys Ala Thr
       450                 455                 460
   Leu Glu Cys Gln Val Gln Thr Ala Val Asp Phe Gly Asn Ser Tyr Ile
   465                 470                 475                 480
   Ala Glu Met Glu Thr Glu Ser Trp Ile Val Asp Arg Gln Trp Ala Gln
                   485                 490                 495
   Asp Leu Thr Leu Pro Trp Gln Ser Gly Ser Gly Gly Val Trp Arg Glu
               500                 505                 510
   Met His His Leu Val Glu Phe Glu Pro Pro His Ala Ala Thr Ile Arg
           515                 520                 525
   Val Leu Ala Leu Gly Asn Gln Glu Gly Ser Leu Lys Thr Ala Leu Thr
       530                 535                 540
   Gly Ala Met Arg Val Thr Lys Asp Thr Asn Asp Asn Asn Leu Tyr Lys
   545                 550                 555                 560
   Leu His Gly Gly His Val Ser Cys Arg Val Lys Leu Ser Ala Leu Thr
                   565                 570                 575
   Leu Lys Gly Thr Ser Tyr Lys Ile Cys Thr Asp Lys Met Phe Phe Val
               580                 585                 590
   Lys Asn Pro Thr Asp Thr Gly His Gly Thr Val Val Met Gln Val Lys
           595                 600                 605
   Val Ser Lys Gly Ala Pro Cys Arg Ile Pro Val Ile Val Ala Asp Asp
       610                 615                 620
   Leu Thr Ala Ala Ile Asn Lys Gly Ile Leu Val Thr Val Asn Pro Ile
   625                 630                 635                 640
   Ala Ser Thr Asn Asp Asp Glu Val Leu Ile Glu Val Asn Pro Pro Phe
                   645                 650                 655
   Gly Asp Ser Tyr Ile Ile Val Gly Arg Gly Asp Ser Arg Leu Thr Tyr
               660                 665                 670
   Gln Trp His Lys Glu Gly Ser Ser Ile Gly Lys Leu Phe Thr Gln Thr
           675                 680                 685
   Met Lys Gly Val Glu Arg Leu Ala Val Met Gly Asp Thr Ala Trp Asp
       690                 695                 700
   Phe Ser Ser Ala Gly Gly Phe Phe Thr Ser Val Gly Lys Gly Ile His
   705                 710                 715                 720
   Thr Val Phe Gly Ser Ala Phe Gln Gly Leu Phe Gly Gly Leu Asn Trp
                   725                 730                 735
   Ile Thr Lys Val Ile Met Gly Ala Val Leu Ile Trp Val Gly Ile Asn
               740                 745                 750
   Thr Arg Asn Met Thr Met Ser Met Ser Met Ile Leu Val Gly Val Ile
           755                 760                 765
   Met Met Phe Leu Ser Leu Gly Val Gly Ala Asp Gln Gly Cys Ala Ile
       770                 775                 780
   Asn Phe Gly Lys Arg Glu Leu Lys Cys Gly Asp Gly Ile Phe Ile Phe
   785                 790                 795                 800
   Arg Asp Ser Asp Asp Trp Leu Asn Lys Tyr Ser Tyr Tyr Pro Glu Asp
                   805                 810                 815
   Pro Val Lys Leu Ala Ser Ile Val Lys Ala Ser Phe Glu Glu Gly Lys
               820                 825                 830
   Cys Gly Leu Asn Ser Val Asp Ser Leu Glu His Glu Met Trp Arg Ser
           835                 840                 845
   Arg Ala Asp Glu Ile Asn Ala Ile Phe Glu Glu Asn Glu Val Asp Ile
       850                 855                 860
   Ser Val Val Val Gln Asp Pro Lys Asn Val Tyr Gln Arg Gly Thr His
   865                 870                 875                 880
   Pro Phe Ser Arg Ile Arg Asp Gly Leu Gln Tyr Gly Trp Lys Thr Trp
                   885                 890                 895
   Gly Lys Asn Leu Val Phe Ser Pro Gly Arg Lys Asn Gly Ser Phe Ile
               900                 905                 910
   Ile Asp Gly Lys Ser Arg Lys Glu Cys Pro Phe Ser Asn Arg Val Trp
           915                 920                 925
   Asn Ser Phe Gln Ile Glu Glu Phe Gly Thr Gly Val Phe Thr Thr Arg
       930                 935                 940
   Val Tyr Met Asp Ala Val Phe Glu Tyr Thr Ile Asp Cys Asp Gly Ser
   945                 950                 955                 960
   Ile Leu Gly Ala Ala Val Asn Gly Lys Lys Ser Ala His Gly Ser Pro
                   965                 970                 975
   Thr Phe Trp Met Gly Ser His Glu Val Asn Gly Thr Trp Met Ile His
               980                 985                 990
   Thr Leu Glu Ala Leu Asp Tyr Lys Glu Cys Glu Trp Pro Leu Thr His
           995                 1000                1005
   Thr Ile Gly Thr Ser Val Glu Glu Ser Glu Met Phe Met Pro Arg Ser
       1010                1015                1020
   Ile Gly Gly Pro Val Ser Ser His Asn His Ile Pro Gly Tyr Lys Val
   1025                1030                1035                1040
   Gln Thr Asn Gly Pro Trp Met Gln Val Pro Leu Glu Val Lys Arg Glu
                   1045                1050                1055
   Ala Cys Pro Gly Thr Ser Val Ile Ile Asp Gly Asn Cys Asp Gly Arg
               1060                1065                1070
   Gly Lys Ser Thr Arg Ser Thr Thr Asp Ser Gly Lys Val Ile Pro Glu
           1075                1080                1085
   Trp Cys Cys Arg Ser Cys Thr Met Pro Pro Val Ser Phe His Gly Ser
       1090                1095                1100
   Asp Gly Cys Trp Tyr Pro Met Glu Ile Arg Pro Arg Lys Thr His Glu
   1105                1110                1115                1120
   Ser His Leu Val Arg Ser Trp Val Thr Ala Gly Glu Ile His Ala Val
                   1125                1130                1135
   Pro Phe Gly Leu Val Ser Met Met Ile Ala Met Glu Val Val Leu Arg
               1140                1145                1150
   Lys Arg Gln Gly Pro Lys Gln Met Leu Val Gly Gly Val Val Leu Leu
           1155                1160                1165
   Gly Ala Met Leu Val Gly Gln Val Thr Leu Leu Asp Leu Leu Lys Leu
       1170                1175                1180
   Thr Val Ala Val Gly Leu His Phe His Glu Met Asn Asn Gly Gly Asp
   1185                1190                1195                1200
   Ala Met Tyr Met Ala Leu Ile Ala Ala Phe Ser Ile Arg Pro Gly Leu
                   1205                1210                1215
   Leu Ile Gly Phe Gly Leu Arg Thr Leu Trp Ser Pro Arg Glu Arg Leu
               1220                1225                1230
   Val Leu Thr Leu Gly Ala Ala Met Val Glu Ile Ala Leu Gly Gly Val
           1235                1240                1245
   Met Gly Gly Leu Trp Lys Tyr Leu Asn Ala Val Ser Leu Cys Ile Leu
       1250                1255                1260
   Thr Ile Asn Ala Val Ala Ser Arg Lys Ala Ser Asn Thr Ile Leu Pro
   1265                1270                1275                1280
   Leu Met Ala Leu Leu Thr Pro Val Thr Met Ala Glu Val Arg Leu Ala
                   1285                1290                1295
   Ala Met Phe Phe Cys Ala Val Val Ile Ile Gly Val Leu His Gln Asn
               1300                1305                1310
   Phe Lys Asp Thr Ser Met Gln Lys Thr Ile Pro Leu Val Ala Leu Thr
           1315                1320                1325
   Leu Thr Ser Tyr Leu Gly Leu Thr Gln Pro Phe Leu Gly Leu Cys Ala
       1330                1335                1340
   Phe Leu Ala Thr Arg Ile Phe Gly Arg Arg Ser Ile Pro Val Asn Glu
   1345                1350                1355                1360
   Ala Leu Ala Ala Ala Gly Leu Val Gly Val Leu Ala Gly Leu Ala Phe
                   1365                1370                1375
   Gln Glu Met Glu Asn Phe Leu Gly Pro Ile Ala Val Gly Gly Leu Leu
               1380                1385                1390
   Met Met Leu Val Ser Val Ala Gly Arg Val Asp Gly Leu Glu Leu Lys
           1395                1400                1405
   Lys Leu Gly Glu Val Ser Trp Glu Glu Glu Ala Glu Ile Ser Gly Ser
       1410                1415                1420
   Ser Ala Arg Tyr Asp Val Ala Leu Ser Glu Gln Gly Glu Phe Lys Leu
   1425                1430                1435                1440
   Leu Ser Glu Glu Lys Val Pro Trp Asp Gln Val Val Met Thr Ser Leu
                   1445                1450                1455
   Ala Leu Val Gly Ala Ala Leu His Pro Phe Ala Leu Leu Leu Val Leu
               1460                1465                1470
   Ala Gly Trp Leu Phe His Val Arg Gly Ala Arg Arg Ser Gly Asp Val
   1475 1480 1485
   Leu Trp Asp Ile Pro Thr Pro Lys Ile Ile Glu Glu Cys Glu His Leu
   1490 1495 1500
   Glu Asp Gly Ile Tyr Gly Ile Phe Gln Ser Thr Phe Leu Gly Ala Ser
   1505 1510 1515 1520
   Gln Arg Gly Val Gly Val Ala Gln Gly Gly Val Phe His Thr Met Trp
   1525 1530 1535
   His Val Thr Arg Gly Ala Phe Leu Val Arg Asn Gly Lys Lys Leu Ile
   1540 1545 1550
   Pro Ser Trp Ala Ser Val Lys Glu Asp Leu Val Ala Tyr Gly Gly Ser
   1555 1560 1565
   Trp Lys Leu Glu Gly Arg Trp Asp Gly Glu Glu Glu Val Gln Leu Ile
   1570 1575 1580
   Ala Ala Val Pro Gly Lys Asn Val Val Asn Val Gln Thr Lys Pro Ser
   1585 1590 1595 1600
   Leu Phe Lys Val Arg Asn Gly Gly Glu Ile Gly Ala Val Ala Leu Asp
   1605 1610 1615
   Tyr Pro Ser Gly Thr Ser Gly Ser Pro Ile Val Asn Arg Asn Gly Glu
   1620 1625 1630
   Val Ile Gly Leu Tyr Gly Asn Gly Ile Leu Val Gly Asp Asn Ser Phe
   1635 1640 1645
   Val Ser Ala Ile Ser Gln Thr Glu Val Lys Glu Glu Gly Lys Glu Glu
   1650 1655 1660
   Leu Gln Glu Ile Pro Thr Met Leu Lys Lys Gly Met Thr Thr Val Leu
   1665 1670 1675 1680
   Asp Phe His Pro Gly Ala Gly Lys Thr Arg Arg Phe Leu Pro Gln Ile
   1685 1690 1695
   Leu Ala Glu Cys Ala Arg Arg Arg Leu Arg Thr Leu Val Leu Ala Pro
   1700 1705 1710
   Thr Arg Val Val Leu Ser Glu Met Lys Glu Ala Phe His Gly Leu Asp
   1715 1720 1725
   Val Lys Phe His Thr Gln Ala Phe Ser Ala His Gly Ser Gly Arg Glu
   1730 1735 1740
   Val Ile Asp Ala Met Cys His Ala Thr Leu Thr Tyr Arg Met Leu Glu
   1745 1750 1755 1760
   Pro Thr Arg Val Val Asn Trp Glu Val Ile Ile Met Asp Glu Ala His
   1765 1770 1775
   Phe Leu Asp Pro Ala Ser Ile Ala Ala Arg Gly Trp Ala Ala His Arg
   1780 1785 1790
   Ala Arg Ala Asn Glu Ser Ala Thr Ile Leu Met Thr Ala Thr Pro Pro
   1795 1800 1805
   Gly Thr Ser Asp Glu Phe Pro His Ser Asn Gly Glu Ile Glu Asp Val
   1810 1815 1820
   Gln Thr Asp Ile Pro Ser Glu Pro Trp Asn Thr Gly His Asp Trp Ile
   1825 1830 1835 1840
   Leu Ala Asp Lys Arg Pro Thr Ala Trp Phe Leu Pro Ser Ile Arg Ala
   1845 1850 1855
   Ala Asn Val Met Ala Ala Ser Leu Arg Lys Ala Gly Lys Ser Val Val
   1860 1865 1870
   Val Leu Asn Arg Lys Thr Phe Glu Arg Glu Tyr Pro Thr Ile Lys Gln
   1875 1880 1885
   Lys Lys Pro Asp Phe Ile Leu Ala Thr Asp Ile Ala Glu Met Gly Ala
   1890 1895 1900
   Asn Leu Cys Val Glu Arg Val Leu Asp Cys Arg Thr Ala Phe Lys Pro
   1905 1910 1915 1920
   Val Leu Val Asp Glu Gly Arg Lys Val Ala Ile Lys Gly Pro Leu Arg
   1925 1930 1935
   Ile Ser Ala Ser Ser Ala Ala Gln Arg Arg Gly Arg Ile Gly Arg Asn
   1940 1945 1950
   Pro Asn Arg Asp Gly Asp Ser Tyr Tyr Tyr Ser Glu Pro Thr Ser Glu
   1955 1960 1965
   Asn Asn Ala His His Val Cys Trp Leu Glu Ala Ser Met Leu Leu Asp
   1970 1975 1980
   Asn Met Glu Val Arg Gly Gly Met Val Ala Pro Leu Tyr Gly Val Glu
   1985 1990 1995 2000
   Gly Thr Lys Thr Pro Val Ser Pro Gly Glu Met Arg Leu Arg Asp Asp
   2005 2010 2015
   Gln Arg Lys Val Phe Arg Glu Leu Val Arg Asn Cys Asp Leu Pro Val
   2020 2025 2030
   Trp Leu Ser Trp Gln Val Ala Lys Ala Gly Leu Lys Thr Asn Asp Arg
   2035 2040 2045
   Lys Trp Cys Phe Glu Gly Pro Glu Glu His Glu Ile Leu Asn Asp Ser
   2050 2055 2060
   Gly Glu Thr Val Lys Cys Arg Ala Pro Gly Gly Ala Lys Lys Pro Leu
   2065 2070 2075 2080
   Arg Pro Arg Trp Cys Asp Glu Arg Val Ser Ser Asp Gln Ser Ala Leu
   2085 2090 2095
   Ser Glu Phe Ile Lys Phe Ala Glu Gly Arg Arg Gly Ala Ala Glu Val
   2100 2105 2110
   Leu Val Val Leu Ser Glu Leu Pro Asp Phe Leu Ala Lys Lys Gly Gly
   2115 2120 2125
   Glu Ala Met Asp Thr Ile Ser Val Phe Leu His Ser Glu Glu Gly Ser
   2130 2135 2140
   Arg Ala Tyr Arg Asn Ala Leu Ser Met Met Pro Glu Ala Met Thr Ile
   2145 2150 2155 2160
   Val Met Leu Phe Ile Leu Ala Gly Leu Leu Thr Ser Gly Met Val Ile
   2165 2170 2175
   Phe Phe Met Ser Pro Lys Gly Ile Ser Arg Met Ser Met Ala Met Gly
   2180 2185 2190
   Thr Met Ala Gly Cys Gly Tyr Leu Met Phe Leu Gly Gly Val Lys Pro
   2195 2200 2205
   Thr His Ile Ser Tyr Ile Met Leu Ile Phe Phe Val Leu Met Val Val
   2210 2215 2220
   Val Ile Pro Glu Pro Gly Gln Gln Arg Ser Ile Gln Asp Asn Gln Val
   2225 2230 2235 2240
   Ala Tyr Leu Ile Ile Gly Ile Leu Thr Leu Val Ser Ala Val Ala Ala
   2245 2250 2255
   Asn Glu Leu Gly Met Leu Glu Lys Thr Lys Glu Asp Leu Phe Gly Lys
   2260 2265 2270
   Lys Asn Leu Ile Pro Ser Ser Ala Ser Pro Trp Ser Trp Pro Asp Leu
   2275 2280 2285
   Asp Leu Lys Pro Gly Ala Ala Trp Thr Val Tyr Val Gly Ile Val Thr
   2290 2295 2300
   Met Leu Ser Pro Met Leu His His Trp Ile Lys Val Glu Tyr Gly Asn
   2305 2310 2315 2320
   Leu Ser Leu Ser Gly Ile Ala Gln Ser Ala Ser Val Leu Ser Phe Met
   2325 2330 2335
   Asp Lys Gly Ile Pro Phe Met Lys Met Asn Ile Ser Val Ile Met Leu
   2340 2345 2350
   Leu Val Ser Gly Trp Asn Ser Ile Thr Val Met Pro Leu Leu Cys Gly
   2355 2360 2365
   Ile Gly Cys Ala Met Leu His Trp Ser Leu Ile Leu Pro Gly Ile Lys
   2370 2375 2380
   Ala Gln Gln Ser Lys Leu Ala Gln Arg Arg Val Phe His Gly Val Ala
   2385 2390 2395 2400
   Lys Asn Pro Val Val Asp Gly Asn Pro Thr Val Asp Ile Glu Glu Ala
   2405 2410 2415
   Pro Glu Met Pro Ala Leu Tyr Glu Lys Lys Leu Ala Leu Tyr Leu Leu
   2420 2425 2430
   Leu Ala Leu Ser Leu Ala Ser Val Ala Met Cys Arg Thr Pro Phe Ser
   2435 2440 2445
   Leu Ala Glu Gly Ile Val Leu Ala Ser Ala Ala Leu Gly Pro Leu Ile
   2450 2455 2460
   Glu Gly Asn Thr Ser Leu Leu Trp Asn Gly Pro Met Ala Val Ser Met
   2465 2470 2475 2480
   Thr Gly Val Met Arg Gly Asn His Tyr Ala Phe Val Gly Val Met Tyr
   2485 2490 2495
   Asn Leu Trp Lys Met Lys Thr Gly Arg Arg Gly Ser Ala Asn Gly Lys
   2500 2505 2510
   Thr Leu Gly Glu Val Trp Lys Arg Glu Leu Asn Leu Leu Asp Lys Arg
   2515 2520 2525
   Gln Phe Glu Leu Tyr Lys Arg Thr Asp Ile Val Glu Val Asp Arg Asp
   2530 2535 2540
   Thr Ala Arg Arg His Leu Ala Glu Gly Lys Val Asp Thr Gly Val Ala
   2545 2550 2555 2560
   Val Ser Arg Gly Thr Ala Lys Leu Arg Trp Phe His Glu Arg Gly Tyr
   2565 2570 2575
   Val Lys Leu Glu Gly Arg Val Ile Asp Leu Gly Cys Gly Arg Gly Gly
   2580 2585 2590
   Trp Cys Tyr Tyr Ala Ala Ala Gln Lys Glu Val Ser Gly Val Lys Gly
   2595 2600 2605
   Phe Thr Leu Gly Arg Asp Gly His Glu Lys Pro Met Asn Val Gln Ser
   2610 2615 2620
   Leu Gly Trp Asn Ile Ile Thr Phe Lys Asp Lys Thr Asp Ile His Arg
   2625 2630 2635 2640
   Leu Glu Pro Val Lys Cys Asp Thr Leu Leu Cys Asp Ile Gly Glu Ser
   2645 2650 2655
   Ser Ser Ser Ser Val Thr Glu Gly Glu Arg Thr Val Arg Val Leu Asp
   2660 2665 2670
   Thr Val Glu Lys Trp Leu Ala Cys Gly Val Asp Asn Phe Cys Val Lys
   2675 2680 2685
   Val Leu Ala Pro Tyr Met Pro Asp Val Leu Glu Lys Leu Glu Leu Leu
   2690 2695 2700
   Gln Arg Arg Phe Gly Gly Thr Val Ile Arg Asn Pro Leu Ser Arg Asn
   2705 2710 2715 2720
   Ser Thr His Glu Met Tyr Tyr Val Ser Gly Ala Arg Ser Asn Val Thr
   2725 2730 2735
   Phe Thr Val Asn Gln Thr Ser Arg Leu Leu Met Arg Arg Met Arg Arg
   2740 2745 2750
   Pro Thr Gly Lys Val Thr Leu Glu Ala Asp Val Ile Leu Pro Ile Gly
   2755 2760 2765
   Thr Arg Ser Val Glu Thr Asp Lys Gly Pro Leu Asp Lys Glu Ala Ile
   2770 2775 2780
   Glu Glu Arg Val Glu Arg Ile Lys Ser Glu Tyr Met Thr Ser Trp Phe
   2785 2790 2795 2800
   Tyr Asp Asn Asp Asn Pro Tyr Arg Thr Trp His Tyr Cys Gly Ser Tyr
   2805 2810 2815
   Val Thr Lys Thr Ser Gly Ser Ala Ala Ser Met Val Asn Gly Val Ile
   2820 2825 2830
   Lys Ile Leu Thr Tyr Pro Trp Asp Arg Ile Glu Glu Val Thr Arg Met
   2835 2840 2845
   Ala Met Thr Asp Thr Thr Pro Phe Gly Gln Gln Arg Val Phe Lys Glu
   2850 2855 2860
   Lys Val Asp Thr Arg Ala Lys Asp Pro Pro Ala Gly Thr Arg Lys Ile
   2865 2870 2875 2880
   Met Lys Val Val Asn Arg Trp Leu Phe Arg His Leu Ala Arg Glu Lys
   2885 2890 2895
   Asn Pro Arg Leu Cys Thr Lys Glu Glu Phe Ile Ala Lys Val Arg Ser
   2900 2905 2910
   His Ala Ala Ile Gly Ala Tyr Leu Glu Glu Gln Glu Gln Trp Lys Thr
   2915 2920 2925
   Ala Asn Glu Ala Val Gln Asp Pro Lys Phe Trp Glu Leu Val Asp Glu
   2930 2935 2940
   Glu Arg Lys Leu His Gln Gln Gly Arg Cys Arg Thr Cys Val Tyr Asn
   2945 2950 2955 2960
   Met Met Gly Lys Arg Glu Lys Lys Leu Ser Glu Phe Gly Lys Ala Lys
   2965 2970 2975
   Gly Ser Arg Ala Ile Trp Tyr Met Trp Leu Gly Ala Arg Tyr Leu Glu
   2980 2985 2990
   Phe Glu Ala Leu Gly Phe Leu Asn Glu Asp His Trp Ala Ser Arg Glu
   2995 3000 3005
   Asn Ser Gly Gly Gly Val Glu Gly Ile Gly Leu Gln Tyr Leu Gly Tyr
   3010 3015 3020
   Val Ile Arg Asp Leu Ala Ala Met Asp Gly Gly Gly Phe Tyr Ala Asp
   3025 3030 3035 3040
   Asp Thr Ala Gly Trp Asp Thr Arg Ile Thr Glu Ala Asp Leu Asp Asp
   3045 3050 3055
   Glu Gln Glu Ile Leu Asn Tyr Met Ser Pro His His Lys Lys Leu Ala
   3060 3065 3070
   Gln Ala Val Met Glu Met Thr Tyr Lys Asn Lys Val Val Lys Val Leu
   3075 3080 3085
   Arg Pro Ala Pro Gly Gly Lys Ala Tyr Met Asp Val Ile Ser Arg Arg
   3090 3095 3100
   Asp Gln Arg Gly Ser Gly Gln Val Val Thr Tyr Ala Leu Asn Thr Ile
   3105 3110 3115 3120
   Thr Asn Leu Lys Val Gln Leu Ile Arg Met Ala Glu Ala Glu Met Val
   3125 3130 3135
   Ile His His Gln His Val Gln Asp Cys Asp Glu Ser Val Leu Thr Arg
   3140 3145 3150
   Leu Glu Ala Trp Leu Thr Glu His Gly Cys Asn Arg Leu Lys Arg Met
   3155 3160 3165
   Ala Val Ser Gly Asp Asp Cys Val Val Arg Pro Ile Asp Asp Arg Phe
   3170 3175 3180
   Gly Leu Ala Leu Ser His Leu Asn Ala Met Ser Lys Val Arg Lys Asp
   3185 3190 3195 3200
   Ile Ser Glu Trp Gln Pro Ser Lys Gly Trp Asn Asp Trp Glu Asn Val
   3205 3210 3215
   Pro Phe Cys Ser His His Phe His Glu Leu Gln Leu Lys Asp Gly Arg
   3220 3225 3230
   Arg Ile Val Val Pro Cys Arg Glu Gln Asp Glu Leu Ile Gly Arg Gly
   3235 3240 3245
   Arg Val Ser Pro Gly Asn Gly Trp Met Ile Lys Glu Thr Ala Cys Leu
   3250 3255 3260
   Ser Lys Ala Tyr Ala Asn Met Trp Ser Leu Met Tyr Phe His Lys Arg
   3265 3270 3275 3280
   Asp Met Arg Leu Leu Ser Leu Ala Val Ser Ser Ala Val Pro Thr Ser
   3285 3290 3295
   Trp Val Pro Gln Gly Arg Thr Thr Trp Ser Ile His Gly Lys Gly Glu
   3300 3305 3310
   Trp Met Thr Thr Glu Asp Met Leu Glu Val Trp Asn Arg Val Trp Ile
   3315 3320 3325
   Thr Asn Asn Pro His Met Gln Asp Lys Thr Met Val Lys Lys Trp Arg
   3330 3335 3340
   Asp Val Pro Tyr Leu Thr Lys Arg Gln Asp Lys Leu Cys Gly Ser Leu
   3345 3350 3355 3360
   Ile Gly Met Thr Asn Arg Ala Thr Trp Ala Ser His Ile His Leu Val
   3365 3370 3375
   Ile His Arg Ile Arg Thr Leu Ile Gly Gln Glu Lys Tyr Thr Asp Tyr
   3380 3385 3390
   Leu Thr Val Met Asp Arg Tyr Ser Val Asp Ala Asp Leu Gln Leu Gly
   3395 3400 3405
   Glu Leu Ile
   3410
<210> 14
   <211> 3411
   <212> PRT
   <213> Flaviviridae Flavivirus Yellow Fever Virus
<400> 14
   Met Ser Gly Arg Lys Ala Gln Gly Lys Thr Leu Gly Val Asn Met Val
    1               5                  10                  15
   Arg Arg Gly Val Arg Ser Leu Ser Asn Lys Ile Lys Gln Lys Thr Lys
               20                  25                  30
   Gln Ile Gly Asn Arg Pro Gly Pro Ser Arg Gly Val Gln Gly Phe Ile
           35                  40                  45
   Phe Phe Phe Leu Phe Asn Ile Leu Thr Gly Lys Lys Ile Thr Ala His
       50                  55                  60
   Leu Lys Arg Leu Trp Lys Met Leu Asp Pro Arg Gln Gly Leu Ala Val
   65                  70                  75                  80
   Leu Arg Lys Val Lys Arg Val Val Ala Ser Leu Met Arg Gly Leu Ser
                   85                  90                  95
   Ser Arg Lys Arg Arg Ser His Asp Val Leu Thr Val Gln Phe Leu Ile
               100                 105                 110
   Leu Gly Met Leu Leu Met Thr Gly Gly Val Thr Leu Val Arg Lys Asn
           115                 120                 125
   Arg Trp Leu Leu Leu Asn Val Thr Ser Glu Asp Leu Gly Lys Thr Phe
       130                 135                 140
   Ser Val Gly Thr Gly Asn Cys Thr Thr Asn Ile Leu Glu Ala Lys Tyr
   145                 150                 155                 160
   Trp Cys Pro Asp Ser Met Glu Tyr Asn Cys Pro Asn Leu Ser Pro Arg
                   165                 170                 175
   Glu Glu Pro Asp Asp Ile Asp Cys Trp Cys Tyr Gly Val Glu Asn Val
               180                 185                 190
   Arg Val Ala Tyr Gly Lys Cys Asp Ser Ala Gly Arg Ser Arg Arg Ser
           195                 200                 205
   Arg Arg Ala Ile Asp Leu Pro Thr His Glu Asn His Gly Leu Lys Thr
       210                 215                 220
   Arg Gln Glu Lys Trp Met Thr Gly Arg Met Gly Glu Arg Gln Leu Gln
   225                 230                 235                 240
   Lys Ile Glu Arg Trp Phe Val Arg Asn Pro Phe Phe Ala Val Thr Ala
                   245                 250                 255
   Leu Thr Ile Ala Tyr Leu Val Gly Ser Asn Met Thr Gln Arg Val Val
               260                 265                 270
   Ile Ala Leu Leu Val Leu Ala Val Gly Pro Ala Tyr Ser Ala His Cys
           275                 280                 285
   Ile Gly Ile Thr Asp Arg Asp Phe Ile Glu Gly Val His Gly Gly Thr
       290                 295                 300
   Trp Val Ser Ala Thr Leu Glu Gln Asp Lys Cys Val Thr Val Met Ala
   305                 310                 315                 320
   Pro Asp Lys Pro Ser Leu Asp Ile Ser Leu Glu Thr Val Ala Ile Asp
                   325                 330                 335
   Arg Pro Ala Glu Val Arg Lys Val Cys Tyr Asn Ala Val Leu Thr His
               340                 345                 350
   Val Lys Ile Asn Asp Lys Cys Pro Ser Thr Gly Glu Ala His Leu Ala
           355                 360                 365
   Glu Glu Asn Glu Gly Asp Asn Ala Cys Lys Arg Thr Tyr Ser Asp Arg
       370                 375                 380
   Gly Trp Gly Asn Gly Cys Gly Leu Phe Gly Lys Gly Ser Ile Val Ala
   385                 390                 395                 400
   Cys Ala Lys Phe Thr Cys Ala Lys Ser Met Ser Leu Phe Glu Val Asp
                   405                 410                 415
   Gln Thr Lys Ile Gln Tyr Val Ile Arg Ala Gln Leu His Val Gly Ala
               420                 425                 430
   Lys Gln Glu Asn Trp Thr Thr Asp Ile Lys Thr Leu Lys Phe Asp Ala
           435                 440                 445
   Leu Ser Gly Ser Gln Glu Val Glu Phe Ile Gly Tyr Gly Lys Ala Thr
       450                 455                 460
   Leu Glu Cys Gln Val Gln Thr Ala Val Asp Phe Gly Asn Ser Tyr Ile
   465                 470                 475                 480
   Ala Glu Met Glu Thr Glu Ser Trp Ile Val Asp Arg Gln Trp Ala Gln
                   485                 490                 495
   Asp Leu Thr Leu Pro Trp Gln Ser Gly Ser Gly Gly Val Trp Arg Glu
               500                 505                 510
   Met His His Leu Val Glu Phe Glu Pro Pro His Ala Ala Thr Ile Arg
           515                 520                 525
   Val Leu Ala Leu Gly Asn Gln Glu Gly Ser Leu Lys Thr Ala Leu Thr
       530                 535                 540
   Gly Ala Met Arg Val Thr Lys Asp Thr Asn Asp Asn Asn Leu Tyr Lys
   545                 550                 555                 560
   Leu His Gly Gly His Val Ser Cys Arg Val Lys Leu Ser Ala Leu Thr
                   565                 570                 575
   Leu Lys Gly Thr Ser Tyr Lys Ile Cys Thr Asp Lys Met Phe Phe Val
               580                 585                 590
   Lys Asn Pro Thr Asp Thr Gly His Gly Thr Val Val Met Gln Val Lys
           595                 600                 605
   Val Ser Lys Gly Ala Pro Cys Arg Ile Pro Val Ile Val Ala Asp Asp
       610                 615                 620
   Leu Thr Ala Ala Ile Asn Lys Gly Ile Leu Val Thr Val Asn Pro Ile
   625                 630                 635                 640
   Ala Ser Thr Asn Asp Asp Glu Val Leu Ile Glu Val Asn Pro Pro Phe
                   645                 650                 655
   Gly Asp Ser Tyr Ile Ile Val Gly Arg Gly Asp Ser Arg Leu Thr Tyr
               660                 665                 670
   Gln Trp His Lys Glu Gly Ser Ser Ile Gly Lys Leu Phe Thr Gln Thr
           675                 680                 685
   Met Lys Gly Val Glu Arg Leu Ala Val Met Gly Asp Thr Ala Trp Asp
       690                 695                 700
   Phe Ser Ser Ala Gly Gly Phe Phe Thr Ser Val Gly Lys Gly Ile His
   705                 710                 715                 720
   Thr Val Phe Gly Ser Ala Phe Gln Gly Leu Phe Gly Gly Leu Asn Trp
                   725                 730                 735
   Ile Thr Lys Val Ile Met Gly Ala Val Leu Ile Trp Val Gly Ile Asn
               740                 745                 750
   Thr Arg Asn Met Thr Met Ser Met Ser Met Ile Leu Val Gly Val Ile
           755                 760                 765
   Met Met Phe Leu Ser Leu Gly Val Gly Ala Asp Gln Gly Cys Ala Ile
       770                 775                 780
   Asn Phe Gly Lys Arg Glu Leu Lys Cys Gly Asp Gly Ile Phe Ile Phe
   785                 790                 795                 800
   Arg Asp Ser Asp Asp Trp Leu Asn Lys Tyr Ser Tyr Tyr Pro Glu Asp
                   805                 810                 815
   Pro Val Lys Leu Ala Ser Ile Val Lys Ala Ser Phe Glu Glu Gly Lys
               820                 825                 830
   Cys Gly Leu Asn Ser Val Asp Ser Leu Glu His Glu Met Trp Arg Ser
           835                 840                 845
   Arg Ala Asp Glu Ile Asn Ala Ile Phe Glu Glu Asn Glu Val Asp Ile
       850                 855                 860
   Ser Val Val Val Gln Asp Pro Lys Asn Val Tyr Gln Arg Gly Thr His
   865                 870                 875                 880
   Pro Phe Ser Arg Ile Arg Asp Gly Leu Gln Tyr Gly Trp Lys Thr Trp
                   885                 890                 895
   Gly Lys Asn Leu Val Phe Ser Pro Gly Arg Lys Asn Gly Ser Phe Ile
               900                 905                 910
   Ile Asp Gly Lys Ser Arg Lys Glu Cys Pro Phe Ser Asn Arg Val Trp
           915                 920                 925
   Asn Ser Phe Gln Ile Glu Glu Phe Gly Thr Gly Val Phe Thr Thr Arg
       930                 935                 940
   Val Tyr Met Asp Ala Val Phe Glu Tyr Thr Ile Asp Cys Asp Gly Ser
   945                 950                 955                 960
   Ile Leu Gly Ala Ala Val Asn Gly Lys Lys Ser Ala His Gly Ser Pro
                   965                 970                 975
   Thr Phe Trp Met Gly Ser His Glu Val Asn Gly Thr Trp Met Ile His
               980                 985                 990
   Thr Leu Glu Ala Leu Asp Tyr Lys Glu Cys Glu Trp Pro Leu Thr His
           995                 1000                1005
   Thr Ile Gly Thr Ser Val Glu Glu Ser Glu Met Phe Met Pro Arg Ser
       1010                1015                1020
   Ile Gly Gly Pro Val Ser Ser His Asn His Ile Pro Gly Tyr Lys Val
   1025                1030                1035                1040
   Gln Thr Asn Gly Pro Trp Met Gln Val Pro Leu Glu Val Lys Arg Glu
                   1045                1050                1055
   Ala Cys Pro Gly Thr Ser Val Ile Ile Asp Gly Asn Cys Asp Gly Arg
               1060                1065                1070
   Gly Lys Ser Thr Arg Ser Thr Thr Asp Ser Gly Lys Val Ile Pro Glu
           1075                1080                1085
   Trp Cys Cys Arg Ser Cys Thr Met Pro Pro Val Ser Phe His Gly Ser
       1090                1095                1100
   Asp Gly Cys Trp Tyr Pro Met Glu Ile Arg Pro Arg Lys Thr His Glu
   1105 1110 1115 1120
   Ser His Leu Val Arg Ser Trp Val Thr Ala Gly Glu Ile His Ala Val
   1125 1130 1135
   Pro Phe Gly Leu Val Ser Met Met Ile Ala Met Glu Val Val Leu Arg
   1140 1145 1150
   Lys Arg Gln Gly Pro Lys Gln Met Leu Val Gly Gly Val Val Leu Leu
   1155 1160 1165
   Gly Ala Met Leu Val Gly Gln Val Thr Leu Leu Asp Leu Leu Lys Leu
   1170 1175 1180
   Thr Val Ala Val Gly Leu His Phe His Glu Met Asn Asn Gly Gly Asp
   1185 1190 1195 1200
   Ala Met Tyr Met Ala Leu Ile Ala Ala Phe Ser Ile Arg Pro Gly Leu
   1205 1210 1215
   Leu Ile Gly Phe Gly Leu Arg Thr Leu Trp Ser Pro Arg Glu Arg Leu
   1220 1225 1230
   Val Leu Thr Leu Gly Ala Ala Met Val Glu Ile Ala Leu Gly Gly Val
   1235 1240 1245
   Met Gly Gly Leu Trp Lys Tyr Leu Asn Ala Val Ser Leu Cys Ile Leu
   1250 1255 1260
   Thr Ile Asn Ala Val Ala Ser Arg Lys Ala Ser Asn Thr Ile Leu Pro
   1265 1270 1275 1280
   Leu Met Ala Leu Leu Thr Pro Val Thr Met Ala Glu Val Arg Leu Ala
   1285 1290 1295
   Ala Met Phe Phe Cys Ala Val Val Ile Ile Gly Val Leu His Gln Asn
   1300 1305 1310
   Phe Lys Asp Thr Ser Met Gln Lys Thr Ile Pro Leu Val Ala Leu Thr
   1315 1320 1325
   Leu Thr Ser Tyr Leu Gly Leu Thr Gln Pro Phe Leu Gly Leu Cys Ala
   1330 1335 1340
   Phe Leu Ala Thr Arg Ile Phe Gly Arg Arg Ser Ile Pro Val Asn Glu
   1345 1350 1355 1360
   Ala Leu Ala Ala Ala Gly Leu Val Gly Val Leu Ala Gly Leu Ala Phe
   1365 1370 1375
   Gln Glu Met Glu Asn Phe Leu Gly Pro Ile Ala Val Gly Gly Leu Leu
   1380 1385 1390
   Met Met Leu Val Ser Val Ala Gly Arg Val Asp Gly Leu Glu Leu Lys
   1395 1400 1405
   Lys Leu Gly Glu Val Ser Trp Glu Glu Glu Ala Glu Ile Ser Gly Ser
   1410 1415 1420
   Ser Ala Arg Tyr Asp Val Ala Leu Ser Glu Gln Gly Glu Phe Lys Leu
   1425 1430 1435 1440
   Leu Ser Glu Glu Lys Val Pro Trp Asp Gln Val Val Met Thr Ser Leu
   1445 1450 1455
   Ala Leu Val Gly Ala Ala Leu His Pro Phe Ala Leu Leu Leu Val Leu
   1460 1465 1470
   Ala Gly Trp Leu Phe His Val Arg Gly Ala Arg Arg Ser Gly Asp Val
   1475 1480 1485
   Leu Trp Asp Ile Pro Thr Pro Lys Ile Ile Glu Glu Cys Glu His Leu
   1490 1495 1500
   Glu Asp Gly Ile Tyr Gly Ile Phe Gln Ser Thr Phe Leu Gly Ala Ser
   1505 1510 1515 1520
   Gln Arg Gly Val Gly Val Ala Gln Gly Gly Val Phe His Thr Met Trp
   1525 1530 1535
   His Val Thr Arg Gly Ala Phe Leu Val Arg Asn Gly Lys Lys Leu Ile
   1540 1545 1550
   Pro Ser Trp Ala Ser Val Lys Glu Asp Leu Val Ala Tyr Gly Gly Ser
   1555 1560 1565
   Trp Lys Leu Glu Gly Arg Trp Asp Gly Glu Glu Glu Val Gln Leu Ile
   1570 1575 1580
   Ala Ala Val Pro Gly Lys Asn Val Val Asn Val Gln Thr Lys Pro Ser
   1585 1590 1595 1600
   Leu Phe Lys Val Arg Asn Gly Gly Glu Ile Gly Ala Val Ala Leu Asp
   1605 1610 1615
   Tyr Pro Ser Gly Thr Ser Gly Ser Pro Ile Val Asn Arg Asn Gly Glu
               1620                1625                1630
   Val Ile Gly Leu Tyr Gly Asn Gly Ile Leu Val Gly Asp Asn Ser Phe
           1635                1640                1645
   Val Ser Ala Ile Ser Gln Thr Glu Val Lys Glu Glu Gly Lys Glu Glu
       1650                1655                1660
   Leu Gln Glu Ile Pro Thr Met Leu Lys Lys Gly Met Thr Thr Val Leu
   1665                1670                1675                1680
   Asp Phe His Pro Gly Ala Gly Lys Thr Arg Arg Phe Leu Pro Gln Ile
                   1685                1690                1695
   Leu Ala Glu Cys Ala Arg Arg Arg Leu Arg Thr Leu Val Leu Ala Pro
               1700                1705                1710
   Thr Arg Val Val Leu Ser Glu Met Lys Glu Ala Phe His Gly Leu Asp
           1715                1720                1725
   Val Lys Phe His Thr Gln Ala Phe Ser Ala His Gly Ser Gly Arg Glu
       1730                1735                1740
   Val Ile Asp Ala Met Cys His Ala Thr Leu Thr Tyr Arg Met Leu Glu
   1745                1750                1755                1760
   Pro Thr Arg Val Val Asn Trp Glu Val Ile Ile Met Asp Glu Ala His
                   1765                1770                1775
   Phe Leu Asp Pro Ala Ser Ile Ala Ala Arg Gly Trp Ala Ala His Arg
               1780                1785                1790
   Ala Arg Ala Asn Glu Ser Ala Thr Ile Leu Met Thr Ala Thr Pro Pro
           1795                1800                1805
   Gly Thr Ser Asp Glu Phe Pro His Ser Asn Gly Glu Ile Glu Asp Val
       1810                1815                1820
   Gln Thr Asp Ile Pro Ser Glu Pro Trp Asn Thr Gly His Asp Trp Ile
   1825                1830                1835                1840
   Leu Ala Asp Lys Arg Pro Thr Ala Trp Phe Leu Pro Ser Ile Arg Ala
                   1845                1850                1855
   Ala Asn Val Met Ala Ala Ser Leu Arg Lys Ala Gly Lys Ser Val Val
               1860                1865                1870
   Val Leu Asn Arg Lys Thr Phe Glu Arg Glu Tyr Pro Thr Ile Lys Gln
           1875                1880                1885
   Lys Lys Pro Asp Phe Ile Leu Ala Thr Asp Ile Ala Glu Met Gly Ala
       1890                1895                1900
   Asn Leu Cys Val Glu Arg Val Leu Asp Cys Arg Thr Ala Phe Lys Pro
   1905                1910                1915                1920
   Val Leu Val Asp Glu Gly Arg Lys Val Ala Ile Lys Gly Pro Leu Arg
                   1925                1930                1935
   Ile Ser Ala Ser Ser Ala Ala Gln Arg Arg Gly Arg Ile Gly Arg Asn
               1940                1945                1950
   Pro Asn Arg Asp Gly Asp Ser Tyr Tyr Tyr Ser Glu Pro Thr Ser Glu
           1955                1960                1965
   Asn Asn Ala His His Val Cys Trp Leu Glu Ala Ser Met Leu Leu Asp
       1970                1975                1980
   Asn Met Glu Val Arg Gly Gly Met Val Ala Pro Leu Tyr Gly Val Glu
   1985                1990                1995                2000
   Gly Thr Lys Thr Pro Val Ser Pro Gly Glu Met Arg Leu Arg Asp Asp
                   2005                2010                2015
   Gln Arg Lys Val Phe Arg Glu Leu Val Arg Asn Cys Asp Leu Pro Val
               2020                2025                2030
   Trp Leu Ser Trp Gln Val Ala Lys Ala Gly Leu Lys Thr Asn Asp Arg
           2035                2040                2045
   Lys Trp Cys Phe Glu Gly Pro Glu Glu His Glu Ile Leu Asn Asp Ser
       2050                2055                2060
   Gly Glu Thr Val Lys Cys Arg Ala Pro Gly Gly Ala Lys Lys Pro Leu
   2065                2070                2075                2080
   Arg Pro Arg Trp Cys Asp Glu Arg Val Ser Ser Asp Gln Ser Ala Leu
                   2085                2090                2095
   Ser Glu Phe Ile Lys Phe Ala Glu Gly Arg Arg Gly Ala Ala Glu Val
               2100                2105                2110
   Leu Val Val Leu Ser Glu Leu Pro Asp Phe Leu Ala Lys Lys Gly Gly
           2115                2120                2125
   Glu Ala Met Asp Thr Ile Ser Val Phe Leu His Ser Glu Glu Gly Ser
       2130                2135                2140
   Arg Ala Tyr Arg Asn Ala Leu Ser Met Met Pro Glu Ala Met Thr Ile
   2145 2150 2155 2160
   Val Met Leu Phe Ile Leu Ala Gly Leu Leu Thr Ser Gly Met Val Ile
   2165 2170 2175
   Phe Phe Met Ser Pro Lys Gly Ile Ser Arg Met Ser Met Ala Met Gly
   2180 2185 2190
   Thr Met Ala Gly Cys Gly Tyr Leu Met Phe Leu Gly Gly Val Lys Pro
   2195 2200 2205
   Thr His Ile Ser Tyr Ile Met Leu Ile Phe Phe Val Leu Met Val Val
   2210 2215 2220
   Val Ile Pro Glu Pro Gly Gln Gln Arg Ser Ile Gln Asp Asn Gln Val
   2225 2230 2235 2240
   Ala Tyr Leu Ile Ile Gly Ile Leu Thr Leu Val Ser Ala Val Ala Ala
   2245 2250 2255
   Asn Glu Leu Gly Met Leu Glu Lys Thr Lys Glu Asp Leu Phe Gly Lys
   2260 2265 2270
   Lys Asn Leu Ile Pro Ser Ser Ala Ser Pro Trp Ser Trp Pro Asp Leu
   2275 2280 2285
   Asp Leu Lys Pro Gly Ala Ala Trp Thr Val Tyr Val Gly Ile Val Thr
   2290 2295 2300
   Met Leu Ser Pro Met Leu His His Trp Ile Lys Val Glu Tyr Gly Asn
   2305 2310 2315 2320
   Leu Ser Leu Ser Gly Ile Ala Gln Ser Ala Ser Val Leu Ser Phe Met
   2325 2330 2335
   Asp Lys Gly Ile Pro Phe Met Lys Met Asn Ile Ser Val Ile Met Leu
   2340 2345 2350
   Leu Val Ser Gly Trp Asn Ser Ile Thr Val Met Pro Leu Leu Cys Gly
   2355 2360 2365
   Ile Gly Cys Ala Met Leu His Trp Ser Leu Ile Leu Pro Gly Ile Lys
   2370 2375 2380
   Ala Gln Gln Ser Lys Leu Ala Gln Arg Arg Val Phe His Gly Val Ala
   2385 2390 2395 2400
   Lys Asn Pro Val Val Asp Gly Asn Pro Thr Val Asp Ile Glu Glu Ala
   2405 2410 2415
   Pro Glu Met Pro Ala Leu Tyr Glu Lys Lys Leu Ala Leu Tyr Leu Leu
   2420 2425 2430
   Leu Ala Leu Ser Leu Ala Ser Val Ala Met Cys Arg Thr Pro Phe Ser
   2435 2440 2445
   Leu Ala Glu Gly Ile Val Leu Ala Ser Ala Ala Leu Gly Pro Leu Ile
   2450 2455 2460
   Glu Gly Asn Thr Ser Leu Leu Trp Asn Gly Pro Met Ala Val Ser Met
   2465 2470 2475 2480
   Thr Gly Val Met Arg Gly Asn His Tyr Ala Phe Val Gly Val Met Tyr
   2485 2490 2495
   Asn Leu Trp Lys Met Lys Thr Gly Arg Arg Gly Ser Ala Asn Gly Lys
   2500 2505 2510
   Thr Leu Gly Glu Val Trp Lys Arg Glu Leu Asn Leu Leu Asp Lys Arg
   2515 2520 2525
   Gln Phe Glu Leu Tyr Lys Arg Thr Asp Ile Val Glu Val Asp Arg Asp
   2530 2535 2540
   Thr Ala Arg Arg His Leu Ala Glu Gly Lys Val Asp Thr Gly Val Ala
   2545 2550 2555 2560
   Val Ser Arg Gly Thr Ala Lys Leu Arg Trp Phe His Glu Arg Gly Tyr
   2565 2570 2575
   Val Lys Leu Glu Gly Arg Val Ile Asp Leu Gly Cys Gly Arg Gly Gly
   2580 2585 2590
   Trp Cys Tyr Tyr Ala Ala Ala Gln Lys Glu Val Ser Gly Val Lys Gly
   2595 2600 2605
   Phe Thr Leu Gly Arg Asp Gly His Glu Lys Pro Met Asn Val Gln Ser
   2610 2615 2620
   Leu Gly Trp Asn Ile Ile Thr Phe Lys Asp Lys Thr Asp Ile His Arg
   2625 2630 2635 2640
   Leu Glu Pro Val Lys Cys Asp Thr Leu Leu Cys Asp Ile Gly Glu Ser
   2645 2650 2655
   Ser Ser Ser Ser Val Thr Glu Gly Glu Arg Thr Val Arg Val Leu Asp
                   2660                2665                2670
   Thr Val Glu Lys Trp Leu Ala Cys Gly Val Asp Asn Phe Cys Val Lys
               2675                2680                2685
   Val Leu Ala Pro Tyr Met Pro Asp Val Leu Glu Lys Leu Glu Leu Leu
           2690                2695                2700
   Gln Arg Arg Phe Gly Gly Thr Val Ile Arg Asn Pro Leu Ser Arg Asn
   2705                2710                2715                2720
   Ser Thr His Glu Met Tyr Tyr Val Ser Gly Ala Arg Ser Asn Val Thr
                   2725                2730                2735
   Phe Thr Val Asn Gln Thr Ser Arg Leu Leu Met Arg Arg Met Arg Arg
               2740                2745                2750
   Pro Thr Gly Lys Val Thr Leu Glu Ala Asp Val Ile Leu Pro Ile Gly
               2755                2760                2765
   Thr Arg Ser Val Glu Thr Asp Lys Gly Pro Leu Asp Lys Glu Ala Ile
           2770                2775                2780
   Glu Glu Arg Val Glu Arg Ile Lys Ser Glu Tyr Met Thr Ser Trp Phe
   2785                2790                2795                2800
   Tyr Asp Asn Asp Asn Pro Tyr Arg Thr Trp His Tyr Cys Gly Ser Tyr
                   2805                2810                2815
   Val Thr Lys Thr Ser Gly Ser Ala Ala Ser Met Val Asn Gly Val Ile
               2820                2825                2830
   Lys Ile Leu Thr Tyr Pro Trp Asp Arg Ile Glu Glu Val Thr Arg Met
           2835                2840                2845
   Ala Met Thr Asp Thr Thr Pro Phe Gly Gln Gln Arg Val Phe Lys Glu
       2850                2855                2860
   Lys Val Asp Thr Arg Ala Lys Asp Pro Pro Ala Gly Thr Arg Lys Ile
   2865                2870                2875                2880
   Met Lys Val Val Asn Arg Trp Leu Phe Arg His Leu Ala Arg Glu Lys
                   2885                2890                2895
   Asn Pro Arg Leu Cys Thr Lys Glu Glu Phe Ile Ala Lys Val Arg Ser
               2900                2905                2910
   His Ala Ala Ile Gly Ala Tyr Leu Glu Glu Gln Glu Gln Trp Lys Thr
           2915                2920                2925
   Ala Asn Glu Ala Val Gln Asp Pro Lys Phe Trp Glu Leu Val Asp Glu
       2930                2935                2940
   Glu Arg Lys Leu His Gln Gln Gly Arg Cys Arg Thr Cys Val Tyr Asn
   2945                2950                2955                2960
   Met Met Gly Lys Arg Glu Lys Lys Leu Ser Glu Phe Gly Lys Ala Lys
                   2965                2970                2975
   Gly Ser Arg Ala Ile Trp Tyr Met Trp Leu Gly Ala Arg Tyr Leu Glu
               2980                2985                2990
   Phe Glu Ala Leu Gly Phe Leu Asn Glu Asp His Trp Ala Ser Arg Glu
           2995                3000                3005
   Asn Ser Gly Gly Gly Val Glu Gly Ile Gly Leu Gln Tyr Leu Gly Tyr
       3010                3015                3020
   Val Ile Arg Asp Leu Ala Ala Met Asp Gly Gly Gly Phe Tyr Ala Asp
   3025                3030                3035                3040
   Asp Thr Ala Gly Trp Asp Thr Arg Ile Thr Glu Ala Asp Leu Asp Asp
                   3045                3050                3055
   Glu Gln Glu Ile Leu Asn Tyr Met Ser Pro His His Lys Lys Leu Ala
               3060                3065                3070
   Gln Ala Val Met Glu Met Thr Tyr Lys Asn Lys Val Val Lys Val Leu
           3075                3080                3085
   Arg Pro Ala Pro Gly Gly Lys Ala Tyr Met Asp Val Ile Ser Arg Arg
       3090                3095                3100
   Asp Gln Arg Gly Ser Gly Gln Val Val Thr Tyr Ala Leu Asn Thr Ile
   3105                3110                3115                3120
   Thr Asn Leu Lys Val Gln Leu Ile Arg Met Ala Glu Ala Glu Met Val
                   3125                3130                3135
   Ile His His Gln His Val Gln Asp Cys Asp Glu Ser Val Leu Thr Arg
               3140                3145                3150
   Leu Glu Ala Trp Leu Thr Glu His Gly Cys Asn Arg Leu Lys Arg Met
           3155                3160                3165
   Ala Val Ser Gly Asp Asp Cys Val Val Arg Pro Ile Asp Asp Arg Phe
       3170                3175                3180
   Gly Leu Ala Leu Ser His Leu Asn Ala Met Ser Lys Val Arg Lys Asp
   3185                3190                3195                3200
   Ile Ser Glu Trp Gln Pro Ser Lys Gly Trp Asn Asp Trp Glu Asn Val
                   3205                3210                3215
   Pro Phe Cys Ser His His Phe His Glu Leu Gln Leu Lys Asp Gly Arg
               3220                3225                3230
   Arg Ile Val Val Pro Cys Arg Glu Gln Asp Glu Leu Ile Gly Arg Gly
           3235                3240                3245
   Arg Val Ser Pro Gly Asn Gly Trp Met Ile Lys Glu Thr Ala Cys Leu
       3250                3255                3260
   Ser Lys Ala Tyr Ala Asn Met Trp Ser Leu Met Tyr Phe His Lys Arg
   3265                3270                3275                3280
   Asp Met Arg Leu Leu Ser Leu Ala Val Ser Ser Ala Val Pro Thr Ser
                   3285                3290                3295
   Trp Val Pro Gln Gly Arg Thr Thr Trp Ser Ile His Gly Lys Gly Glu
               3300                3305                3310
   Trp Met Thr Thr Glu Asp Met Leu Glu Val Trp Asn Arg Val Trp Ile
           3315                3320                3325
   Thr Asn Asn Pro His Met Gln Asp Lys Thr Met Val Lys Lys Trp Arg
       3330                3335                3340
   Asp Val Pro Tyr Leu Thr Lys Arg Gln Asp Lys Leu Cys Gly Ser Leu
   3345                3350                3355                3360
   Ile Gly Met Thr Asn Arg Ala Thr Trp Ala Ser His Ile His Leu Val
                   3365                3370                3375
   Ile His Arg Ile Arg Thr Leu Ile Gly Gln Glu Lys Tyr Thr Asp Tyr
               3380                3385                3390
   Leu Thr Val Met Asp Arg Tyr Ser Val Asp Ala Asp Leu Gln Leu Gly
           3395                3400                3405
   Glu Leu Ile
       3410
<210> 15
   <211> 10862
   <212> DNA
   <213> Flaviviridae Flavivirus Yellow Fever Virus
<400> 15
   agtaaatcct gtgtgctaat tgaggtgcat tggtctgcaa atcgagttgc taggcaataa 60
   acacatttgg attaatttta atcgttcgtt gagcgattag cagagaactg accagaacat 120
   gtctggtcgt aaagctcagg gaaaaaccct gggcgtcaat atggtacgac gaggagttcg 180
   ctccttgtca aacaaaataa aacaaaaaac aaaacaaatt ggaaacagac ctggaccttc 240
   aagaggtgtt caaggattta tctttttctt tttgttcaac attttgactg gaaaaaagat 300
   cacagcccac ctaaagaggt tgtggaaaat gctggaccca agacaaggct tggctgttct 360
   aaggaaagtc aagagagtgg tggccagttt gatgagagga ttgtcctcaa ggaaacgccg 420
   ttcccatgat gttctgactg tgcaattcct aattttggga atgctgttga tgacgggtgg 480
   agtgaccttg gtgcggaaaa acagatggtt gctcctaaat gtgacatctg aggacctcgg 540
   gaaaacattc tctgtgggca caggcaactg cacaacaaac attttggaag ccaagtactg 600
   gtgcccagac tcaatggaat acaactgtcc caatctcagt ccaagagagg agccagatga 660
   cattgattgc tggtgctatg gggtggaaaa cgttagagtc gcatatggta agtgtgactc 720
   agcaggcagg tctaggaggt caagaagggc cattgacttg cctacgcatg aaaaccatgg 780
   tttgaagacc cggcaagaaa aatggatgac tggaagaatg ggtgaaaggc aactccaaaa 840
   gattgagaga tggttcgtga ggaacccctt ttttgcagtg acggctctga ccattgccta 900
   ccttgtggga agcaacatga cgcaacgagt cgtgattgcc ctactggtct tggctgttgg 960
   tccggcctac tcagctcact gcattggaat tactgacagg gatttcattg agggggtgca 1020
   tggaggaact tgggtttcag ctaccctgga gcaagacaag tgtgtcactg ttatggcccc 1080
   tgacaagcct tcattggaca tctcactaga gacagtagcc attgatagac ctgctgaggt 1140
   gaggaaagtg tgttacaatg cagttctcac tcatgtgaag attaatgaca agtgccccag 1200
   cactggagag gcccacctag ctgaagagaa cgaaggggac aatgcgtgca agcgcactta 1260
   ttctgataga ggctggggca atggctgtgg cctatttggg aaagggagca ttgtggcatg 1320
   cgccaaattc acttgtgcca aatccatgag tttgtttgag gttgatcaga ccaaaattca 1380
   gtatgtcatc agagcacaat tgcatgtagg ggccaagcag gaaaattgga ctaccgacat 1440
   taagactctc aagtttgatg ccctgtcagg ctcccaggaa gtcgagttca ttgggtatgg 1500
   aaaagctaca ctggaatgcc aggtgcaaac tgcggtggac tttggtaaca gttacatcgc 1560
   tgagatggaa acagagagct ggatagtgga cagacagtgg gcccaggact tgaccctgcc 1620
   atggcagagt ggaagtggcg gggtgtggag agagatgcat catcttgtcg aatttgaacc 1680
   tccgcatgcc gccactatca gagtactggc cctgggaaac caggaaggct ccttgaaaac 1740
   agctcttact ggcgcaatga gggttacaaa ggacacaaat gacaacaacc tttacaaact 1800
   acatggtgga catgtttctt gcagagtgaa attgtcagct ttgacactca aggggacatc 1860
   ctacaaaata tgcactgaca aaatgttttt tgtcaagaac ccaactgaca ctggccatgg 1920
   cactgttgtg atgcaggtga aagtgtcaaa aggagccccc tgcaggattc cagtgatagt 1980
   agctgatgat cttacagcgg caatcaataa aggcattttg gttacagtta accccatcgc 2040
   ctcaaccaat gatgatgaag tgctgattga ggtgaaccca ccttttggag acagctacat 2100
   tatcgttggg agaggagatt cacgtctcac ttaccagtgg cacaaagagg gaagctcaat 2160
   aggaaagttg ttcactcaga ccatgaaagg cgtggaacgc ctggccgtca tgggagacac 2220
   cgcctgggat ttcagctccg ctggagggtt cttcacttcg gttgggaaag gaattcatac 2280
   ggtgtttggc tctgcctttc aggggctatt tggcggcttg aactggataa caaaggtcat 2340
   catgggggcg gtacttatat gggttggcat caacacaaga aacatgacaa tgtccatgag 2400
   catgatcttg gtaggagtga tcatgatgtt tttgtctcta ggagttgggg cggatcaagg 2460
   atgcgccatc aactttggca agagagagct caagtgcgga gatggtatct tcatatttag 2520
   agactctgat gactggctga acaagtactc atactatcca gaagatcctg tgaagcttgc 2580
   atcaatagtg aaagcctctt ttgaagaagg gaagtgtggc ctaaattcag ttgactccct 2640
   tgagcatgag atgtggagaa gcagggcaga tgagatcaat gccatttttg aggaaaacga 2700
   ggtggacatt tctgttgtcg tgcaggatcc aaagaatgtt taccagagag gaactcatcc 2760
   attttccaga attcgggatg gtctgcagta tggttggaag acttggggta agaaccttgt 2820
   gttctcccca gggaggaaga atggaagctt catcatagat ggaaagtcca ggaaagaatg 2880
   cccgttttca aaccgggtct ggaattcttt ccagatagag gagtttggga cgggagtgtt 2940
   caccacacgc gtgtacatgg acgcagtctt tgaatacacc atagactgcg atggatctat 3000
   cttgggtgca gcggtgaacg gaaaaaagag tgcccatggc tctccaacat tttggatggg 3060
   aagtcatgaa gtaaatggga catggatgat ccacaccttg gaggcattag attacaagga 3120
   gtgtgagtgg ccactgacac atacgattgg aacatcagtt gaagagagtg aaatgttcat 3180
   gccgagatca atcggaggcc cagttagctc tcacaatcat atccctggat acaaggttca 3240
   gacgaacgga ccttggatgc aggtaccact agaagtgaag agagaagctt gcccagggac 3300
   tagcgtgatc attgatggca actgtgatgg acggggaaaa tcaaccagat ccaccacgga 3360
   tagcgggaaa gttattcctg aatggtgttg ccgctcctgc acaatgccgc ctgtgagctt 3420
   ccatggtagt gatgggtgtt ggtatcccat ggaaattagg ccaaggaaaa cgcatgaaag 3480
   ccatctggtg cgctcctggg ttacagctgg agaaatacat gctgtccctt ttggtttggt 3540
   gagcatgatg atagcaatgg aagtggtcct aaggaaaaga cagggaccaa agcaaatgtt 3600
   ggttggagga gtagtgctct tgggagcaat gctggtcggg caagtaactc tccttgattt 3660
   gctgaaactc acagtggctg tgggattgca tttccatgag atgaacaatg gaggagacgc 3720
   catgtatatg gcgttgattg ctgccttttc aatcagacca gggctgctca tcggctttgg 3780
   gctcaggacc ctatggagcc ctcgggaacg ccttgtgctg accctaggag cagccatggt 3840
   ggagattgcc ttgggtggcg tgatgggcgg cctgtggaag tatctaaatg cagtttctct 3900
   ctgcatcctg acaataaatg ctgttgcttc taggaaagca tcaaatacca tcttgcccct 3960
   catggctctg ttgacacctg tcactatggc tgaggtgaga cttgccgcaa tgttcttttg 4020
   tgccgtggtt atcatagggg tccttcacca gaatttcaag gacacctcca tgcagaagac 4080
   tatacctctg gtggccctca cactcacatc ttacctgggc ttgacacaac cttttttggg 4140
   cctgtgtgca tttctggcaa cccgcatatt tgggcgaagg agtatcccag tgaatgaggc 4200
   actcgcagca gctggtctag tgggagtgct ggcaggactg gcttttcagg agatggagaa 4260
   cttccttggt ccgattgcag ttggaggact cctgatgatg ctggttagcg tggctgggag 4320
   ggtggatggg ctagagctca agaagcttgg tgaagtttca tgggaagagg aggcggagat 4380
   cagcgggagt tccgcccgct atgatgtggc actcagtgaa caaggggagt tcaagctgct 4440
   ttctgaagag aaagtgccat gggaccaggt tgtgatgacc tcgctggcct tggttggggc 4500
   tgccctccat ccatttgctc ttctgctggt ccttgctggg tggctgtttc atgtcagggg 4560
   agctaggaga agtggggatg tcttgtggga tattcccact cctaagatca tcgaggaatg 4620
   tgaacatctg gaggatggga tttatggcat attccagtca accttcttgg gggcctccca 4680
   gcgaggagtg ggagtggcac agggaggggt gttccacaca atgtggcatg tcacaagagg 4740
   agctttcctt gtcaggaatg gcaagaagtt gattccatct tgggcttcag taaaggaaga 4800
   ccttgtcgcc tatggtggct catggaagtt ggaaggcaga tgggatggag aggaagaggt 4860
   ccagttgatc gcggctgttc caggaaagaa cgtggtcaac gtccagacaa aaccgagctt 4920
   gttcaaagtg aggaatgggg gagaaatcgg ggctgtcgct cttgactatc cgagtggcac 4980
   ttcaggatct cctattgtta acaggaacgg agaggtgatt gggctgtacg gcaatggcat 5040
   ccttgtcggt gacaactcct tcgtgtccgc catatcccag actgaggtga aggaagaagg 5100
   aaaggaggag ctccaagaga tcccgacaat gctaaagaaa ggaatgacaa ctgtccttga 5160
   ttttcatcct ggagctggga agacaagacg tttcctccca cagatcttgg ccgagtgcgc 5220
   acggagacgc ttgcgcactc ttgtgttggc ccccaccagg gttgttcttt ctgaaatgaa 5280
   ggaggctttt cacggcctgg acgtgaaatt ccacacacag gctttttccg ctcacggcag 5340
   cgggagagaa gtcattgatg ctatgtgcca tgccacccta acttacagga tgttggaacc 5400
   aactagggtt gttaactggg aagtgatcat tatggatgaa gcccattttt tggatccagc 5460
   tagcatagcc gctagaggtt gggcagcgca cagagctagg gcaaatgaaa gtgcaacaat 5520
   cttgatgaca gccacaccgc ctgggactag tgatgaattt ccacattcaa atggtgaaat 5580
   agaagatgtt caaacggaca tacccagtga gccctggaac acagggcatg actggatcct 5640
   ggctgacaaa aggcccacgg catggttcct tccatccatc agagctgcaa atgtcatggc 5700
   tgcctctttg cgtaaggctg gaaagagtgt ggtggtcctg aacaggaaaa cctttgagag 5760
   agaatacccc acgataaagc agaagaaacc tgactttata ttggccactg acatagctga 5820
   aatgggagcc aacctttgcg tggagcgagt gctggattgc aggacggctt ttaagcctgt 5880
   gcttgtggat gaagggagga aggtggcaat aaaagggcca cttcgtatct ccgcatcctc 5940
   tgctgctcaa aggagggggc gcattgggag aaatcccaac agagatggag actcatacta 6000
   ctattctgag cctacaagtg aaaataatgc ccaccacgtc tgctggttgg aggcctcaat 6060
   gctcttggac aacatggagg tgaggggtgg aatggtcgcc ccactctatg gcgttgaagg 6120
   aactaaaaca ccagtttccc ctggtgaaat gagactgagg gatgaccaga ggaaagtctt 6180
   cagagaacta gtgaggaatt gtgacctgcc cgtttggctt tcgtggcaag tggccaaggc 6240
   tggtttgaag acgaatgatc gtaagtggtg ttttgaaggc cctgaggaac atgagatctt 6300
   gaatgacagc ggtgaaacag tgaagtgcag ggctcctgga ggagcaaaga agcctctgcg 6360
   cccaaggtgg tgtgatgaaa gggtgtcatc tgaccagagt gcgctgtctg aatttattaa 6420
   gtttgctgaa ggtaggaggg gagctgctga agtgctagtt gtgctgagtg aactccctga 6480
   tttcctggct aaaaaaggtg gagaggcaat ggataccatc agtgtgtttc tccactctga 6540
   ggaaggctct agggcttacc gcaatgcact atcaatgatg cctgaggcaa tgacaatagt 6600
   catgctgttt atactggctg gactactgac atcgggaatg gtcatctttt tcatgtctcc 6660
   caaaggcatc agtagaatgt ctatggcgat gggcacaatg gccggctgtg gatatctcat 6720
   gttccttgga ggcgtcaaac ccactcacat ctcctatatc atgctcatat tctttgtcct 6780
   gatggtggtt gtgatccccg agccagggca acaaaggtcc atccaagaca accaagtggc 6840
   atacctcatt attggcatcc tgacgctggt ttcagcggtg gcagccaacg agctaggcat 6900
   gctggagaaa accaaagagg acctctttgg gaagaagaac ttaattccat ctagtgcttc 6960
   accctggagt tggccggatc ttgacctgaa gccaggagct gcctggacag tgtacgttgg 7020
   cattgttaca atgctctctc caatgttgca ccactggatc aaagtcgaat atggcaacct 7080
   gtctctgtct ggaatagccc agtcagcctc agtcctttct ttcatggaca aggggatacc 7140
   attcatgaag atgaatatct cggtcataat gctgctggtc agtggctgga attcaataac 7200
   agtgatgcct ctgctctgtg gcatagggtg cgccatgctc cactggtctc tcattttacc 7260
   tggaatcaaa gcgcagcagt caaagcttgc acagagaagg gtgttccatg gcgttgccaa 7320
   gaaccctgtg gttgatggga atccaacagt tgacattgag gaagctcctg aaatgcctgc 7380
   cctttatgag aagaaactgg ctctatatct ccttcttgct ctcagcctag cttctgttgc 7440
   catgtgcaga acgccctttt cattggctga aggcattgtc ctagcatcag ctgccctagg 7500
   gccgctcata gagggaaaca ccagccttct ttggaatgga cccatggctg tctccatgac 7560
   aggagtcatg agggggaatc actatgcttt tgtgggagtc atgtacaatc tatggaagat 7620
   gaaaactgga cgccggggga gcgcgaatgg aaaaactttg ggtgaagtct ggaagaggga 7680
   actgaatctg ttggacaagc gacagtttga gttgtataaa aggaccgaca ttgtggaggt 7740
   ggatcgtgat acggcacgca ggcatttggc cgaagggaag gtggacaccg gggtggcggt 7800
   ctccaggggg accgcaaagt taaggtggtt ccatgagcgt ggctatgtca agctggaagg 7860
   tagggtgatt gacctggggt gtggccgcgg aggctggtgt tactacgctg ctgcgcaaaa 7920
   ggaagtgagt ggggtcaaag gatttactct tggaagagac ggccatgaga aacccatgaa 7980
   tgtgcaaagt ctgggatgga acatcatcac cttcaaggac aaaactgata tccaccgcct 8040
   agaaccagtg aaatgtgaca cccttttgtg tgacattgga gagtcatcat cgtcatcggt 8100
   cacagagggg gaaaggaccg tgagagttct tgatactgta gaaaaatggc tggcttgtgg 8160
   ggttgacaac ttctgtgtga aggtgttagc tccatacatg ccagatgttc tcgagaaact 8220
   ggaattgctc caaaggaggt ttggcggaac agtgatcagg aaccctctct ccaggaattc 8280
   cactcatgaa atgtactacg tgtctggagc ccgcagcaat gtcacattta ctgtgaacca 8340
   aacatcccgc ctcctgatga ggagaatgag gcgtccaact ggaaaagtga ccctggaggc 8400
   tgacgtcatc ctcccaattg ggacacgcag tgttgagaca gacaagggac ccctggacaa 8460
   agaggccata gaagaaaggg ttgagaggat aaaatctgag tacatgacct cttggtttta 8520
   tgacaatgac aacccctaca ggacctggca ctactgtggc tcctatgtca caaaaacctc 8580
   aggaagtgcg gcgagcatgg taaatggtgt tattaaaatt ctgacatatc catgggacag 8640
   gatagaggag gtcacaagaa tggcaatgac tgacacaacc ccttttggac agcaaagagt 8700
   gtttaaagaa aaagttgaca ccagagcaaa ggatccacca gcgggaacta ggaagatcat 8760
   gaaagttgtc aacaggtggc tgttccgcca cctggccaga gaaaagaacc ccagactgtg 8820
   cacaaaggaa gaatttattg caaaagtccg aagtcatgca gccattggag cttacctgga 8880
   agaacaagaa cagtggaaga ctgccaatga ggctgtccaa gacccaaagt tctgggaact 8940
   ggtggatgaa gaaaggaagc tgcaccaaca aggcaggtgt cggacttgtg tgtacaacat 9000
   gatggggaaa agagagaaga agctgtcaga gtttgggaaa gcaaagggaa gccgtgccat 9060
   atggtatatg tggctgggag cgcggtatct tgagtttgag gccctgggat tcctgaatga 9120
   ggaccattgg gcttccaggg aaaactcagg aggaggagtg gaaggcattg gcttacaata 9180
   cctaggatat gtgatcagag acctggctgc aatggatggt ggtggattct acgcggatga 9240
   caccgctgga tgggacacgc gcatcacaga ggcagacctt gatgatgaac aggagatctt 9300
   gaactacatg agcccacatc acaaaaaact ggcacaagca gtgatggaaa tgacatacaa 9360
   gaacaaagtg gtgaaagtgt tgagaccagc cccaggaggg aaagcctaca tggatgtcat 9420
   aagtcgacga gaccagagag gatccgggca ggtagtgact tatgctctga acaccatcac 9480
   caacttgaaa gtccaattga tcagaatggc agaagcagag atggtgatac atcaccaaca 9540
   tgttcaagat tgtgatgaat cagttctgac caggctggag gcatggctca ctgagcacgg 9600
   atgtaacaga ctgaagagga tggcggtgag tggagacgac tgtgtggtcc ggcccatcga 9660
   tgacaggttc ggcctggccc tgtcccatct caacgccatg tccaaggtta gaaaggacat 9720
   atctgaatgg cagccatcaa aagggtggaa tgattgggag aatgtgccct tctgttccca 9780
   ccacttccat gaactacagc tgaaggatgg caggaggatt gtggtgcctt gccgagaaca 9840
   ggacgagctc attgggagag gaagggtgtc tccaggaaac ggctggatga tcaaggaaac 9900
   agcttgcctc agcaaagcct atgccaacat gtggtcactg atgtattttc acaaaaggga 9960
   catgaggcta ctgtcattgg ctgtttcctc agctgttccc acctcatggg ttccacaagg 10020
   acgcacaaca tggtcgattc atgggaaagg ggagtggatg accacggaag acatgcttga 10080
   ggtgtggaac agagtatgga taaccaacaa cccacacatg caggacaaga caatggtgaa 10140
   aaaatggaga gatgtccctt atctaaccaa gagacaagac aagctgtgcg gatcactgat 10200
   tggaatgacc aataggccca cctgggcctc ccacatccat ttggtcatcc atcgtatccg 10260
   aacgctgatt ggacaggaga aatacactga ctacctaaca gtcatggaca ggtattctgt 10320
   ggatgctgac ctgcaactgg gtgagcttat ctgaaacacc atctaacagg aataaccggg 10380
   atacaaacca cgggtggaga accggactcc ccacaacctg aaaccgggat ataaaccacg 10440
   gctggagaac cggactccgc acttaaaatg aaacagaaac cgggataaaa actacggatg 10500
   gagaaccgga ctccacacat tgagacagaa gaagttgtca gcccagaacc ccacacgagt 10560
   tttgccactg ctaagctgtg aggcagtgca ggctgggaca gccgacctcc aggttgcgaa 10620
   aaacctggtt tctgggacct cccaccccag agtaaaaaga acggagcctc cgctaccacc 10680
   ctcccacgtg gtggtagaaa gacggggtct agaggttaga ggagaccctc cagggaacaa 10740
   atagtgggac catattgacg ccagggaaag accggagtgg ttctctgctt ttcctccaga 10800
   ggtctgtgag cacagtttgc tcaagaataa gcagaccttt ggatgacaaa cacaaaacca 10860
   ct                                                                10862

## Description

### BACKGROUND OF THE INVENTION

The Yellow Fever virus is endemic, that is, continuously present with low levels of infection in some tropical areas of Africa and the Americas, where it regularly amplifies into epidemics. Other parts of the world, including coastal regions of South America, the Caribbean islands, and Central and North America, are infested with the mosquito vector capable of transmitting the virus and are therefore considered at risk for yellow fever epidemics (World Health Organization Fact Sheet No. 100, revised December, 2001).

For example, in Africa alone, thirty-three countries with a combined population of 508 million, are at risk (*Id*.). Each year, the World Health Organization (WHO) estimates there are 200,000 cases of yellow fever, with 30,000 deaths (*Id*.). Travel to these tropical regions also is believed to result in a small number of imported cases in countries generally free of yellow fever. Although yellow fever cases have not been reported in Asia, "this region is at risk because the appropriate primates and mosquitoes are present" (*Id*.).

The Yellow Fever (YF) virus is in the genus *Flavivirus*, in the family Flaviviridae. In the so-called "jungle" or "sylvan cycle", the YF virus is enzootic, maintained and transmitted by canopy breeding mosquitoes to monkeys in the rainforests. The "urban cycle" begins when humans become infected by entering the rainforests and are bitten by YF-infected mosquitoes. The "urban cycle" continues with peridomestic transmission from humans to mosquitoes and thence to other humans, and can result in yellow fever epidemics in villages and cities. Illness ranges in severity from a self-limited febrile illness to severe hepatitis and fatal hemorrhagic disease.

Unvaccinated humans, including both native people and travelers to YF endemic areas are at significant risk of YF infection when occupational and other activities bring them in contact with infected mosquitoes in the sylvan cycle or the urban cycle.

Patients with yellow fever may be viremic, i.e., have virus in their blood, for 3 to 6 days during the early phase of illness. This phase may be followed by a short period of symptom remission.

The toxic phase develops as the fever returns, with clinical symptoms including, for example, high fever and nausea, hemorrhagic symptoms, including hematemesis (black vomit), epistaxis (nose bleed), gum bleeding, and petechial and purpuric hemorrhages (bruising). Deepening jaundice and proteinuria frequently occur in severe cases.

In the late stages of disease, patients can develop hypotension, shock, metabolic acidosis, acute tubular necrosis, myocardial dysfunction, and cardiac arrhythmia. Confusion, seizures, and coma can also occur, as well as complications such as secondary bacterial infections and kidney failure.

There is no specific treatment for yellow fever. Steps to prevent yellow fever include use of insect repellent, protective clothing, and vaccination with the available, but risky attenuated vaccine.

Live, attenuated vaccines produced from the 17D substrain, are available, but adverse events associated with the attenuated vaccine can lead to a severe infection with the live 17D virus, and serious and fatal adverse neurotropic and viscerotropic events, the latter resembling the severe infection by the wild-type YF virus. Thus there is a need for a safer, inactivated, non-replicating vaccine that will elicit a neutralizing antibody response while eliminating the potential for neurotropic and viscerotropic adverse events.

Thus, there is an on-going need for an effective, inactivated, "killed" or non-replicating vaccine in order to avoid the potential for neurotropic and viscerotropic adverse events associated with the currently available attenuated YF 17D vaccine. Further, there is a need for an improved vaccine produced in Vero cells without animal-derived proteins, a vaccine that can be safely used for persons for whom the live vaccine is contraindicated or for whom warnings appear on the label. Such individuals include immuno-suppressed persons, persons with thymic disease, egg-allergic, young infants, and the elderly.

A problem with any potential inactivated virus is that it may need to be delivered at a higher titer than the existing live attenuated vaccines, because the latter can expand antigenic mass during cycles of replication in the host whereas an inactivated vaccine contains a fixed dose of antigen. Therefore, in order to develop a sufficiently potent inactivated vaccine, it is desirable to modify the YF virus in order to produce a high yield of virus in the conditioned medium (also called the supernatant fluid) of a cell culture. It is highly desirable to use the attenuated 17D vaccine strain for vaccine manufacturing, since the 17D strain can be manipulated at a lower level of biocontainment than the wild-type virulent YF virus. However, the attenuated 17D vaccine strain yields in cell culture are inherently lower than yields of wild-type virus. For these reasons, modifications of the 17D vaccine strain to achieve higher yields in cell culture used for vaccine production would be useful.

Galller, R. et al. (1997) disclosed modified YF 17D strains with amino acid mutations in the envelope protein, NS1 and/or NS3 protein and discuss their usefulness for the development as vaccine.

### BRIEF SUMMARY OF THE INVENTION

The Yellow Fever virus is the prototype species in the genus *Flavivirus*, in the family *Flaviviridae*. Structural and functional studies of the E protein of tick-borne encephalitis (TBE) virus, a fast-growing, virulent member of the flavivirus genus, indicate that Domains I and II in the E protein of TBE participate in an acidic pH-dependent conformational change that facilitates flavivirus membrane fusion with the host and subsequent infectivity. The junction of Domains I and II function as a 'molecular hinge' resulting in a major rearrangement of these domains from of the normal dimeric structure of the E protein at acid pH into a homotrimeric state. Rey FA et al. The envelope glycoprotein from tick-borne encephalitis virus at 2 Å resolution. Nature 375: 291-298 (1995); Heinz FX et al. Structural changes and functional control of the tick-borne encephalitis virus glycoprotein E by the heterodimeric association with protein prM. Virology 198: 109-117 (1994); Mandl CW et al. Antigenic structure of the flavivirus envelope protein E at the molecular level, using tick-borne encephalitis virus as a model. Journal of Virology 63(2): 564-571 (1989); Harrison SC. Viral membrane fusion. Nature structural and molecular biology 15(7): 690-698 (2008); Stiasny K et al. Molecular mechanisms of flavivirus membrane fusion. Amino acids DOI 10.1007/s00726-009-0370-4, published on line 01 November 2009.

Lys 160 in the E protein of Yellow Fever virus is located in the molecular hinge region between Domains I and II. Mutations in this region could alter the acid-dependent conformational change in region Domain I of the E protein required for fusion and virus internalization into the cell cytoplasm. Without being bound by theory, higher yields seen with the lysine to arginine change at amino acid 160 in Domain I of the E protein of the adapted Yellow Fever virus strain may be due to an increased affinity for protons that arginine provides as compared with lysine, that results in enhanced membrane fusion with the host and more efficient infectivity. In regard to the invention, it is important to note that the side chains of lysine and arginine have pKa values of 10.53 and 12.48, respectively, indicating a one hundred fold greater affinity for protons in arginine than in lysine. The increased affinity for protons that the side chain of arginine shows relative to lysine's side chain may enhance the rate and efficiency of E protein conformational change at the molecular hinge, membrane fusion, and flavivirus infectivity, resulting in higher yields of virus in the adapted virus strain.

Other members within the genus *Flavivirus* include West Nile, dengue, and Japanese encephalitis viruses. The non-structural proteins found in West Nile Virus are known to be directly or indirectly involved in viral RNA synthesis. Amino acid substitutions in the non-structural proteins of these viruses have been shown to affect the yields of mutant viruses grown in Vero cells. For example, a proline to leucine substitution at amino acid 250 in the NS1 protein of the flavivirus Kunjin, a West Nile Virus subtype, grows at 100-fold lower titers than wild-type virus. Similarly, mutation of the C-terminal sites in the NS2A protein of yellow fever virus was shown to be lethal for virus replication. Brinton MA. The molecular biology of west nile virus: a new invader of the western hemisphere. Annual Review of Microbiology 56: 371-402 (2002).

In a first aspect, the disclosure provides a nucleic acid molecule comprising a sequence encoding a modified non-structural protein 4B of the Yellow Fever virus, wherein said nucleic acid molecule comprises a nucleotide mutation in the codon corresponding to the amino acid at position 113 of the non-structural protein 4B of SEQ ID NO: 14 resulting in a change from AUA to AUG.

In a second aspect, the disclosure provides a nucleic acid molecule of SEQ ID NO 11 comprising a sequence encoding an envelope protein, an NS1 non-structural protein, an NS2A non-structural protein, of the Yellow Fever virus, wherein said proteins comprise an amino acid mutation at position 160 of the envelope protein, at position 317 of the NS 1 protein, at position 170 of the NS2A protein.

### BRIEF DESCRIPTION OF THE DRAWINGS

FIG. 1 is a schematic representation of the passage history of Vero cells during the manufacture of the disclosed yellow fever vaccine.
FIG. 2 is a schematic representation of the preparation of the virus seeds.
FIG. 3A is a schematic of the process used for 10 serial passages (PI through P10) to modify the nucleotide sequence of the viral genome virus to develop a seed virus with enhanced growth in Vero cells for preparation of an inactivated Yellow Fever virus candidate.
FIG. 3B is a graphical representation of the virus replication for passage one (P1) and passage 11 (P11) of the initial experiment, in which P11 virus differs from P1 by a single mutation at E160 (lys→arg)
FIG. 3C is a graphical representation of a repeat passaging study of passage one (Bp1, Cp1) and passage 11 (B-p11, C-p11) virus performed in a series of experiments: Series B and C.
FIG. 4A-L depicts the consensus alignment of the P1 and P11 nucleic acid sequences. The starting nucleic acid sequence, P1, is identified herein as SEQ ID NO: 1. A comparison of the P1 passage and the P11 passage revealed a genetic mutation at nucleotide residue #211 of SEQ ID NO: 1, and a second mutation at nucleotide residue #1452 of SEQ ID NO: 1. Thus, "P1 consensus" corresponds to SEQ ID NO.1; "P11 consensus" corresponds to SEQ ID NO.2 having the codon mutation at envelope protein amino acid position 160.

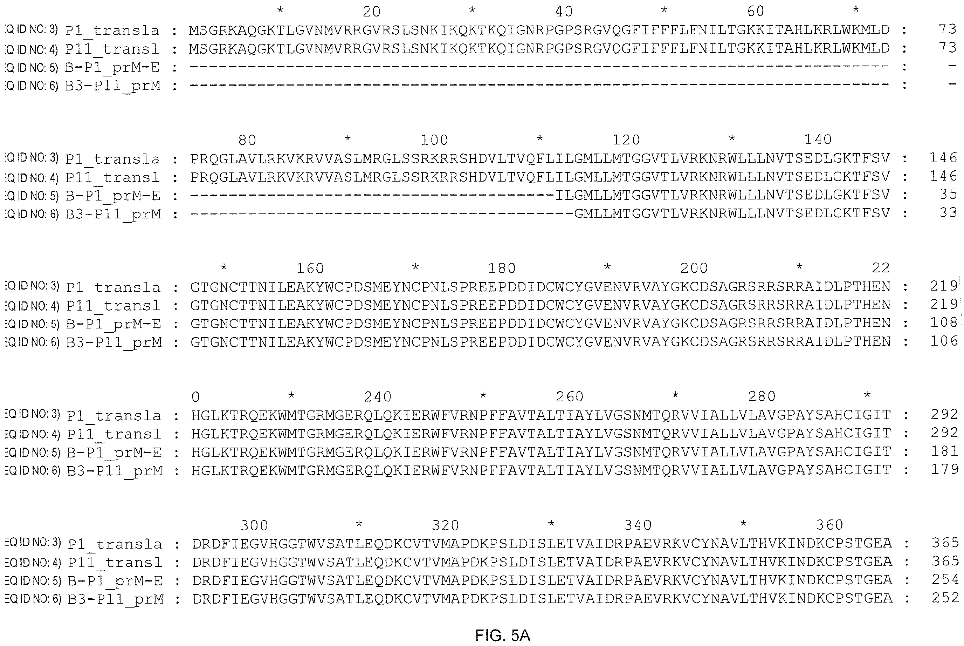

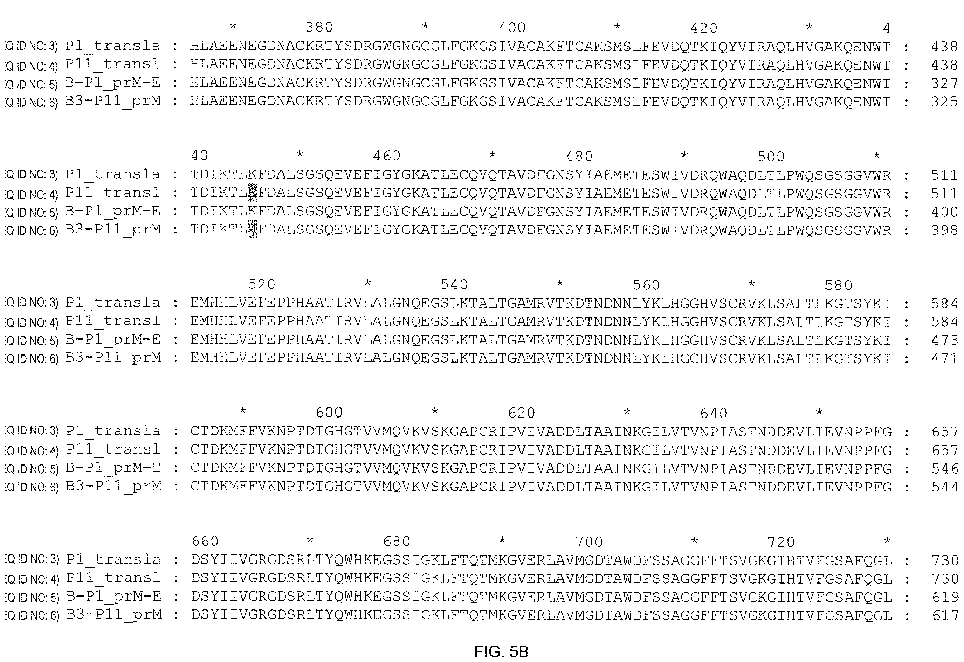

FIG. 5A-J depicts the amino acid sequence of P1 and P11, with the Series B-P1 and Series B3-P11 amino acid sequences from the repeat passaging study. The amino acid sequence for P1 is identified herein as SEQ ID NO: 3. A comparison of the amino acid sequence for P1 and that of P11 (SEQ ID NO.4) revealed a mutation at amino acid residue 160 of the envelope protein (E160) (amino acid 445 of the P1 amino acid sequence in Fig 5B). Series B-P1 and Series B3-P11 present partial amino acid sequences from the repeat passaging study. The amino acid sequence for B-P1 is identified herein as SEQ ID NO: 5. A comparison of the amino acid sequence for B-P1 and that of B3-P11 (SEQ ID NO.6) revealed a mutation at amino acid residue 160 of the envelope protein (E160) in B3-P11 (amino acid 445 of the P1 amino acid sequence).
FIG. 6 depicts the comparative 50% plaque reduction neutralization test (PRNT50) titers between treatment groups of BALB/c and CD-1 strain mice in a preliminary mouse study (M-9003-002) of the efficacy of inactivated Yellow Fever vaccine.
FIG. 7 is a graphical representation of PRNT50 antibody titers for the preliminary mouse study (M-9003-002).

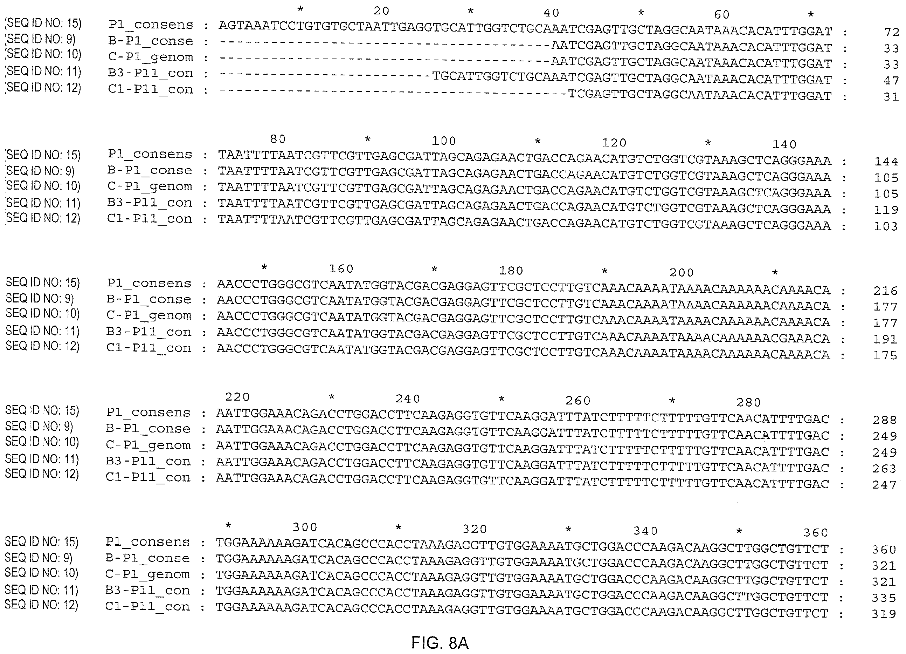

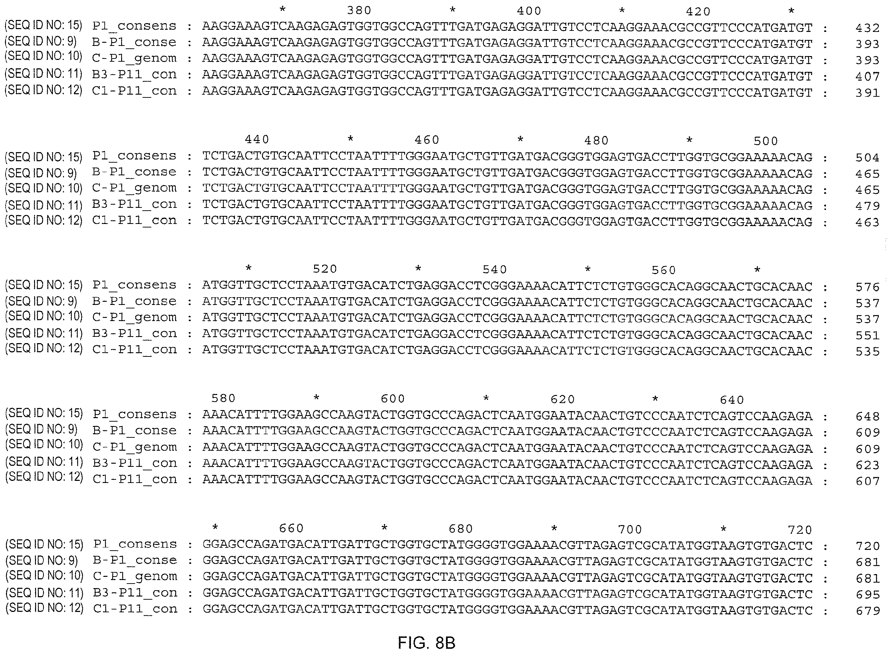

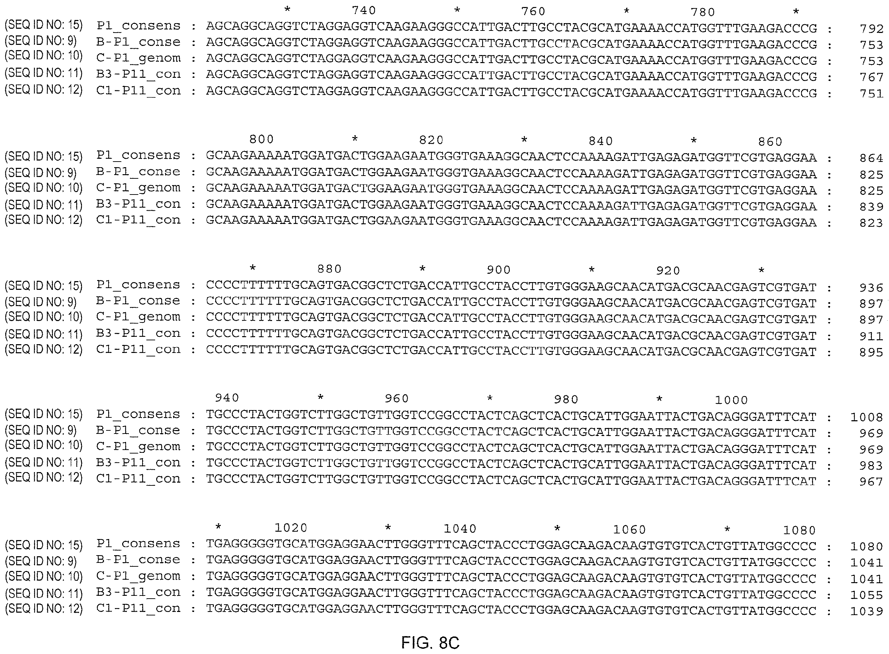

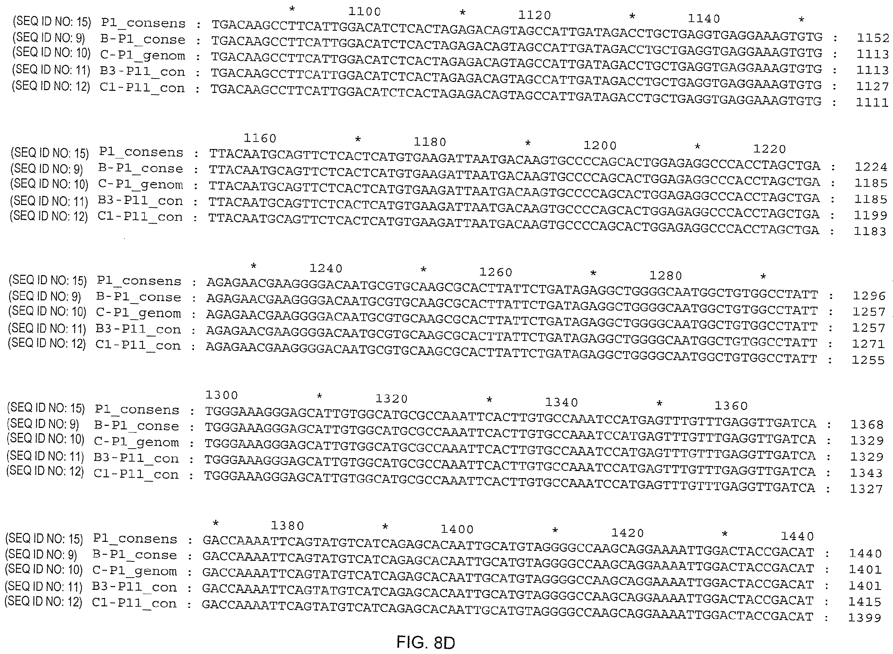

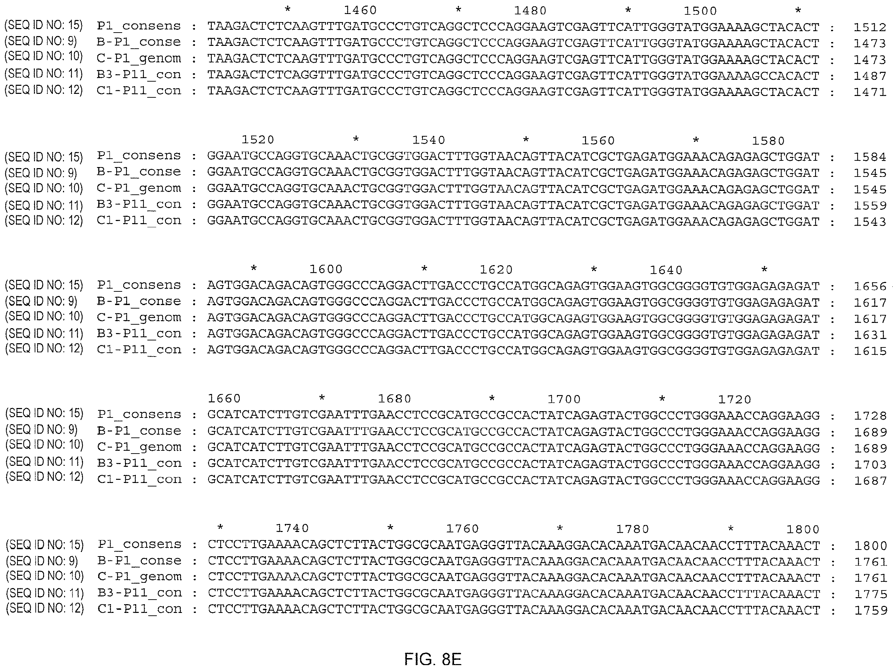

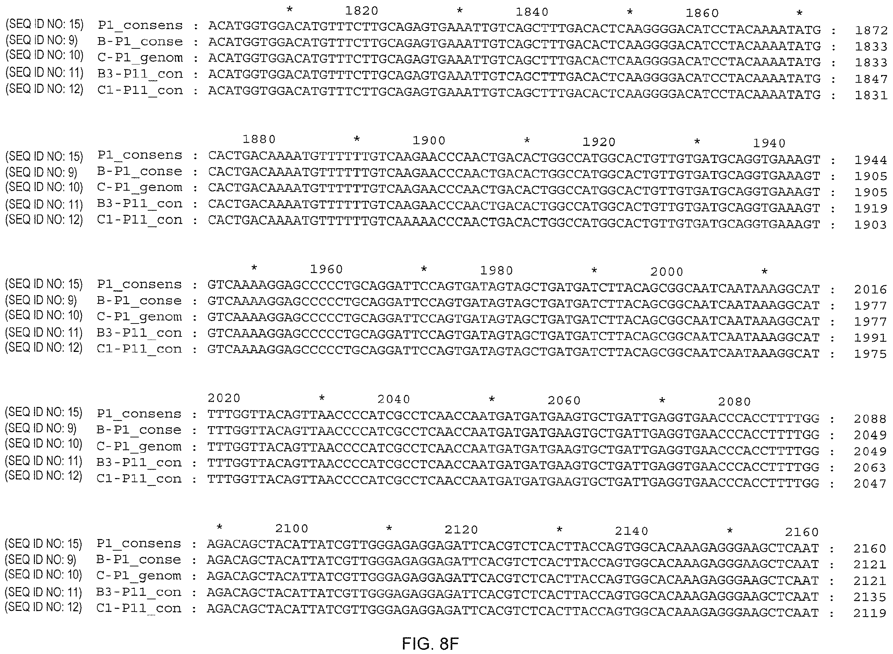

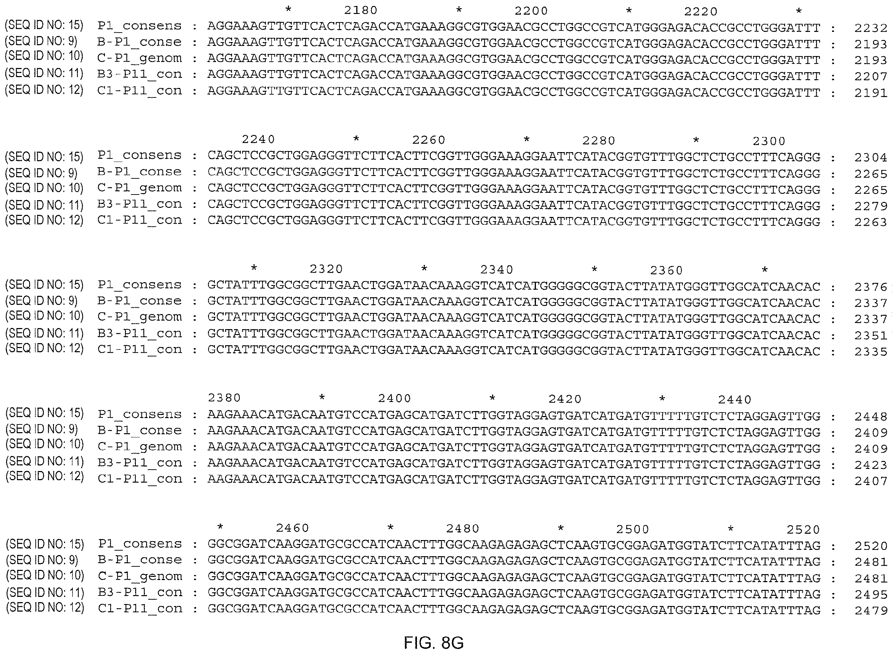

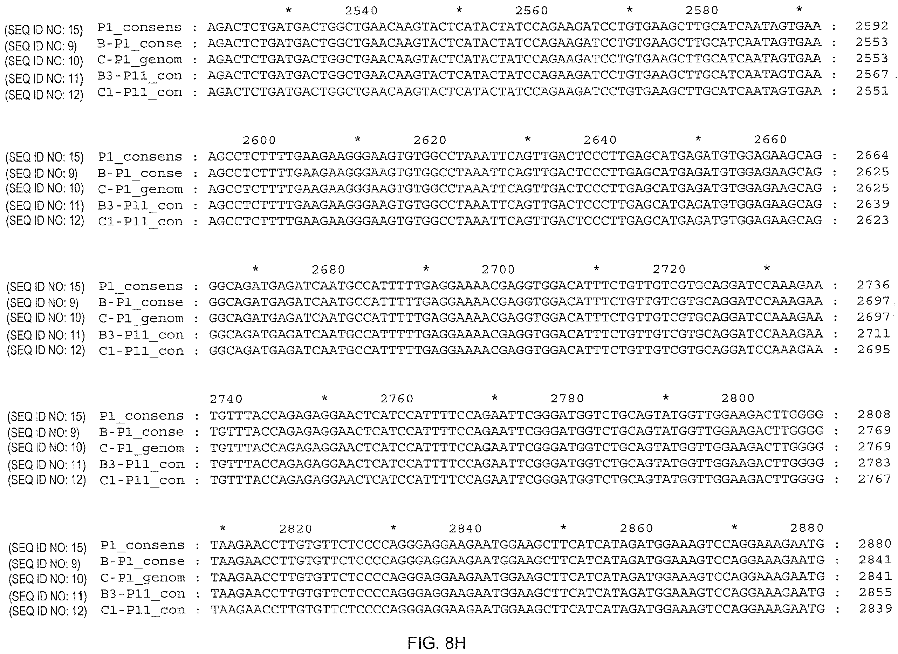

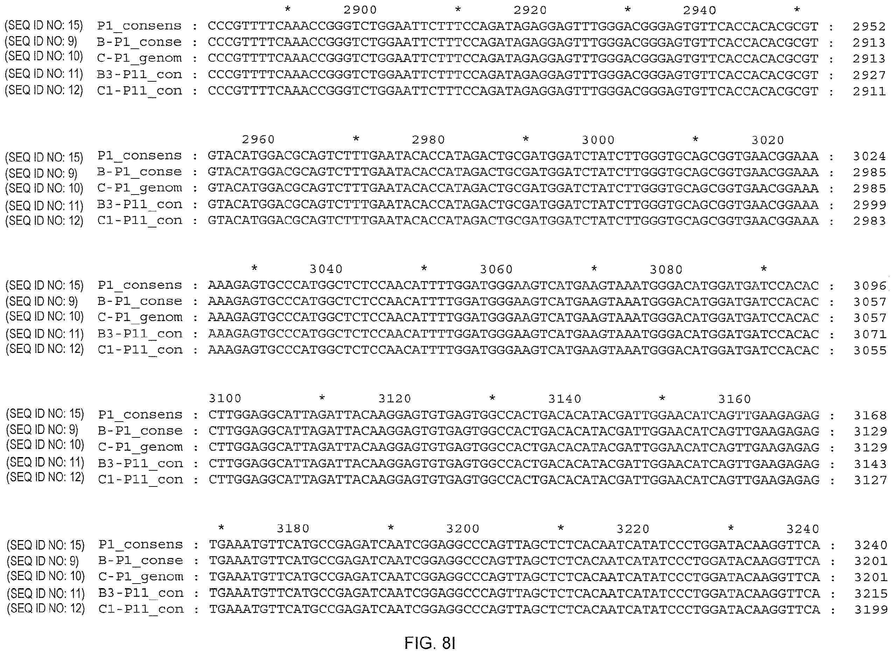

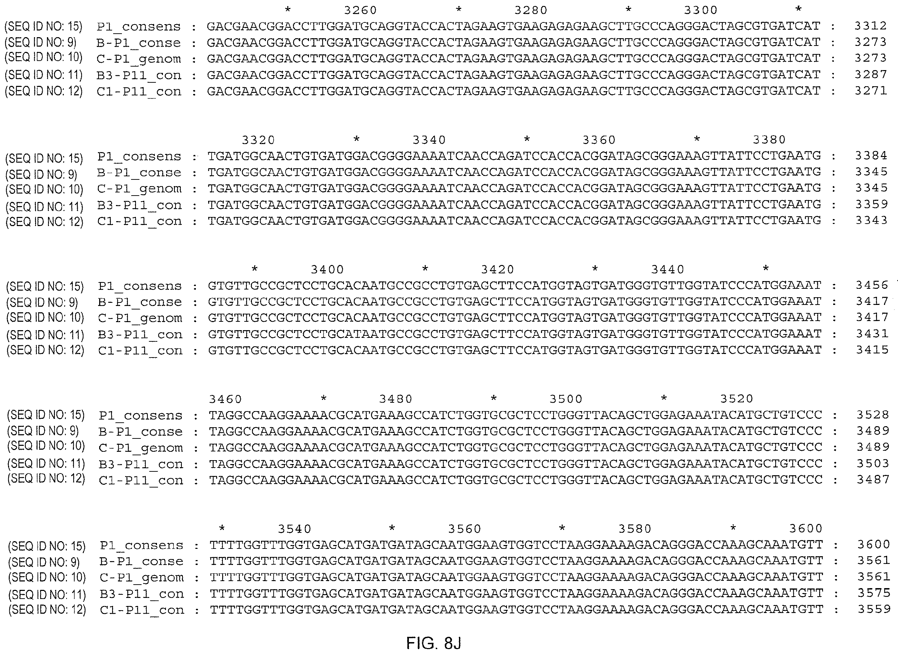

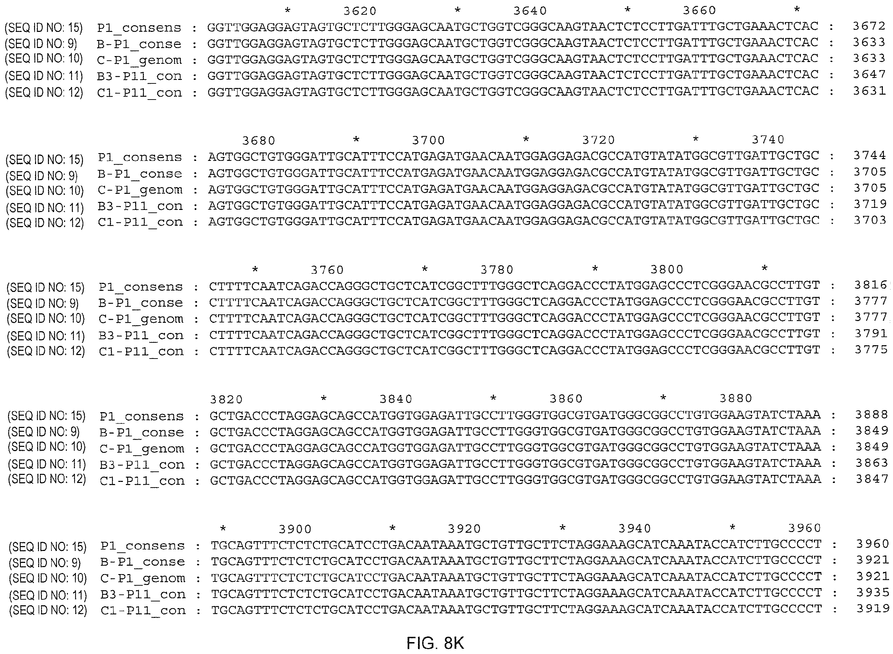

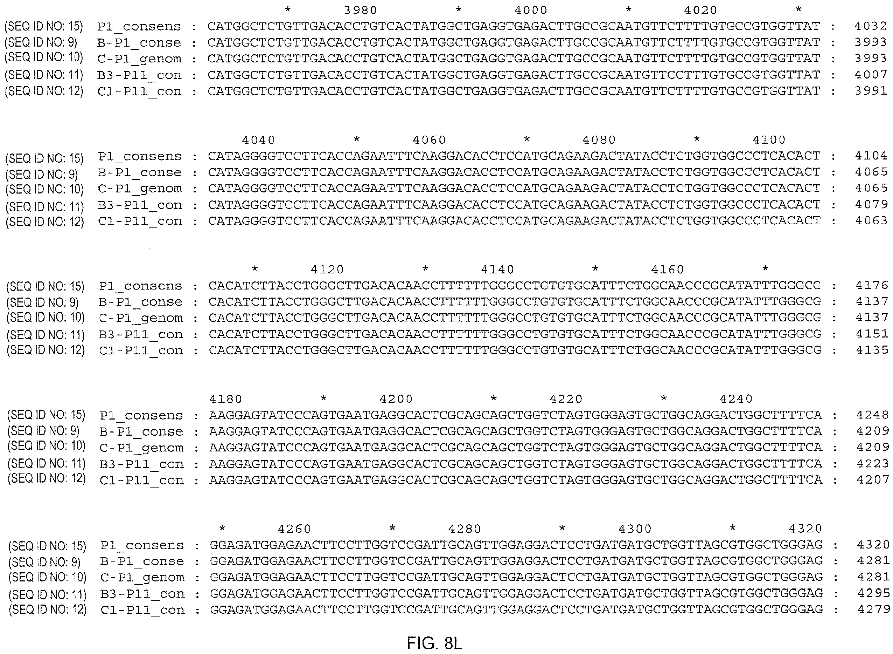

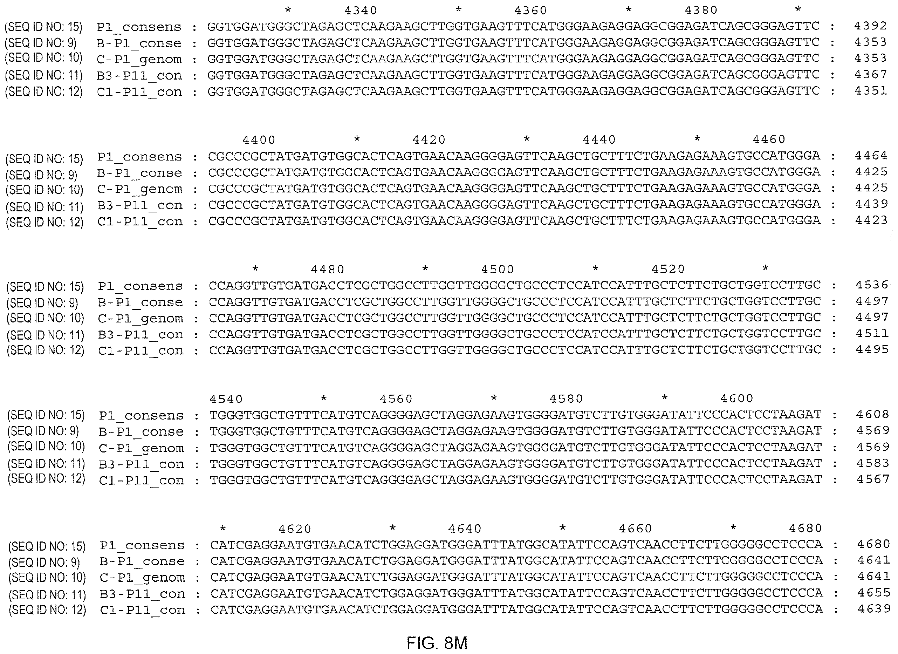

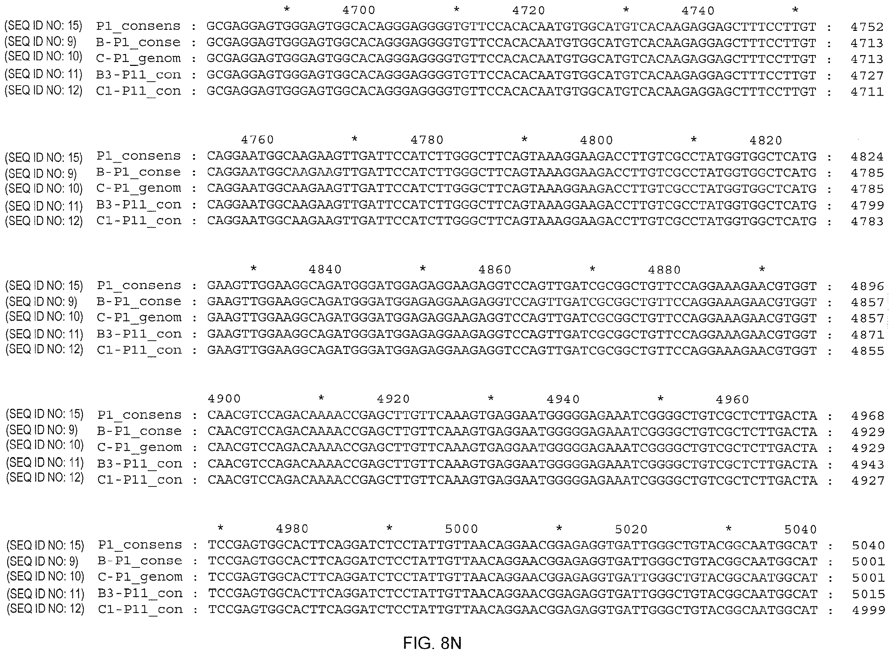

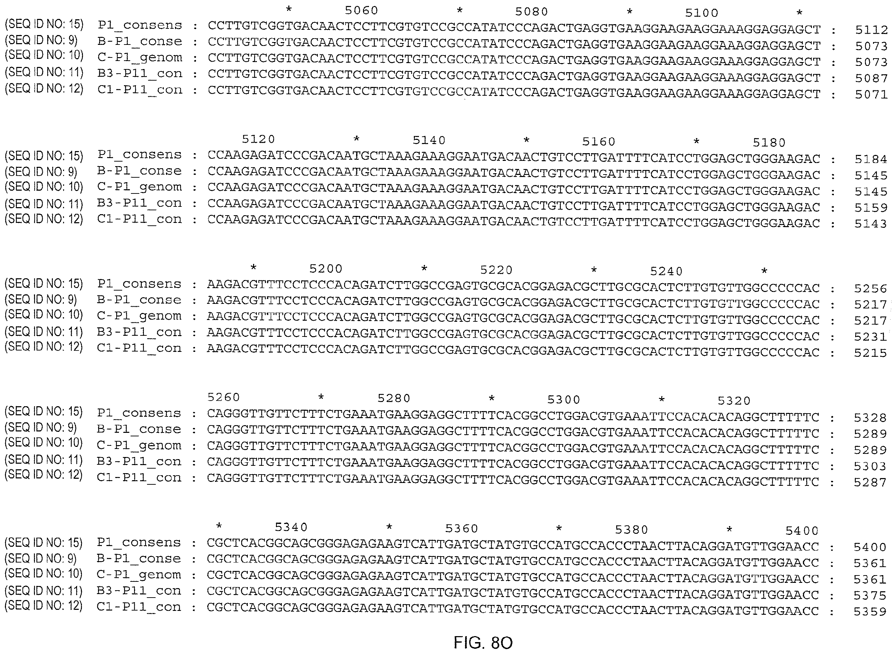

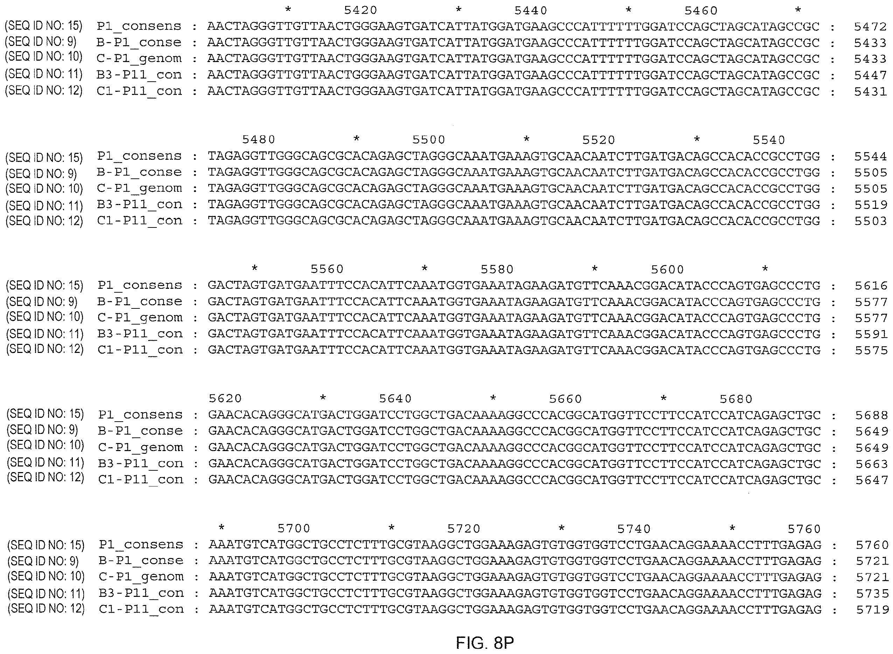

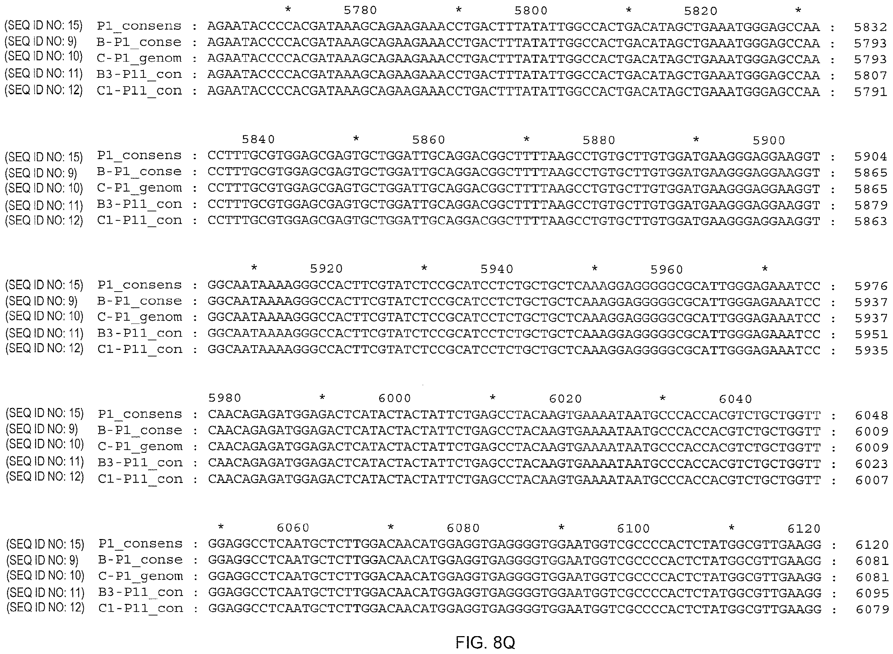

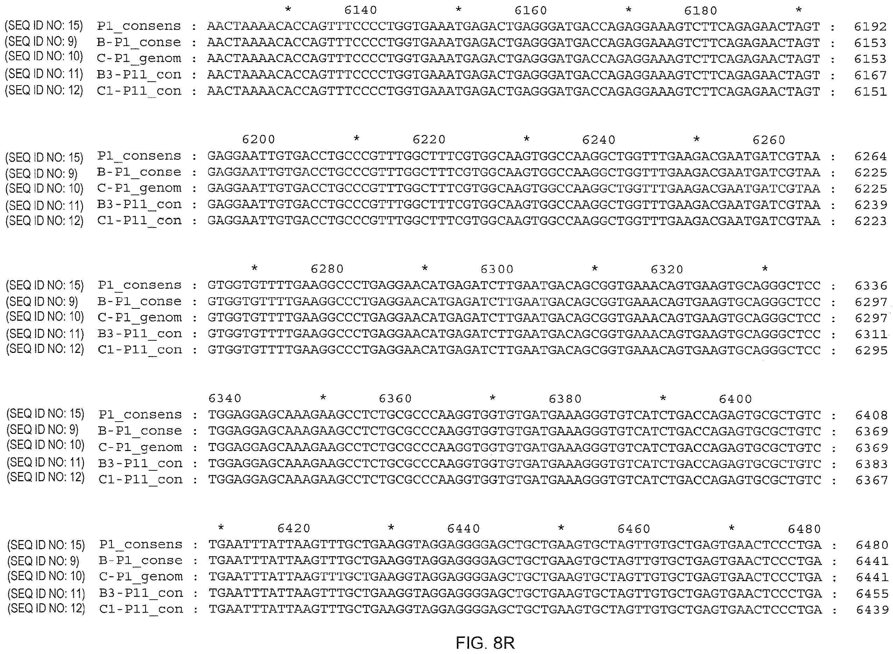

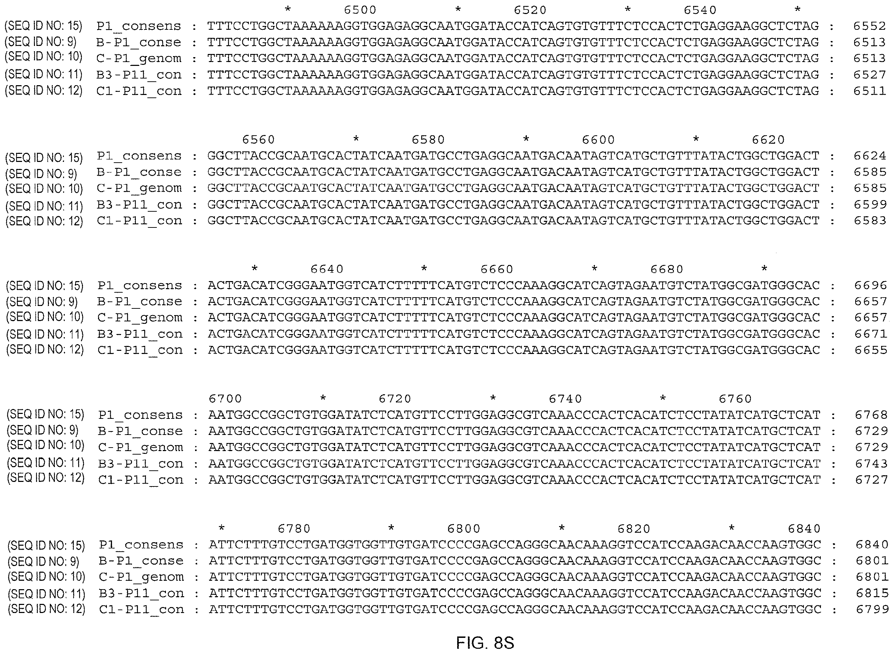

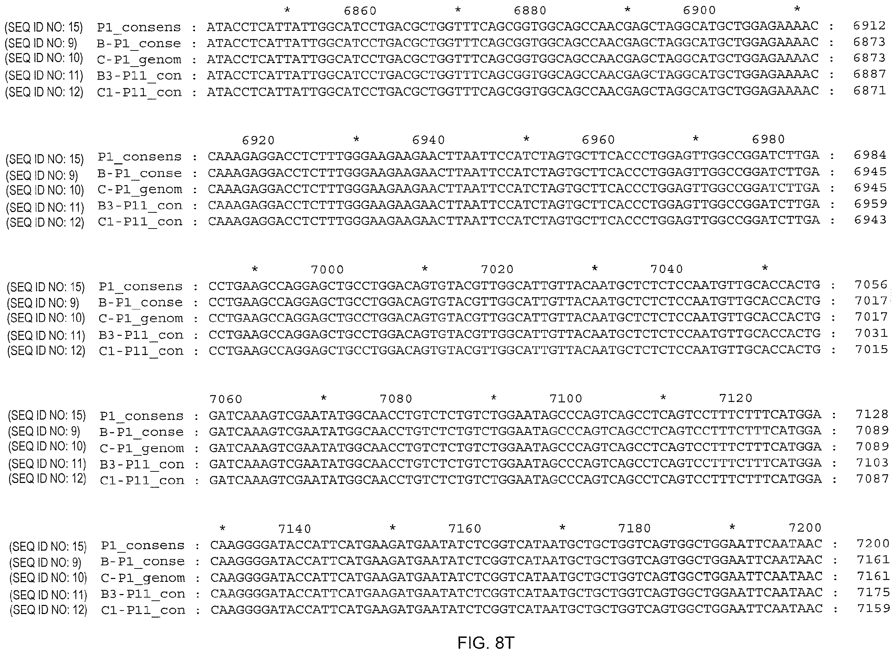

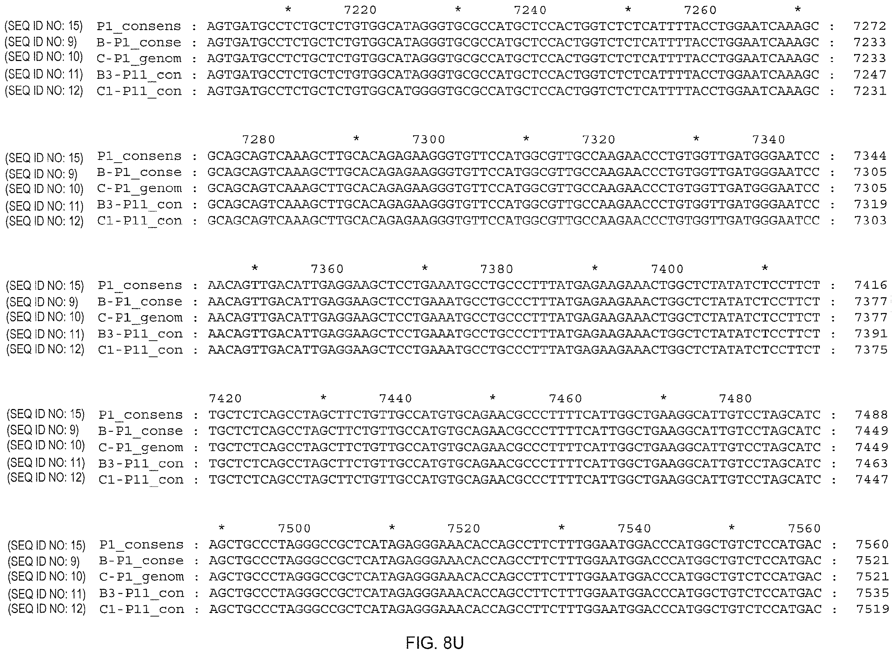

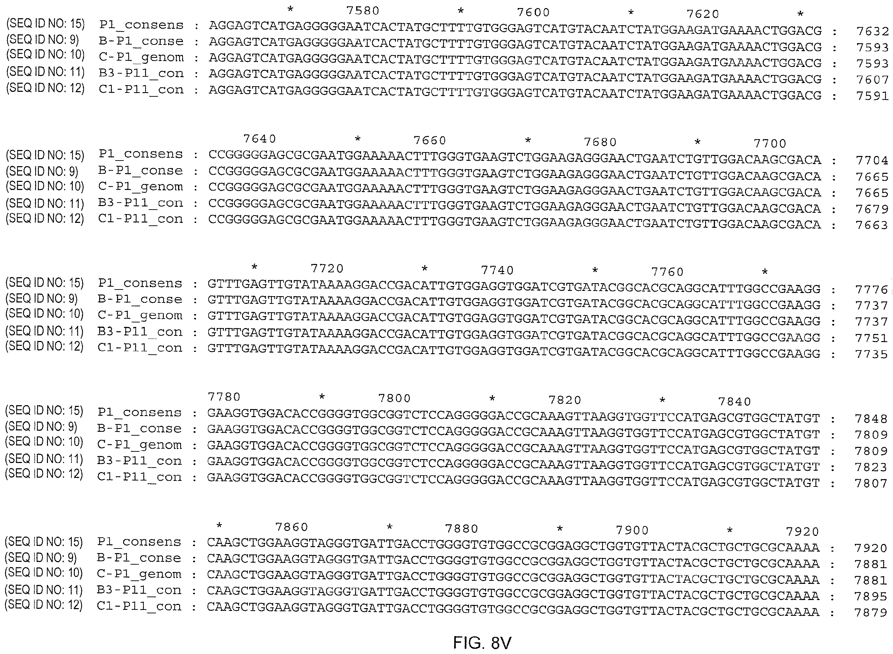

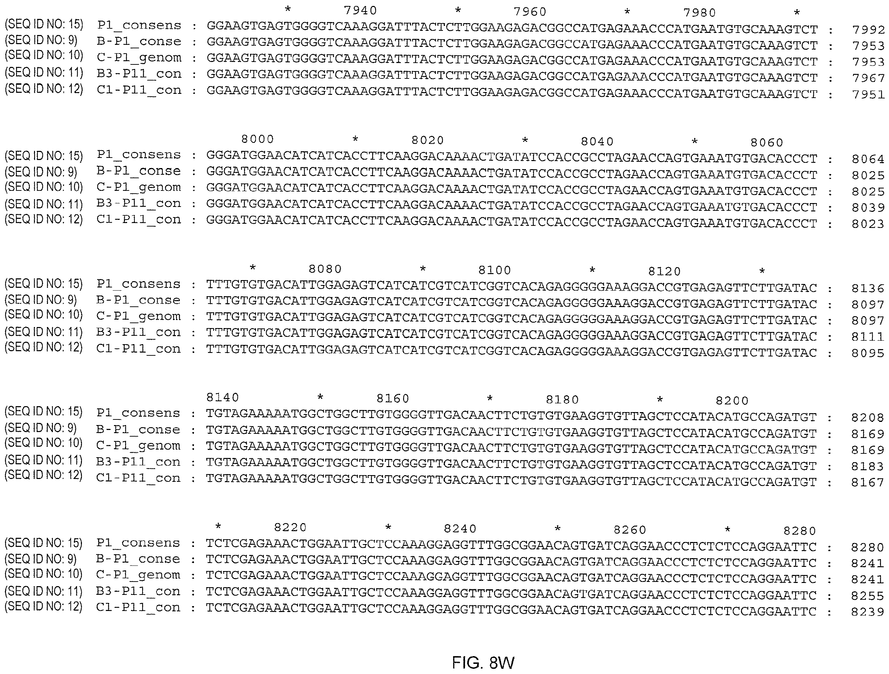

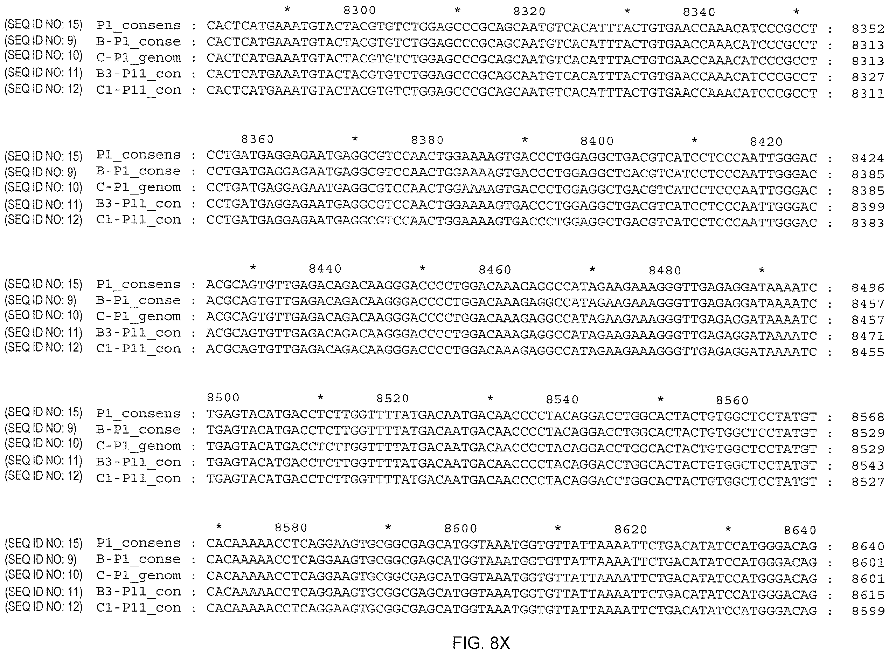

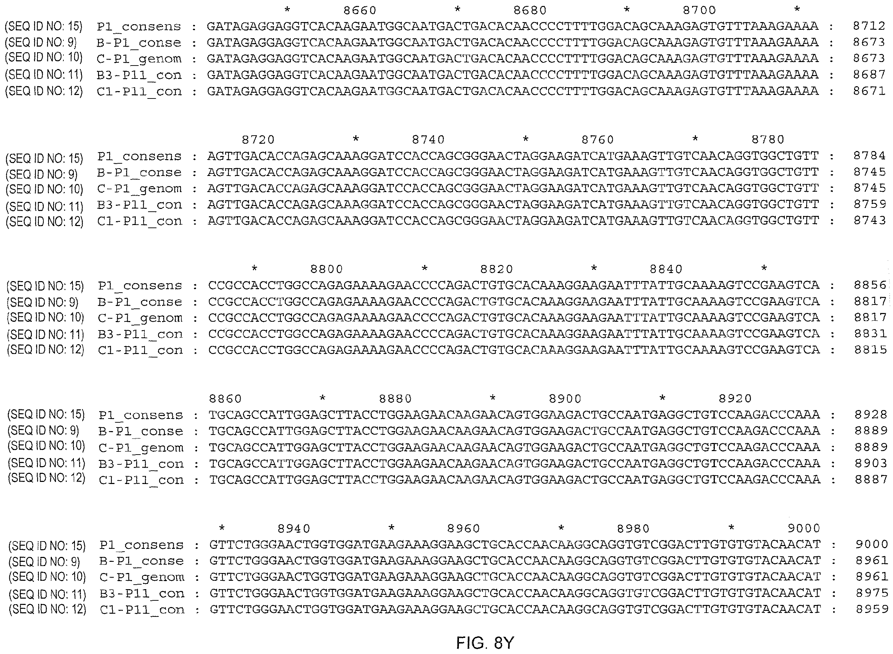

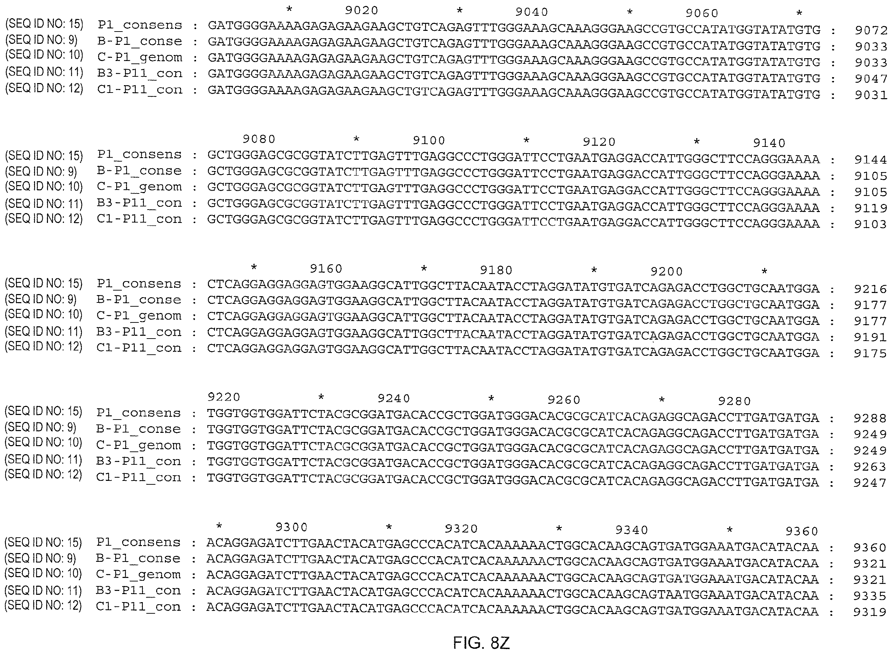

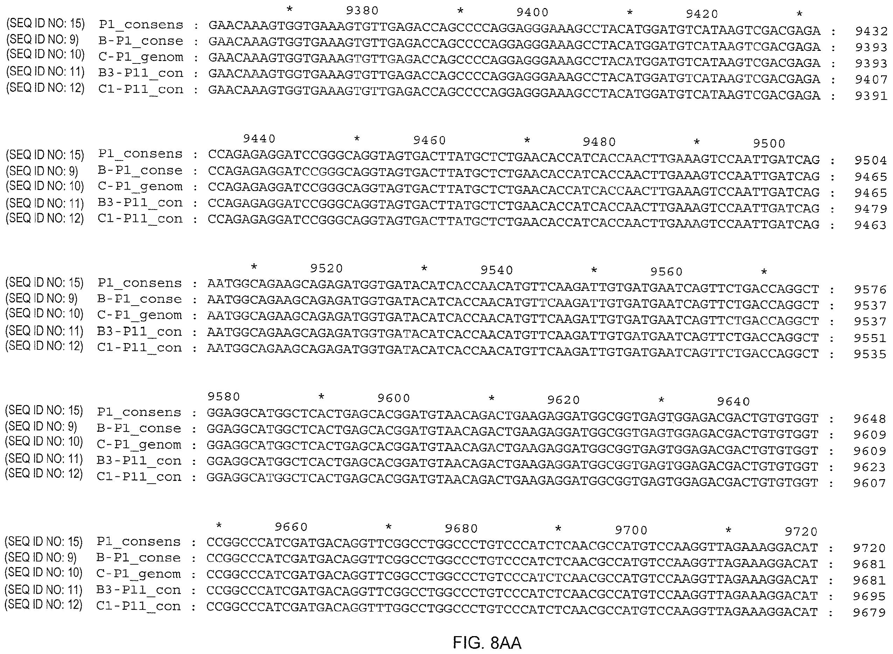

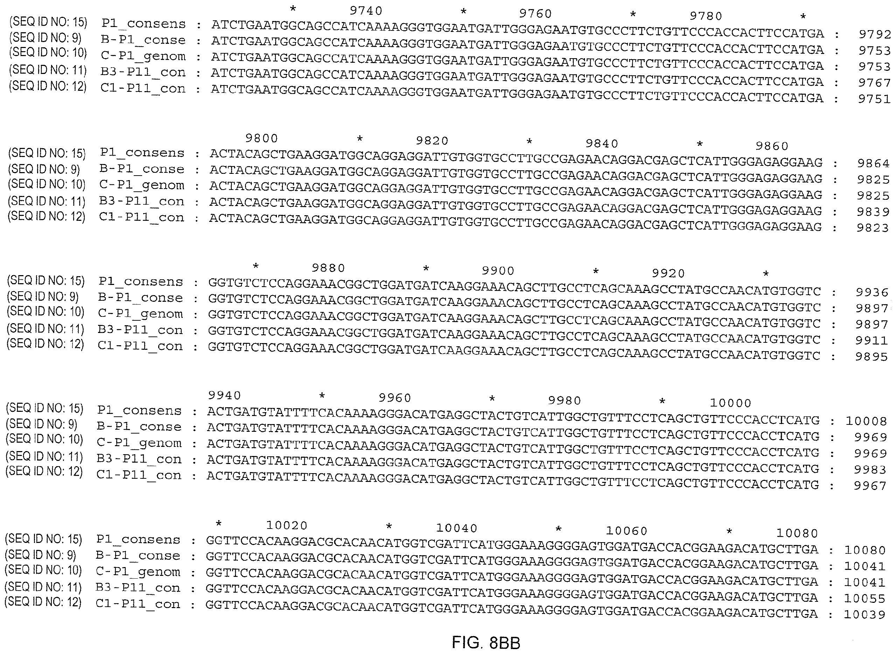

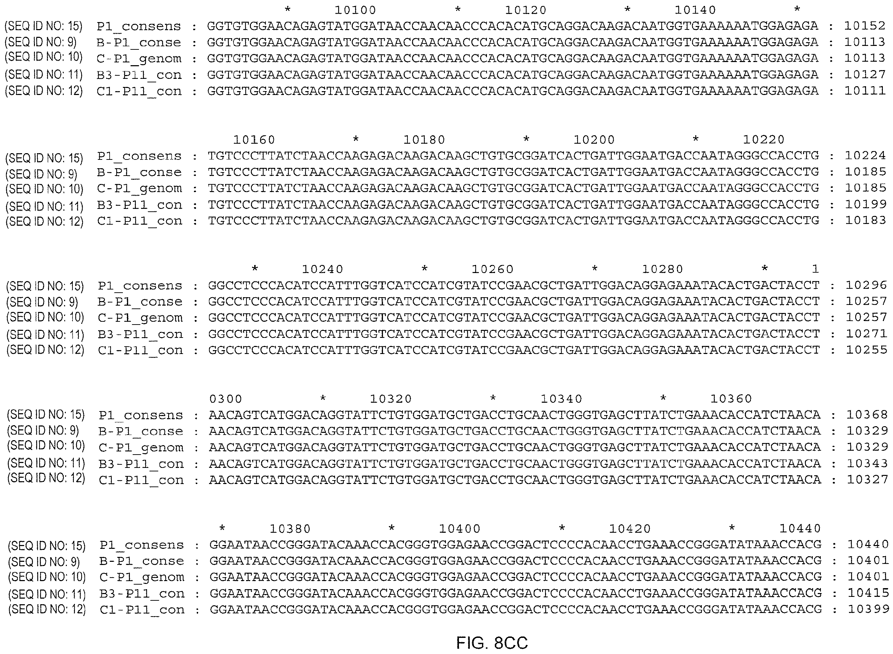

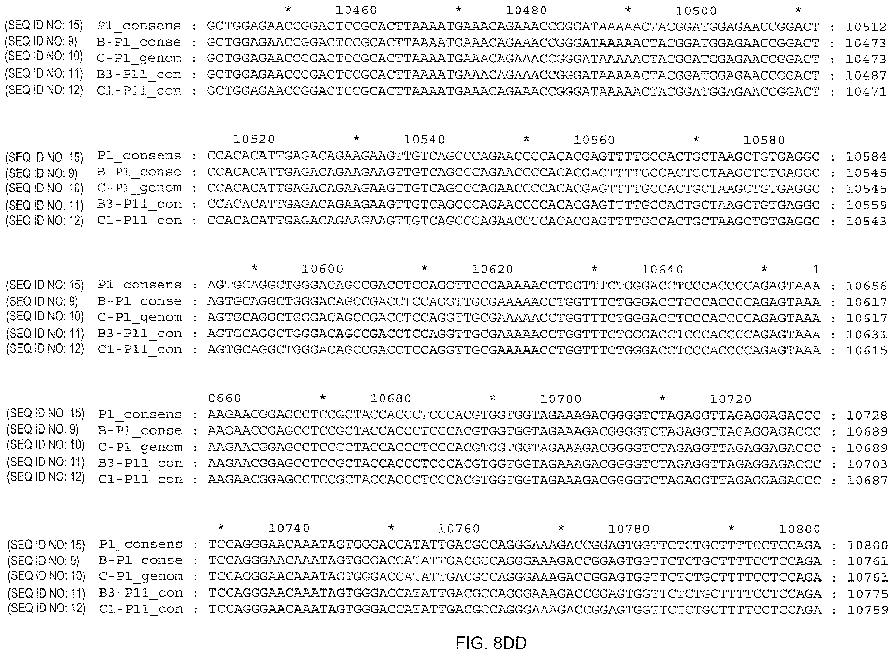

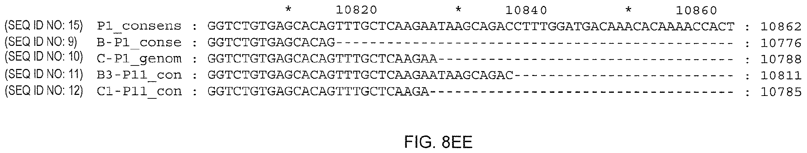

FIG. 8A-EE depicts the consensus alignment of the P1, B-P1, C-P1, B3-P11 and C1-P11 nucleic acid sequences. The nucleic acid sequence, P1, is identified herein as SEQ ID NO: 15. The nucleic acid sequence, B-P1, is identified herein as SEQ ID NO: 9. The nucleic acid sequence, B3-P11, is identified herein as SEQ ID NO: 11. The nucleic acid sequence, C-P1, is identified herein as SEQ ID NO: 10. The nucleic acid sequence, C1-P11, is identified herein as SEQ ID NO: 12. A comparison of the B-P1 passage and the B3-P11 passage revealed a genetic mutation in B3-P11 at nucleotide residue #1452 of SEQ ID NO: 15, a second mutation in B3-P11 at nucleotide residue #3402 of SEQ ID NO: 15, and a third mutation in B3-P11 at nucleotide residue #4016 of SEQ ID NO: 15. A comparison of the C-P1 passage and the C1-P11 passage revealed a genetic mutation in C1-P11 at nucleotide residue #7225 of SEQ ID NO: 15. SEQ ID NO: 11 corresponds to B3-P11, and has the codon mutations at envelope protein amino acid position 160, non-structural protein 1 amino acid position 317, and non-structural protein 2A amino acid position 170. SEQ ID NO.12 corresponds to C1-P11, and has the codon mutation at non-structural protein 4B amino acid position 113.

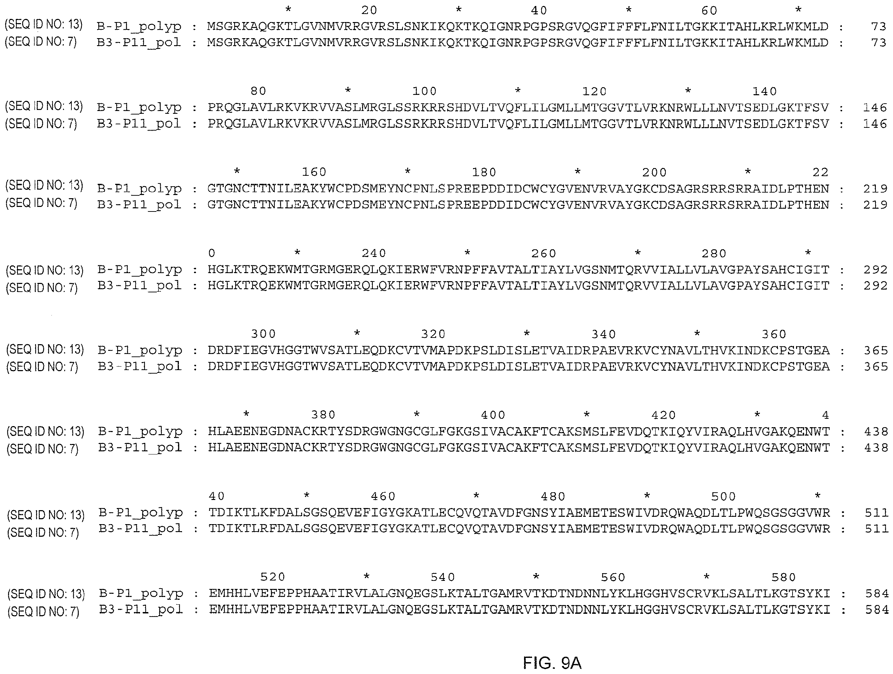

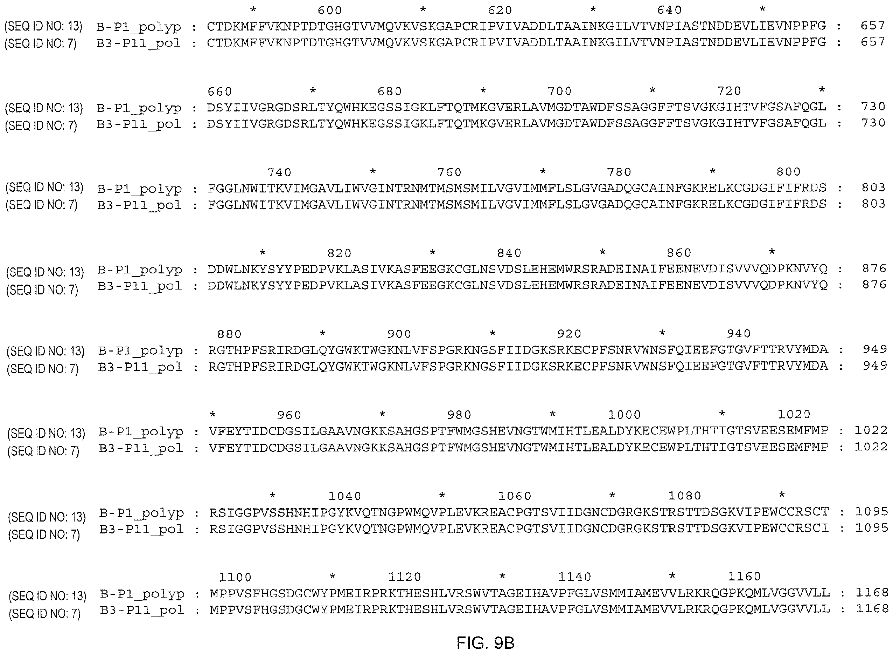

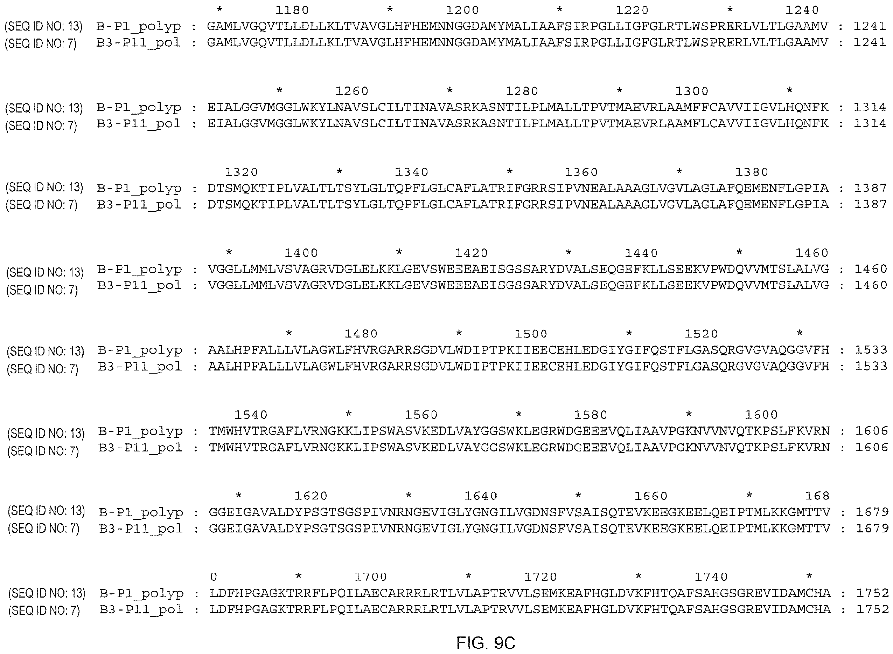

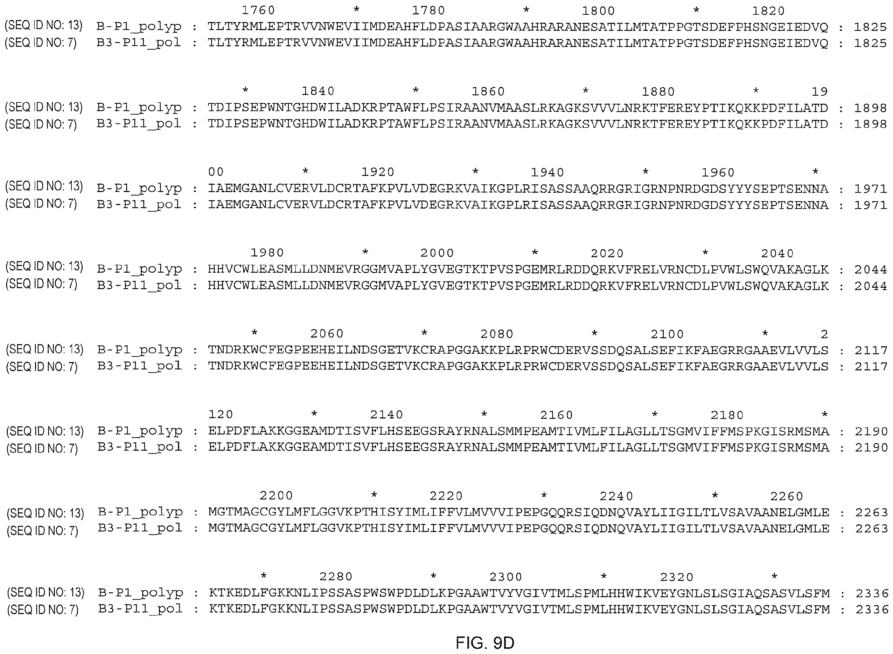

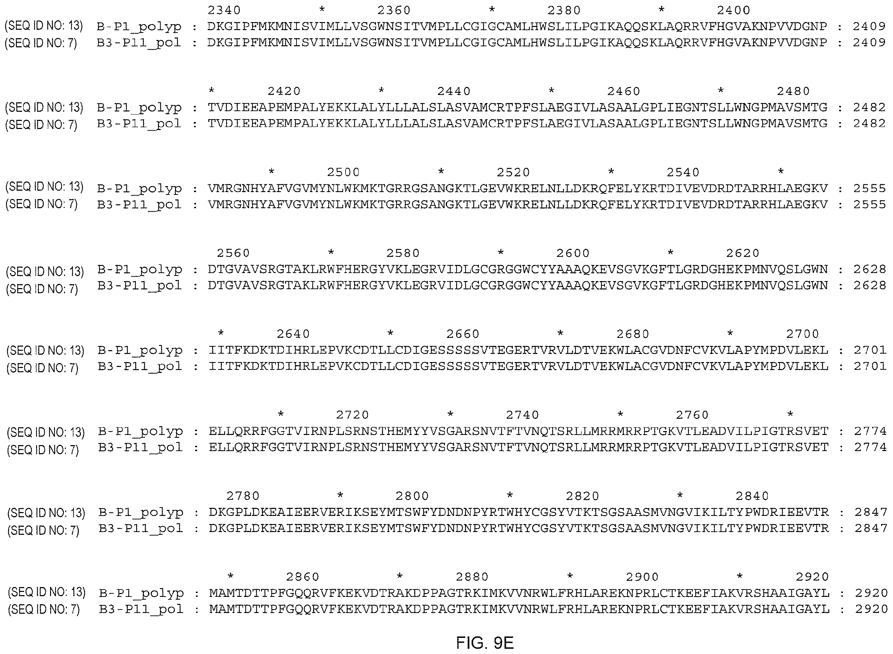

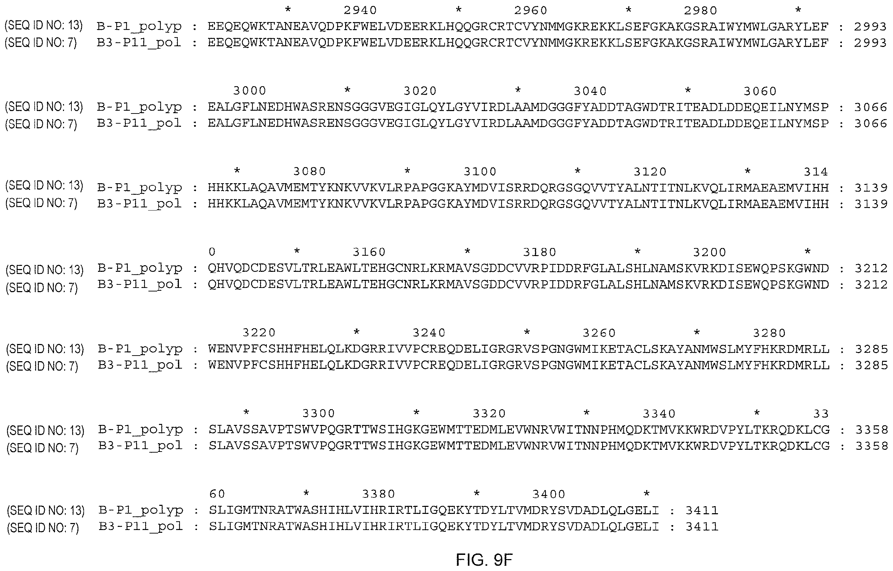

FIG. 9A-F depicts the amino acid sequence of B-P1 and B3-P11 from the repeat passaging study. The amino acid sequence for B-P1 is identified herein as SEQ ID NO: 13. A comparison of the amino acid sequence for B-P1 and that of B3-P11 (SEQ ID NO: 7) revealed a mutation at amino acid residue 160 of the envelope protein (E160) (amino acid 445 in Fig 9A), a mutation at amino acid residue 317 of the non-structural protein 1 (NS1-317) (amino acid 1095 in Fig 9B), and a mutation at amino acid residue 170 of the non-structural protein 2A (NS2A-170) (amino acid 1300 in Fig 9C). Series B-P1 and Series B3-P11 present complete amino acid sequences from the repeat passaging study.

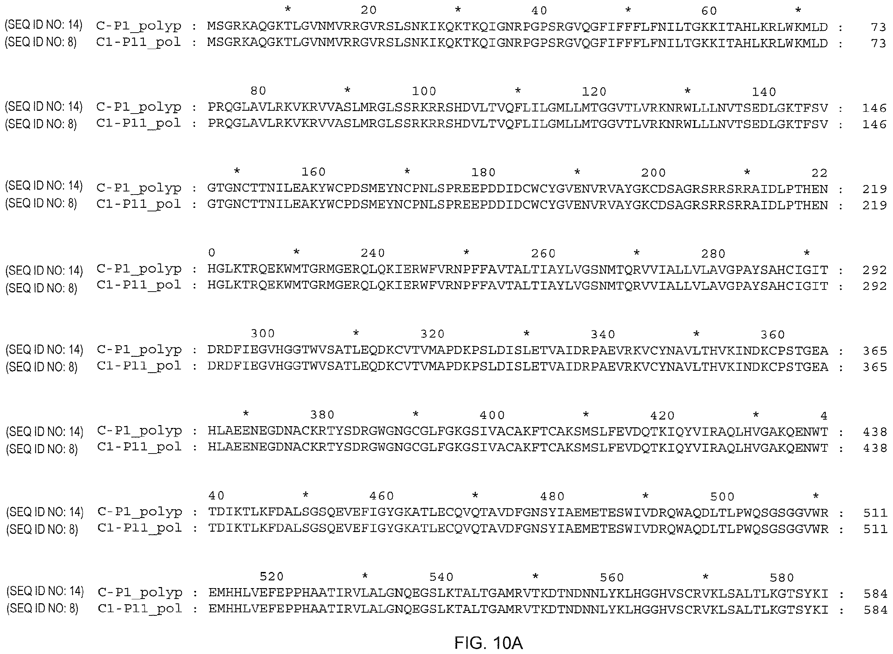

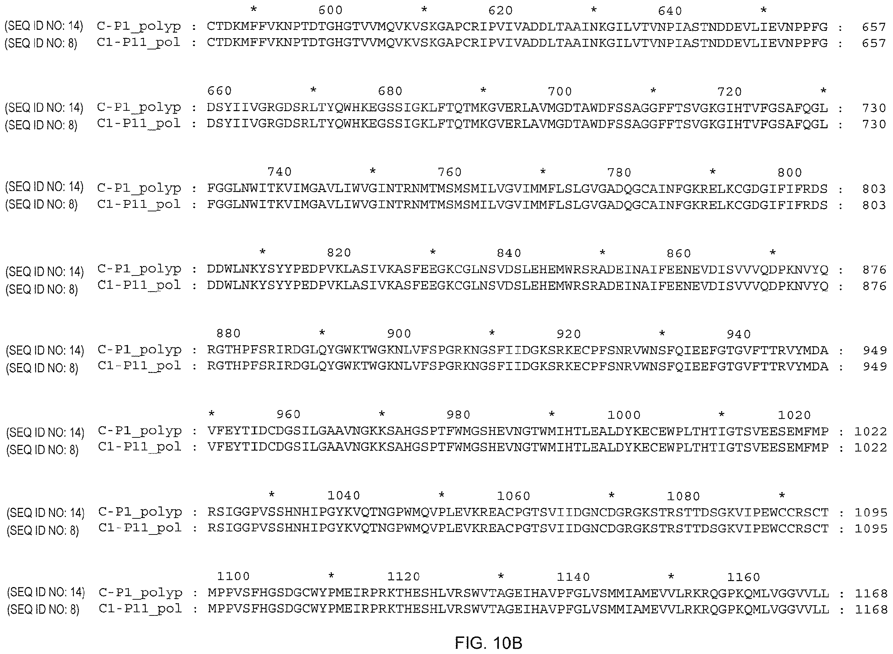

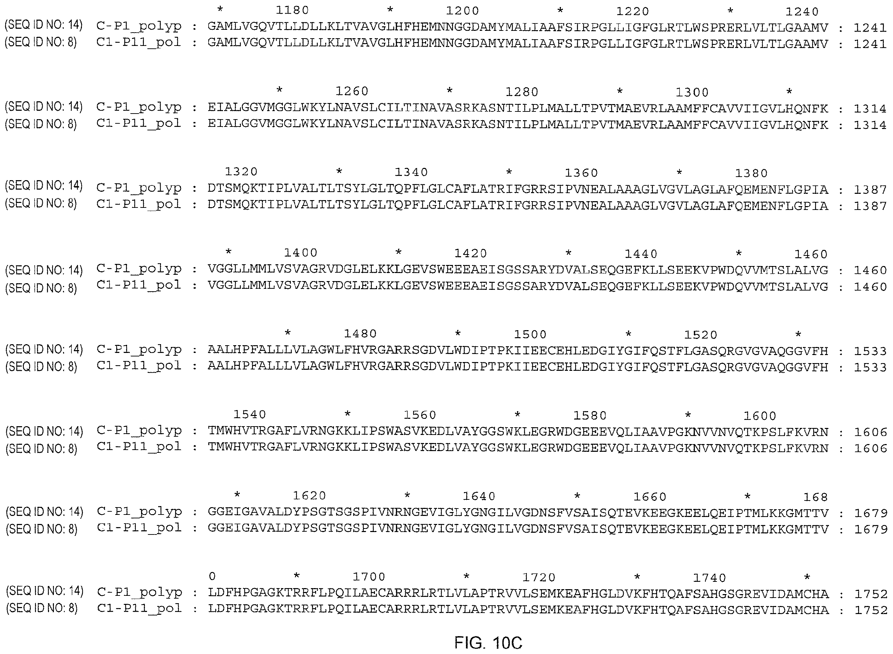

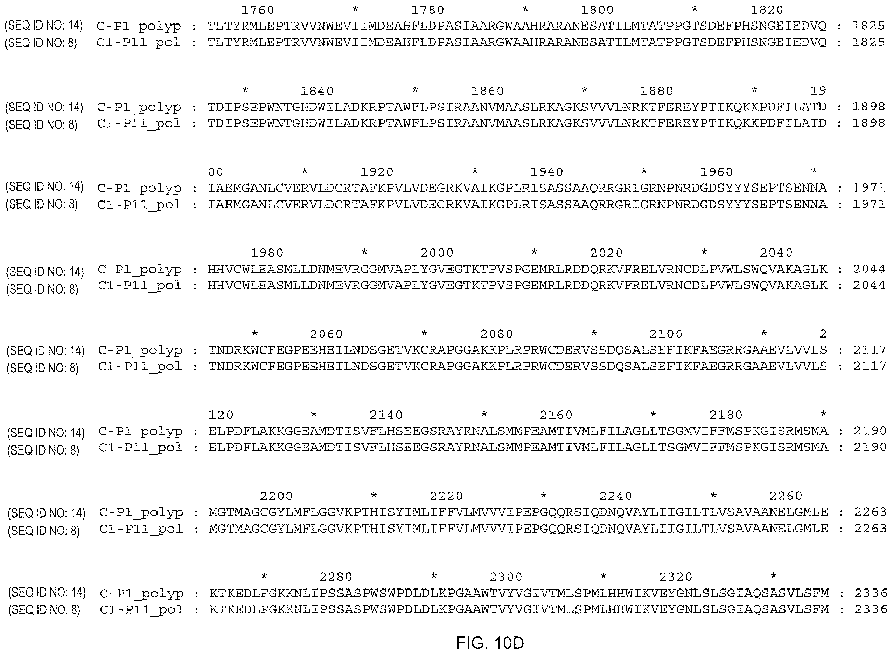

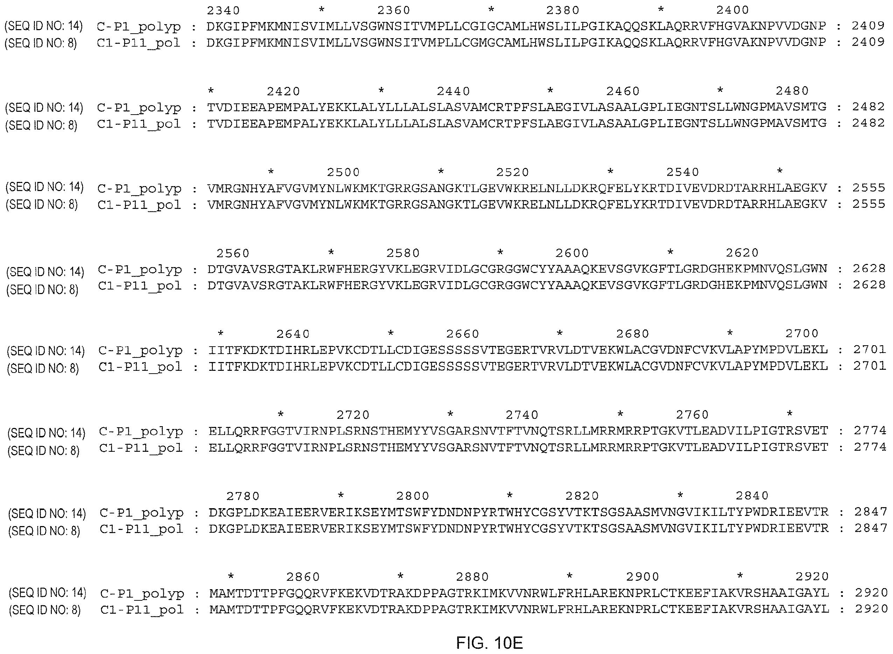

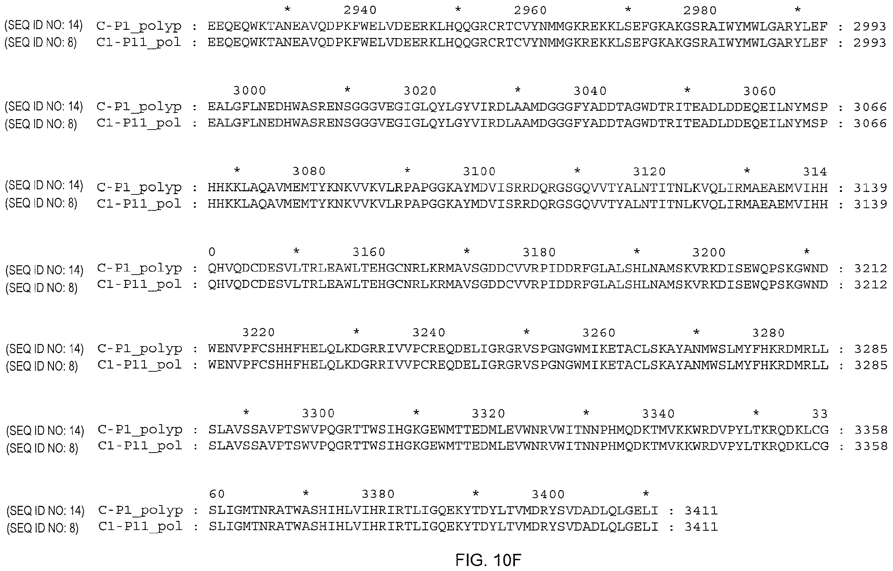

FIG 10A-F depicts the amino acid sequence of C-P1 and C1-P11 from the repeat passaging study. The amino acid sequence for C-P1 is identified herein as SEQ ID NO: 14. A comparison of the amino acid sequence for C-P1 and that of C1-P11 (SEQ ID NO: 8) revealed a mutation at amino acid residue 113 of the non-structural protein 4B (NS4B-113) (amino acid 2369 in Fig 10E). Series C-P1 and Series C1-P11 present complete amino acid sequences from the repeat passaging study.

### DETAILED DESCRIPTION OF THE PREFERRED EMBODIMENTS

A description of preferred embodiments of the invention follows. It will be understood that the particular embodiments of the invention are shown by way of illustration. At the outset, the invention is defined by the claims, with a more detailed description following. The features and other details of the compositions and methods disclosed herein are outlined below.

### Overview of Approach and Benefits

Disclosed herein is a method of producing an inactivated Yellow Fever virus candidate, the method comprising the serial passage of the YF 17D virus (i.e., an "unadapted virus") in certified African green monkey kidney cells (VERO) to increase the titer to yield a sufficient antigenic mass to induce a protective immune response and/or modify the nucleotide sequence of the viral genome. This method has been repeated and shown to be reproducible.

The virus has been purified from host cell DNA and proteins by depth filtration, ultrafiltration, diafiltration, and chromatographic separation. The method is described in WO2011011660. The purified virus may be inactivated by using a method that ensures preservation of critical, neutralizing epitopes. For example, the virus can be inactivated using formalin, heat, UV, gamma irradiation or beta-propiolactone. A purified, inactivated virus may be formulated with an adjuvant, such as adsorbed to aluminum hydroxide adjuvant, and stored as a liquid at temperatures of from about 2 degrees Celsius (2°C) to about 8 degrees Celsius (8°C).

A vaccine containing the purified, inactivated virus is believed to be safer than the currently available attenuated, live YF virus vaccine because the disclosed inactivated YF virus vaccine is non-replicating. The improved vaccine can be manufactured by modern methods in Vero cells without animal derived proteins, and therefore it can be used safely in persons (including egg-allergic persons) for whom the live vaccine (produced in hens' eggs) is contraindicated or for whom warnings appear in the label. Such warnings would include, for example warnings to immunosuppressed persons, persons with thymic disease, egg-allergic persons, infants <9 months, and the elderly.

### Adaptation of Yellow Fever Virus for Robust Production In Vero Cells:

The Vero cells used in the virus development phase were obtained from the World Health Organization (W.H.O.) seed lot, WHO Vero 10-87 Cell Bank at Passage 134. The WHO Vero 10-87 Cell Bank was originally made by the Institut Merieux using the ATCC Vero cell line CCL81 at Passage 129. The cells were thawed into OptiPRO™ SFM (serum-free medium) supplemented with 5% fetal bovine serum which was removed 24 hours later and replaced with OptiPRO™ SFM medium without fetal bovine serum. The serum, certified as being of USA origin, was gamma irradiated and had been tested for adventitious agents by the manufacturer; additional testing for sterility, mycoplasma, and adventitious viruses was performed on this material by WuXi AppTec. All subsequent passages of Vero cells to make the cell banks, virus seeds, and vaccine were made in OptiPRO™ SFM without serum. No other animal derived materials or products were used in producing the cell banks or the final vaccine according to an embodiment of the invention.

### Preparation of Vero Cell Banks:

Master and Working Cell banks were prepared according to cGMP and were tested and characterized according to FDA Points to Consider. The Vero cells had an established provenance and were free from regulatory concerns about Bovine spongiform encephalitis (BSE). Serum-free growth medium was employed in propagating cells.

### Passage history of Vero cells during manufacture of seed viruses and vaccine lots:

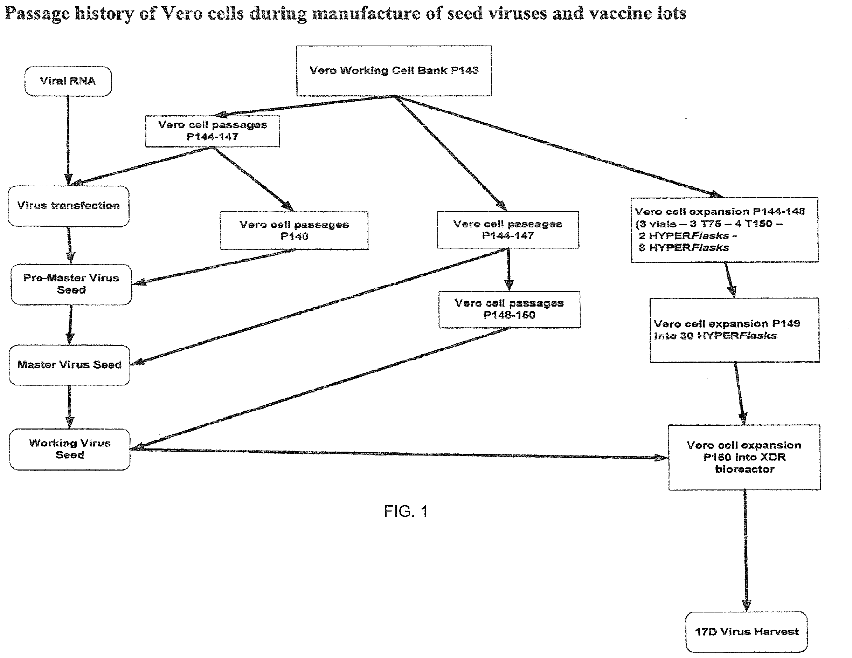

The passage history of Vero cells during the manufacture of the disclosed yellow fever vaccine is shown schematically in FIG. 1. The WHO cells were received at Passage 134, the Master Cell Bank (MCB) and Working Cell bank (MWCB) were banked at Passages 139 and 143 respectively. The cells were further expanded a maximum of 11 passages to Passage 154 during cell expansion in stationary cultures prior to seeding of the bioreactor used for virus production. The estimated number of population doublings in the bioreactor is calculated to be 1 to 3.

### Preparation of Master and Working Virus Seeds:

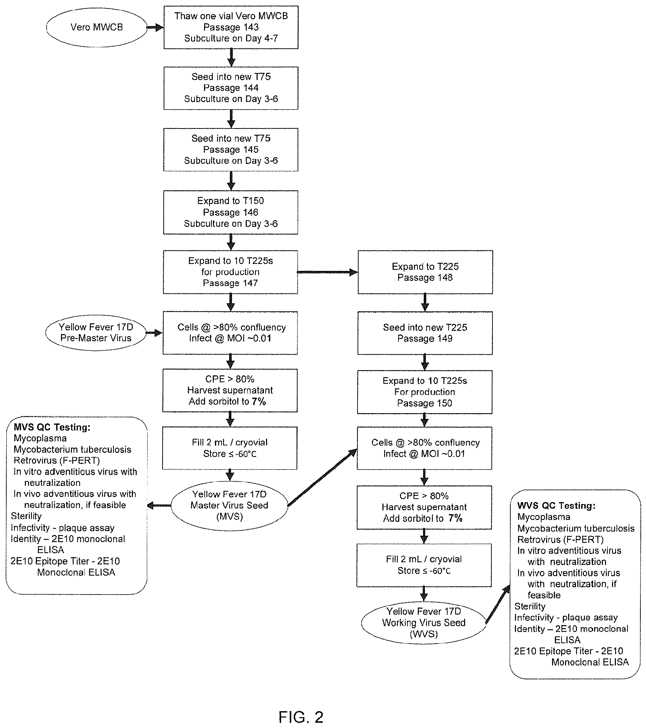

FIG. 2 is a schematic representation of the preparation of Virus seeds according to an aspect of the disclosure. An important safety factor for the disclosed vaccine is the use of the attenuated YF 17D vaccine for manufacture. The attenuated virus used as a starting material was a commercial vaccine, YF-VAX® (Sanofi Pasteur, Swiftwater PA) which had undergone various tests for adventitious agents. The original YF-VAX® material used to inoculate Vero cells was derived from embryonated hens' eggs, and contained hydrolyzed porcine gelatin as a stabilizer. However, the likelihood of carry-over of an adventitious agent from eggs was mitigated by use of RNA transfection to produce the Pre-Master Virus Seed.

The cells were propagated in OptiPro-SFM medium (Invitrogen, Grand Island, NY). To develop the modified Yellow Fever (YF) virus that will grow to high titers in Vero cells, initially the YF-17D virus at a 0.01 multiplicity of infection (MOI) was used to infect a T-25 flask with a confluent layer of Vero cells. The cell culture was incubated at 37°C and 5 percent CO₂.

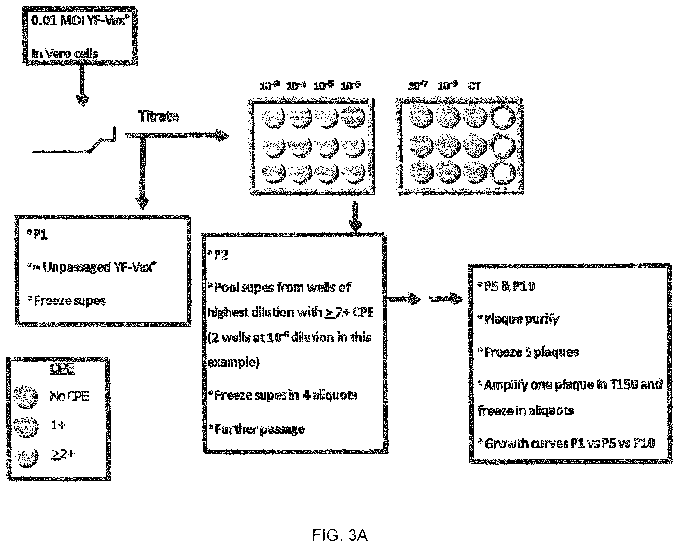

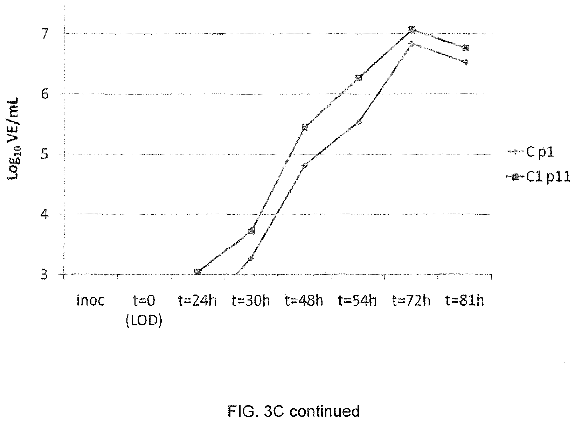

Once cytopathic effect (CPE) was observed in about 2 + (50%) of the cells, aliquots of the culture were prepared, labeled as passage one (P1) and stored at -80 °C for use as the inoculum to continue the serial passages. A schematic of the procedure used to make P1 through P10 is shown in FIG. 3A.

An aliquot of the Passage 1 (P1) virus was diluted 10⁻¹ through 10⁻⁸ and each dilution was inoculated onto confluent monolayers of three (3) Vero cell cultures propagated in sterile 12 well plates from which growth medium had been removed.

Log₁₀ dilutions were prepared by transferring 0.2 ml of virus to 1.8 ml of phosphate buffered saline (PBS) to equal a 10⁻¹ dilution. The virus plus PBS was mixed and then a new pipette was used to transfer 0.2 ml to 1.8 ml of PBS = 10⁻², and then repeated through 10⁻⁸ dilution. Twelve well confluent monolayers of Vero cell culture were labeled and log₁₀ dilutions of the P1 material (negative control, 10⁻¹ (3 wells), 10⁻² (3 wells), 10⁻³ (3 wells), 10⁻⁴ (3 wells), 10⁻⁵ (3 wells), 10⁻⁶ (3 wells), 10⁻⁷ (3 wells) and 10⁻⁸ (3 wells) were prepared and inoculated onto medium-free cultures using a new pipette for each dilution of inoculum. The negative control cultures were inoculated with a similar volume of PBS. After inoculating the cultures they were incubated at 37°C for 1 hour with intermittent rocking and then 1.0 ml of maintenance medium was added per culture. Cells were observed each day for cytopathic effect (CPE) and recorded as 1+ (25% of the cell monolayer effected), 2+ (50% of the cell monolayer effected), 3+ (75% of the cell monolayer effected) and 100% (all of the cell monolayer effected). Estimates of CPE were based on a comparison with the control cells. The plaque assay was also performed on the same dilutions of inoculum to verify that the CPE represented viral infectivity.

Once CPE (2 +) developed in these cultures, five 0.5 ml aliquots of the medium were harvested from the cultures that received the highest dilution or next to the highest dilution of inoculum. The five aliquots were prepared and stored as passage 2 (P2) at -80°C. The strategy was to select the virus population that replicated at or near the highest log₁₀ dilution based on the appearance of CPE in the cells. As such, the virus population selected would be the population that was best adapted to replicate in the cells with possible genetic changes that will allow for an increase in viral titer.

Subsequently, log₁₀ dilutions were prepare of an aliquot of the P2 virus and used to infect cultures of Vero cell propagated in 12-well plates as described for passage one YF virus. Similar methods were employed to complete 10 serial passages of the virus.

### P10 and P11:

At each serial passage, each of the aliquots used as the inoculum was also tested to determine the infectivity titers by plaque assay in Vero cells. At passage 10, five single, well isolated plaques, each representing progeny from a single infectious virus particle, were selected at the highest dilution that yielded plaques. Each plaque was suspended in 0.3 ml of medium containing Human Serum Albumin (HSA) to protect the virus infectivity during freezing and stored at -80°C.

The series of passages (PI to P10) of the YF 17D virus in Vero static cultures at dilutions of 10⁻¹ to 10⁻⁸ were performed at the University of Texas Medical Branch (Galveston, Texas). The strategy was to select the virus population that replicated at or near the highest log10 dilution based on the microscopic appearance of CPE in the Vero cells. The virus population that showed cytopathic effects at the highest dilution, the P10 harvest, was selected as the optimized, "high-yield" virus. The high yield virus population that showed CPE at the highest dilution was sequenced.

### The High Yield Virus:

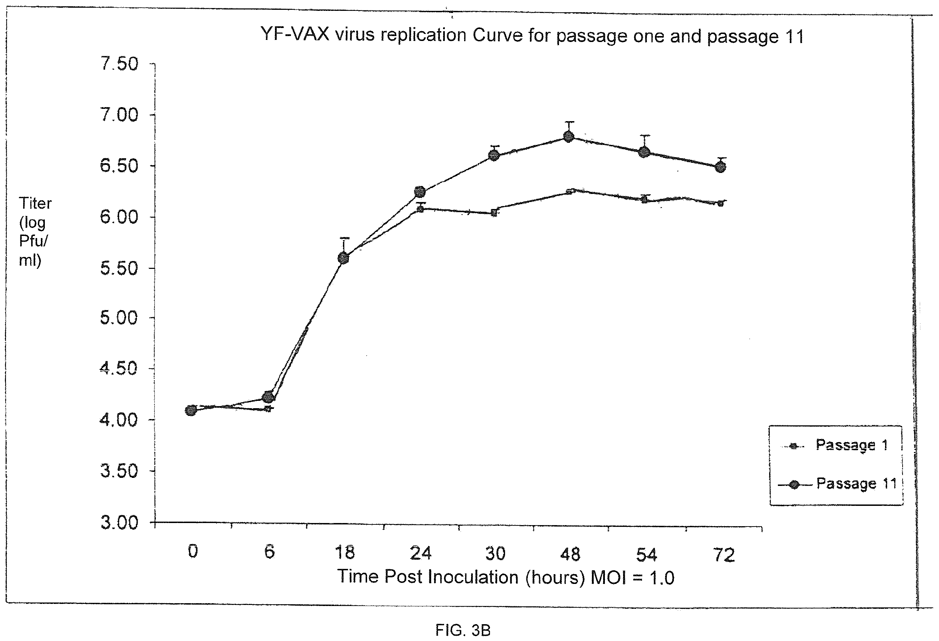

The "high yield" virus was adapted for increased replication in Vero cells by 10 serial virus passages at terminal dilution in Vero cells. At Virus Passage 10, a single plaque forming unit was picked and passed in fluid culture to produce a mini-seed stock at Virus Passage 11. The graph in FIG. 3B shows comparative growth curves of P1 and P11 viruses, that had been inoculated at high MOI; the data indicate that the P11 virus has a higher peak titer than the P1 virus. This virus (P11) showed a 3-7 fold increased replication capacity in in Vero cells compared to the YF 17D at Virus Passage 1. The Virus Passage 11 virus stock was used for RNA extraction and the RNA used to produce cGMP grade virus seeds.

### RNA Sequence of the Vero Adapted 17D Virus (P11)

The full genomic consensus sequences of the viruses at P1 and P11 from the original YF-VAX® were determined. Two genetic mutations or nucleotide differences were found, as shown in Table 1 below. One nucleotide difference lies in the capsid (C) gene and one in the envelope (E) gene. The term "capsid" as used herein, refers to the shell of protein that surrounds and protects the nucleic acid of a virus. The change in the C gene was silent (no amino acid change), whereas the E gene mutation resulted in an amino acid (Lys→Arg) mutation.

**Table 1. RNA sequence and mutations in the YF 17D virus adapted to Vero cells**

| | **Nucleotide Change** | | **Amino Acid Change** | | | **Codon** | |
|---|---|---|---|---|---|---|---|
| **NT residue** # | **P1** | **P11** | **P1** | **P11** | **Location** | **P1** | **P11** |
| 211 | A | G | Threonine | Threonine | C31 | ACA | ACG |
| 1452 | A | G | Lysine | Arginine | E160 | AAG | AGG |

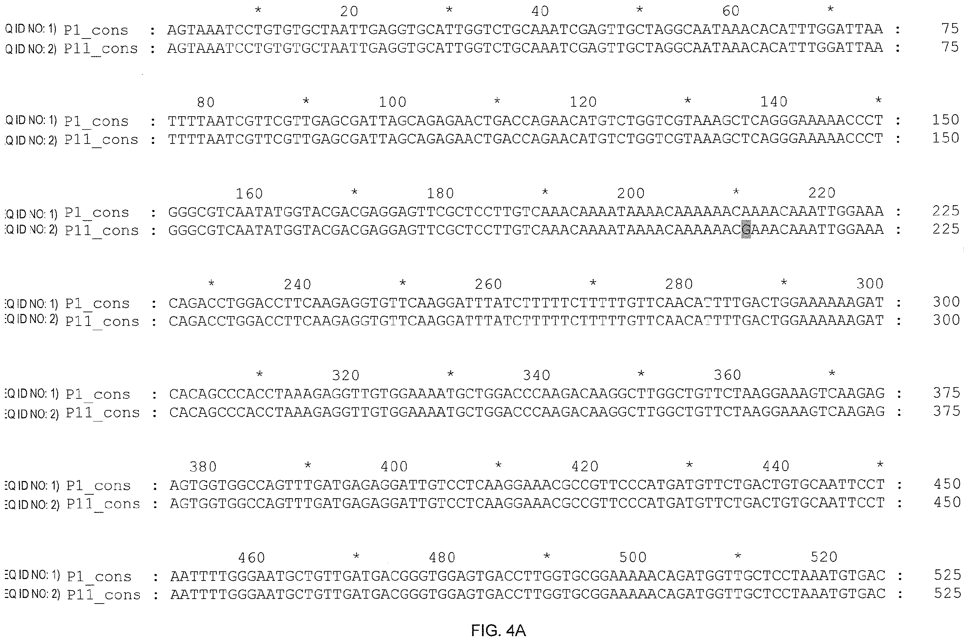

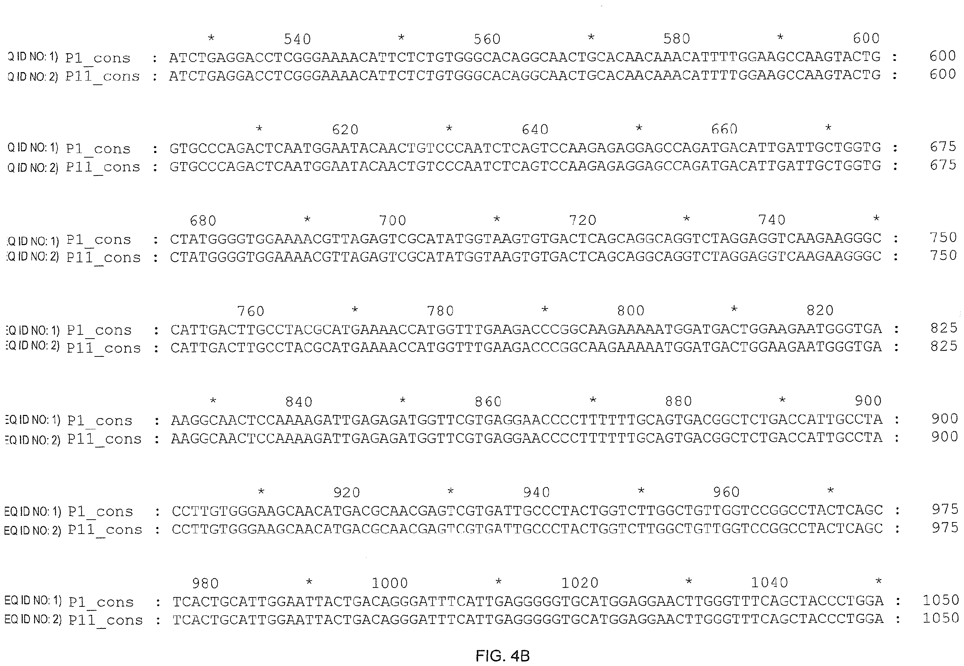

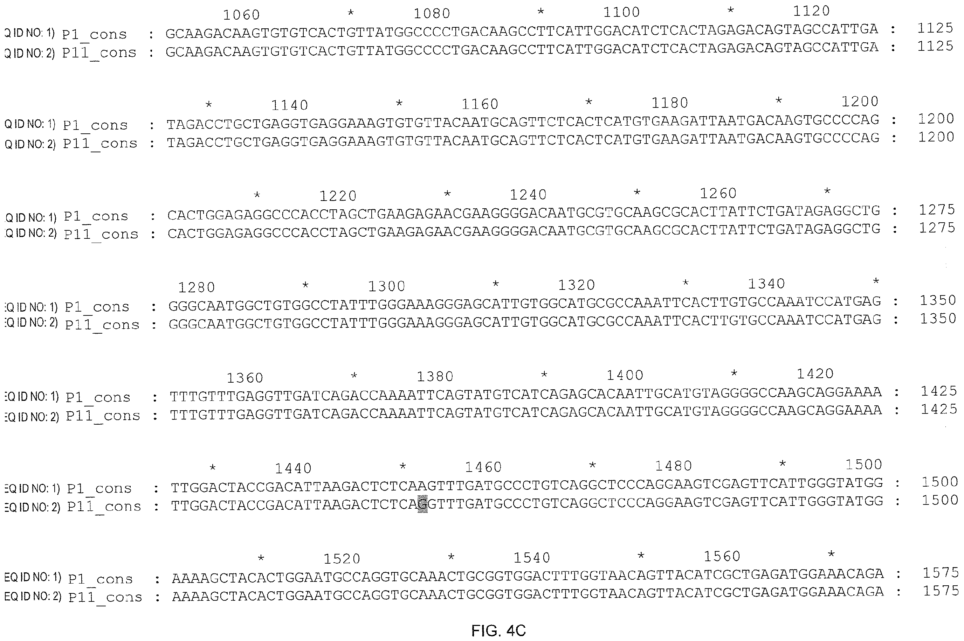

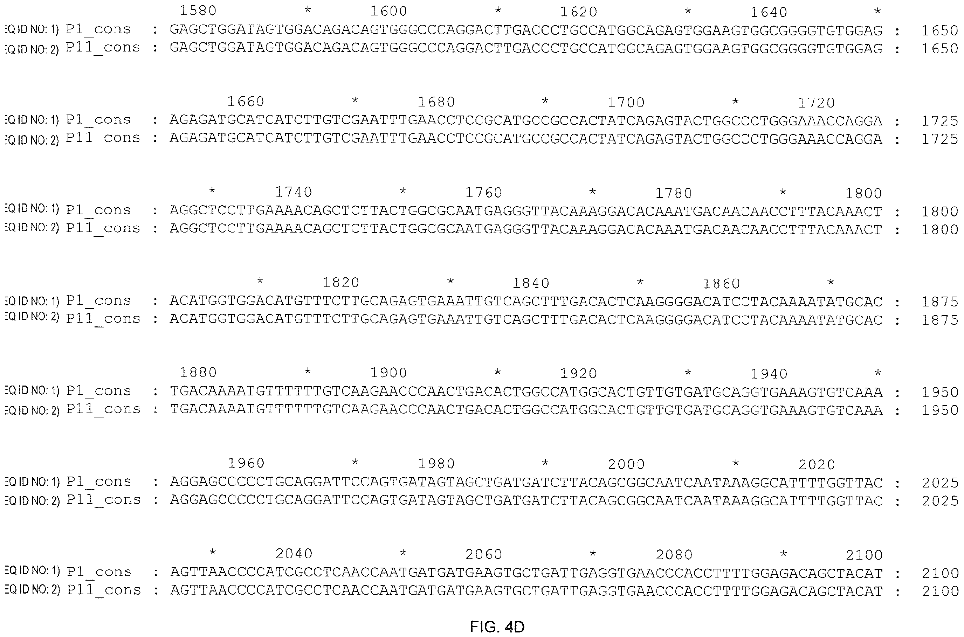

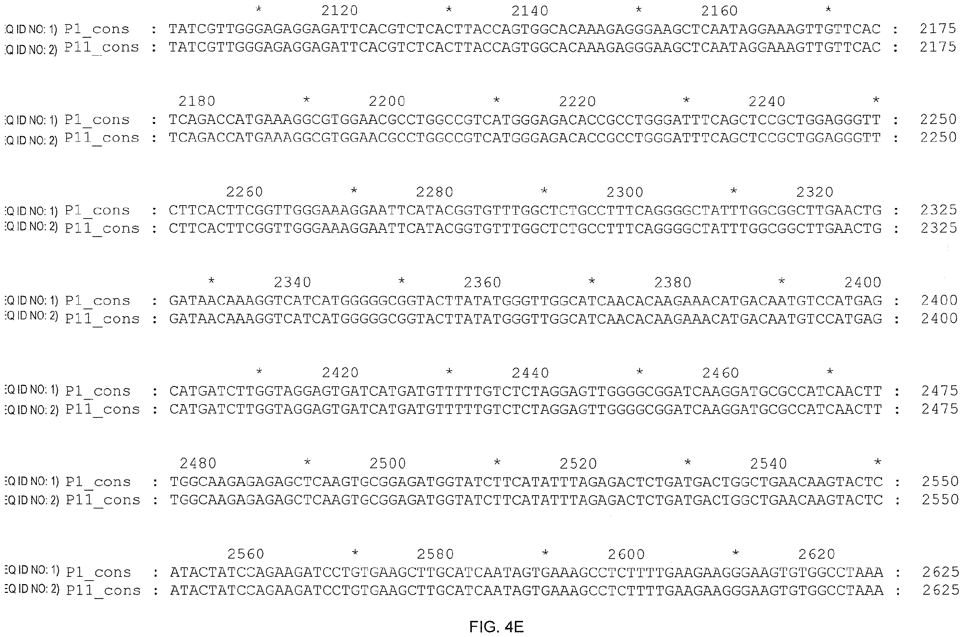

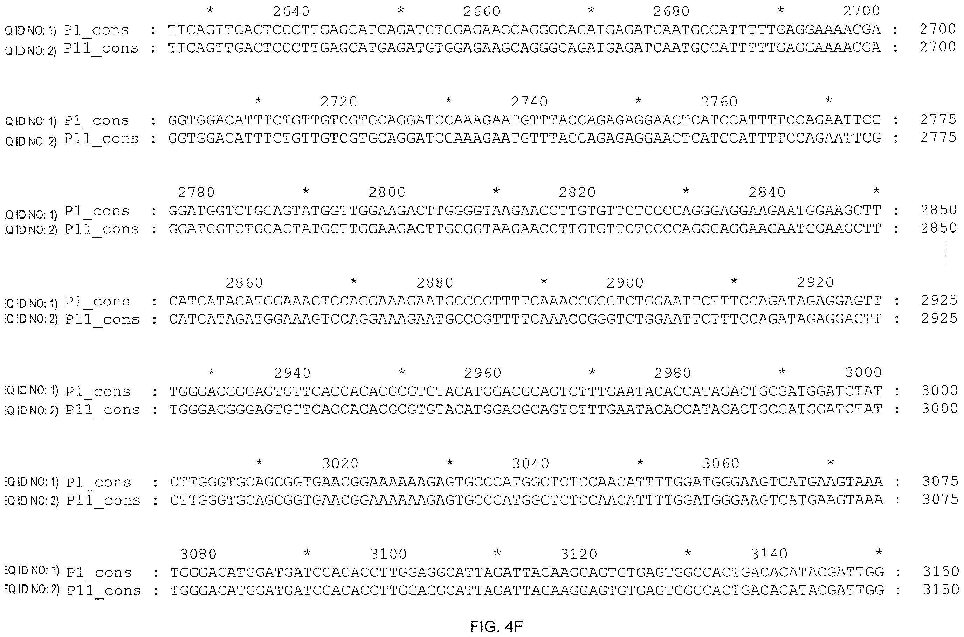

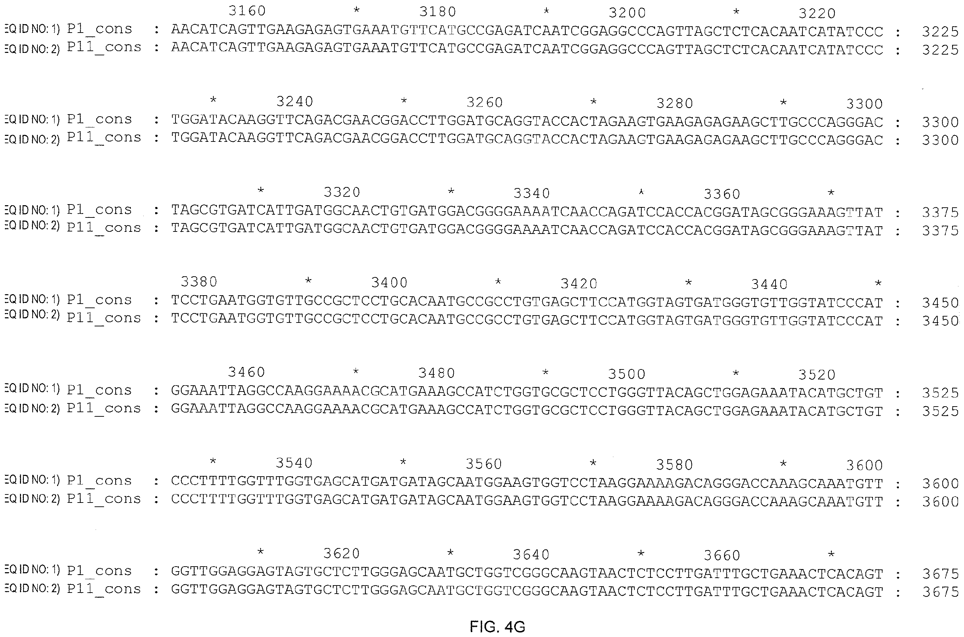

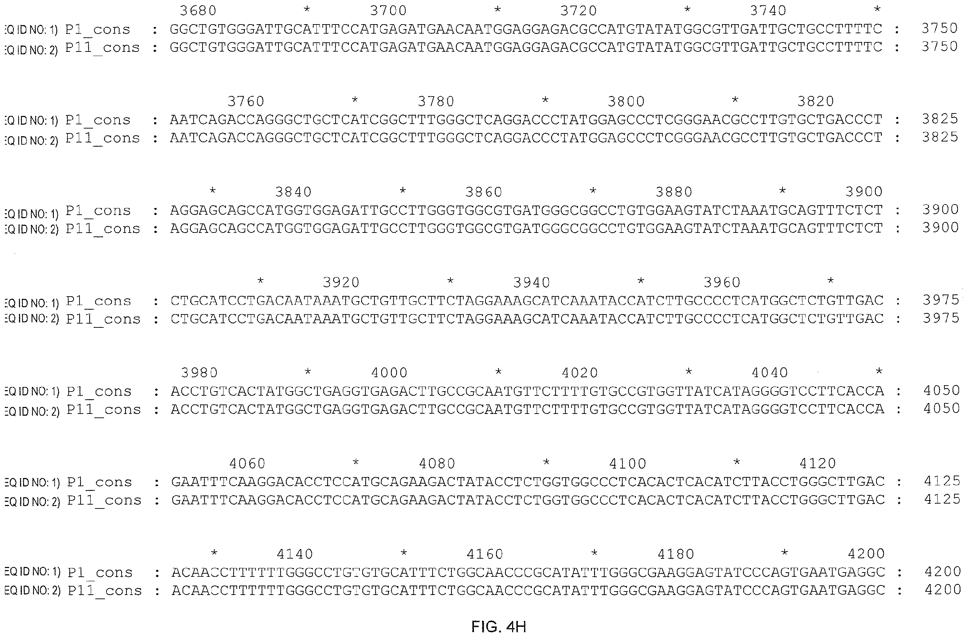

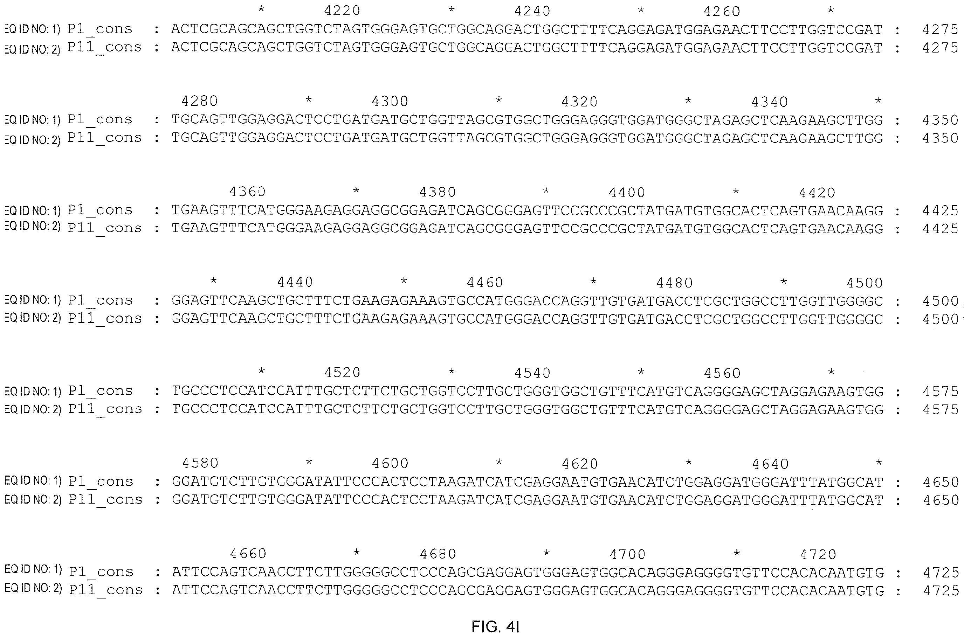

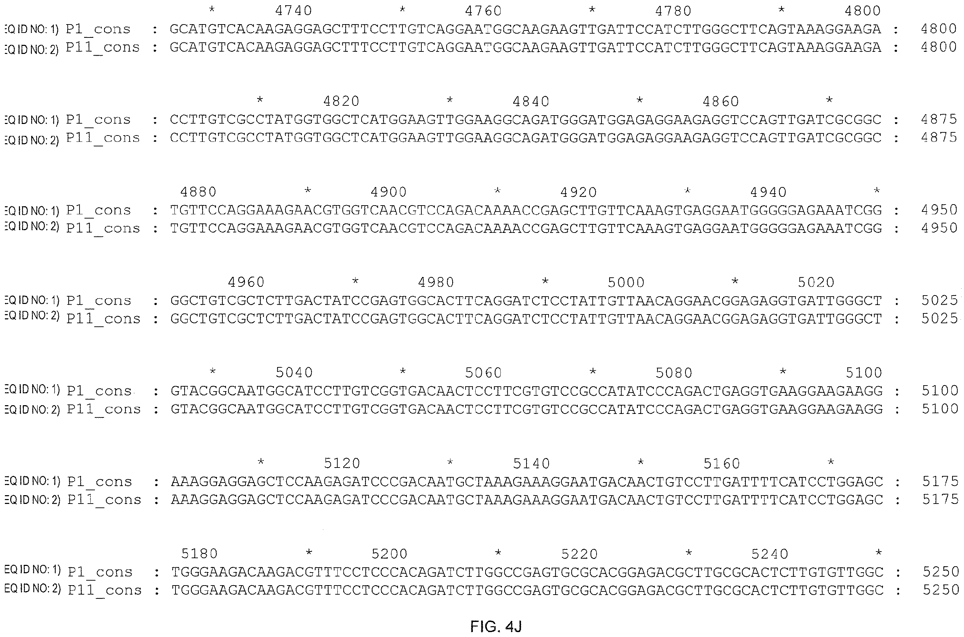

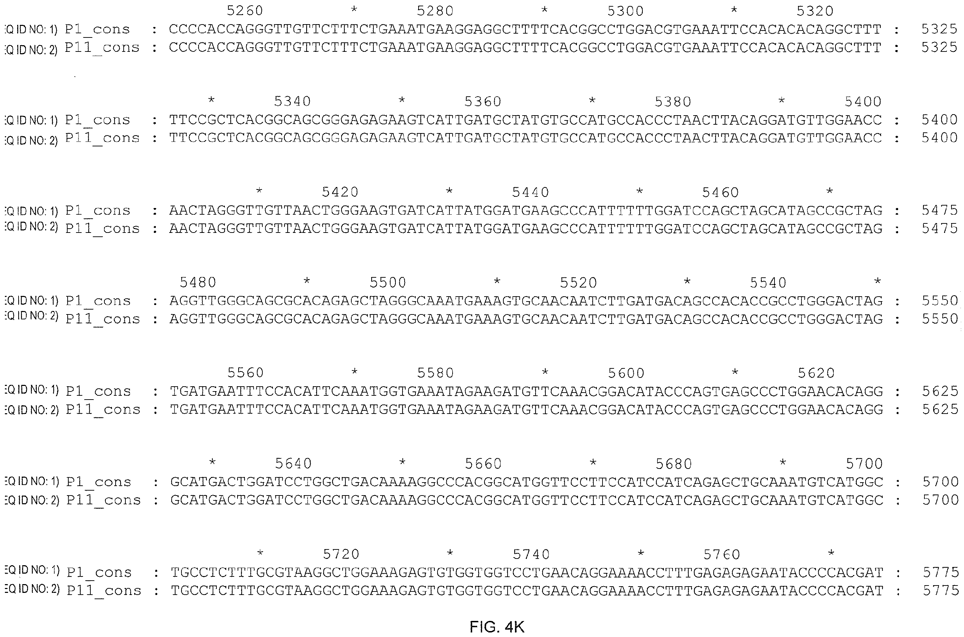

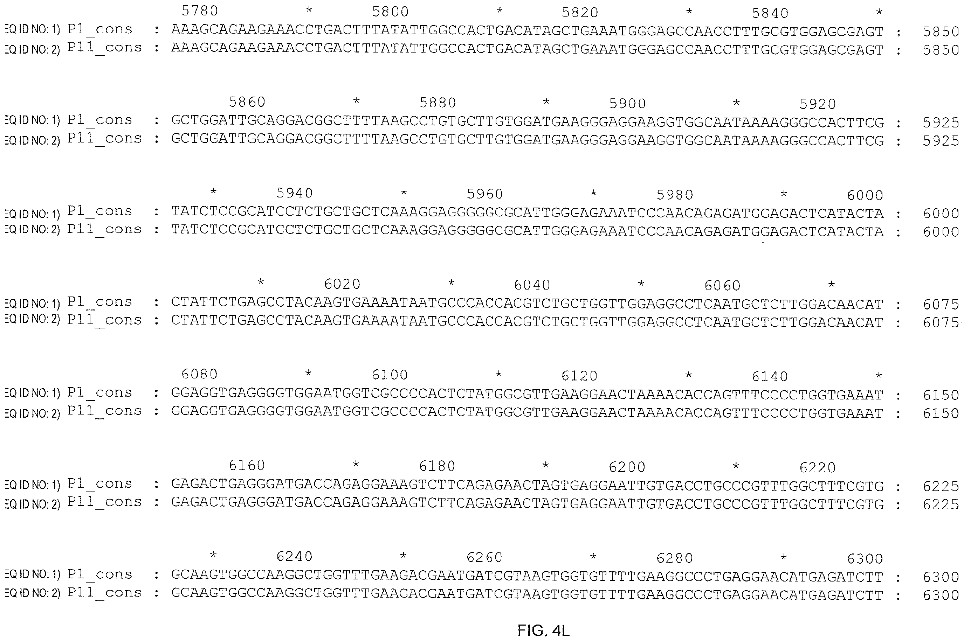

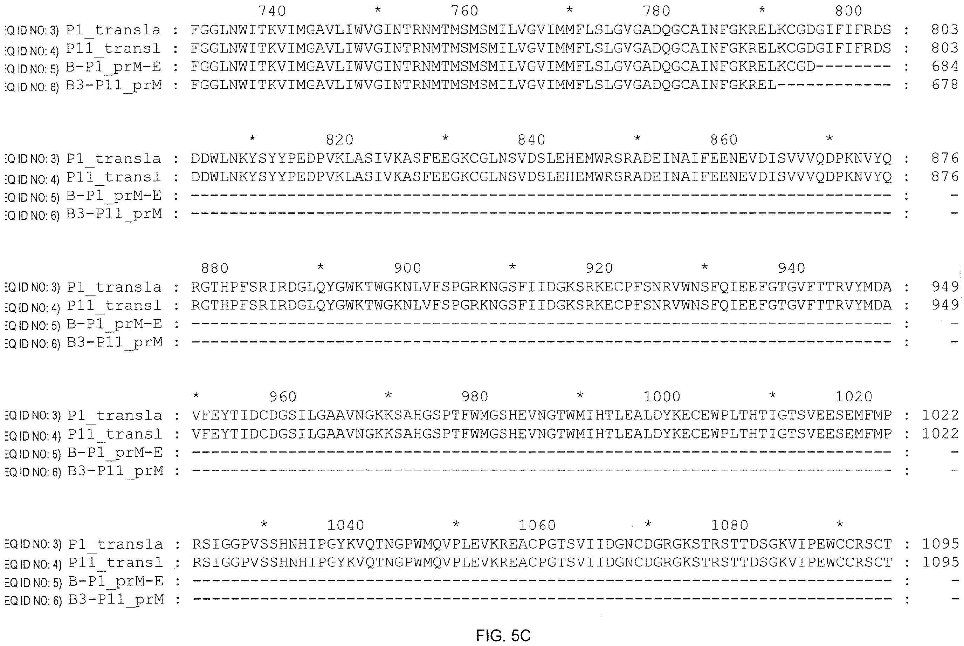

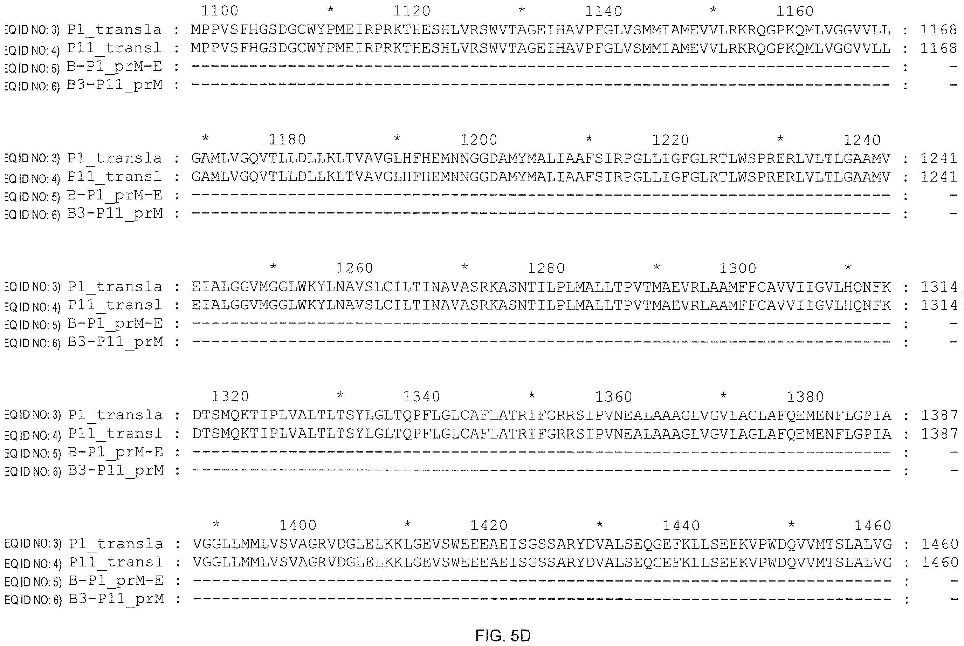

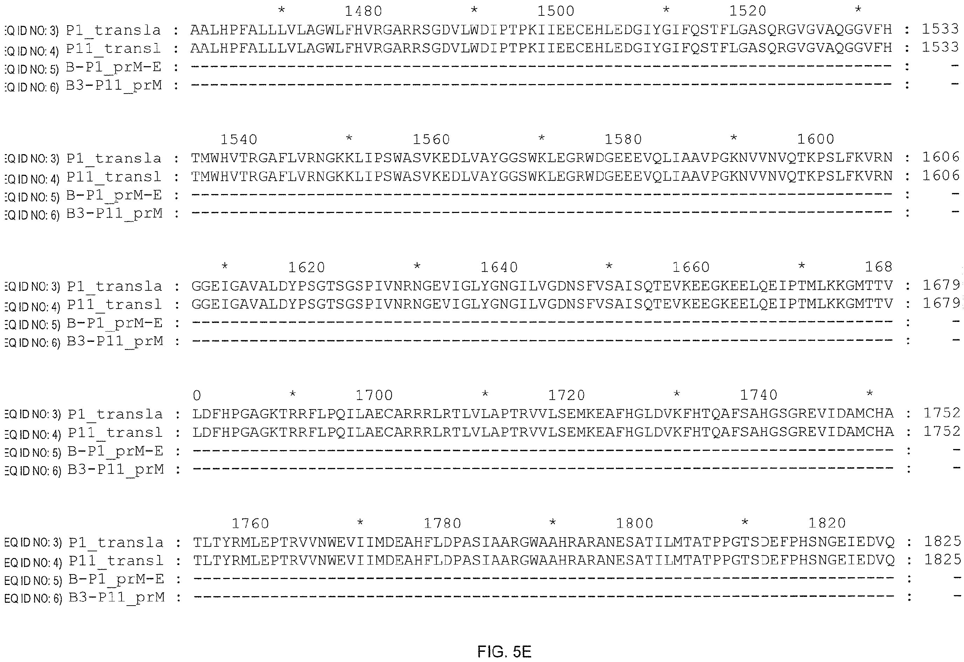

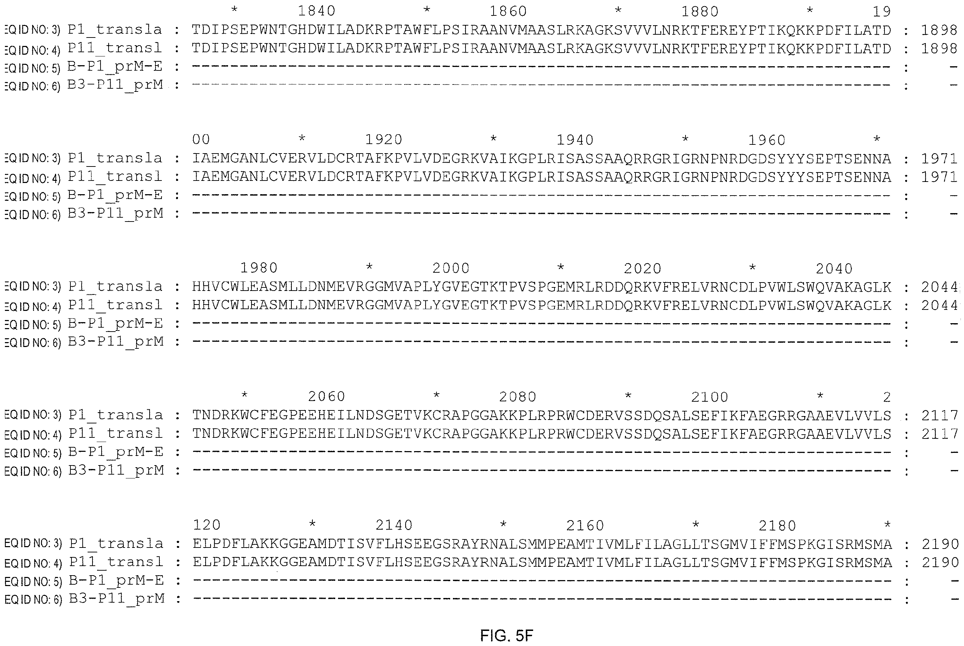

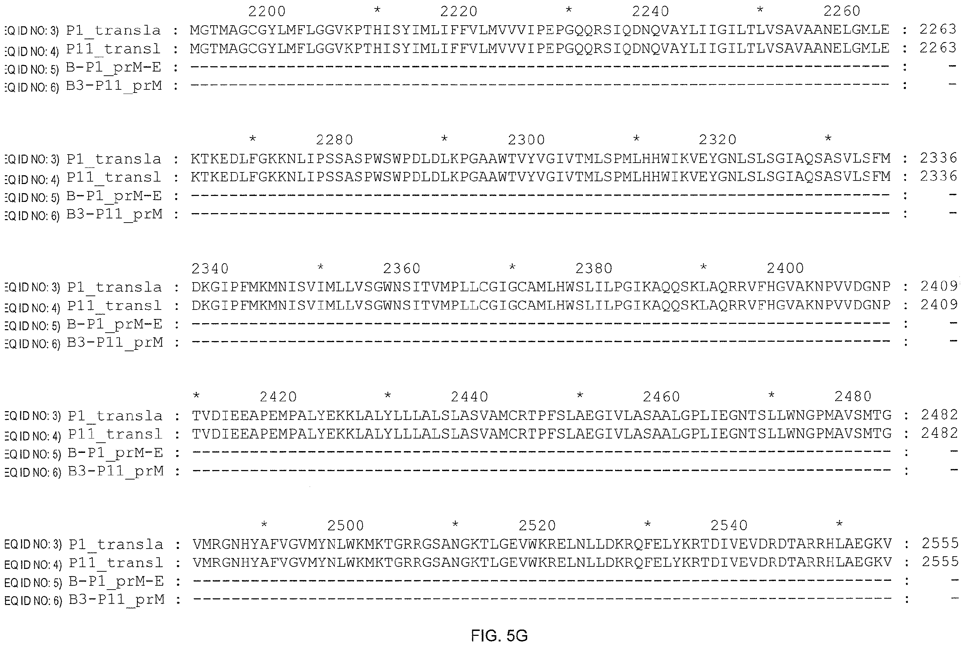

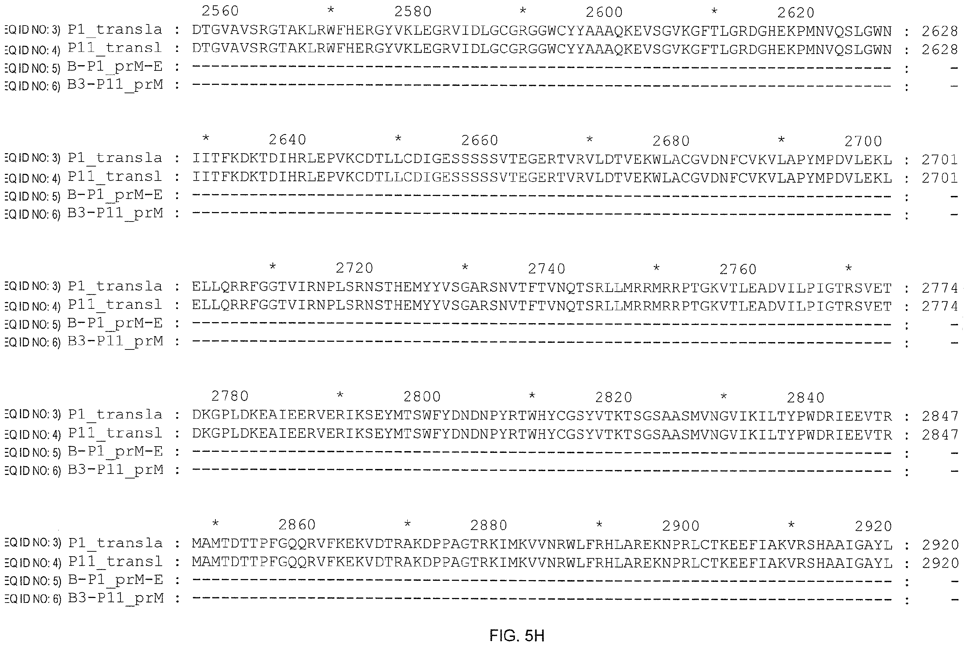

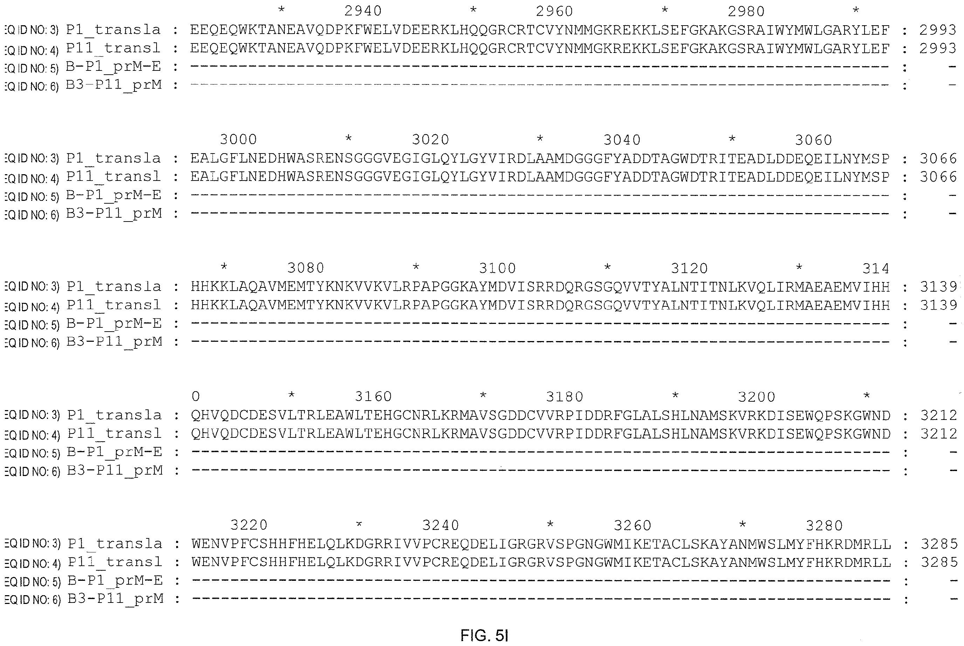

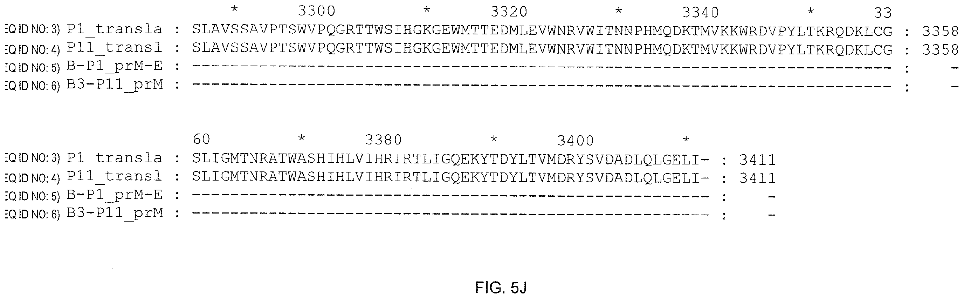

The first mutation was an A to G conversion at nucleotide residue #211, according to SEQ ID NO: 1, which resulted in a change in the codon for the amino acid at position 31 of the capsid protein (C31) from ACA to ACG. This mutation, however, did not change the amino acid residue at this position. The second mutation was an A to G conversion at nucleotide residue #1452, according to SEQ ID NO: 1, which resulted in a change in the codon for the amino acid at position 160 of the envelope protein (E160) from AAG to AGG. This mutation resulted in a Lysine to Arginine substitution at this position. A consensus alignment of the nucleic acid and amino acid sequences for P1 and P11 are depicted in Figures 4 and 5.

### Plaque Purification of P10 Harvest:

As described above, virus from P10 was purified by plaque formation. The virus isolated from one plaque was inoculated into a T 150 flask. The conditioned medium from this flask was harvested when 50 percent of the cells exhibited CPE. This material was aliquoted in one mL aliquots and designated P11. The P11 virus was then used as the source of RNA for transfection of Vero cells. The P11 titer of plaque forming units was determined to be 8.5 X 10⁷ plaque forming units (PFU). The RNA isolated from the P 11 virus was used to transfect cells to produce a Pre-Master Seed. The Pre-Master Seed virus was passaged in additional cultures of Vero cells to produce a Master and Working Virus Seed stock.

### Manufacture of Master Virus Seed:

The Master Virus Seed (MVS) was produced in Vero cells under serum-free conditions using a single vial of the Pre-Master Seed as the virus inoculum, as represented schematically in FIG. 2. Cells from the Manufacturer's Working Cell Bank (MWCB) of Vero cells at Passage 143 were expanded to eleven (11) 225 cm³ T-flasks. Once the cells became confluent, one flask was trypsinized and used to determine cell number and also to seed additional flasks used to produce the Working Virus Seed. The OptiPRO™ SFM medium was removed from the remaining 10 T-flasks and the cells were inoculated with Pre-Master Seed virus at a multiplicity of infection (MOI) ∼0.01 PFU/cell. The virus was allowed to adsorb for 60 (± 5) minutes at 37°± 2°C, after which pre-warmed OptiPRO™ SFM medium was added to the flasks. The infected culture was then incubated at 37°± 2°C with ∼5% CO₂.

After 3 days, when CPE was observed in ≥ 80% of the cell population, the virus propagation process was terminated by harvesting the cell culture fluid. The virus-containing culture fluid was pooled from all flasks, centrifuged to remove cell debris, and mixed with sterile 70% sorbitol to a final sorbitol concentration of 7%. This mixture was filled into 4 mL cryovials at 2 mL per vial and frozen at ≤ -60°C. The frozen virus stock constitutes the YF 17D MVS.

As shown in FIG. 2, the highest Vero cell passage level used for production of the MVS was 147.

### Manufacture of Working Virus Seed:

The Working Virus Seed (WVS) was produced as shown in FIG. 2, from a single vial of the MVS under cGMP conditions. Starting with cells in the 11th T225 flask used to determine the cell density in the production of MVS, four T225 flasks were seeded at a cell density 1 x 10⁶ viable cells per flasks, Passage 147. The cells were passaged into 4 new T225 flasks to allow time for the production of the Master Seed Stock. Cells at Passage 148 were then seeded into eleven T225 flasks for the production of the WVS.

When the cells were greater than 80% confluent, the cell density in one flask was determined. This cell density was used to estimate the cell density in the remaining ten flasks and the cells in the 10 flasks were infected with virus from the MVS at a MOI of 0.01 PFU/cell. To perform the infection, the medium was removed from the flasks and then diluted virus was added in phosphate buffered saline. After one hour fresh medium was added to each flask and the cells were returned to the incubator. The cells were observed microscopically for CPE. When CPE was greater than 80% the virus was harvested. The medium from the 10 flasks was centrifuged to remove cellular debris and the clarified supernatant was pooled into one vessel. Sorbitol (final concentration 7%) was added to the virus-containing supernatant as a cryo-preservative. The pooled virus was aliquoted into 4 mL cryovials, two mL per vial. The filled vials were stored at ≤-60°C. Once frozen, one vial from the end of the bank was tested in a plaque assay in Vero cells to determine the virus titer.

### Increase in Titer Achieved in P11 Compared to P1:

The original YF virus and P11 harvest of YF virus were titrated by plaque assay in Vero cells to determine the infectivity titers expressed as plaque forming units (PFU) (Table 1). The original YF-VAX 17D vaccine contained 10^{3.7} log₁₀ per ml in Vero cells. The peak titer for passage one was 6.68 log₁₀ per ml and remained at about the same titer through P6 and then increased significantly to 7.67 log₁₀ by P10. Thus, in this experiment, there was a 1.0 log₁₀ (10-fold) increase in the titer of the passage 10 (7.67 log₁₀) over the titer (6.68 log₁₀) of the P1 virus (see Table 2).

Virus growth curves were also performed concurrently on the P1 and P11 viruses. Growth curves was performed by infecting duplicate 75 cm² flasks of Vero cells at high MOI of 1.0 and a second growth curve was performed using a low MOI of 0.001. At high MOI it is expected that all cells are infected at initiation of the culture, while at low MOI, virus released by a small number of cells initially infected would infect the remaining cells of the culture; thus, virus in a low-MOI growth curve would be expected to be somewhat delayed compared to a high-MOI culture. At times 0, 6, 18, 24, 30, 48, 54 and 72 hr post inoculation, conditioned medium (2mL) was removed from the cultures, stabilized with 2% HSA and frozen (duplicate one ml samples) at - 80°C. Log₁₀ dilutions of each sample were tested in Vero cells to determine the infectivity titer and the growth curves were plotted over time.

**Table 2. Peak infectivity titer for each sequential passage of YF virus**

| Passages of YF-VAX in Vero cells | Highest Dilutions yielding plaques | Average # of plaques | Peak infectivity titer (PFU/ml) | Conversion of plaque forming units to equal the infectivity titer in log₁₀ PFU per ml |
|---|---|---|---|---|
| 0 | 10⁻³ | 1 | 5 X 10³ | 3.7 |
| 1 | 10⁻⁵ | 9.67 | 4.83 X 10⁶ | 6.68 |
| 2 | 10⁻⁵ | 12.67 | 6.33 X 10⁶ | 6.80 |
| 3 | 10⁻⁴ | 21.67 | 1.08 X 10⁶ | 6.03 |
| 4 | 10⁻⁵ | 12.67 | 6.33 X 10⁶ | 6.80 |
| 6 | 10⁻⁶ | 1.00 | 5.00 X 10⁶ | 6.70 |
| 8 | 10⁻⁶ | 3.00 | 1.50 X 10⁷ | 7.18 |
| 9 | 10⁻⁶ | 5.67 | 2.83 X 10⁷ | 7.45 |
| 10 | 10⁻⁶ | 9.33 | 4.67 X 10⁷ | 7.67 |

The growth curve results using an MOI of 1.0 indicated that the P1 YF virus increased from a titer of 4.09 log₁₀ at 0 hours; or at the time of inoculation to a maximum titer of 6.28 log₁₀ at 48 hours post inoculation (PI) and the titers showed a slight decrease of 6.21 and 6.18 log₁₀ at 60 and 72 hours PI, respectively. The results for passage 11 (P11) showed an increase in titers over the passage one virus (P1). At the time of inoculation, the titer was 4.15 log₁₀ and reached a maximum titer of 6.83 log₁₀ at 48 hours P.I. and had decreased to a titer of 6.54 log₁₀ at 72 hours P.I (see Table 3). The peak virus titer at approximately 48 hours for the P11 virus was 0.55 log₁₀ or 3.5 times higher than for the P1 virus.

**Table 3. Growth curve of Yellow Fever 17D Passage 1 and Passage 11 virus at high MOI (1.0)**

| Time points (hr) | **0** | **6** | **18** | **24** | **30** | **48** | **54** | **72** |
|---|---|---|---|---|---|---|---|---|
| Passage 1 | 4.15 | 4.11 | 5.63 | 6.09 | 6.05 | 6.28 | 6.21 | 6.18 |
| Passage 11 | 4.09 | 4.22 | 5.60 | 6.27 | 6.63 | 6.83 | 6.68 | 6.54 |
| P1 STDEV | 0.11 | 0.02 | 0.17 | 0.08 | 0.05 | 0.03 | 0.05 | 0.04 |
| P11 STDEV | 0.04 | 0.08 | 0.21 | 0.02 | 0.10 | 0.14 | 0.18 | 0.10 |

As compared to the growth curve using high MOI, the pattern of the growth curve using an MOI of 0.001 showed a lag in replication but maximum titers were higher. At the time of inoculation, the titers were 1.7 and 0.57 log₁₀ for the passage 1 and 11, respectively. There was a linear increase in titers and by 72 hours PI, maximum titers of 7.35 and 8.17 log₁₀ had been attained by P1 and P11, respectively. The peak virus titer at approximately 72 hours for the P11 virus was 0.82 log₁₀ or 6.6 times higher than for the P1 virus. These results indicated that the serial passage of YF-VAX produced a substantial increase in titer and that this approach appears to be promising for developing an inactivated YF vaccine (see Table 4).

**Table 4. Growth curve of Yellow Fever 17D Passage 1 and Passage 11 virus at low MOI (0.001)**

| Time points (hr) | **0** | **6** | **18** | **24** | **30** | **48** | **54** | **72** |
|---|---|---|---|---|---|---|---|---|
| Passage 1 | 1.70 | 2.00 | 2.57 | 4.14 | 4.72 | 6.44 | 7.19 | 7.35 |
| Passage 11 | 0.57 | 0.67 | 3.01 | 4.44 | 5.18 | 7.04 | 7.38 | 8.17 |
| P1 STDEV | 0.00 | 0.30 | 0.19 | 0.07 | 0.06 | 0.04 | 0.02 | 0.03 |
| P11 STDEV | 0.98 | 1.15 | 0.14 | 0.05 | 0.05 | 0.02 | 0.10 | 0.10 |

These results indicated that the serial passage of the YF virus produced a substantial increase in titer. Next, as described above, the sequence analysis of P1 and P11 was performed, the comparative results of which show that the serial passages may have resulted in two genetic mutations in the YF virus, one of which resulted in an amino acid change.

The disclosed modified YF virus produced by the serial passage of the attenuated YF 17D virus vaccine in certified African green monkey kidney cells (Vero) showed enhanced productivity in cells. The methods of the invention involve vaccination of subjects with the modified, inactivated YF virus to produce immunity to Yellow Fever.

### Vaccine Production in Bioreactors:

Bioreactors containing approximately 5 g/L of Cytodex 1 microcarriers were seeded with approximately 5 x 10⁵ Vero cells/mL in OptiPRO™ SFM medium. The cells were allowed to propagate for 3 to 4 days until cells attached to the microcarriers achieved a density of ≥ 7 x 10⁵ nuclei per mL. For virus inoculation, the agitation and parameter controls are turned off and the microcarriers and cells are allowed to settle. Approximately 75% of the medium volume was removed through a 90 µm sieve tube which is designed to retain microcarriers in the reactor. WVS virus is introduced at a MOI of ∼0.01 PFU/cell. Low agitation was applied at this low volume for about 1 hour to allow virus to adsorb to and infect cells. Fresh medium was added to the full volume before agitation and parameter controls are returned to their original settings. On day 3 or 4 post infection, 75% of the conditioned medium was removed, and the reactor was re-fed with fresh medium. The culture was allowed to proceed for 2 or 3 more days and on Day 5, 6, or 7 post infection the conditioned medium was harvested. To ensure biosafety, harvest samples were taken from the bioreactor immediately before microcarrier removal and tested for sterility, mycoplasma, retroviruses and adventitious viruses (*in vitro* assay).

The reactor mixing was stopped to allow for settling of the microcarriers. The culture is transferred from the bioreactor through a 90 µm sieve tube into a bioprocess bag. The 90 µm sieve reduces the amount of microcarriers and large particulates from transferring into the harvest. This was the Virus Harvest. The Virus Harvest was sampled and tested for infectivity, potency, identity, endotoxin, sterility, residual Vero cell DNA, and residual Vero cell proteins.

### Virus Purification and Inactivation:

The culture conditioned medium was harvested, clarified in two steps, digested with BENZONASE®, purified by ultrafiltration and diafiltration and then sterile filtered to generate the Live Virus Bulk. The Live Virus Bulk was then inactivated by treatment with β-propiolactone (BPL) which permeates the virus envelope and disrupts the viral RNA by alkylating purine residues, rendering the virus inactive. The inactivated virus is further purified by cellufine sulfate column chromatography and diluted to the desired viral concentration to form the Bulk Vaccine Drug Substance.

### Repeat of YFV 17D Passaging Study:

Experiments were performed to repeat the passage of YF virus from unpassaged virus stock through P11 using similar techniques as in the original passage series.

### Preparation of the Virus Stocks:

Vero cells were maintained under serum-free conditions throughout the study, using OptiPRO SFM.

The initial source of the YFV 17D virus was from a single vial of YF-VAX® (Sanofi Pasteur, Swiftwater PA). The vial was originally reconstituted and dispensed into aliquots. One of these aliquots was used for the repeat experiments. The repeat serial passaging was performed in duplicate such that there were two runs of the study, performed in parallel, referred to here as series B and C.

At each passage of the virus, the virus sample was diluted in serial 10-fold dilutions, and the diluted virus was used to inoculate Vero cells seeded in 12 well plates. The serial dilutions performed at each passage were inoculated in duplicate such that one set of plates was used for the preparing the next passage of virus, inoculating 4 wells per dilution, and the other set of plates was used to determine the titer of the passaged virus, inoculating 2 wells per dilution.

For the serial passages of the virus, the dilution selected for passaging the virus was the last dilution where generalized cytopathic effect (CPE) was observed, three to four days after infection. The media from the four wells was pooled for the next passage. The titer of the virus was determined by plaque assay using an immunostain to visualize and count the plaques. The immunostain method allowed for determining the titer after 3 days of infection.

For the initial passage of the virus, 0.3 ml of the YF-VAX aliquot was diluted into 3 mL final, using OptiPRO SFM, for a 10⁻¹ dilution. The diluted virus was divided equally into three aliquots. From each of these aliquots, serial 10-fold dilutions were made to 10⁻⁵, making two dilution series (B and C). This is referred to here as the P0 → P1 passage. From the plaque assay inoculated using the dilution series, of the P0 virus was determined, and for the plates inoculated for passage, the P1 virus was generated. Each round of the passaging is summarized in the Table 5.

**Table 5: Serial Passages of YFV 17D (Results same for series B and C)**

| Passage | Dilutions plated | Dilution harvested for next passage |
|---|---|---|
| P0 (initial vial) | N/A | N/A |
| P0 → P1 | 10⁻¹ to 10⁻⁵ | 10⁻³ |
| P1 → P2 | 10⁻² to 10⁻⁷ | 10⁻⁵ |
| P2 → P3 | 10⁻³ to 10⁻⁸ | 10⁻⁵ |
| P3 →P4 | 10⁻³ to 10⁻⁹ | 10⁻⁵ |
| P4 →P5 | 10⁻³ to 10⁻⁹ | 10⁻⁵ |
| P5 →P6 | 10⁻³ to 10⁻⁹ | 10⁻⁴ |
| P6 →P7 | 10⁻³ to 10⁻⁹ | 10⁻⁵ |
| P7 →P8 | 10⁻³ to 10⁻⁷ | 10⁻⁴ |
| P8→P9 | 10⁻³ to 10⁻⁷ | 10⁻⁵ |
| P9 →P10 | 10⁻³ to 10⁻⁷ | 10⁻⁵ |

The passaging was repeated for 10 serial passages of the virus. Once the virus was harvested from the last passage, the titers were generated for the P10 virus from each series. The P10 viruses were then diluted for inoculating cells such that only one plaque per well would develop after inoculation. Well-isolated plaques could then be picked from the wells. From the B series, six well-isolated plaques were picked, and from C, two were picked. The picked plaques were used to inoculate T25 flasks to generate the P1 1 virus stocks for growth curve studies.

### Growth curve analysis:

For the growth curve studies, the P1 stock virus from each series was compared to the P11 stocks for each series. Since the volume of the P1 stocks would have been limiting for this, an aliquot of P1 virus from each series was diluted threefold, then aliquots made from the diluted virus to generate P1 stocks for the growth curve. For the P11 stocks, three stocks from the B series were analyzed, and the two from the C series. Prior to the growth curve studies, aliquots of the virus stocks were assayed to confirm the level of infectivity.

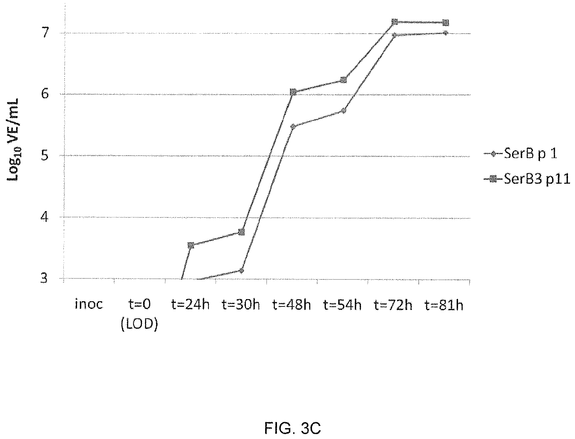

The growth curve analysis was performed by infecting Vero cells in T25 flasks, at low MOI of 0.001 PFU/cell. The study was conducted under serum free conditions using OptiPRO SFM as the culture medium. After diluting the virus stocks to achieve the target 0.001 MOI, a sample was reserved to confirm the titer of the inoculum. The virus inocula were allowed to adsorb to the cells for approximately one hour. After adsorption, the monolayers were washed three times then the cultures were fed with 8 ml of medium. At each time point, 1 mL was removed from each culture, and 1 mL fresh media was added back. The reserved one mL of medium was clarified by centrifugation and stored at -80°C in the presence of sorbitol, until ready to assay. The time points for which samples were taken were 0, 24, 30, 48, 54, 72, and 81 hours after infection. A plaque assay was performed on all samples. The results of the study are detailed in Figure 3C.

For both the B and C series, there was one P11 virus stock that was shown to replicate to higher titers than the P1 virus stock from the series. The P1 stocks for both B and C, and the B3-P11 stock and the C1-P11 stock were selected for sequence analysis. The sequence analysis illustrates that the B3 stock enjoys the same Lys→Arg mutation at E160 as was observed in the original passage series, as well as a Threonine (Thr) → Isoleucine (Ile) mutation at amino acid position 317 in non-structural protein 1 (NS1-317), and a Phenylalanine (Phe) → Leucine (Leu) mutation at amino acid position 170 in non-structural protein 2A (NS2A-170). While, the C1 stock did not carry the same mutation in the E gene, further study of the C1 stock genome is ongoing, and has revealed a mutation at amino acid position 113 in non-structural protein NS4B (NS4B-113).

Table 6 summarizes the nucleotide and amino acid changes found in the modified Yellow Fever viruses obtained from the original and repeat passage studies.

**Table 6:**

| Nucleotide and amino acid changes in the consensus sequence between passage 1 (P1) and P11 in three separate passage series of yellow fever 17D vaccine (YF-VAX®) in Vero cells. The position of the altered nucleotide or amino acid in the designated viral protein (or non-coding region, NCR) is shown. Some nucleotide changes were silent (did not result in corresponding amino acid mutations). | | | | | | |
|---|---|---|---|---|---|---|
| **Protein** | **Original passage series** | | **Repeat Series B** | | **Repeat Series C** | |
| | **Nucleotide** | **Amino acid** | **Nucleotide** | **Amino acid** | **Nucleotide** | **Amino acid** |
| 5'NCR | | | | | | |
| prM | | | | | | |
| E | 211 A→G | | 211 A→G | | | |
| | 1452 A→G | 160 K→R | 1452 A→G | 160 K→R | | |
| | | | 1507 T→C | | | |
| | | | | | 1897 G→A | |
| NS1 | | | 3402 C→T | 317 T→1 | | |
| NS2a | | | 4016 T→C | 170 F→L | | |
| NS2b | | | | | | |
| NS3 | | | | | | |
| NS4a | | | | | | |
| NS4b | | | | | 7225 A→G | 113 I→M |
| NS5 | | | | | | |
| 3'NCR | | | 9343 G→A | | | |
| | | | | | 9670 C→T | |

A Yellow Fever viral strain was produced to develop a safer, inactivated, non-replicating vaccine that will elicit a neutralizing antibody response while eliminating the potential for neurotropic and viscerotropic adverse events for the prevention of human disease. Additional Yellow Fever virus strains are produced to develop safer, inactivated, non-replicating vaccines that will elicit a neutralizing antibody response while eliminating the potential for neurotropic and viscerotropic adverse events for the prevention of human disease.

The cells are selected from Vero cells. Other cells suitable for propagation of the Yellow Fever virus may be utilized, including but not limited to, primary chick embryo, primary duck embryo, primary dog kidney, primary rabbit kidney, WI-38, MRC-5, or fetal rhesus lung.

The chemically inactivated viral vaccines can be administered and formulated, for example, as a sterile aqueous solution containing between 10² and 10⁸, e.g., or between 10⁶ and 10⁷, inactivated equivalents of infectious units (e.g., plaque-forming units (PFU) or tissue culture infectious doses) in a dose volume of from about 0.1 to about 1.0 ml, or about 0.5 ml. to be administered by, for example, subcutaneous, intramuscular, epidermal, or intradermal routes. In addition, in an appropriate formulation, a mucosal route, such as the intranasal oral route, can be selected. Selection of an appropriate amount of virus to administer can be determined by those of skill in this art, and this amount can vary due to numerous factors, e.g., the size and general health of the subject to whom the virus is to be administered. The subject can be vaccinated a single time or, if necessary, follow-up immunization can take place.

As is noted above, the vaccines can be administered as primary prophylactic agents to a subject that is at risk of Yellow Fever virus infection. Also, although not required, adjuvants can be used to enhance the immunogenicity of the Yellow Fever virus vaccines. Selection of appropriate adjuvants can readily be carried out by those of skill in this art.

Also as is noted above, the live virus can be inactivated by treatment with β-propiolactone (BPL), rendering the virus inactive. Other suitable methods of virus inactivation include, but are not limited to, formalin, ultraviolet radiation, ethylenimine, acetylethylenimine, and binary ethylenimine.

### EXEMPLIFICATION

The examples below are intended to further illustrate certain preferred embodiments of the invention.

### Antibody Responses in Mice:

The neutralizing antibody responses in female, outbred BALB/c and CD-1 mice after immunization with inactivated yellow fever vaccine compared to live virus was assessed. Yellow fever (YF) virus was inactivated with beta propiolactone (BPL), formulated with alum adjuvant and injected by the intramuscular route as two or three doses, each separated by 14 days. Two dose levels of virus were tested in BALB/c mice, the high dose level only was tested in CD1 mice. Sera taken at 14 days after the last immunization were tested for neutralizing antibody activity.

### Preimmunization Procedures:

Female BALB/c and CD-1 strain mice (6 weeks of age) were acclimated in designated isolators in a restricted virus animal facility. Serum sample were collected Study Day 28 or 42 upon sacrifice. Mice were housed at 5 mice per cage and each animal was uniquely identified on the cage cards, and by ear notch. Mice were acclimated for a week prior to the initiation of any treatments. Mice received sterilized food and water and were housed in sterilized polycarbonate cages with sterilized bedding with a 12-hour light cycle (on at 6am and off at 6 pm). General health was evaluated by technical staff daily and by a veterinarian weekly and as needed for health issues. Body weights were collected on Day 0 prior to immunization and on Day 28 and 42.

### Immunization Procedure:

Body weight was determined on Day 0 prior to immunization. Immunization was given by either the *i*.*m*. (alum formulations) or *s*.*c*. (live virus or inactivated vaccine with Freund's adjuvant) route. Injections were given with mice under light anesthesia with suboptimal dose of ketamine/xylazine mixture. For *s*.*c*. route with live virus, a volume of 100 µl of vaccine in a 1 ml syringe fitted with a 27 gauge needle is injected between the skin and underlying layers of tissue in the scapular region on the backs of mice. For *i*.*m*. administration, a volume of 100 µl of vaccine in a 0.5 ml insulin syringe is injected into the muscle bundles of 2 rear upper legs of mice (50 µl/leg).

### Sacrifice:

Mice were sacrificed 28 or 42 days after the first vaccination. Body weight was determined on all mice on Study Day 28 and prior to sacrifice. Blood was collected for neutralizing antibody testing. Blood (0.7-1.0 ml) was removed by cardiac puncture from mice anesthetized with light ketamine/xylazine treatment before they are humanely terminated by ketamine/xylazine overdose.

### Experimental design:

Alum-formulated vaccine prepared the day prior to immunization as a suspension and the vaccine was well mixed prior to filling each syringe. Alum-formulated preparations were administered by the *i*.*m*. route, a volume of 100 µl of vaccine in a 0.5 ml insulin syringe was injected into the muscle bundles of 2 rear upper legs of mice (50 µl/leg).

Live Yellow Fever (YF) vaccine was reconstituted with 0.6 ml of saline to a virus concentration of approximately 1.1 x 10⁵ pfu/ml. A dose of 1 x 10⁴ PFU (i.e. 1/10^{th} the human dose) was delivered in a volume of 100 µl of sterile saline administered on day 0 *s*.*c*.

Freund's adjuvanted vaccine was formulated the day of vaccination by placing 2 ml of antigen solution into a glass syringe and 2 ml of the adjuvant into another glass syringe. The syringes were connected through the luer fitting to the 3-way valve. The plunger from the antigen solution was carefully depressed first, pushing the antigen into the oil of the adjuvant. The plungers were alternately pushed, to mix the adjuvant and the antigen solution into an emulsion (approximately 8 to 10 minutes). A 0.5 ml volume was delivered *s*.*c*. between the skin and underlying layers of tissue in the scapular region on the backs of mice (Formulation with Freund's adjuvant).

Live Yellow Fever (YF Vax™) vaccine was reconstituted with 0.6 ml of supplied saline to a virus concentration of approximately 1.1 x 10⁵ PFU/ml.

The inactivated whole virion vaccine adsorbed to 0.2% aluminum hydroxide ("alum") adjuvant was prepared no more than 2 weeks prior to day of dosing.

### Preliminary Mouse Studies

Groups of 5 mice each were dosed with as outlined in Table 7. Serum samples were collected by cardiac puncture 14 or 28 days post last vaccination.

**Table 7**

| **Group** | **# Mice** | **Strain** | **Vaccine** (Volume = 0.1 ml) | **Route** | **Vaccination schedule** | **Neut. Ab** |
|---|---|---|---|---|---|---|
| 1 | 5 | BALB/c | 10⁸ BPL-inactivated in 0.2% alum | IM | Day 0, 14 | Day 28 |
| 2 | 5 | BALB/c | 10⁸ BPL-inactivated in 0.2% alum | IM | Day 0, 14, 28 | Day 42 |
| 3 | 5 | BALB/c | 10⁷ BPL-inactivated in 0.2% alum | IM | Day 0, 14 | Day 28 |
| 4 | 5 | BALB/c | 10⁷ BPL-inactivated in 0.2% alum | IM | Day 0, 14, 28 | Day 42 |
| 5 | 5 | BALB/c | 10⁸ BPL-inactivated in Freund's complete/incomplete | SC | Day 0, 14, 28 | Day 42 |
| 6 | 5 | BALB/c | 10⁷ BPL-inactivated Freund's complete/incomplete | SC | Day 0, 14, 28 | Day 42 |
| 7 | 5 | BALB/c | 10⁸ BPL-inactivated no adjuvant | IM | Day 0, 14, 28 | Day 42 |
| 8 | 5 | BALB/c | Live YF Vax® | sc | Day 0 | Day 28 |
| 9 | 5 | CD1 | 10⁸ BPL-inactivated in 0.2% alum | IM | Day 0, 14 | Day 28 |
| 10 | 5 | CD1 | 10⁸ BPL-inactivated in 0.2% alum | IM | Day 0, 14, 28 | Day 42 |
| 11 | 5 | BALB/c | 0.2% alum | IM | Day 0, 14, 28 | Day 42 |

Plaque reduction neutralization activity in mouse sera

Plaque reduction neutralization test was performed using a dilution of 17D virus which, in the absence of neutralization, produces 10-40 plaque forming units per well in 12 well plates. An equal volume of serially diluted mouse serum was incubated with virus for 16-20 h at 4°C and then the inoculated into duplicate wells of Vero cells in 12 well plates. After virus absorption for 60 minutes at 37°C, the wells are overlaid with medium containing 0.75% methylcellulose, incubated for 4 days at 37°C, fixed and stained with crystal violet and plaques counted using a stereomicroscope over light box. The 50% plaque reduction titer represents the final mouse serum dilution resulting in less than 50% of the average plaque counts when no serum is added.

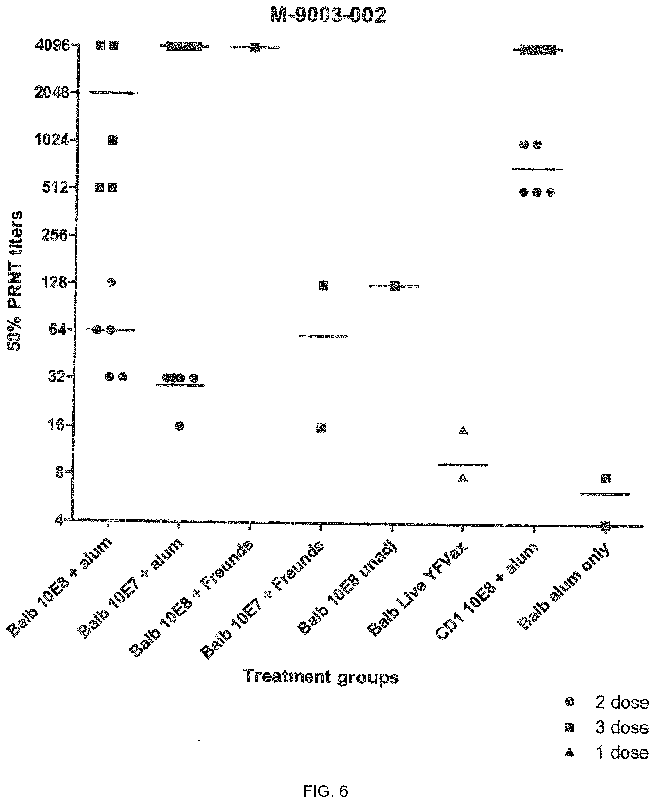

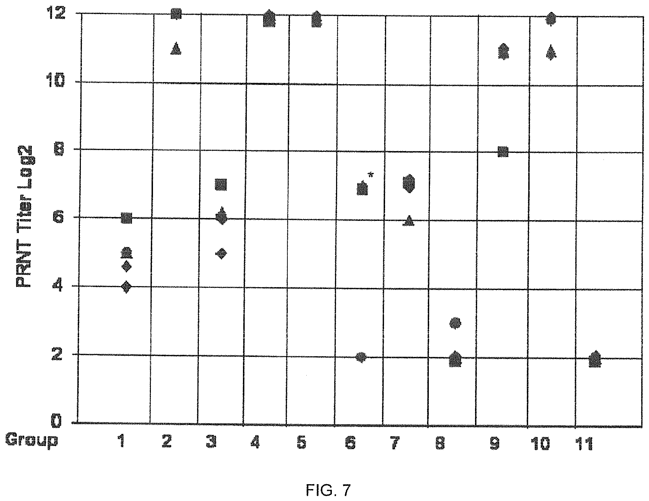

The plaque reduction neutralization test (PRNT) responses and titers are shown in Table 8 and FIGS. 6 and 7. The PRNT test is currently the generally accepted standard for antibodies against Yellow Fever virus. All mice, regardless of strain, receiving 2 or 3 doses of inactivated vaccine given either without adjuvant (Group 7), with alum (Groups 1, 2, 3, 4, 9, 10, 11), or with Freund's adjuvant (Groups 5, 6) developed neutralizing antibody responses. Titers of greater than 4096 were found in 5 of 5 BALB/c mice immunized with 3 doses of alum bound inactivated virus at the 10⁷ EU/dose. These titers were higher than BALB/c mice immunized with 3 doses of inactivated virus delivered with Freund's adjuvant (Group 6, titers 16-128). CD1 mice immunized with 2 doses of alum bound inactivated virus at the 10⁸ EU/dose level achieved higher titers (Group 9; titers 512-1024) than did similarly immunized BALB/c mice (Group 3; titers of 32-64). Only 1 in 5 mice receiving live YF Vax® (Group 8) mounted a neutralizing antibody response that was above the baseline levels in the mice receiving alum only (Groups 11).

In FIG. 7, each symbol represents an individual mouse. Treatment groups are shown in Tables 7 and 8. For Group 6 (*) the highest dilution of serum tested was 1:128. For Group 9 (**) the highest dilution of serum tested was 1:2048.

This study demonstrates that robust neutralizing antibody titers can be achieved in mice immunized with 2 or more inoculations of the disclosed inactivated YF virus delivered with alum. Outbred CD1 mice had higher antibody responses than an inbred strain (BALB/c). Alum was a superior adjuvant to Freund's, but this result could also be related to the route of immunization (SC for Freund's vs. IM for alum). Additional studies will be performed to determine if immunogenicity can be achieved with a single dose of vaccine.

**Table 8 Mice with plaque reduction neutralization activity**

| **Group** | **Strain of mice** | **Vaccine** | **Schedule** | | **% Positive (**+**/total)** |
|---|---|---|---|---|---|
| | | | **Vaccination** | **Sacrifice** | |
| 1 | BALB/c | 10⁸ BPL-inactivated in 0.2% alum | Day 0, 14 | Day 28 | 100% (5/5) |
| 2 | BALB/c | 10⁸ BPL-inactivated in 0.2% alum | Day 0, 14, 28 | Day 42 | 100% (5/5) |
| 3 | BALB/c | 10⁷ BPL-inactivated in 0.2% alum | Day 0, 14 | Day 28 | 100% (5/5) |
| 4 | BALB/c | 10⁷ BPL-inactivated in 0.2% alum | Day 0, 14, 28 | Day 42 | 100% (5/5) |
| 5 | BALB/c | 10⁸ BPL-inactivated in Freund's complete/incomplete | Day 0, 14, 28 | Day 42 | 100% (5/5) |
| 6 | BALB/c | 10⁷ BPL-inactivated in Freund's complete/incomplete | Day 0, 14, 28 | Day 42 | 100% (5/5) |
| 7 | BALB/c | 10⁸ BPL-inactivated no adjuvant | Day 0, 14, 28 | Day 42 | 100% (5/5) |
| 8 | BALB/c | Live YF Vax® | Day 0 | Day 28 | 20% (1/5) |
| 9 | CD1 | 10⁸ BPL-inactivated in 0.2% alum | Day 0, 14 | Day 28 | 100% (5/5) |
| 10 | CD1 | 10⁸ BPL-inactivated in 0.2% alum | Day 0, 14, 28 | Day 42 | 100% (5/5) |
| 11 | BALB/c | 0.2% alum | Day 0, 14, 28 | Day 42 | 0% (0/5) |

## Claims

1. A modified Yellow Fever virus strain YF 17D comprising a nucleic acid sequence having at least one nucleic acid mutation relative to the nucleic acid sequence of Yellow Fever virus strain YF 17D, wherein said at least one nucleic acid mutation is a mutation in the nucleic acid sequence encoding a non-structural protein 4B, in the codon corresponding to the amino acid at position 113 of a non-structural protein 4B of SEQ ID NO: 14 resulting in a change from AUA to AUG.

2. The modified Yellow Fever virus strain according to claim 1, wherein the nucleic acid mutation in the codon for the amino acid at position 113 of a non-structural protein 4B results in a codon change from isoleucine to methionine.

3. The modified Yellow Fever virus according to claim 2 comprising a nucleic acid of SEQ ID NO: 12 and/or an amino acid sequence of SEQ ID NO: 8.

4. A modified Yellow Fever virus strain YF 17D comprising a nucleic acid sequence having three nucleic acid mutations relative to the nucleic acid sequence of Yellow Fever virus strain YF 17D, wherein said three nucleic acid mutations are a mutation in the nucleic ac*i*d sequence encoding a non-structural protein 1, a mutation in the nucleic acid sequence encoding a non-structural protein 2A and a mutation in the nucleic acid sequence encoding the envelope protein according to SEQ ID NO: 11, wherein the mutation in the nucleic acid sequence encoding a non-structural protein 1 comprises a mutation in the codon corresponding to the threonine at position 317 of non-structural protein 1 and wherein the mutation in the nucleic acid sequence encoding a non-structural protein 2A comprises a mutation in the codon corresponding to the phenylalanine at position 170 of the non-structural protein 2A and wherein the mutation in the nucleic acid sequence encoding the envelope protein comprises a mutation in the codon corresponding to the lysine at position 160 of the envelope protein, wherein said modified Yellow Fever virus strain has increased propagation in cells and a higher yield in the conditioned medium of a cell culture relative to unadapted Yellow Fever virus YF 17D.

5. The modified Yellow Fever virus according to claim 4, wherein the mutation in the codon for the amino acid at position 317 of non-structural protein 1 results in a codon change from threonine to isoleucine and wherein the mutation in the codon for the amino acid at position 170 of the non-structural protein 2A results in a codon change from phenylalanine to leucine and wherein the mutation in the codon for the amino acid at position 160 of the envelope protein results in a codon change from lysine to arginine.

6. The modified Yellow Fever virus according to claim 4, wherein the mutation in the nucleic acid sequence encoding the envelope protein comprises a further mutated codon within 10 Angstroms or twenty amino acids from amino acid 160 and results in a pKa value of the side chain of the mutated amino acid at that position that is higher than the pKa value of the side chain of the original amino acid at that position, or wherein the nucleic acid sequence encoding the envelope protein comprises a further mutated codon in the molecular hinge region between Domain I and II of the envelope protein and results in a pKa value of the side chain of the mutated amino acid at that position that is higher than the pKa value of the side chain of the original amino acid at that position.

7. The modified Yellow Fever virus according to claim 5, comprising a nucleic acid of SEQ ID NO: 11 and/or an amino acid sequence of SEQ ID NO: 7

## Patentansprüche

1. Modifizierter Gelbfiebervirusstamm YF 17D, umfassend eine Nukleinsäuresequenz, die zumindest eine Nukleinsäuremutation im Verhältnis zur Nukleinsäuresequenz des Gelbfiebervirusstamm YF 17D aufweist, wobei die zumindest eine Nukleinsäuremutation eine Mutation in der Nukleinsäuresequenz, die für ein nicht strukturelles Protein 4B kodiert, in dem Codon ist, das der Aminosäure an Position 113 eines nicht strukturellen Proteins 4B der SEQ ID NO: 14 entspricht, was eine Änderung von AUA zu AUG zur Folge hat.

2. Modifizierter Gelbfiebervirusstamm nach Anspruch 1, wobei die Nukleinsäuremutation in dem Codon für die Aminosäure an Position 113 eines nicht strukturellen Proteins 4B eine Codonänderung von Isoleucin zu Methionin zur Folge hat.

3. Modifizierter Gelbfiebervirusstamm nach Anspruch 2, umfassend eine Nukleinsäure der SEQ ID NO: 12 und/oder eine Aminosäuresequenz der SEQ ID NO: 8.

4. Modifizierter Gelbfiebervirusstamm YF 17D, umfassend eine Nukleinsäuresequenz, die drei Nukleinsäuremutationen im Verhältnis zur Nukleinsäuresequenz des Gelbfiebervirusstamm YF 17D aufweist, wobei die drei Nukleinsäuremutationen eine Mutation in der Nukleinsäuresequenz, die für ein nicht strukturelles Protein 1 kodiert, eine Mutation in der Nukleinsäuresequenz, die für ein nicht strukturelles Protein 2A kodiert, und eine Mutation in der Nukleinsäuresequenz, die für das Hüllprotein gemäß SEQ ID NO: 11 kodiert, sind, wobei die Mutation in der Nukleinsäuresequenz, die für ein nicht strukturelles Protein 1 kodiert, eine Mutation in dem Codon umfasst, das dem Threonin an Position 317 des nicht strukturellen Proteins 1 entspricht, und wobei die Mutation in der Nukleinsäuresequenz, die für ein nicht strukturelles Protein 2A kodiert, eine Mutation in dem Codon umfasst, das dem Phenylalanin an Position 170 des nicht strukturellen Proteins 2A entspricht, und wobei die Mutation in der Nukleinsäuresequenz, die für das Hüllprotein gemäß SEQ ID NO: 11 kodiert, eine Mutation in dem Codon umfasst, das dem Lysin an Position 160 des Hüllproteins entspricht, wobei der modifizierte Gelbfiebervirusstamm im Verhältnis zum nicht angepassten Gelbfiebervirus YF 17D eine erhöhte Verbreitung in Zellen und eine höhere Ausbeute in dem konditionierten Medium einer Zellkultur aufweist.

5. Modifiziertes Gelbfiebervirus nach Anspruch 4, wobei die Mutation in dem Codon für die Aminosäure an Position 317 des nicht strukturellen Proteins 1 eine Codonänderung von Threonin zu Isoleucin zur Folge hat und wobei die Mutation in dem Codon für die Aminosäure an Position 170 des nicht strukturellen Proteins 2A eine Codonänderung von Phenylalanin zu Leucin zur Folge hat und wobei Mutation in dem Codon für die Aminosäure an Position 160 des Hüllproteins eine Codonänderung von Lysin zu Arginin zur Folge hat.

6. Modifiziertes Gelbfiebervirus nach Anspruch 4, wobei die Mutation in der Nukleinsäuresequenz, die für das Hüllprotein kodiert, ein weiteres mutiertes Codon binnen 10 Ängström oder zwanzig Aminosäuren von der Aminosäure 160 umfasst und einen pKa-Wert der Seitenkette der mutierten Aminosäure an dieser Position zur Folge hat, der höher ist als der pKa-Wert der Seitenkette der ursprünglichen Aminosäure an dieser Position, oder wobei die Nukleinsäuresequenz, die für das Hüllprotein kodiert, ein weiteres mutiertes Codon in der molekularen Gelenkregion zwischen den Domänen I und II des Hüllproteins umfasst und einen pKa-Wert der Seitenkette der mutierten Aminosäure an dieser Position zur Folge hat, der höher ist als der pKa-Wert der Seitenkette der ursprünglichen Aminosäure an dieser Position.

7. Modifiziertes Gelbfiebervirus nach Anspruch 5, umfassend eine Nukleinsäure der SEQ ID NO: 11 und/oder eine Aminosäuresequenz der SEQ ID NO: 7.

## Revendications

1. Souche YF 17D du virus de la fièvre jaune modifiée comprenant une séquence d'acide nucléique comportant au moins une mutation d'acide nucléique par rapport à la séquence d'acide nucléique de la souche YF 17D du virus de la fièvre jaune, dans laquelle ladite au moins une mutation d'acide nucléique est une mutation dans la séquence d'acide nucléique codant pour une protéine non structurale 4B, dans le codon correspondant à l'acide aminé au niveau de la position 113 d'une protéine non structurale 4B de SEQ ID NO : 14 produisant un changement de AUA en AUG.

2. Souche du virus de la fièvre jaune modifiée selon la revendication 1, dans laquelle la mutation d'acide nucléique dans le codon pour l'acide aminé au niveau de la position 113 d'une protéine non structurale 4B produit un changement de codon de l'isoleucine en méthionine.

3. Virus de la fièvre jaune modifié selon la revendication 2 comprenant un acide nucléique de SEQ ID NO : 12 et/ou une séquence d'acides aminés de SEQ ID NO : 8.

4. Souche YF 17D du virus de la fièvre jaune modifiée comprenant une séquence d'acide nucléique comportant trois mutations d'acide nucléique par rapport à la séquence d'acide nucléique de la souche YF 17D du virus de la fièvre jaune, dans laquelle lesdites trois mutations d'acide nucléique sont une mutation dans la séquence d'acide nucléique codant pour une protéine non structurale 1, une mutation dans la séquence d'acide nucléique codant pour une protéine non structurale 2A et une mutation dans la séquence d'acide nucléique codant pour la protéine de l'enveloppe selon SEQ ID NO : 11, dans laquelle la mutation dans la séquence d'acide nucléique codant pour une protéine non structurale 1 comprend une mutation dans le codon correspondant à la thréonine au niveau de la position 317 de la protéine non structurale 1 et dans laquelle la mutation dans la séquence d'acide nucléique codant pour une protéine non structurale 2A comprend une mutation dans le codon correspondant à la phénylalanine au niveau de la position 170 de la protéine non structurale 2A et dans laquelle la mutation dans la séquence d'acide nucléique codant pour la protéine de l'enveloppe comprend une mutation dans le codon correspondant à la lysine au niveau de la position 160 de la protéine de l'enveloppe, dans laquelle ladite souche du virus de la fièvre jaune modifiée présente une propagation augmentée dans les cellules et un rendement supérieur dans le milieu conditionné d'une culture cellulaire par rapport au virus de la fièvre jaune YF 17D non adapté.

5. Virus de la fièvre jaune modifié selon la revendication 4, dans lequel la mutation dans le codon pour l'acide aminé au niveau de la position 317 de la protéine non structurale 1 produit un changement de codon de la thréonine en isoleucine et dans lequel la mutation dans le codon pour l'acide aminé au niveau de la position 170 de la protéine non structurale 2A produit un changement de codon de la phénylalanine à la leucine et dans lequel la mutation dans le codon pour l'acide aminé au niveau de la position 160 de la protéine de l'enveloppe produit un changement de codon de la lysine en arginine.

6. Virus de la fièvre jaune modifié selon la revendication 4, dans lequel la mutation dans la séquence d'acide nucléique codant pour la protéine de l'enveloppe comprend un autre codon muté à 10 angströms ou vingt acides aminés à partir de l'acide aminé 160 et produit une valeur de pKa de la chaîne latérale de l'acide aminé muté à cette position qui est supérieure à la valeur de pKa de la chaîne latérale de l'acide aminé d'origine à cette position, ou dans lequel la séquence d'acide nucléique codant pour la protéine de l'enveloppe comprend un autre codon muté dans la région charnière moléculaire entre les domaines I et II de la protéine de l'enveloppe et produit une valeur de pKa de la chaîne latérale de l'acide aminé muté à cette position qui est supérieure à la valeur de pKa de la chaîne latérale de l'acide aminé d'origine à cette position.

7. Virus de la fièvre jaune modifié selon la revendication 5, comprenant un acide nucléique de SEQ ID NO : 11 et/ou une séquence d'acides aminés de SEQ ID NO : 7.
